# EUROPEAN PATENT APPLICATION

(11) **EP 3 865 180 A1**
(43) Date of publication of application: **18.08.2021**
(21) Application number: 20305141.2
(22) Date of filing: 13.02.2020
(51) Int. Cl.: A61P 31/14, C07K 14/165, C12N 15/86

(54) **LIVE RECOMBINANT MEASLES VIRUS EXPRESSING CORONAVIRUS ANTIGENS - ITS USE IN ELICITING IMMUNITY AGAINST CORONAVIRUSES**

(71) Applicant: Institut Pasteur, 75724 Paris Cedex 15 (FR)
(72) Inventor: ESCRIOU, Nicolas, Robert, Xavier, 75724 Paris Cedex 15 (FR); TANGY, Frédéric, 75724 Paris Cedex 15 (FR)
(74) Representative: Ernest Gutmann - Yves Plasseraud S.A.S.

(57) **Abstract**

The invention relates to the field of immunity against Coronaviruses. In this respect, the invention provides vectorized antigens derived from Coronaviruses that trigger an immune response against Coronaviruses. The invention accordingly relates to an active ingredient which is a live attenuated recombinant measles virus expressing Coronavirus antigen(s) and to its use in the elicitation of immunity, in particular protective immunity against 2019-cCoV strain and advantageously broad-spectrum protective immunity against various strains of Coronaviruses.

## Description

The invention relates to the field of immunity against Coronaviruses. In this respect, the invention provides vectorized antigens derived from Coronaviruses that trigger an immune response against Coronaviruses. The invention accordingly relates to an active ingredient which is a live attenuated recombinant measles virus expressing Coronavirus antigen(s) and to its use in the elicitation of immunity, in particular protective immunity against 2019-cCoV strain and advantageously broad-spectrum protective immunity against various strains of Coronaviruses. The invention also relates to polypeptides derived from the native antigens of coronavirus 2019-nCoV wherein the polypeptides have useful properties to design efficient immunogens in particular to design a vaccine candidate against coronavirus infection. The invention also relates to polynucleotides encoding the native antigens of coronavirus 2019-nCoV or encoding polypeptides derived from the native antigens of coronavirus 2019-nCoV, in particular polynucleotides adapted for expression by a recombinant measles virus or for improved recovery from producing cells.

The invention is also directed to means for the preparation of recombinant measles virus expressing the polypeptides obtained from antigens of coronavirus 2019-nCoV and to recombinant measles virus thus obtained.

The invention also concerns an immunogenic composition comprising recombinant measles virus expressing the polypeptides obtained from antigens of coronavirus 2019-nCoV. The invention also relates to the use of such immunogenic composition for the elicitation of a protective immune response in an animal host, in particular a mammalian host, especially a human host, against coronavirus 2019-nCoV and optionally against other coronaviruses or against disease caused by the infection. The invention also relates to a method for the treatment of a host in need thereof, in particular for prophylactic treatment, against the infection by coronavirus 2019-nCoV and optionally against other coronaviruses or against disease caused by the infection.

Coronaviruses are enveloped, positive-sense single-stranded RNA viruses with large genome (from 26 to 32kb). Four genera (alpha, beta, gamma and delta) have been described and among them betacoronavirus has been subdivided in four lineages (A, B, C and D). Among the host identified for coronaviruses avian and mammalian specified, including humans have been especially shown to be infected either by strains circulating annually or by strains capable of giving rise to pandemic outbreaks. Human coronaviruses include annual strains HCoV-OC43, HCoV- 229E, HCoV-HKU1 HCoV-NL63 and pandemic strains such as SARS-CoV (Severe Acute Respiratory Syndrome coronavirus) isolated in 2003 or MERS-CoV (Middle East Respiratory Syndrome coronavirus) isolated in 2012 is still circulating. SARS-CoV and MERS-CoV belong to the betacoronavirus lineage B and lineage C respectively. These coronaviruses are airborne transmitted and have been shown to allow human-to-human transmission.

Beside these known strains, a new Coronavirus strain designated 2019-nCoV (or more recently designated SARS-CoV-2 or SARS-CoV-2Covid-19) has been identified in 2019 as currently infecting people in China and that may spread around the world resulting in severe illness or death for a large number of the infected human hosts. This highly pathogenic stain emerged into the human population from animal reservoirs and has already proved to be responsible of high case-fatality rates including by human-to-human transmission causing great concerns for a coronavirus pandemic.

It is accordingly necessary to propose vaccine candidates that would be capable of providing protection against Coronavirus 2019-nCoV or possibly against a broader range of coronavirus strains, e.g., epidemic or pandemic strains. For the purpose of the present invention virus strain 2019-nCoV will be described in particular by reference to its nucleotide sequence (wild type sequence) disclosed in Genbank as MN908947 sequence and publicly available from NBCBI since 20^{th} January 2020 and that has been updated since that date as MN908947.3.

### Biology of the coronavirus

The coronavirus replicates in the cytoplasm of the host cells. The 5' end of the RNA genome has a capped structure and the 3' end contains a polyA tail. The envelope of the virus comprises, at its surface, peplomeric structures called spicules (or spike protein).

The genome comprises the following open reading frames or ORFs, from its 5' end to its 3' end: ORF1a and ORF1b corresponding to the proteins of the transcription-replication complex, and ORF-S, ORF-E, ORF-M and ORF-N corresponding to the structural proteins S, E, M and N. It also comprises ORFs corresponding to proteins of unknown function encoded by the region situated between ORF-S and ORF-E and overlapping the latter, the region situated between ORF-M and ORF-N, and the region included in ORF-N.

The S protein is a membrane glycoprotein (200-220 kDa) existing in the form of spicules or spikes emerging from the surface of the viral envelope. It is responsible for the attachment of the virus to the receptors of the host cell and for inducing the fusion of the viral envelope with the cell membrane. The S protein may be functionally divided into two sub-regions S1 and S2 wherein S1 forms the head of the S protein involved in the binding to the virus receptor on host cells and S2 forms a stalk structure.

The small envelope protein (E), also called sM (*small membrane*), which is a nonglycosylated transmembrane protein of about 10 kDa, is the protein present in the smallest quantity in the virion. It is involved in the coronavirus budding process which occurs at the level of the intermediate compartment in the endoplasmic reticulum and the Golgi apparatus.

The M protein or matrix protein (25-30 kDa) is a more abundant membrane glycoprotein which is integrated into the viral particle by an M/E interaction, whereas the incorporation of S into the particles is directed by an S/M interaction. It appears to be important for the viral maturation of coronaviruses and for the determination of the site where the viral particles are assembled.

The N protein or nucleocapsid protein (45-50 kDa) which is the most conserved among the coronavirus structural proteins is necessary for encapsidating the genomic RNA and then for directing its incorporation into the virion. This protein is probably also involved in the replication of the RNA.

When the host cell is infected, the reading frame (ORF) situated in 5' of the viral genome is translated into a polyprotein which is cleaved by the viral proteases and then releases several nonstructural proteins such as the RNA-dependent RNA polymerase (Rep) and the ATPase helicase (Hel). These two proteins are involved in the replication of the viral genome and in the generation of transcripts which are used in the synthesis of the viral proteins. The mechanisms by which these subgenomic mRNAs are produced are not completely understood; however, recent facts indicate that the sequences for regulation of transcription at the 5' end of each gene represent signals which regulate the discontinuous transcription of the subgenomic mRNAs.

The proteins of the viral membrane (S, E and M proteins) are inserted into the intermediate compartment, whereas the replicated RNA (+ strand) is assembled with the N (nucleocapsid) protein. This protein-RNA complex then combines with the M protein contained in the membranes of the endoplasmic reticulum and the viral particles form when the nucleocapsid complex buds into the endoplasmic reticulum. The virus then migrates across the Golgi complex and eventually leaves the cell, for example by exocytosis. The site of attachment of the virus to the host cell is at the level of the S protein.

With the aim of developing a vaccine against existing or emerging, possibly pandemic coronaviruses, in particular a vaccine that could be used in children (in particular in young children or babies) or in adult population or both, the inventors designed a strategy based on the expression of polypeptides derived from selected antigens (or suitable portion(s) thereof) by a measles virus vector, wherein in particular the measles virus (MV or MeV) is selected among live attenuated measles viruses such as vaccine measles viruses, preferably the Schwarz strain.

Measles virus is a non-segmented single-stranded, negative-sense enveloped RNA virus of the genus *Morbilivirus* within the family of *Paramyxoviridae.* This virus has been isolated in 1954 (Enders, J. F., and T. C. Peebles. 1954. Propagation in tissue cultures of cytopathogenic agents from patients with measles. Proc. Soc. Exp. Biol. Med. 86:277-286.), and live attenuated vaccines have been derived from this virus since then to provide vaccine strains and in particular from the Schwarz strain. Measles vaccines have been administered to hundreds of millions of children over the last 30 years and have proved its efficiency and safety. It is produced on a large scale in many countries and is distributed at low cost. For all these reasons, the inventors used attenuated Measles viruses to generate recombinant Measles virus particles expressing, advantageously stably expressing polypeptides derived from structural antigens of coronavirus 2019-nCoV.

The invention accordingly proposes a new approach to provide coronavirus antigens or polypeptides derived therefrom including spike derived antigens to the immune system of the host and especially provides use of measles virus vector to express such polypeptides or antigens, in particular for the elicitation of an immune response in a mammalian host, especially a human host, to confer protection, especially preventive protection, against the disease caused by coronavirus in particular 2019-nCoV stain. This approach using measles virus as a vector of the immunogenic polypeptides of coronavirus also takes benefits from the vector properties in particular of the immune properties of the vector to improve the quality of the immune response in the host. The inventors hence provide a recombinant infectious live attenuated measles virus, such as recombinant measles virus obtained using the Schwarz strain, capable of eliciting an immune response in mammalian, in particular in human individuals that would be effective and long lasting against illness resulting from coronavirus infection, especially from 2019-nCoV infection.

The invention thus relates to the use of measles virus as a vector to express coronavirus immunogens or epitopes of coronavirus antigens wherein said immunogens or epitopes encompass or derive from polypeptides derived from the wild type antigens of the coronavirus 2019-nCoV as generally described hereabove such as the S, E, N, ORF3a, ORF8, ORF7a and M proteins of a coronavirus or specifically described for the 2019-nCoV strain and illustrated in the present description.

The expression "*polypeptide*" or "*polypeptide of a coronavirus in particular of 2019-nCoV*" define a molecule resulting from a concatenation of amino acid residues. This molecule is suitable for expression by the recombinant measles virus particles of polypeptides that may thus be the wild type antigens, fragments thereof that comprise epitopes sufficient for the elicitation of an immune response in a mammalian host, or mutated or truncated antigens, wherein the mutations or truncations or the fragments resulting from deletions of amino acid residues or of regions of the native antigen preserve the immunogenic properties of the antigen and enable their production or their use in immunogenic compositions.Accordingly mutated antigens or fragments of antigens may have improved stability in cells and/or enable recovery of solubilized forms of the antigens and/or multimeric forms of the antigens, in particular trimers thereof (such as for the spike derived antigens). The polypeptides disclosed herein especially originate from the CoV, in particular from coronavirus 2019-nCoV, and are structural proteins that may be identical to native proteins or alternatively that may be derived thereof by mutation, especially targeted point mutations, including by substitution (in particular by conservative amino acid residues) or by addition of amino acid residues or by secondary modification after translation (including glycosylation) or by deletion of portions of the native proteins(s) resulting in fragments having a shortened size with respect to the native protein of reference. Fragments are encompassed within the present invention to the extent that they bear epitopes of the native protein suitable for the elicitation of an immune response in a host in particular in a mammalian host, especially a human host, preferably a response that enables the protection against CoV, in particular coronavirus 2019-nCoV. Epitopes are in particular of the type of B epitopes involved in the elicitation of a humoral immune response through the activation of the production of antibodies in a host to whom the protein has been administered or in whom it is expressed following administration of the infectious replicative particles of the invention. Epitopes may alternatively be of the type of T epitopes involved in elicitation of Cell Mediated Immune response (CMI response). Fragments may have a size representing more than 50% of the amino-acid sequence size of the native protein of CoV, in particular of coronavirus 2019-nCoV, preferably at least 90% or 95%. Alternatively, fragments may be short polypeptides with at least 10 amino acid residues, which harbor epitope(s) of the native protein. Fragments in this respect also include polyepitopes as defined herein. In a particular embodiment the polypeptide is a fragment of the native antigen that contains or consists in the soluble portion of the antigen and/or is point mutated (such as with 1, 2 or by less than 5% substitutions in the amino acid residues of the native antigen). Mutations may be designed to improve its stability in the cells. The polypeptides (such as the S polypeptide) may in particular be expressed as trimeric forms of the coronavirus native or modified antigens.The words "polypeptide" and "antigens" are used interchangeably to define a "polypeptide of the coronavirus in particular of 2019-nCoV" according to the invention in accordance with the definition provided herein. The amino acid sequence of a polypeptide is hence either identical to a counterpart in an antigen of a strain of CoV, in particular 2019-cCoV, including for a polypeptide which is a native mature or precursor of a protein of CoV or is modified by mutation or deletion to define an antigenic or an immunogenic fragment thereof or a mutant thereof.

In particular a fragment or a mutant having at least 50%, at least 80%, in particular advantageously at least 90% or preferably at least 95% amino acid sequence identity to a naturally occurring CoV polypeptide. Amino acid sequence identity can be determined by alignment by one skilled in the art using manual alignments or using the numerous alignment programs available. Fragments or mutants of CoV proteins of the invention may be defined with respect to the particular amino acid sequences illustrated herein.

In a first aspect of the invention heterologous polypeptides for expression by the recombinant measles virus, are derived from glycoprotein S of a coronavirus, in particular of 2019-nCoV: they may be the S polypeptide as such in its glycosylated or non-glycosylated form or they may be fragments thereof such as immunogenic fragments S1 and/or S2 or shorter fragments thereof comprising epitopes suitable to elicit an immune response in the context of the recombinant virus particles. Particular fragments of S or mutated fragments of S according to the invention are the polypeptides listed below encoded by the nucleotide sequence disclosed hereafter or having the amino acid sequence described herein: S polypeptide of 2019-nCoV, stab-S polypeptide of 2019-nCoV, Secto polypeptide of 2019-nCoV, stab-Secto polypeptide of 2019-nCoV, S1 polypeptide of 2019-nCoV, S2 polypeptide of 2019-nCoV, tri-Secto polypeptide of 2019-nCoV, tristab-Secto polypeptide of 2019-nCoV.The fragments may be obtained from the wild type sequence or may be mutated and/or deleted with respect to the wild type sequence.

In another aspect of the invention heterologous polypeptides for expression by the recombinant measles virus, are derived from one of the following antigens of a coronavirus, in particular of 2019-nCoV:, E, N, ORF3a, ORF8, ORF7a or M proteins, in particular N protein.The invention thus relates to a nucleic acid construct which comprises:
(1) a cDNA molecule encoding a full length, infectious antigenomic (+) RNA strand of a measles virus (MV);
(2) a first heterologous polynucleotide encoding at least one polypeptide of a coronavirus (CoV), in particular of coronavirus 2019-nCoV, in particular said first polynucleotide encoding at least one polypeptide consisting of the spike (S) polypeptide of a coronavirus (CoV), in particular of coronavirus 2019-nCoV, or at least one polypeptide consisting of an immunogenic fragment thereof or an antigenic fragment thereof including those especially disclosed above,
and wherein the first heterologous polynucleotide is operatively cloned within an additional transcription unit (ATU) inserted within the cDNA of the antigenomic (+) RNA to provide a recombinant MV-CoV, in particular MV-CoVS nucleic acid molecule.

In a particular embodiment, the first heterologous polynucleotide is operatively cloned in an ATU2 located between the P gene and the M gene of the MV or in an ATU3 located downstream of the H gene of the MV .

A nucleic acid construct according to the invention is in particular a purified DNA molecule, obtained or obtainable by recombination of various polynucleotides of different origins, operably linked together. It is also and interchangeably designated as a cDNA as a result of the designation as a cDNA, of the molecule encoding a full length, infectious antigenomic (+) RNA strand of a measles virus (MV).

The expression "*operatively linked*" "*operably linked*" refers to the functional link existing between the different polynucleotides of the nucleic acid construct of the invention such that said different polynucleotides and nucleic acid construct are efficiently transcribed and if appropriate translated, in particular in cells or cell lines, especially in cells or cell lines used as part of a rescue system for the production or amplification of recombinant infectious MV particles of the invention or in host cells, especially in mammalian or in human cells.

In a particular embodiment of the invention, the nucleic acid construct further comprises a second heterologous polynucleotide encoding at least one polypeptide, an immunogenic fragment thereof or an antigenic fragment thereof (including a wild type or a mutated fragment), of a coronavirus, in particular of coronavirus 2019-nCoV, wherein said polypeptide is different from at least one polypeptide encoded by the first heterologous polynucleotide and in particular is selected from the group consisting of the nucleocapsid (N) polypeptide, the matrix (M), the E polypeptide, the ORF8 polypeptide, the ORF7a polypeptide and the ORF3a polypeptide, or immunogenic or antigenic fragments thereof or mutated fragments thereof, the second heterologous polynucleotide being operatively cloned within an ATU at a location distinct from the location of the first cloned heterologous polynucleotide.

The ATU for cloning of the second heterologous polynucleotide is located at a different location with respect to the ATU used for cloning the first heterologous polynucleotide, in particular is located upstream the N gene of the MV in the ATU1, or in particular within an ATU at a location between the P gene and the M gene of the MV in the ATU2 or in particular within an ATU at a location downstream of the H gene of the MV in the ATU3.

In a further aspect the invention concerns a nucleic acid construct which comprises:
(1) a cDNA molecule encoding a full length, infectious antigenomic (+) RNA strand of a measles virus (MV);
(2) a heterologous polynucleotide encoding at least one polypeptide of a coronavirus (CoV), in particular of coronavirus 2019-nCoV, selected from the group consisting of the nucleocapsid (N) polypeptide, the matrix (M), the E polypeptide, the ORF8 polypeptide, the ORF7a polypeptide and the ORF3a polypeptide, or immunogenic or antigenic fragments thereof or mutated fragments thereof, the second heterologous polynucleotide being operatively cloned within an ATU.

ATU sequences, especially ATU1, ATU2, ATU3 used for the invention are sequences known from the skilled person and comprise, for use in steps of cloning into cDNA of MV, cis-acting sequences necessary for MV-dependent expression of a transgene, such as a promoter of the gene preceding, in MV cDNA, the insert represented by the polynucleotide, e.g., the first or the second polynucleotide encoding at least one polypeptide of a coronavirus, in particular encoding the polypeptide consisting of the spike (S) polypeptide of a coronavirus (CoV), in particular of coronavirus 2019-nCoV, or consisting of an immunogenic fragment thereof or an antigenic fragment thereof, or in particular encoding the nucleocapsid (N) polypeptide, the matrix (M), the E polypeptide, the ORF8 polypeptide, the ORF7a polypeptide or the ORF3a polypeptide, or immunogenic or antigenic fragments thereof or mutated fragments thereof and a multiple cloning sites cassette for insertion of said polynucleotide. The ATU comprises in addition to the intergenic sequence of the genes (including Gene Start (GS) promoting the transcription and Gene End (GE) of the P gene of MV terminating the transcription of the insert (heterologous polynucleotide) a polylinker sequence for the insertion of the heterologous polynucleotide. ATU sequences are illustrated in the constructs of the invention.

When used to carry out the invention, the ATU is advantageously located within the N-terminal sequence of the cDNA molecule encoding the full-length (+)RNA strand of the antigenome of the MV and is especially located upstream from the N gene (ATU1) or between the P and M genes of this virus (ATU2) or between the H and L genes (ATU3). It has been observed that the transcription of the viral RNA of MV follows a gradient from the 5' to the 3' end. This explains that, when inserted in the 5' end of the coding sequence of the cDNA, the ATU will enable a higher expression of the heterologous DNA sequence (e.g. the polynucleotide encoding at least one polypeptide such as a polypeptide consisting of the spike (S) polypeptide of a coronavirus (CoV), in particular of coronavirus 2019-nCoV, or consisting of an immunogenic fragment thereof or an antigenic fragment thereof) that it contains.

The polynucleotide encoding at least one polypeptide consisting of the spike (S) polypeptide of a coronavirus (CoV), in particular of coronavirus 2019-nCoV, or consisting of an immunogenic fragment thereof or an antigenic fragment thereof, may thus be inserted in any intergenic region of the cDNA molecule of the MV in particular in an ATU. Particular constructs of the invention are those illustrated in the examples.

In a preferred embodiment of the invention, the polynucleotide encoding at least one polypeptide consisting of the spike (S) polypeptide of a CoV, in particular of coronavirus 2019-nCoV, or consisting of an immunogenic fragment thereof or an antigenic fragment thereof, is inserted in the intergenic region of the P and M genes of the MV cDNA molecule, in particular in an ATU, such as ATU2. ATU3 is preferred to insert the second heterologous polynucleotide of the invention if present in the construct.

In a particular embodiment of the invention, the construct is prepared by cloning a polynucleotide encoding at least one polypeptide in particular consisting of the spike (S) E, N, ORF3a, ORF8, ORF7a or M polypeptide of a coronavirus (CoV), in particular of coronavirus 2019-nCoV (such as a S polypeptide having the sequence disclosed in Genbank MN908947.3 or any of the polypeptides derived from the native S antigens and illustrated herein, especially as fragments of S or modified fragments of S), or consisting of an immunogenic fragment thereof or an antigenic fragment thereof (including a mutated fragment) as disclosed herein, in the cDNA encoding a full-length, infectious antigenomic (+) RNA strand of a MV.

Alternatively, a nucleic acid construct of the invention may be prepared using steps of synthesis of nucleic acid fragments or polymerization from a template, including by PCR.

The nucleic acid construct of the invention and the MV-CoV of the invention encodes or expresses at least one polypeptide consisting of S, E, N, ORF3a, ORF8, ORF7a or M proteins of a coronavirus or specifically described for the 2019-nCoV strain, in particular the spike (S) polypeptide of a coronavirus (CoV), in particular of coronavirus 2019-nCoV, or consisting of an immunogenic fragment thereof or an antigenic fragment thereof.

According to a preferred embodiment, the invention also concerns modifications and optimization of the polynucleotide to allow an efficient expression of the at least one polypeptide consisting of S, E, N, ORF3a, ORF8, ORF7a or M proteins of a coronavirus or specifically described for the 2019-nCoV strain, in particular polypeptide consisting of the spike (S) polypeptide of a coronavirus (CoV), in particular of coronavirus 2019-nCoV, or consisting of an immunogenic fragment thereof or an antigenic fragment thereof, at the surface of chimeric infectious particles of MV-CoV in the host, in particular the human host.

According to this embodiment, optimization of the polynucleotide sequence can be operated avoiding cis-active domains of nucleic acid molecules: internal TATA-boxes, chi-sites and ribosomal entry sites; AT-rich or GC-rich sequence stretches; ARE, INS, CRS sequence elements; repeat sequences and RNA secondary structures ; cryptic splice donor and acceptor sites, branch points.

The optimized polynucleotides may also be codon optimized for expression in a specific cell type. This optimization allows increasing the efficiency of chimeric infectious particles production in cells without impacting the expressed protein(s).

In a particular embodiment of the invention, the polynucleotide encoding at least one polypeptide consisting of the spike (S) polypeptide of a coronavirus (CoV), in particular of coronavirus 2019-nCoV, or consisting of an immunogenic fragment thereof or an antigenic fragment thereof, has been optimized for a human codon usage.

The optimization of the polynucleotide encoding at least one polypeptide consisting of the spike (S) polypeptide of a coronavirus (CoV), in particular of coronavirus 2019-nCoV, or consisting of an immunogenic fragment thereof or an antigenic fragment thereof may be performed by modification of the wobble position in codons without impacting the identity of the amino acid residue translated from said codon with respect to the original one.

Optimization is also performed to avoid editing-like sequences from Measles virus. The editing of transcript of Measles virus is a process which occurs in particular in the transcript encoded by the *P* gene of Measles virus. This editing, by the insertion of extra G residues at a specific site within the *P* transcript, gives rise to a new protein truncated compared to the P protein. Addition of only a single G residue results in the expression of the V protein, which contains a unique carboxyl terminus (Cattaneo R et al., Cell. 1989 Mar 10;56(5):759-64).

In a particular embodiment of the invention, *measles editing-like sequences* have been deleted from said polynucleotide encoding at least one polypeptide consisting of the spike (S) polypeptide of a coronavirus (CoV), in particular of coronavirus 2019-nCoV, or consisting of an immunogenic fragment thereof or an antigenic fragment thereof. The following *measles editing-like sequences* can be mutated: AAAGGG, AAAAGG, GGGAAA, GGGGAA, TTAAA, AAAA, as well as their complementary sequence: TTCCCC, TTTCCC, CCTTTT, CCCCTT, TTTAA, TTTT. For example, AAAGGG can be mutated in AAAGGC, AAAAGG can be mutated in AGAAGG or in TAAAGG or in GAAAGG, and GGGAAA in GCGAAA.

In a particular embodiment of the invention, the native and codon-optimized nucleotide sequences of the polynucleotide encoding particular peptides/proteins/antigen as well as the amino acid sequences of these peptides/proteins/antigen of the invention are the sequences disclosed as SEQ ID NOs: X-X and mentioned in Table 1 below.

Codon-optimized genes are most useful both to promote high level expression of poorly transcribed / translated genes and to recover Measles-based vaccine candidates with high and stable antigen expression. However, they suffer from major drawbacks, which are intrinsically linked to their design and final codon (most used codons in the final host genome) and nucleotide (high GC/AT ratio) compositions. Thus codon-optimized genes most often promote such high level translation that, if highly transcribed, leads to saturation of the translational and post-translational cellular machineries and associated consequences on the quality of the expressed protein and on the cells itself (RE and golgi stress). Their naturally high GC/ composition (usually more than 55-60%) is also certainly not optimal for engineering of stable recombinant MV vectors since the GC composition of the MV genome is much lower (47.4%). the inventors thus decided to engineer specifically optimized genes for the MV platform with the following features in the polynucleotides encoding the polypeptide(s) of a coronavirus, in particular of 2019-nCoV:
- absence of MV editing (AnGn, n≥3)- and core gene end (A4CKT)-like sequences on both strands,
- removal, where applicable, of internal TATA-boxes, chi-sites and ribosomal entry sites (for translation efficiency),
- removal, where applicable, of AT-rich or GC-rich sequence stretches, RNA instability motifs, repeat sequences and RNA secondary structures (for transcription efficiency, mRNA stability and also translation efficiency),
- balanced codon composition, avoiding, where applicable, rare codons and high usage of the most frequent codons (for efficiency, accuracy and speed of translation without saturation of the translation machinery),
- target GC composition of 44-50% (to adjust to that of MV genome).

In addition,a criteria was added for the removal of (cryptic) splice donor and acceptor sites in higher eucaryotes, which is of no importance for the MV platform but allow to check for the synthetic gene expression by transient transfection in mammalian cells. BsiWI and BssHII restriction sites were added at the 5' and 3' ends, respectively, of the designed nucleotide sequences and appropriate spacer sequence were inserted so that the resulting cDNAs comply with the "rule of six", which stipulates that the number of nucleotides of the MV genome must be a multiple of 6. The resulting cDNAs were named S_MV optimized synthetic gene and N_MV optimized synthetic gene and will be synthesized at Geneart (ThermoFisher) facilities and their nucleic acid sequence is herein disclosed as SEQ ID NO.36 and SEQ. 37 respectively.

In a particular embodiment of the invention, the transfer vector plasmid ... has the optimized sequence of CoV of SEQ ID NO: 34 or 35, as mentioned in Table 1 below.

As used herein, the expression "*encoding*" defines the ability of the nucleic acid molecules to be transcribed and where appropriate translated for product expression into selected cells or cell lines. Accordingly, the nucleic acid construct may comprise regulatory elements controlling the transcription of the coding sequences, in particular promoters and termination sequences for the transcription and possibly enhancer and other cis-acting elements. These regulatory elements may be heterologous with respect to the CoV, in particular the coronavirus 2019-nCoV polynucleotide sequences.

In a particular embodiment of the invention, the first heterologous polynucleotide encodes the wild type S polypeptide of SEQ ID NO 3, or a fragment thereof consisting of the S1 domain of SEQ ID NO 11 or the S2 domain of SEQ ID NO 13 of the S polypeptide, or a variant thereof that has 1, 2 or more amino acid residue substitution(s), in particular less than 10, or less than 5 amino acid residue substitutions. The substitutions may in particular be designed to improve stability. According to a particular embodiment the first heterologous polynucleotide encodes a polypeptide selected in the group of polypeptides characterized by one of the amino acid sequences of SEQ ID NO 5, 7, 9, 15, 17 and 19.

In a particular embodiment of the invention, a single polypeptide of a coronavirus, in particular of coronavirus 2019-nCoV, is encoded by the nucleic acid construct and said polypeptide is the S polypeptide or a portion or fragment thereof as described herein.

In a particular embodiment of the nucleic acid construct of the invention, the second heterologous polynucleotide encodes (i) the N polypeptide of SEQ ID NO 22, an immunogenic fragment thereof or an antigenic fragment thereof, or a variant of the N polypeptide by substitution of 1, 2 or less than 10 amino acid residue(s), in particular less than 5 amino acid residues and/or (ii) the M polypeptide or its endodomain, (iii) the E polypeptide,of SEQ ID NO. 23 (iv) the ORF8 polypeptide ,of SEQ ID NO. 25, (v) the ORF7a polypeptide, of SEQ ID NO. 27 and/or (vi) the ORF3a polypeptide, of SEQ ID NO. 226 of a coronavirus, in particular of 2019-nCoV. In a preferred embodiment of the invention, the heterologous polynucleotide encoding the N polypeptide has the sequence of SEQ ID NO. 20, 21 or 37.

In a preferred embodiment of the invention, the heterologous polynucleotide encoding the S polypeptide, S1 polypeptide or S2 polypeptide, an immunogenic fragment thereof or an antigenic fragment or a mutated variant thereof comprises or consists in the open reading frame of the wild type gene or has a codon-optimized open reading frame(s) (coORF) for expression in mammalian cells and/or in drosophila cells, in particular, the heterologous polynucleotide comprises one of the following sequences:
- SEQ ID NO: 1 or 2 or 36 which encodes the S polypeptide or,
- SEQ ID NO: 10 which encodes the S1 polypeptide or,
- SEQ ID NO: 12 which encodes the S2 polypeptide or,
- SEQ ID NO: 4 which encodes the stab-S polypeptide
- SEQ ID NO: 6 which encodes the Secto polypeptide or,
- SEQ ID NO: 8 which encodes the stab-Secto polypeptide or,
- SEQ ID NO:14 which encodes the stab-S2 polypeptide or,
- SEQ ID NO: 16 which encodes the tri-Secto polypeptide or,
- SEQ ID NO: 18 which encodes the tristab-Secto polypeptide .

In a particular embodiment of the invention, the nucleic acid construct is a cDNA construct comprising from 5' to 3' end the following polynucleotides coding for ORFs:
(a) a polynucleotide encoding the N protein of the MV;
(b) a polynucleotide encoding the P protein of the MV;
(c) the first heterologous polynucleotide encoding at least one polypeptide, an immunogenic fragment thereof or an antigenic fragment thereof, of a coronavirus, in particular of coronavirus 2019-nCoV, said at least one polypeptide consisting of the S polypeptide and wherein the first heterologous polynucleotide is operatively cloned within an additional transcription unit (ATU) inserted within the cDNA of the antigenomic (+) RNA, in particular within ATU2;
(d) a polynucleotide encoding the M protein of the MV;
(e) a polynucleotide encoding the F protein of the MV;
(f) a polynucleotide encoding the H protein of the MV;
(g) a polynucleotide encoding the L protein of the MV;
and wherein said polynucleotides are operatively linked within the nucleic acid construct and are under the control of a viral replication and transcriptional regulatory elements such as MV leader and trailer sequences and are framed by a T7 promoter and a T7 terminator and additionally are framed by restrictions sites suitable for cloning in a vector to provide a recombinant MV-CoV expression cassette.

In another embodiment the first nucleic acid construct is replaced by the second nucleic acid construct.

The expressions "*N protein*", "*P protein*", "*M protein*", "F protein" , "*H protein*" and "*L protein*" refer respectively to the nucleoprotein (N), the phosphoprotein (P), the matrix protein (M), the fusion protein (F), the hemagglutinin protein (H) and the RNA polymerase large protein (L) of a MV. These components have been identified in the prior art and are especially disclosed in Fields, Virology (Knipe & Howley, 2001).

In the construct of the invention the polynucleotide sequences disclosed herein in respect of MV sequences taken together with the added polynucleotide sequences that will remain in the replicon of the recombinant genome comply with the "rule of six" featuring the requirement that the MV genome be an exact multiple of six nucleotides in length for reverse genetics for correctly take place in order to enable efficient rescue.

In a particular embodiment of the invention, the sequence of the recombinant MV-CoV nucleic acid molecule between the first nucleotide of the cDNA encoding the MV antigenome and the last nucleotide of the cDNA encoding the MV antigenome is a multiple of 6 nucleotides.

The "rule of six" accordingly also applies to this construct prepared according to the invention that comprises the sequences encoding the coronavirus antigen(s).

The "*rule of six*" is expressed in the fact that the total number of nucleotides present in a nucleic acid representing the MV(+) strand RNA genome or in nucleic acid constructs comprising same is a multiple of six. The "rule of six" has been acknowledged in the state of the art as a requirement regarding the total number of nucleotides in the genome of the MV, which enables efficient or optimized replication of the MV genomic RNA. In the embodiments of the present invention defining a nucleic acid construct that meets the rule of six, said rule applies to the nucleic acid construct specifying the cDNA encoding the full-length MV (+) strand RNA genome and all inserted sequences, when taken individually or collectively

In particular, the nucleic acid construct of the invention complies with the rule of six (6) of the MV genome when recombined with the polynucleotide encoding at least one polypeptide consisting of the spike (S) polypeptide of a coronavirus (CoV), in particular of coronavirus 2019-nCoV, or consisting of an immunogenic fragment thereof or an antigenic fragment thereof, taken together with the cDNA molecule encoding the full-length, infectious antigenomic (+) RNA strand of the MV consist of a number of nucleotides that is a multiple of six. In a particular embodiment, the rule of six applies to the cDNA encoding the full-length infectious antigenomic (+) RNA strand of the MV and to the polynucleotide cloned into said cDNA and encoding at least one polypeptide consisting of the spike (S) polypeptide of a CoV, in particular of coronavirus 2019-nCoV, or consisting of an immunogenic fragment thereof or an antigenic fragment thereof. Alternatively, compliance with the rule of six may be determined taking into account the whole construct or the transcript obtained from the construct in cells used for the rescue of recombinant measles virus.

The organization of the genome of MVs and their replication and transcription process have been fully identified in the prior art and are especially disclosed in Horikami S.M. and Moyer S.A. (Curr. Top. Microbiol. Immunol. (1995) 191, 35-50) or in Combredet C. et al (Journal of Virology, Nov 2003, p11546-11554) for the Schwarz vaccination strain of the virus or for broadly considered negative-sense RNA viruses, in Neumann G. et al (Journal of General Virology (2002) 83, 2635-2662).

In a preferred embodiment of the invention, the measles virus is an attenuated virus strain.

An "*attenuated strain*" of measles virus is defined as a strain that is avirulent or less virulent than the parent strain in the same host, while maintaining immunogenicity and possibly adjuvanticity when administered in a host *i.e*., preserving immunodominant T and B cell epitopes and possibly the adjuvanticity such as the induction of T cell costimulatory proteins or the cytokine IL-12.

An attenuated strain of a MV accordingly refers to a strain which has been serially passaged on selected cells and, possibly, adapted to other cells to produce seed strains suitable for the preparation of vaccine strains, harboring a stable genome which would not allow reversion to pathogenicity nor integration in host chromosomes. As a particular "attenuated strain", an approved strain for a vaccine is an attenuated strain suitable for the invention when it meets the criteria defined by the FDA (US Food and Drug Administration) *i.e*., it meets safety, efficacy, quality and reproducibility criteria, after rigorous reviews of laboratory and clinical data (www.fda.gov/cber/vaccine/vacappr.htm).

Particular attenuated strains that can be used to implement the present invention and especially to derive the MV cDNA of the nucleic acid construct are the Schwarz strain, the Zagreb strain, the AIK-C strain and the Moraten strain, more preferably the Schwarz strain. All these strains have been described in the prior art and access to them is provided in particular as commercial vaccines.

In a particular embodiment of the invention, the recombinant DNA or cDNA of the MV-CoV molecule is placed under the control of heterologous expression control sequences. The insertion of such a control for the expression of the DNA/cDNA, is favorable when the expression of thisDNA/cDNA is sought in cell types which do not enable full transcription of the DNA/cDNA with its native control sequences.

In a particular embodiment of the invention, the heterologous expression control sequence comprises the T7 promoter and T7 terminator sequences. These sequences are respectively located 5' and 3' of the coding sequence for the full length antigenomic (+)RNA strand of MV and from the adjacent sequences around this coding sequence. Accordingly in a particular embodiment the nucleic acid construct of the invention comprises these additional control sequences.

In a particular embodiment of the invention, the recombinant nucleic acid molecule or the nucleic acid construct encoding the antigenomic RNA of the measles virus recombined with the heterologous polynucleotide, which is defined herein is further modified i.e., comprises additional nucleotide sequences or motifs.

In a preferred embodiment, the nucleic acid construct or the recombinant nucleic acid molecule encoding the antigenomic RNA of the measles virus recombined with the heterologous polynucleotide according to the invention further comprises, at its 5'-end, adjacent to the first nucleotide of the nucleotide sequence encoding the full-length antigenomic (+)RNA strand of the MV approved vaccine strain, a GGG motif followed by a hammerhead ribozyme sequence and also comprises, at its 3'-end, adjacent to the last nucleotide of said nucleotide sequence encoding the full length anti-genomic (+)RNA strand, the sequence of a ribozyme. The Hepatitis delta virus ribozyme (δ) is advantageously hence provided at the 3'-end, adjacent to the last nucleotide of said nucleotide sequence encoding the full length anti-genomic (+)RNA strand.

The GGG motif placed at the 5' end, adjacent to the first nucleotide of the above coding sequence improves the efficiency of the transcription of said cDNA coding sequence. As a requirement for the proper assembly of measles virus particles is the fact that the cDNA encoding the antigenomic (+)RNA of the nucleic acid construct of the invention complies with the rule of six, when the GGG motif is added, a ribozyme is also added at the 5' end of the coding sequence of the cDNA, 3' from the GGG motif, in order to enable cleavage of the transcript at the first coding nucleotide of the full-length antigenomic (+)RNA strand of MV.

In a particular embodiment of the invention, in order to prepare the nucleic acid construct of the invention, the preparation of a cDNA molecule encoding the full-length antigenomic (+) RNA of a MV disclosed in the prior art is achieved by known methods. Said cDNA provides especially the genome vector when it is inserted in a vector such as a plasmid. A particular cDNA molecule suitable for the preparation of the nucleic acid construct of the invention is the one obtained using the Schwarz strain of MV. Accordingly, the cDNA coding for the antigenome of the measles virus used within the present invention may be obtained as disclosed in WO2004/000876 or may be obtained from plasmid pTM-MVSchw deposited by Institut Pasteur at the Collection Nationale de Culture de Microorganismes (CNCM), 28 rue du Dr Roux, 75724 Paris Cedex 15, France, under No I-2889 on June 12, 2002, the sequence of which is disclosed in WO2004/000876 incorporated herein by reference. The plasmid pTM-MVSchw has been obtained from a Bluescript plasmid and comprises the polynucleotide coding for the full-length measles virus (+) RNA strand of the Schwarz strain placed under the control of the promoter of the T7 RNA polymerase. It has 18967 nucleotides and a sequence represented as SEQ ID NO: 28. cDNA molecules (also designated cDNA of the measles virus or MV cDNA for convenience) from other MV strains may be similarly obtained starting from the nucleic acid purified from viral particles of attenuated MV such as those described herein.

The cDNA coding for the antigenome of the measles virus used within the present invention may also be obtained from plasmid pTM2-MVSchw-gfp deposited by Institut Pasteur at the Collection Nationale de Culture de Microorganismes (CNCM), 28 rue du Dr Roux, 75724 Paris Cedex 15, France, under No I-2890 on June 12, 2002. It has 19795 nucleotides and a sequence represented as SEQ ID NO: 29. This plasmid contains the sequence encoding the eGFP marker that may be deleted.

The above cited pTM-MVSchw plasmids may also be used for the preparation of the nucleic acid constructs of the invention, by cloning the heterologous polynucleotide encoding a polypeptide derived from an antigen of a coronavirus, in particular of the 2019-nCoV strain, (in particular a polypeptide derived from the S antigen as disclosed herein) in the cDNA encoding the antigenome of the measles virus, using one or more ATU inserted at position known for insertion of ATU1 or ATU2 or ATU3.

In a particular embodiment, the nucleic acid construct of the invention comprises or consists in the recombinant MV-CoV nucleic acid molecule located from position 1 to position 20152 in the sequence of of SEQ ID NO. 34 or SEQ ID NO. 35. This construct encodes the S polypeptide of the 2019-nCoV respectively located in the ATU2 or in the ATU3 inserted in the cDNA encoding the measles virus antigenome.

In a particular embodiment, the invention relates to a nucleic acid construct derived from the above by replacement of the sequence encoding the S protein by a polynucleotide encoding another polypeptide of a coronavirus, in particular of the 2019-nCoV, such as the sequence of a fragment of the S antigen as disclosed herein, in particular a nucleotide sequence encoding one of the stab-S, Secto, stab-Secto, S1, S2, stab-S2, tri-Secto, tristab-Secto polypeptide in particular a polynucleotide of sequence disclosed as SEQ ID NO: 4, 6, 8, 10, 12, 14, 16 or 18 or a polynucleotide encoding the amino acid sequence of SEQ ID NO: 5, 7, 9, 11, 13, 15 17 or 19 respectively. In such embodiment, the polynucleotide region to be replaced in the sequence of SEQ ID NO 34 is from position 3538 to position 7362 and the polynucleotide region to be replaced in the sequence of SEQ ID NO 35 is from position 9340 to position 13164.

In another embodiment the invention relates to a nucleic acid construct derived from the above by replacement of the sequence encoding the S protein by a polynucleotide encoding another polypeptide of a coronavirus, in particular of the 2019-nCoV, such as the sequence encoding the N, E, M, ORF3a, ORF7a, ORF8 polypeptide of a coronavirus, in particular of a coronavirus 2019-cCoV, or an antigenic or immunogenic fragment thereof that may be obtained using the a sequence disclosed in Genbank MN908947.3 or tha may have of the herein disclosed nucleotide sequences.

In a preferred embodiment of the invention, the nucleic acid construct comprises or consists of a recombinant MV-CoV nucleic acid molecule that comprises a second heterologous polynucleotide that encodes the N polypeptide of a coronavirus in particular of the 2019-nCoV, or an immunogenic fragment thereof or an antigenic fragment thereof, said second heterologous polynucleotide being cloned in a ATU at a different location from ATU2.

In a particular embodiment such nucleic acid construct is inserted, in particular cloned in an expression vector or a transfer vector, for example in a plasmid. Examples of suitable plasmids are the pTM plasmid know from Combredet et al (2003) or from WO 04/00876 of the pKM plasmid disclosed herein.

Any herein described nucleic acid construct of the invention is suitable and intended for the preparation of recombinant infectious replicative measles - coronavirus virus (MV-CoV) and accordingly said nucleic acid construct: (i) is used for insertion in a transfer genome vector that as a result comprises the cDNA molecule of the measles virus, especially of the Schwarz strain, for the production of said MV-CoV and yield of at least one polypeptide of a coronavirus (CoV), in particular of coronavirus 2019-nCoV, or consisting of an immunogenic fragment thereof or an antigenic fragment thereof, in particular a polypeptide which is consisting the spike (S) polypeptide or an antigenic or an immunogenic fragment thereof as disclosed herein or (ii) is such transfer vector, especially plasmid vector.

The nucleic acid construct may also be used for the production of viral-like particles (VLPs), in particular CoV VLPs

As an example, the pTM-MVSchw plasmid or the pTM2-MVSchw plasmid is suitable to prepare the transfer vector, by insertion of the CoV polynucleotide(s) necessary for the expression of at least one polypeptide consisting of the spike (S) polypeptide or another antigen such as N, M, E, ORF7a, ORF3a or ORF8 of a coronavirus (CoV), in particular of coronavirus 2019-nCoV, or consisting of an immunogenic fragment thereof or an antigenic fragment thereof. Alternatively such transfer vector may be the pKM vectors described in detail in the examples, including pKP-MVSchw-ATU1(eGFP), pKP-MVSchw-ATU2(eGFP), pKP-MVSchw-ATU3(eGFP) wherein the nucleotide sequence of the eGFP is replaced by the polynucleotide encoding the spike (S) polypeptide or another antigen such as N, M, E, ORF7a, ORF3a or ORF8 of a coronavirus (CoV), in particular of coronavirus 2019-nCoV, or consisting of an immunogenic fragment thereof or an antigenic fragment thereof. The herein disclosed sequences enable the person skilled in the art to have access to the position of the inserts contained in the plasmids to design and prepare insert substitution especially using the disclosure in the examples.

All the plasmids cited herein by reference to their deposit at the CNCM have been deposited at the Collection Nationale de Cultures de Microorganismes, 25 rue du Docteur Roux, 75724 Paris Cedex 15 (France).

According to a particular aspect of a nucleic acid construct, the invention relates to a transfer vector, in particular a plasmid vector, suitable for the rescue of a recombinant Measles virus (MV) which comprises the nucleic acid construct according to the invention, in particular a transfer vector selected from the group consisting of plasmid of SEQ ID NO: 28 (pTM-MVSchwarz), plasmid of SEQ ID NO: 29 (pTM-MVSchwarz-ATU2- CNCM I-3034 deposited on May 26, 2003 with the insertion of the GFPbis coding sequence), plasmid pTM-MVSchwarz-ATU3 (CNCM I-3037 deposited on May 26, 2003 with the insertion of the GFP coding sequence), plasmid of SEQ ID NO: 30 (pKP-MVSchwarz), plasmid of SEQ ID NO: 31 (pKP-MVSchwarz-ATU1), plasmid of SEQ ID NO: 32 (pKP-MVSchwarz-ATU2) and plasmid of SEQ ID NO: 33 (pKP-MVSchwarz-ATU3) wherein said transfer vector is recombined with (i) a first heterologous DNA polynucleotide encoding at least one polypeptide consisting of the spike polypeptide of a coronavirus, in particular of coronavirus 2019-nCoV, or consisting of an immunogenic fragment thereof or an antigenic fragment thereof has been operatively cloned within an additional transcription unit (ATU) inserted within the cDNA of the antigenomic (+) RNA or with (ii) a second heterologous polynucleotide encoding another polypeptide of a coronavirus, in particular of the 2019-nCoV, such as the sequence of the N, E, M, ORF3a, ORF7a, ORF8 polypeptide of a coronavirus,in particular of 2019-nCoV such as a sequence derived from the sequence disclosed in Genbank MN908947.3 or corresponding to a sequence disclosed herein.

In a particular embodiment, the transfer vector is a plasmid, especially one of the above plasmids recombined with a recombinant DNA MV-CoV sequence wherein the sequence encoding a polypeptide of 2019-nCoV is selected from the group consisting of:
- SEQ ID NO: 1 or 2 or 36 (construct S);
- SEQ ID NO: 4 (construct stab-S);
- SEQ ID NO: 6 (construct Secto);
- SED ID NO: 8 (construct stab-Secto);
- SEQ ID NO: 10 (construct S1),
- SEQ ID NO: 12 (construct S2),
- SEQ ID NO: 14 (construct stab-S2),
- SEQ ID NO: 16 (construct tri-Secto),
- SEQ ID NO: 18 (construct tristab-Secto) and
- SEQ ID NO: 21 or 37 (construct N).

When the sequence encoding the eGFP is present in the plasmid it is advantageously substituted by a sequence selected in the group defined above that is inserted in an ATU.

In a particular embodiment, the transfer vector is derived from the plasmids selected among:
- pKP-MSchwarz deposited under No. CNCM I-5493 on February 12, 2020,
- pKM-ATU2(eGFP) deposited under No. CNCM I- 5494 on February 12, 2020,
- pKM-ATU3(eGFP) deposited under No. CNCM I- 5495 on February 12, 2020, in particular is one of these plasmids comprising a polynucleotide selected among the polynucleotides having the sequence of SEQ ID NO: 1, 2 or 36 or 4, 6, 8, 10, 12, 14, 16, 18 21 or 37 inserted in an ATU in particular to replace the eGFP coding sequence.
   or is one of the plasmids selected among,
- pKM-ATU2-S_2019-nCoV deposited under No. CNCM I-5496 on February 12, 2020,
- pKM-ATU3-S_2019-nCoV deposited under No. CNCM I- 5497 on February 12, 2020.

The invention also concerns the use of said transfer vector to transform cells suitable for rescue of viral MV-CoV particles, in particular to transfect or to transduce such cells respectively with plasmids or with viral vectors harboring the nucleic acid construct of the invention, said cells being selected for their capacity to express required MV proteins for appropriate replication, transcription and encapsidation of the recombinant genome of the virus corresponding to the nucleic acid construct of the invention in recombinant infectious replicating MV-CoV particles.

In a preferred embodiment, the invention relates to a host cell which is a helper cell, an amplification cell or a production cell, transfected with the nucleic acid construct according to the invention or with the transfer plasmid vector according to the invention, or infected with the recombinant measles virus according to the invention, in particular a mammalian cell, VERO NK cells, CEF cells, human embryonic kidney cell line 293 or lines derived therefrom (293T or 293T-T7 cells deposited at the CNCM (Paris France) under number I-3618 déposé le 14 Juin 2006) or MRC5 cells.

Polynucleotides are thus present in said cells, which encode proteins that include in particular the N, P and L proteins of a MV (*i.e*., native MV proteins or functional variants thereof capable of forming ribonucleoprotein (RNP) complexes as a replicon), as stably expressed proteins at least for the N and P proteins or as or transitorily expressed proteins, functional in the transcription and replication of the recombinant viral MV-CoV particles. The N and P proteins may be expressed in the cells from a plasmid comprising their coding sequences or may be expressed from a DNA molecule inserted in the genome of the cell. The L protein may be expressed from a different plasmid. It may be expressed transitory. The helper cell is also capable of expressing a RNA polymerase suitable to enable the synthesis of the recombinant RNA derived from the nucleic acid construct of the invention, possibly as a stably expressed RNA polymerase. The RNA polymerase may be the T7 phage polymerase or its nuclear form (nlsT7).

In an embodiment of the invention, the cDNA clone of MV is from the same MV strain as the N protein and/or the P protein and/or the L protein. In another embodiment of the invention, the cDNA clone of a MV is from a different strain of virus than the N protein and/or the P protein and/or the L protein.

The invention also relates to a process for the preparation of recombinant infectious measles virus (MV) particles comprising:
1) transferring, in particular transfecting, the nucleic acid construct of the invention or the transfer vector containing such nucleic acid construct in a helper cell line which also expresses proteins necessary for transcription, replication and encapsidation of the antigenomic (+)RNA sequence of MV from its cDNA and under conditions enabling viral particles assembly; and
2) recovering the recombinant infectious MV-CoV particles expressing at least one polypeptide consisting of the spike (S) polypeptide of a coronavirus (CoV), in particular of coronavirus 2019-nCoV, or consisting of an immunogenic fragment thereof or an antigenic fragment thereof.

In a particular embodiment of the invention, this process comprises:
1) transfecting helper cells with a nucleic acid construct according to the invention and with a transfer vector, wherein said helper cells are capable of expressing helper functions to express an RNA polymerase, and to express the N, P and L proteins of a MV virus ;
2) co-cultivating said transfected helper cells of step 1) with passaged cells suitable for the passage of the MV attenuated strain from which the cDNA originates ;
3) recovering the recombinant infectious MV-CoV particles expressing at least one polypeptide consisting of the spike (S) polypeptide of a coronavirus (CoV), in particular of coronavirus 2019-nCoV, or consisting of an immunogenic fragment thereof or an antigenic fragment thereof.

In another particular embodiment of the invention, the method for the production of recombinant infectious MV-CoV particles comprises :
1) recombining a cell or a culture of cells stably producing a RNA polymerase, the N protein of a MV and the P protein of a MV, with a nucleic acid construct of the invention and with a vector comprising a nucleic acid encoding the L protein of a MV, and
2) recovering the recombinant infectious MV-CoV particles from said recombinant cell or culture of recombinant cells.

In a particular embodiment of said process, recombinant MV are produced, which express at least one polypeptide consisting of the spike (S) polypeptide of a coronavirus (CoV), in particular of coronavirus 2019-nCoV, or consisting of an immunogenic fragment thereof or an antigenic fragment thereof, in particular CoV VLPs expressing the same CoV protein(s).

The invention thus relates to recombinant infectious replicating MV-CoV particles that may be recovered from rescue helper cells or in production cells. Optionally, VLP expressing the CoV antigens disclosed in accordance with the invention may additionally be recovered.

In a particular embodiment, the recombinant MV are produced, which express at least one polypeptide consisting of the spike (S) polypeptide of a coronavirus (CoV), in particular of coronavirus 2019-nCoV, or consisting of an immunogenic fragment thereof or an antigenic fragment thereof according to the various embodiments disclosed herein.

In a particular embodiment, the recombinant MV particles express at least one polypeptide consisting of the N, E, M, ORF7a, ORF3a, ORF8 polypeptide of a coronavirus (CoV), in particular of coronavirus 2019-nCoV, or consisting of an immunogenic fragment thereof or an antigenic fragment thereof according to the various embodiments disclosed herein.

In a particular embodiment, the recombinant MV particles express at least one polypeptide consisting of the spike (S) polypeptide of a coronavirus (CoV), in particular of coronavirus 2019-nCoV, or consisting of an immunogenic fragment thereof or an antigenic fragment thereof according to the various embodiments disclosed herein and additionally express at least one polypeptide consisting of the N, E, M, ORF7a, ORF3a, ORF8 polypeptide of a coronavirus (CoV), in particular of coronavirus 2019-nCoV, or consisting of an immunogenic fragment thereof or an antigenic fragment thereof according to the various embodiments disclosed herein.

In a particular embodiment of the invention, the particles are obtained from a measles virus which is an attenuated virus strain selected from the group consisting of the Schwarz strain according to all embodiments disclosed herein, the Zagreb strain, the AIK-C strain, the Moraten strain, the Philips strain, the Beckenham 4A strain, the Beckenham 16 strain, the CAM-70 strain, the TD 97 strain, the Leningrad-16 strain, the Shanghai 191 strain and the Belgrade strain, in particular the Schwarz strain.

In a particular embodiment the recombinant measles virus, in particular the recombinant measles virus of the Schwarz strain, comprises in its genome a nucleic acid construct which encodes at least one polypeptide consisting of the spike polypeptide of a coronavirus, in particular of coronavirus 2019-nCoV, or an immunogenic fragment thereof or an antigenic fragment thereof or one polypeptide consisting of the N, E, M, ORF7a, ORF3a, ORF8 polypeptide of a coronavirus (CoV), in particular of coronavirus 2019-nCoV, or consisting of an immunogenic fragment thereof or an antigenic fragment thereof, in particular comprises in its genome a transcript of a nucleotide construct of the invention, in particular a nucleic acid construct which is a replicon of a transfer vector of the invention, said nucleic acid construct being operatively linked with said genome in an expression cassette.

In a particular embodiment, the nucleotide sequence of the nucleic acid molecule encoding the polypeptide of a coronavirus, in particular of 2019-nCoV is selected from the group consisting of SEQ ID NO.1, 2, 4, 6, 8, 10, 12, 14, 16, 18, 20, 21 and 36.

The invention also relates to a process for rescuing recombinant measles virus expressing at least one polypeptide consisting of at least one of the (i) N, E, M, ORF7a, ORF3a, ORF8 polypeptide of a coronavirus (CoV), in particular of coronavirus 2019-nCoV, or consisting of an immunogenic fragment thereof or an antigenic fragment thereof according to the various embodiments described herein or (ii) a polypeptide consisting of the spike (S) polypeptide of a coronavirus, in particular of 2019-nCoV or an immunogenic fragment thereof or an antigenic fragment thereof according to the various embodiments described herein comprising:
(a) co-transfecting cells, in particular helper cells, in particular HEK293 helper cells, stably expressing T7 RNA polymerase and measles virus N and P proteins with (i) the nucleic acid construct according to the invention or with the transfer plasmid vector according to the invention that encodes said at least one polypeptide, and with (ii) a vector, especially a plasmid, encoding the MV L polymerase,
(b) maintaining the transfected cells in conditions suitable for the production of recombinant measles virus;
(c) infecting cells enabling propagation of the recombinant measles virus by co-cultivating them with the transfected cells of step (b), in particular VERO cells;
(d) harvesting the recombinant measles virus expressing at least the polypeptide of the coronavirus or an immunogenic fragment or an antigenic fragment thereof of a coronavirus, in particular of 2019-nCoV.

According to a particular embodiment of said process, the transfer vector plasmid has the sequence of SEQ ID NO: 34, SEQ ID NO: 35, or is one of the vectors deposited at the CNCM and disclosed herein under numbers I-5496 or I-5497.

As used herein, the term "*recombining*" means introducing at least one polynucleotide into a cell, for example under the form of a vector, said polynucleotide integrating (entirely or partially) or not integrating into the cell genome (such as defined above).

According to a particular embodiment, recombination can be obtained with a first polynucleotide, which is the nucleic acid construct of the invention. Recombination can, also or alternatively, encompass introducing a polynucleotide, which is a vector encoding a RNA polymerase large protein (L) of a MV, whose definition, nature and stability of expression has been described herein.

In accordance with the invention, the cell or cell lines or a culture of cells stably producing a RNA polymerase, a nucleoprotein (N) of a measles virus and a polymerase cofactor phosphoprotein (P) of a measles virus is a cell or cell line as defined in the present specification or a culture of cells as defined in the present specification, *i.e*., are also recombinant cells to the extent that they have been modified by the introduction of one or more polynucleotides as defined above. In a particular embodiment of the invention, the cell or cell line or culture of cells, stably producing the RNA polymerase, the N and P proteins, does not produce the L protein of a measles virus or does not stably produce the L protein of a measles virus, *e.g*., enabling its transitory expression or production.

The production of recombinant infectious replicating MV-CoV particles of the invention may involve a transfer of cells transformed as described herein. The term "*transfer*" as used herein refers to the plating of the recombinant cells onto a different type of cells, and particularly onto monolayers of a different type of cells. These latter cells are competent to sustain both the replication and the production of infectious MV-CoV particles, *i.e*., respectively the formation of infectious viruses inside the cell and possibly the release of these infectious viruses outside of the cells. This transfer results in the co-culture of the recombinant cells of the invention with competent cells as defined in the previous sentence. The above transfer may be an additional, *i.e*., optional, step when the recombinant cells are not sufficiently efficient virus-producing culture, *i.e*., when infectious MV-CoV particles cannot be efficiently recovered from these recombinant cells. This step is introduced after further recombination of the recombinant cells of the invention with nucleic acid construct of the invention, and optionally a vector comprising a nucleic acid encoding a RNA polymerase large protein (L) of a measles virus.

In a particular embodiment of the invention, a transfer step is required since the recombinant cells, usually chosen for their capacity to be easily recombined are not efficient enough in the sustaining and production of recombinant infectious MV-CoV particles. In said embodiment, the cell or cell line or culture of cells of step 1) of the above-defined methods is a recombinant cell or cell line or culture of recombinant cells according to the invention.

Cells suitable for the preparation of the recombinant cells of the invention are prokaryotic or eukaryotic cells, particularly animal or plant cells, and more particularly mammalian cells such as human cells or non-human mammalian cells or avian cells or yeast cells. In a particular embodiment, cells, before recombination of its genome, are isolated from either a primary culture or a cell line. Cells of the invention may be dividing or non-dividing cells.

According to a preferred embodiment, helper cells are derived from human embryonic kidney cell line 293, which cell line 293 is deposited with the ATCC under No. CRL-1573. Particular cell line 293 is the cell line disclosed in the international application WO2008/078198 and referred to in the following examples.

According to another aspect of this process, the cells suitable for passage are CEF cells. CEF cells can be prepared from fertilized chicken eggs as obtained from EARL Morizeau, 8 rue Moulin, 28190 Dangers, France, or from any other producer of fertilized chicken eggs.

The process which is disclosed according to the present invention is used advantageously for the production of infectious replicative MV-CoV particles that may be used in an immunogenic composition. Optionally VLPs expressing CoV antigens may also be expressed that are appropriate for use in immunization compositions. Accordingly the invention concerns the recombinant MV-CoV particles of the invention for use in the elicitation of a humoral, especially a protective, in particular a neutralizing humoral response and/or a cellular response in an animal host, in particular a mammalian host, especially in a human being. The recombinant MV-CoV particles are in particular for use in the elicitation of a prophylactic response against infection by a coronavirus, in particular 2019-nCoV.

The invention thus relates to an immunogenic composition, advantageously a vaccine composition comprising (i) an effective dose of the recombinant measles virus according to the invention, and/or of the recombinant VLPs according to the invention and (ii) a pharmaceutically acceptable vehicle, wherein the composition or the vaccine elicits a humoral, especially a protective, in particular a neutralizing humoral response and/or a cellular response in an animal host, especially in a human being, in particular after a single immunization, against the polypeptide(s) of the coronavirus, in particular of 2019-nCoV or their fragments, that it expresses.

In a particular embodiment of the invention, said composition is used in the elicitation of a protective, and preferentially prophylactic, immune response against 2019-nCoV or against 2019-nCoV and against further distinct coronavirus(es), by the elicitation of antibodies recognizing coronavirus protein(s) or antigenic fragment(s) thereof, and/or by the elicitation of a cellular and/or humoral and cellular response against the Coronavirus, in a host in need thereof, in particular a human host, in particular a child. Preferably, said composition is devoid of added adjuvant.

The invention also relates to an immunogenic or vaccine composition comprising (i) an effective dose of the recombinant measles virus according to the invention, and/or of the recombinant VLPs according to the invention and (ii) a pharmaceutically acceptable vehicle for use in the prevention or treatment of an infection by CoV, in particular 2019-nCoV or in the prevention of clinical outcomes of infection by CoV in a host in need thereof, in particular a human host, in particular a child. In a particular embodiment, said composition is for administration to children, adolescents or travelers.

Said composition may comprise a suitable vehicle for administration *e.g*. a pharmaceutically acceptable vehicle to a host, especially a human host and may further comprise but not necessarily adjuvant to enhance immune response in a host. The inventors have indeed shown that the administration of the active ingredients of the invention may elicit an immune response without the need for external adjuvantation.

Such a vaccine composition comprises advantageously active principles (active ingredients) which comprise recombinant infectious replicating MV-CoV particles rescued from the vector and constructs as defined herein optionally associated with VLPs comprising the same CoV proteins.

In the context of the invention, the terms "associated" or "*in association*" refer to the presence, in a unique composition, of both recombinant infectious replicating MV-CoV particles and the above-mentioned CoV proteins, in particular as VLPs, usually as physically separate entities.

The administration scheme and dosage regime may require a unique administration of a selected dose of the recombinant infectious replicating MV-CoV particles according to the invention in association with the above-mentioned CoV proteins, in particular in association with CoV-VLPs expressing the same CoV proteins.

Alternatively it may require administration of multiple doses.

In a particular embodiment the administration is performed in accordance with a prime-boost regimen. Priming and boosting may be achieved with identical active ingredients consisting of said recombinant infectious replicating MV-CoV particles in association with the above-mentioned CoV proteins, in particular in association with CoV-VLPs expressing the same CoV proteins.

Alternatively priming and boosting administration may be achieved with different active ingredients, involving said recombinant infectious replicating MV-CoV particles in association with the above-mentioned CoV proteins, in particular in association with CoV-VLPs expressing the same CoV proteins, in at least one of the administration steps and other active immunogens of CoV, such as the above-mentioned CoV polypeptides or CoV-VLPs expressing the same CoV proteins, in other administration steps.

Administration of recombinant infectious replicating MV-CoV particles according to the invention in association with CoV-VLPs expressing the same CoV proteins elicits an immune response and may elicit antibodies that are cross-reactive for various CoV strains. Accordingly, administration of the active ingredients according to the invention, when prepared with the coding sequences of a particular strain of CoV, may elicit an immune response against a group of strains of CoV.

Considering that the currently known doses for human MV vaccines are in the range of 10³ to 10⁵ TCID50, a suitable dose of recombinant MV-CoV to be administered may be in the range of 0.1 to 10ng, in particular 0.2 to 6ng, and possibly as low as 0.2 to 2ng.

According to a particular embodiment of the invention, the immunogenic or vaccine composition defined herein may also be used for protection against an infection by the measles virus.

The invention also relates to a method for preventing a coronavirus virus related disease, in particular a disease related to infection by 2019-nCoV said method comprising the immunization of a mammalian, especially a human, in particular a child, by the injection, in particular by subcutaneous injection, of recombinant measles virus according to the invention.

The invention also relates to a method for treating a coronavirus virus related disease, in particular a disease related to infection by 2019-nCoV said method comprising the immunization of a mammalian, especially a human, in particular a child, by the injection, in particular subcutaneous injection, of recombinant measles virus according to the invention.

The invention also relates to a nucleic acid molecule that encodes a polypeptide of 2019-nCoV and which has been modified with respect to the native sequence. In particular the invention relates to the nucleic acid molecule comprising or consisting of a polynucleotide of sequence as disclosed in the Table 1 below for

| | |
|---|---|
| **S** polypeptide of nCoV | SEQ ID NO. 1 or NO.2 |
| **stab-S** polypeptide of nCoV | SEQ ID NO. 4 |
| **Secto** polypeptide of nCoV | SEQ ID NO. 6 |
| **stab-Secto** polypeptide of nCoV | SEQ ID NO. 8 |
| **S1** polypeptide of nCoV | SEQ ID NO. 10 |
| **S2** polypeptide of nCoV | SEQ ID NO. 12 |
| **stab-S2** polypeptide of nCoV | SEQ ID NO. 14 |
| **tri-Secto** polypeptide of nCoV | SEQ ID NO. 16 |
| **tristab-Secto** polypeptide of nCoV | SEQ ID NO. 18 |

The invention also concerns the plasmids disclosed hereunder:

| | |
|---|---|
| **pKP-MVSchw** | SEQ ID NO. 30 |
| **pKP-MVSchw-ATU1(eGFP)** | SEQ ID NO. 31 |
| **pKP-MVSchw-ATU2(eGFP)** | SEQ ID NO. 32 |
| **pKP-MVSchw-ATU3(eGFP)** | SEQ ID NO. 33 |
| **pKM-ATU2-S_2019-nCoV (optimized sequence)** | SEQ ID NO. 34 |
| **pKM-ATU3-S_2019-nCoV (optimized sequence)** | SEQ ID NO. 35 |

In a particular aspect the invention relates to the plasmid pKP-MVSchwarz deposited under No. CNCM I-5493 on February 12, 2020 or the plasmid pKP-MVSchw having the sequence of SEQ ID NO:30. This plasmid may be used as plasmid for cloning anu polynucleotide.

**Table 1. Native and codon/MV-optimized nucleotide sequences of the polynucleotide encoding particular peptides/proteins as well as amino acid sequences of these peptides/proteins used in the invention.**

| **Name of the compound, *i.e.* peptide/protein/antigen (abbreviation)** | **SEQ ID NO of the native nucleotide sequence of the polynucleotide encoding the compound** | **SEQ ID NO of the codon-optimized nucleotide sequence of the polynucleotide encoding the compound** | **SEQ ID NO of the MV-optimized nucleotide sequence of the polynucleotide encoding the compound** | **SEQ ID NO of the amino acid sequence of the compound** |
|---|---|---|---|---|
| **S** polypeptide of nCoV | 1 | 2 | 36 | 3 |
| **stab-S** polypeptide of nCoV | | 4 | | 5 |
| **Secto** polypeptide of nCoV | | 6 | | 7 |
| **stab-Secto** polypeptide of nCoV | | 8 | | 9 |
| **S1** polypeptide of nCoV | | 10 | | 11 |
| **S2** polypeptide of nCoV | | 12 | | 13 |
| **stab-S2** polypeptide of nCoV | | 14 | | 15 |
| **tri-Secto** polypeptide of nCoV | | 16 | | 17 |
| **tristab-Secto** polypeptide of nCoV | | 18 | | 19 |
| **N** polypeptide of nCoV | 20 | 21 | 37 | 22 |
| **E** polypeptide of nCoV | | | | 23 |
| **M** polypeptide of nCoV | | | | 24 |
| **ORF8** polypeptide of nCoV | | | | 25 |
| **ORF3a** polypeptide of nCoV | | | | 26 |
| **ORF7a** polypeptide of nCoV | | | | 27 |

| **Name of the transfer vector plasmid and elements** | **SEQ ID NO** |
|---|---|
| **pTM-MVSchw** | 28 |
| **pTM2-MVSchw-gfp** | 29 |
| **pKP-MVSchw** | 30 |
| **pKP-MVSchw-ATU1(eGFP)** | 31 |
| **pKP-MVSchw-ATU2(eGFP)** | 32 |
| **pKP-MVSchw-ATU3(eGFP)** | 33 |
| **pKM-ATU2-S_2019-nCoV (optimized sequence)** | 34 |
| **pKM-ATU3-S_2019-nCoV (optimized sequence)** | 35 |
| **pTM3-MVSchw-gfp** | 38 |
| **ATU1(eGFP)** | 39 |
| **ATU2(eGFP)** | 40 |
| **ATU3(eGFP)** | 41 |

The disclosed sequences are further characterized by the following annotations relating to the nucleotide or amino acid residues:
**Native nucleotide sequence of the spike (S) polypeptide of nCoV (SEQ ID NO: 1)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..3846 |
| | /cds_type="ORF" |
| | /product="spike protein (S)" |
| | /label="2019-nCoV_S ORF" |
| Site | join(3853..3858,3853^3854) |
| | /site_type="restriction site" |
| | /note="Name: BssHII" |
| | /note="Pattern: gcgcgc" |
| | /label="BssHII" |

**Codon-optimized nucleotide sequence of the spike (s) polypeptide of nCoV (SEQ ID NO: 2)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| sig_peptide | 25..69 |
| | /note="predicted from Phobius" |
| | /note="predicted from SignalP-5.0" |
| | /label="Signal_peptide" |
| CDS | 25..3849 |
| | /product="spike protein (S)" |
| | /label="2019-nCoV_Sopt ORF" |
| Site | 2068..2079 |
| | /site_type="cleavage site" |
| | /note="polybasic furin-like cleavage site" |
| | /label="predicted S1/S2 cleavage site" |
| Region | 3664..3732 |
| | /site_type="transmembrane-region" |
| | /note="predicted from TMHMM2.0" |
| | /note="predicted from PHOBIUS" |
| | /label="TM domain" |
| Site | join(3853..3858,3853^3854) |
| | /site_type="restriction site" |
| | /note="Name: BssHII" |
| | /note="Pattern: gcgcgc" |
| | /label="BssHII" |

**Codon-optimized nucleotide sequence of a stabilized form of the spike (stab-S) polypeptide of nCoV (SEQ ID NO: 4)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| sig_peptide | 25..69 |
| | /note="predicted from Phobius" |
| | /note="predicted from SignalP-5.0" |
| | /label="Signal_peptide" |
| CDS | 25..3849 |
| | /product="stabilized spike protein (S)" |
| | /label="2019-nCoV_stab-Sopt ORF" |
| Site | 2068..2079 |
| | /site_type="cleavage site" |
| | /note="polybasic furin-like cleavage site" |
| | /label="predicted S1/S2 cleavage site" |
| Site | 2980..2985 |
| | /site_type="mutagenized site" |
| | /function="stabilizing double mutation" |
| | /label="K986P + V987P mutations" |
| Region | 3664..3732 |
| | /site_type="transmembrane-region" |
| | /note="predicted from TMHMM2.0" |
| | /note="predicted from PHOBIUS" |
| | /label="TM domain" |
| Site | join(3853..3858,3853^3854) |
| | /site_type="restriction site" |
| | /note="Name: BssHII" |
| | /note="Pattern: gcgcgc" |
| | /label="BssHII" |

**Stabilized form of the spike (stab-S) polypeptide of nCoV (SEQ ID NO: 5)**

| FEATURES | Location/Qualifiers |
|---|---|
| Site | 986..987 |
| | /site_type="mutagenized site" |
| | /label="K986P + V987P mutations" |

**Codon-optimized nucleotide sequence of the soluble and monomeric form of the spike (Secto) ectodomain polypeptide of nCoV (SEQ ID NO: 6)**

| FEATURES | Location/Qualifiers |
|---|---|
| Site | join(17..22,17^18) |
| | /site_type="restriction site" |
| | /note="Name: BsiWI" |
| | /note="Pattern: cgtacg" |
| | /label="BsiWI" |
| sig_peptide | 29..73 |
| | /note="predicted from Phobius" |
| | /note="predicted from SignalP-5.0" |
| | /label="Signal_peptide" |
| CDS | 29..3667 |
| | /product="soluble S ectodomain" |
| | /label="2019-NCoV_Secto-opt ORF" |
| Site | 2072..2083 |
| | /site_type="cleavage site" |
| | /note="polybasic furin-like cleavage site" |
| | /label="predicted S1/S2 cleavage site" |
| Site | join(3671..3676,3671^3672) |
| | /site_type="restriction site" |
| | /note="Name: BssHII" |
| | /note="Pattern: gcgcgc" |
| | /label="BssHII" |

**Codon-optimized nucleotide sequence of the stabilized form of the spike (stab-Secto) ectodomain polypeptide of nCoV (SEQ ID NO: 8)**

| FEATURES | Location/Qualifiers |
|---|---|
| Site | join(17..22,17^18) |
| | /site_type="restriction site" |
| | /note="Name: BsiWI" |
| | /note="Pattern: cgtacg" |
| | /label="BsiWI" |
| sig_peptide | 29..73 |
| | /note="predicted from Phobius" |
| | /note="predicted from SignalP-5.0" |
| | /label="Signal_peptide" |
| CDS | 29..3667 |
| | /product="stabilized soluble S ectodomain" |
| | /label="2019-NCoV_stab-Secto-opt ORF" |
| Site | 2072..2083 |
| | /site_type="cleavage site" |
| | /note="polybasic furin-like cleavage site" |
| | /label="predicted S1/S2 cleavage site" |
| Site | 2984..2989 |
| | /site_type="mutagenized site" |
| | /function="stabilizing double mutation" |
| | /label="K986P + V987P mutations" |
| Site | join(3671..3676,3671^3672) |
| | /site_type="restriction site" |
| | /note="Name: BssHII" |
| | /note="Pattern: gcgcgc" |
| | /label="BssHII" |

**Stabilized form of the spike (stab-Secto) ectodomain polypeptide of nCoV (SEQ ID NO: 9)**

| FEATURES | Location/Qualifiers |
|---|---|
| Site | 986..987 |
| | /site_type="mutagenized site" |
| | /label="K986P + V987P mutations" |

**Codon-optimized nucleotide sequence of the S1 polypeptide of nCoV (SEQ ID NO: 10)**

| FEATURES | Location/Qualifiers |
|---|---|
| Site | join(17..22,17^18) |
| | /site_type="restriction site" |
| | /note="Name: BsiWI" |
| | /note="Pattern: cgtacg" |
| | /label="BsiWI" |
| sig_peptide | 29..73 |
| | /note="predicted from Phobius" |
| | /note="predicted from SignalP-5.0" |
| | /label="Signal_peptide" |
| CDS | 29..2077 |
| | /product="soluble S1 subdomain" |
| | /label="2019-NCoV_S1 opt ORF" |
| Site | join(2081..2086,2081^2082) |
| | /site_type="restriction site" |
| | /note="Name: BssHII" |
| | /note="Pattern: gcgcgc" |
| | /label="BssHII" |

**Codon-optimized nucleotide sequence of the S2 polypeptide of nCoV (SEQ ID NO: 12)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..1842 |
| | /product="S2 domain linked to TM and cytoplasmic domain |
| | and fused to signal peptide" |
| | /label="S2-TM-cyto_2019-NCoV-opt" |
| sig_peptide | 25..69 |
| | /note="predicted from Phobius" |
| | /note="predicted from SignalP-5.0" |
| Site | join(67..72,67^68) |
| | /site_type="restriction site" |
| | /note="Name: NgoMIV" |
| | /note="Pattern: gccggc" |
| | /label="NgoMIV" |
| Region | 67..72 |
| | /region_type="Connecting peptide" |
| | /label="Ala-Gly linker" |
| Region | 73..1656 |
| | /region_type="Domain" |
| | /label="S2 domain" |
| Region | 1657..1725 |
| | /site_type="transmembrane-region" |
| | /note="predicted from TMHMM2.0" |
| | /note="predicted from PHOBIUS" |
| | /label="TM domain" |
| Region | 1726..1836 |
| | /region_type="Cytoplasmic" |
| | /label="cytoplasmic domain" |
| Site | join(1843..1848,1843^1844) |
| | /site_type="restriction site" |
| | /note="Name: BssHII" |
| | /note="Pattern: gcgcgc" |
| | /label="BssHII" |

**S2 polypeptide of nCoV (SEQ ID NO: 13)**

| FEATURES | Location/Qualifiers |
|---|---|
| sig_peptide | 1..15 |
| | /label="signal_peptide" |
| Region | 15..16 |
| | /region_type="Connecting peptide" |
| | /label="AG linker" |
| Region | 17..544 |
| | /region_type="Extracellular" |
| | /label="S2_domain" |
| Region | 545..567 |
| | /site_type="transmembrane-region" |
| | /label="TM_domain" |
| Region | 568..604 |
| | /region_type="Cytoplasmic" |
| | /label="cytoplasmic_domain" |

**Codon-optimized nucleotide sequence of the stabilized form of the S2 (stab-S2) polypeptide of nCoV (SEQ ID NO: 14)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..1842 |
| | /product="stabilized S2 domain linked to TM and |
| | cytoplasmic domain and fused to signal peptide" |
| | /label="stab-S2-TM-cyto_2019-NCoV-opt" |
| sig_peptide | 25..69 |
| | /note="predicted from Phobius" |
| | /note="predicted from SignalP-5.0" |
| Site | join(67..72,67^68) |
| | /site_type="restriction site" |
| | /note="Name: NgoMIV" |
| | /note="Pattern: gccggc" |
| | /label="NgoMIV" |
| Region | 67..72 |
| | /region_type="Connecting peptide" |
| | /label="Ala-Gly linker" |
| Region | 73..1656 |
| | /region_type="Domain" |
| | /label="S2 domain (stabilized)" |
| Site | 973..978 |
| | /site_type="mutagenized site" |
| | /note="stabilizing double mutation" |
| | /label="K986P + V987P mutations" |
| Region | 1657..1725 |
| | /site_type="transmembrane-region" |
| | /note="predicted from TMHMM2.0" |
| | /note="predicted from PHOBIUS" |
| | /label="TM domain" |
| Region | 1726..1836 |
| | /region_type="Cytoplasmic" |
| | /label="cytoplasmic domain" |
| Site | join(1843..1848,1843^1844) |
| | /site_type="restriction site" |
| | /note="Name: BssHII" |
| | /note="Pattern: gcgcgc" |
| | /label="BssHII" |

**Stabilized form of the S2 polypeptide (stab-S2) of nCoV (SEQ ID NO: 15)**

| FEATURES | Location/Qualifiers |
|---|---|
| sig_peptide | 1..15 |
| | /label="signal_peptide" |
| Region | 15..16 |
| | /region_type="Connecting peptide" |
| | /label="AG linker" |
| Region | 17..544 |
| | /region_type="Extracellular" |
| | /label="S2_domain" |
| Site | 317..318 |
| | /site_type="mutagenized site" |
| | /label="K986P + V987P mutations" |
| Region | 545..567 |
| | /site_type="transmembrane-region" |
| | /label="TM_domain" |
| Region | 568..604 |
| | /region_type="Cytoplasmic" |
| | /label="cytoplasmic_domain" |

**Codon-optimized nucleotide sequence of the soluble and trimerized form of the spike (tri-Secto) polypeptide of nCoV (SEQ ID NO: 16)**

| FEATURES | Location/Qualifiers |
|---|---|
| Site | join(17..22,17^18) |
| | /site_type="restriction site" |
| | /note="Name: BsiWI" |
| | /note="Pattern: cgtacg" |
| | /label="BsiWI" |
| CDS | 29..3754 |
| | /product="trimerized soluble S ectodomain" |
| | /label="2019-NCoV_tri-Secto-opt ORF" |
| sig_peptide | 29..73 |
| | /note="predicted from Phobius" |
| | /note="predicted from SignalP-5.0" |
| | /label="Signal_peptide" |
| Region | 74..3661 |
| | /region_type="Domain" |
| | /label="S ectodomain" |
| Site | 2072..2083 |
| | /site_type="cleavage site" |
| | /note="polybasic furin-like cleavage site" |
| | /label="predicted S1/S2 cleavage site" |
| Region | 3662..3670 |
| | /region_type="Connecting peptide" |
| | /label="SGG linker" |
| Region | 3668..3748 |
| | /region_type="Coiled coil" |
| | /function="trimerization foldon" |
| | /label="T4 fibritin foldon" |
| Site | join(3755..3760,3755^3756) |
| | /site_type="restriction site" |
| | /note="Name: BssHII" |
| | /note="Pattern: gcgcgc" |
| | /label="BssHII" |

**Soluble and trimerized form of the spike (tri-Secto) polypeptide of nCoV (SEQ ID NO: 17)**

| FEATURES | Location/Qualifiers |
|---|---|
| sig_peptide | 1..15 |
| | /label="signal_peptide" |
| Region | 16..1211 |
| | /region_type="Extracellular" |
| | /label="S_ectodomain" |
| Region | 1212..1214 |
| | /region_type="Connecting peptide" |
| | /label="SGG_linker" |
| Region | 1214..1240 |
| | /region_type="Coiled coil" |
| | /label="T4_fibritin_foldon" |

**Codon-optimized nucleotide sequence of the stabilized and trimeric form of the spike ectodomain (tristab-Secto) polypeptide of nCoV (SEQ ID NO: 18)**

| FEATURES | Location/Qualifiers |
|---|---|
| Site | join(17..22,17^18) |
| | /site_type="restriction site" |
| | /note="Name: BsiWI" |
| | /note="Pattern: cgtacg" |
| | /label="BsiWI" |
| CDS | 29..3754 |
| | /product="stabilized and trimerized soluble S ectodomain" |
| | /label="2019-NCoV_tristab-Secto-opt ORF" |
| sig_peptide | 29..73 |
| | /note="predicted from Phobius" |
| | /note="predicted from SignalP-5.0" |
| | /label="Signal_peptide" |
| Region | 74..3661 |
| | /region_type="Domain" |
| | /label="S ectodomain (stabilized)" |
| Site | 2072..2083 |
| | /site_type="cleavage site" |
| | /note="polybasic furin-like cleavage site" |
| | /label="predicted S1/S2 cleavage site" |
| Site | 2984..2989 |
| | /site_type="mutagenized site" |
| | /function="stabilizing double mutation" |
| | /label="K986P + V987P mutations" |
| Region | 3662..3670 |
| | /region_type="Connecting peptide" |
| | /label="SGG linker" |
| Region | 3668..3748 |
| | /region_type="Coiled coil" |
| | /function="trimerization foldon" |
| | /label="T4 fibritin foldon" |
| Site | join(3755..3760,3755^3756) |
| | /site_type="restriction site" |
| | /note="Name: BssHII" |
| | /note="Pattern: gcgcgc" |
| | /label="BssHII" |

**Stabilized and trimeric form of the spike ectodomain (tristab-Secto) polypeptide of nCoV (SEQ ID NO: 19)**

| FEATURES | Location/Qualifiers |
|---|---|
| sig_peptide | 1..15 |
| | /label="signal_peptide" |
| Region | 16..1211 |
| | /region_type="Extracellular" |
| | /label="S_ectodomain" |
| Site | 986..987 |
| | /site_type="mutagenized site" |
| | /label="K986P + V987P mutations" |
| Region | 1212..1214 |
| | /region_type="Connecting peptide" |
| | /label="SGG_linker" |
| Region | 1214..1240 |
| | /region_type="Coiled coil" |
| | /label="T4_fibritin_foldon" |

**Native nucleotide sequence of the N polypeptide of CoV (SEQ ID NO: 20)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..1284 |
| | /cds_type="ORF" |
| | /product="nucleoprotein (N)" |
| | /label="2019-nCoV_N ORF" |
| Site | join(1291..1296, 1291^1292) |
| | /site_type="restriction site" |
| | /note="Name: BssHII" |
| | /note="Pattern: gcgcgc" |
| | /label="BssHII" |

**Codon-optimized nucleotide sequence of the N polypeptide of CoV (SEQ ID NO: 21)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..1287 |
| | /product="nucleoprotein (N)" |
| | /label="2019-nCoV_Nopt ORF" |
| Site | join(1291..1296, 1291 ^1292) |
| | /site_type="restriction site" |
| | /note="Name: BssHII" |
| | /note="Pattern: gcgcgc" |
| | /label="BssHII" |

**pKP-MVSchw (SEQ ID NO: 30)**

| | |
|---|---|
| LOCUS | pKP-MVSchw 17858 bp DNA circular UNA |
| DEFINITION | Abbreviated names : pKP-MSchwarz, pKM |

| COMMENT FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..15976 |
| | /label="MVSchwarz_antigenome" |
| misc_signal | 83..134 |
| | /standard_name="leader" |
| misc_feature | 135..137 |
| | /function="nontranscribed intergenic trinucleotide" |
| | /standard_name="Gene Junction" |
| | /label="leader-N GJ" |
| promoter | 138..148 |
| | /function="start of transcription" |
| | /label="N gene start" |
| CDS | 190..1767 |
| | /codon_start=1 |
| | /product="nucleocapsid protein" |
| | /label="N ORF" |
| terminator | 1816..1826 |
| | /function="polyadenylation signal" |
| | /label="N gene end" |
| misc_feature | 1827..1829 |
| | /function="nontranscribed intergenic trinucleotide" |
| | /standard_name="gene junction" |
| | /label="N-P GJ" |
| promoter | 1830..1840 |
| | /function="restart of transcription" |
| | /label="P/V/C gene start" |
| CDS | join(1889..2580,2580..2787) |
| | /note="addition of an additional G at 2499 to generate V |
| | mRNA" |
| | /label="V ORF" |
| CDS | 1889..3412 |
| | /codon_start=1 |
| | /product="phosphoprotein" |
| | /label="P ORF" |
| CDS | 1911..2471 |
| | /codon_start=1 |
| | /product="nonstructural C protein" |
| | /label="C ORF" |
| misc_signal | 2570..2586 |
| | editing of mRNA" |
| | /note="addition of an additional G at 2499 to generate V |
| | mRNA" |
| | /label="editing motif" |
| Site | join(3446..3451,3446^3447) |
| | /site_type="restriction site" |
| | /note="Name: Sall" |
| | /note="Pattern: gtcgac" |
| | /label="Sall" |
| Site | join(3455..3460,3455^3456) |
| | /site_type="restriction site" |
| | /note="Name: Spel" |
| | /note="Pattern: actagt" |
| | /label="Spel" |
| terminator | 3474..3484 |
| | /function="polyadenylation signal" |
| | /label="P/V/C gene end" |
| misc_feature | 3485..3487 |
| | /function="nontranscribed intergenic trinucleotide" |
| | /label="P-M GJ" |
| promoter | 3488..3498 |
| | /function="restart of transcription" |
| | /label="M gene start" |
| CDS | 3520..4527 |
| | /codon_start=1 |
| | /product="matrix protein" |
| | /label="M ORF" |
| terminator | 4943..4953 |
| | /function="polyadenylation signal" /label="M gene end" |
| misc_feature | 4954..4956 |
| | /function="non transcribed intergenic trinucleotide" |
| | /standard_name="Gene Junction" |
| | /label="M-F GJ" |
| promoter | 4957..4967 |
| | /function="restart of transcription" |
| | /label="F gene start" |
| CDS | 5531..7192 |
| | /codon_start=1 |
| | /product="fusion protein (alternate product)" |
| | /label="F ORF (alt.)" |
| CDS | 5540..7192 |
| | /codon_start=1 |
| | /product="fusion protein (main product)" |
| | /label="F ORF" |
| Site | join (6353..6358,6353^6354) |
| | /site_type="restriction site" |
| | /note="Name: Sall" |
| | /note="Pattern: gtcgac" |
| | /label="Sall" |
| terminator | 7319..7329 |
| | /function="polyadenylation signal" /label="F gene end" |
| misc_feature | 7330..7332 |
| | /function="nontranscribed intergenic trinucleotide" |
| | /standard_name="Gene Junction" |
| | /label="F-H GJ" |
| promoter | 7333..7343 |
| | /function="restart of transcription" |
| | /label="H gene start" |
| CDS | 7353..9206 |
| | /codon_start=1 |
| | /product="hemagglutinin protein" |
| | /label="H ORF" |
| Site | join(9257..9262,9257^9258) |
| | /site_type="restriction site" |
| | /note="Name: Spel" |
| | /note="Pattern: actagt" |
| | /label="Spel" |
| terminator | 9280..9290 |
| | /function="polyadenylation signal" |
| | /label="H gene end" |
| misc_feature | 9291..9293 |
| | /function="nontranscribed intergenic trinucleotide" |
| | /standard_name="Gene Junction" |
| | /label="H-L GJ" |
| promoter | 9294..9304 |
| | /function="restart of transcription" |
| | /label="L gene start" |
| CDS | 9316..15867 |
| | /codon_start=1 |
| | /product="large polymerase" |
| | /label="L ORF" |
| terminator | 15926..15936 |
| | /function="polyadenylation signal" |
| | /label="L gene end" |
| misc_feature | 15937..15939 |
| | /function="non transcribed intergenic trinucleotide" |
| | /standard_name="Gene Junction" |
| | /label="L-trailer GJ" |
| misc_signal | 15940..15976 |
| | /standard_name="trailer" |
| Site | 15977..16060 |
| | /site_type="cleavage site" |
| | /function="self-cleaves before position a to generate an |
| | antigenome with an exact 3' end" |
| | /label="genomic HDV ribozyme" |
| Region | 16074..16196 |
| | /note="nt 24107..24229 from bact. T7 DNA" |
| | /label="full terminator region from bacteriophage T7" |
| terminator | 16131..16177 |
| | /label="T7 terminator" |
| Site | join(16203..16210,16204^16205) |
| | /site_type="restriction site" |
| | /note="Name: Notl" |
| | /note="Pattern: gcggccgc" |
| | /label="Notl" |
| misc_feature | 16211..17858 |
| | /label="plasmid_backbone" |
| CDS | 16261..17070 |
| | /function="kanamycin resistance" |
| | /label="kanaR" |
| rep_origin | 17134..17807 |
| | /label="pUC origin" |

**pKP-MVSchw-ATU1(eGFP) (SEQ ID NO: 31)**

| | |
|---|---|
| LOCUS | pKP-MVSchw-ATU1(eGFP) 18734 bp DNA circular UNA |
| DEFINITION | Abbreviated names : pKM-ATU1(eGFP) ; pKM1-eGFP |

| COMMENT FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..16852 |
| | /label="MVSchw-ATU1-eGFP_antigenome" |
| misc_feature | 135..137 |
| | /function="nontranscribed intergenic trinucleotide" |
| | /standard_name="Gene Junction" |
| | /note="identical to leader-N GJ" |
| | /label="leader-ATU1 GJ" |
| misc_feature | 135..1010 |
| | /feature_type="Insertion" |
| | /function="additional transcription unit" |
| | /label="ATU1" |
| promoter | 138..148 |
| | /function="start of transcription" |
| | /note="identical to N gene start" |
| | /label="ATU1 gene start" |
| Site | join(190..195,190^191) |
| | /site_type="restriction site" |
| | /note="Name: Mlul" |
| | /note="Pattern: acgcgt" |
| | /label="Mlul" |
| Site | join(193..198,193^194) |
| | /site_type="restriction site" |
| | /note="Name: BsiWI" |
| | /note="Pattern: cgtacg" |
| | /label="BsiWI" |
| CDS | 199..918 |
| | /product="enhanced Green Fluorescent Protein" |
| | /label="eGFP ORF" |
| Site | join(919..924,919^920) |
| | /site_type="restriction site" |
| | /note="Name: BssHII" |
| | /note="Pattern: gcgcgc" |
| | /label="BssHII" |
| Site | join(925..930,927^928) |
| | /site_type="restriction site" |
| | /note="Name: Afel" |
| | /note="Pattern: agcgct" |
| | /label="Afel" |
| Site | join(933..938,937^938) |
| | /site_type="restriction site" |
| | /note="Name: Aatll" |
| | /note="Pattern: gacgtc" |
| | /label="Aatll" |
| terminator | 1000..1010 |
| | /function="polyadenylation signal" |
| | /note="corresponds to N gene end sequence" |
| | /label="engineered ATU1 gene end" |
| misc_feature | 1011..1013 |
| | /function="nontranscribed intergenic trinucleotide" |
| | /standard_name="Gene Junction" |
| | /note="original leader-N GJ" |
| | /label="ATU1-N GJ" |
| promoter | 1014..1024 |
| | /function="start of transcription" |
| | /note="original N gene start" |
| | /label="N gene start" |
| Site | 16853..16936 |
| | /site_type="cleavage site" |
| | /function="self-cleaves before position a to generate an |
| | antigenome with an exact 3' end" |
| | /label="genomic HDV ribozyme" |
| terminator | 17007..17053 |
| | /label="T7 terminator" |
| Site | join(17079..17086,17080^17081) |
| | /site_type="restriction site" |
| | /note="Name: Notl" |
| | /note="Pattern: gcggccgc" |
| | /label="Notl" |
| misc_feature | 17087..18734 |
| | /label="plasmid_backbone" |
| CDS | 17137..17946 |
| | /function="kanamycin resistance" |
| | /label="kanaR" |
| rep_origin | 18010..18683 |
| | /label="pUC origin" |

**pKP-MVSchw-ATU2(eGFP) (SEQ ID NO: 32)**

| FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..16804 |
| | /label="MVSchw-ATU2-eGFP_antigenome" |
| misc_feature | 3460..4287 |
| | /featu re_type="Insertion" |
| | /function="additional transcription unit" |
| | /label="ATU2" |
| terminator | 3476..3486 |
| | /function="polyadenylation signal" |
| | /note="corresponds to N gene end sequence" |
| | /label="engineered P/V/C gene end" |
| misc_feature | 3487..3489 |
| | /function="nontranscribed intergenic trinucleotide" |
| | /standard_name="gene junction" |
| | /label="P-ATU2 GJ" |
| promoter | 3490..3500 |
| | /function="restart of transcription" |
| | /note="corresponds to P/V/C gene start sequence" |
| | /label="ATU2 gene start" |
| Site | join(3523..3528,3523^3524) |
| | /site_type="restriction site" |
| | /note="Name: Mlul" |
| | /note="Pattern: acgcgt" |
| | /label="Mlul" |
| Site | join(3526..3531,3526^3527) |
| | /site_type="restriction site" |
| | /note="Name: BsiWI" |
| | /note="Pattern: cgtacg" |
| | /label="BsiWI" |
| CDS | 3532..4251 |
| | /cds_type="ORF" |
| | /product="enhanced Green Fluorescent Protein" |
| | /label="eGFP ORF" |
| Site | join(4252..4257,4252^4253) |
| | /site_type="restriction site" |
| | /note="Name: BssHII" |
| | /note="Pattern: gcgcgc" |
| | /label="BssHII" |
| Site | join(4258..4263,4260^4261) |
| | /site_type="restriction site" |
| | /note="Name: Afel" |
| | /note="Pattern: agcgct" |
| | /label="Afel" |
| Site | join(4266..4271,4270^4271) |
| | /site_type="restriction site" |
| | /note="Name: Aatll" |
| | /note="Pattern: gacgtc" |
| | /label="Aatll" |
| terminator | 4302..4312 |
| | /function="polyadenylation signal" |
| | /label="P/V/C gene end" |
| misc_feature | 4313..4315 |
| | /function="nontranscribed intergenic trinucleotide" |
| | /label="P-M GJ" |
| promoter | 4316..4326 |
| | /function="restart of transcription" /label="M gene start" |
| terminator | 5771..5781 |
| | /function="polyadenylation signal" |
| | /label="M gene end" |
| misc_feature | 5782..5784 |
| | /function="non transcribed intergenic trinucleotide" |
| | /standard_name="Gene Junction" |
| | /label="M-F GJ" |
| promoter | 5785..5795 |
| | /function="restart of transcription" |
| | /label="F gene start" |
| Site | 16805..16888 |
| | /site_type="cleavage site" |
| | /function="self-cleaves before position a to generate an |
| | antigenome with an exact 3' end" |
| | /label="genomic HDV ribozyme" |
| terminator | 16959..17005 |
| | /label="T7 terminator" |
| Site | join(17031..17038,17032^17033) |
| | /site_type="restriction site" |
| | /note="Name: Notl" |
| | /note="Pattern: gcggccgc" |
| | /label="Notl" |
| misc_feature | 17039..18686 |
| | /label="plasmid_backbone" |
| CDS | 17089..17898 |
| | /function="kanamycin resistance" |
| | /label="kanaR" |
| rep_origin | 17962..18635 |
| /label="pUC | /label="pUC origin" |

**pKP-MVSchw-ATU3(eGFP) (SEQ ID NO: 33)**

| | |
|---|---|
| LOCUS | pKP-MVSchw-ATU3(eGFP) 18686 bp DNA circular UNA |
| DEFINITION | Abbreviated names : pKM-ATU3(eGFP); pKM3-eGFP |

| COMMENT FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..16804 |
| | /label="MVSchw-ATU3-eGFP_antigenome" |
| misc_feature | 9258..10085 |
| | /feature_type="Insertion" |
| | /function="additional transcription unit" |
| | /label="ATU3" |
| terminator | 9278..9288 |
| | /function="polyadenylation signal" |
| | /note="corresponds to N gene end sequence" |
| | /label="engineered P/V/C gene end" |
| misc_feature | 9289..9291 |
| | /function="nontranscribed intergenic trinucleotide" |
| | /standard_name="gene junction" |
| | /label="P-ATU2 GJ" |
| promoter | 9292..9302 |
| | /function="restart of transcription" |
| | /note="corresponds to P/V/C gene start sequence" |
| | /label="ATU2 gene start" |
| Site | join(9325..9330,9325^9326) |
| | /site_type="restriction site" |
| | /note="Name: Mlul" |
| | /note="Pattern: acgcgt" |
| | /label="Mlul" |
| Site | join(9328..9333,9328^9329) |
| | /site_type="restriction site" |
| | /note="Name: BsiWI" |
| | /note="Pattern: cgtacg" |
| | /label="BsiWI" |
| CDS | 9334..10053 |
| | /cds_type="ORF" |
| | /product="enhanced Green Fluorescent Protein" |
| | /label="eGFP ORF" |
| Site | join(10054..1 0059, 10054^10055) |
| | /site_type="restriction site" |
| | /note="Name: BssHII" |
| | /note="Pattern: gcgcgc" |
| | /label="BssHII" |
| Site | join(10060..1 0065, 10062^10063) |
| | /site_type="restriction site" |
| | /note="Name: Afel" |
| | /note="Pattern: agcgct" |
| | /label="Afel" |
| Site | join(10068..1 0073, 10072^10073) |
| | /site_type="restriction site" |
| | /note="Name: Aatll" |
| | /note="Pattern: gacgtc" |
| | /label="Aatll" |
| terminator | 10108..10118 |
| | /function="polyadenylation signal" |
| | /label="H gene end" |
| misc_feature | 10119..10121 |
| | /function="nontranscribed intergenic trinucleotide" |
| | /standard_name="Gene Junction" |
| | /label="H-L GJ" |
| promoter | 10122..10132 |
| | /function="restart of transcription" |
| | /label="L gene start" |
| Site | 16805..16888 |
| | /site_type="cleavage site" |
| | /function="self-cleaves before position a to generate an |
| | antigenome with an exact 3' end" |
| | /label="genomic HDV ribozyme" |
| terminator | 16959..17005 |
| | /label="T7 terminator" |
| Site | join(17031..17038,17032^17033) |
| | /site_type="restriction site" |
| | /note="Name: Notl" |
| | /note="Pattern: gcggccgc" |
| | /label="Notl" |
| misc_feature | 17039..18686 |
| | /label="plasmid_backbone" |
| CDS | 17089..17898 |
| | /function="kanamycin resistance" |
| | /label="kanaR" |
| rep_origin | 17962..18635 |
| | /label="pUC origin" |

**pKM-ATU2-S 2019-nCoV (optimized sequence) (SEQ ID NO: 34)**

| | |
|---|---|
| LOCUS | pKM-ATU2-S_2019-nCoV 21800 bp DNA circular UNA |
| DEFINITION | Abbreviated names : pKM2-S_nCoV |

| COMMENT FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..19918 |
| | /label="MVSchw-ATU2-S_2019-nCoV" |
| misc_feature | 3460..7401 |
| | /featu re_type="Insertion" |
| | /function="additional transcription unit" |
| | /label="ATU2" |
| terminator | 3476..3486 |
| | /function="polyadenylation signal" |
| | /note="corresponds to N gene end sequence" |
| | /label="engineered P/V/C gene end" |
| misc_feature | 3487..3489 |
| | /function="nontranscribed intergenic trinucleotide" |
| | /standard_name="gene junction" |
| | /label="P-ATU2 GJ" |
| promoter | 3490..3500 |
| | /function="restart of transcription" |
| | /note="corresponds to P/V/C gene start sequence" |
| | /label="ATU2 gene start" |
| Site | join(3523..3528,3523^3524) |
| | /site_type="restriction site" |
| | /note="Name: Mlul" |
| | /note="Pattern: acgcgt" |
| | /label="Mlul" |
| Site | join(3526..3531,3526^3527) |
| | /site_type="restriction site" |
| | /note="Name: BsiWI" |
| | /note="Pattern: cgtacg" |
| | /label="BsiWI" |
| CDS | 3538..7362 |
| | /product="spike protein (S)" |
| | /label="2019-nCoV_Sopt ORF" |
| sig_peptide | 3538..3582 |
| | /note="predicted from Phobius" |
| | /note="predicted from SignaIP-5.0" |
| | /label="Signal_peptide" |
| Site | 5581..5592 |
| | /site_type="cleavage site" |
| | /note="polybasic furin-like cleavage site" |
| | /label="predicted S1/S2 cleavage site" |
| Region | 7177..7245 |
| | /site_type="transmembrane-region" |
| | /note="predicted from TMHMM2.0" |
| | /note="predicted from PHOBIUS" |
| | /label="TM domain" |
| Site | join(7366..7371,7366^7367) |
| | /site_type="restriction site" |
| | /note="Name: BssHII" |
| | /note="Pattern: gcgcgc" |
| | /label="BssHII" |
| Site | join(7372..7377,7374^7375) |
| | /site_type="restriction site" |
| | /note="Name: Afel" |
| | /note="Pattern: agcgct" |
| | /label="Afel" |
| Site | join(7380..7385,7384^7385) |
| | /site_type="restriction site" |
| | /note="Name: Aatll" |
| | /note="Pattern: gacgtc" |
| | /label="Aatll" |
| terminator | 7416..7426 |
| | /function="polyadenylation signal" |
| | /label="P/V/C gene end" |
| misc_feature | 7427..7429 |
| | /function="nontranscribed intergenic trinucleotide" |
| | /label="P-M GJ" |
| promoter | 7430..7440 |
| | /function="restart of transcription" |
| | /label="M gene start" |
| terminator | 8885..8895 |
| | /function="polyadenylation signal" |
| | /label="M gene end" |
| misc_feature | 8896..8898 |
| | /function="non transcribed intergenic trinucleotide" |
| | /standard_name="Gene Junction" |
| | /label="M-F GJ" |
| promoter | 8899..8909 |
| | /function="restart of transcription" |
| | /label="F gene start" |
| Site | 19919..20002 |
| | /site_type="cleavage site" |
| | /function="self-cleaves before position a to generate an |
| | antigenome with an exact 3' end" |
| | /label="genomic HDV ribozyme" |
| terminator | 20073..20119 |
| | /label="T7 terminator" |
| Site | join(20145..20152,20146^20147) |
| | /site_type="restriction site" |
| | /note="Name: Notl" |
| | /note="Pattern: gcggccgc" |
| | /label="Notl" |
| misc_feature | 20153..21800 |
| | /label="plasmid_backbone" |
| CDS | 20203..21012 |
| | /function="kanamycin resistance" |
| | /label="kanaR" |
| rep_origin | 21076..21749 |
| | /label="pUC origin" |

**pKM-ATU3-S 2019-nCoV (optimized sequence) (SEQ ID NO: 35)**

| | |
|---|---|
| LOCUS | pKM-ATU3-S_2019-nCoV 21800 bp DNA circular UNA |
| DEFINITION | Abbreviated names : pKM3-S_nCoV |

| COMMENT FEATURES | Location/Qualifiers |
|---|---|
| Site | join(1..8,5^6) |
| | /site_type="restriction site" |
| | /note="Name: AsiSI" |
| | /note="Pattern: gcgatcgc" |
| | /label="AsiSI" |
| promoter | 9..28 |
| | /label="T7 promoter" |
| Site | 29..97 |
| | /site_type="cleavage site" |
| | /function="self-cleaves after position 54 to generate an |
| | antigenome with an exact 5' end" |
| | /label="hammerhead ribozyme" |
| misc_RNA | 83..19918 |
| | /label="MVSchw-ATU3-eGFP_antigenome" |
| misc_feature | 9258..13199 |
| | /feature_type="Insertion" |
| | /function="additional transcription unit" |
| | /label="ATU3" |
| terminator | 9278..9288 |
| | /function="polyadenylation signal" |
| | /note="corresponds to N gene end sequence" |
| | /label="engineered P/V/C gene end" |
| misc_feature | 9289..9291 |
| | /function="nontranscribed intergenic trinucleotide" |
| | /standard_name="gene junction" |
| | /label="P-ATU2 GJ" |
| promoter | 9292..9302 |
| | /function="restart of transcription" |
| | /note="corresponds to P/V/C gene start sequence" |
| | /label="ATU2 gene start" |
| Site | join(9325..9330,9325^9326) |
| | /site_type="restriction site" |
| | /note="Name: Mlul" |
| | /note="Pattern: acgcgt" |
| | /label="Mlul" |
| Site | join(9328..9333,9328^9329) |
| | /site_type="restriction site" |
| | /note="Name: BsiWI" |
| | /note="Pattern: cgtacg" |
| | /label="BsiWI" |
| CDS | 9340..13164 |
| | /product="spike protein (S)" |
| | /label="2019-nCoV_Sopt ORF" |
| sig_peptide | 9340..9384 |
| | /note="predicted from Phobius" |
| | /note="predicted from SignaIP-5.0" |
| | /label="Signal_peptide" |
| Site | 11383..11394 |
| | /site_type="cleavage site" |
| | /note="polybasic furin-like cleavage site" |
| | /label="predicted S1/S2 cleavage site" |
| Region | 12979..13047 |
| | /site_type="transmembrane-region" |
| | /note="predicted from TMHMM2.0" |
| | /note="predicted from PHOBIUS" |
| | /label="TM domain" |
| Site | join(13168..13173,13168^13169) |
| | /site_type="restriction site" |
| | /note="Name: BssHII" |
| | /note="Pattern: gcgcgc" |
| | /label="BssHII" |
| Site | join(13174..13179,13176^13177) |
| | /site_type="restriction site" |
| | /note="Name: Afel" |
| | /note="Pattern: agcgct" |
| | /label="Afel" |
| Site | join(13182..13187,13186^13187) |
| | /site_type="restriction site" |
| | /note="Name: Aatll" |
| | /note="Pattern: gacgtc" |
| | /label="Aatll" |
| terminator | 13222..13232 |
| | /function="polyadenylation signal" |
| | /label="H gene end" |
| misc_feature | 13233..13235 |
| | /function="nontranscribed intergenic trinucleotide" |
| | /standard_name="Gene Junction" |
| | /label="H-L GJ" |
| promoter | 13236..13246 |
| | /function="restart of transcription" |
| | /label="L gene start" |
| Site | 19919..20002 |
| | /site_type="cleavage site" |
| | /function="self-cleaves before position a to generate an |
| | antigenome with an exact 3' end" |
| | /label="genomic HDV ribozyme" |
| terminator | 20073..20119 |
| | /label="T7 terminator" |
| Site | join(20145..20152,20146^20147) |
| | /site_type="restriction site" |
| | /note="Name: Notl" |
| | /note="Pattern: gcggccgc" |
| | /label="Notl" |
| misc_feature | 20153..21800 |
| | /label="plasmid_backbone" |
| CDS | 20203..21012 |
| | /function="kanamycin resistance" |
| | /label="kanaR" |
| rep_origin | 21076..21749 |
| | /label="pUC origin" |

**MV-optimized nucleotide sequence of the spike (S) polypeptide of nCoV (SEQ ID NO: 36)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..3849 |
| | /product="spike protein (S)" |
| | /label="2019-nCoV_Smvopt ORF" |
| Site | join(3853..3858,3853^3854) |
| | /site_type="restriction site" |
| | /note="Name: BssHII" |
| | /note="Pattern: gcgcgc" |
| | /label="BssHII" |

**MV-optimized nucleotide sequence of the N polypeptide of CoV (SEQ ID NO: 37)**

| FEATURES | Location/Qualifiers |
|---|---|
| Site | join(13..18,13^14) |
| | /site_type="restriction site" |
| | /note="Name: BsiWI" |
| | /note="Pattern: cgtacg" |
| | /label="BsiWI" |
| CDS | 25..1287 |
| | /product="nucleoprotein (N)" |
| | /label="2019-nCoV_Nmvopt ORF" |
| Site | join(1291..1296, 1291^ 1292) |
| | /site_type="restriction site" |
| | /note="Name: BssHII" |
| | /note="Pattern: gcgcgc" |
| | /label="BssHII" |

**ATU1(eGFP) (SEQ ID NO : 39)**

| FEATURES | Location/Qualifiers |
|---|---|
| misc_feature | 1..876 |
| | /featu re_type="Insertion" |
| | /function="additional transcription unit" |
| | /label="ATU1" |
| misc_feature | 1..3 |
| | /function="nontranscribed intergenic trinucleotide" |
| | /standard_name="Gene Junction" |
| | /note="identical to leader-N GJ" |
| | /label="leader-ATU1 GJ" |
| promoter | 4..14 |
| | /function="start of transcription" |
| | /note="identical to N gene start" |
| | /label="ATU1 gene start" |
| Site | join(56..61,56^57) |
| | /site_type="restriction site" |
| | /note="Name: Mlul" |
| | /note="Pattern: acgcgt" |
| | /label="Mlul" |
| Site | join(59..64,59^60) |
| | /site_type="restriction site" |
| | /note="Name: BsiWI" |
| | /note="Pattern: cgtacg" |
| | /label="BsiWI" |
| CDS | 65..784 |
| | /product="enhanced Green Fluorescent Protein" |
| | /label="eGFP ORF" |
| Site | join(785..790,785^786) |
| | /site_type="restriction site" |
| | /note="Name: BssHII" |
| | /note="Pattern: gcgcgc" |
| | /label="BssHII" |
| Site | join(791..796,793^794) |
| | /site_type="restriction site" |
| | /note="Name: Afel" |
| | /note="Pattern: agcgct" |
| | /label="Afel" |
| Site | join(799..804,803^804) |
| | /site_type="restriction site" |
| | /note="Name: Aatll" |
| | /note="Pattern: gacgtc" |
| | /label="Aatll" |
| terminator | 866..876 |
| | /function="polyadenylation signal" |
| | /note="corresponds to N gene end sequence" |
| | /label="engineered ATU1 gene end" |

**ATU2(eGFP) (SEQ ID NO: 40)**

| FEATURES | Location/Qualifiers |
|---|---|
| misc_feature | 1..828 |
| | /featu re_type="Insertion" |
| | /function="additional transcription unit" |
| | /label="ATU2" |
| terminator | 17..27 |
| | /function="polyadenylation signal" |
| | /note="corresponds to N gene end sequence" |
| | /label="engineered P/V/C gene end" |
| misc_feature | 28..30 |
| | /function="nontranscribed intergenic trinucleotide" |
| | /standard_name="gene junction" |
| | /label="P-ATU2 GJ" |
| promoter | 31..41 |
| | /function="restart of transcription" |
| | /note="corresponds to P/V/C gene start sequence" |
| | /label="ATU2 gene start" |
| Site | join(64..69,64^65) |
| | /site_type="restriction site" |
| | /note="Name: Mlul" |
| | /note="Pattern: acgcgt" |
| | /label="Mlul" |
| Site | join(67..72,67^68) |
| | /site_type="restriction site" |
| | /note="Name: BsiWI" |
| | /note="Pattern: cgtacg" |
| | /label="BsiWI" |
| CDS | 73..792 |
| | /cds_type="ORF" |
| | /product="enhanced Green Fluorescent Protein" |
| | /label="eGFP ORF" |
| Site | join(793..798,793^794) |
| | /site_type="restriction site" |
| | /note="Name: BssHII" |
| | /note="Pattern: gcgcgc" |
| | /label="BssHII" |
| Site | join(799..804,801^802) |
| | /site_type="restriction site" |
| | /note="Name: Afel" |
| | /note="Pattern: agcgct" |
| | /label="Afel" |
| Site | join(807..812,811^812) |
| | /site_type="restriction site" |
| | /note="Name: Aatll" |
| | /note="Pattern: gacgtc" |
| | /label="Aatll" |

**ATU3(eGFP) (SEQ ID NO: 41)**

| FEATURES | Location/Qualifiers |
|---|---|
| misc_feature | 1..828 |
| | /featu re_type="Insertion" |
| | /function="additional transcription unit" |
| | /label="ATU3" |
| terminator | 21..31 |
| | /function="polyadenylation signal" |
| | /note="corresponds to N gene end sequence" |
| | /label="engineered P/V/C gene end" |
| misc_feature | 32..34 |
| | /function="nontranscribed intergenic trinucleotide" |
| | /standard_name="gene junction" |
| | /label="P-ATU2 GJ" |
| promoter | 35..45 |
| | /function="restart of transcription" |
| | /note="corresponds to P/V/C gene start sequence" |
| | /label="ATU2 gene start" |
| Site | join(68..73,68^69) |
| | /site_type="restriction site" |
| | /note="Name: Mlul" |
| | /note="Pattern: acgcgt" |
| | /label="Mlul" |
| Site | join(71..76,71^72) |
| | /site_type="restriction site" |
| | /note="Name: BsiWI" |
| | /note="Pattern: cgtacg" |
| | /label="BsiWI" |
| CDS | 77..796 |
| | /cds_type="ORF" |
| | /product="enhanced Green Fluorescent Protein" |
| | /label="eGFP ORF" |
| Site | join(797..802,797^798) |
| | /site_type="restriction site" |
| | /note="Name: BssHII" |
| | /note="Pattern: gcgcgc" |
| | /label="BssHII" |
| Site | join(803..808,805^806) |
| | /site_type="restriction site" |
| | /note="Name: Afel" |
| | /note="Pattern: agcgct" |
| | /label="Afel" |
| Site | join(811..816,815^816) |
| | /site_type="restriction site" |
| | /note="Name: Aatll" |
| | /note="Pattern: gacgtc" |
| | /label="Aatll" |

The invention also concerns recombinant virus like particles (VLPs) comprising a S polypeptide or an immunogenic or an antigenic fragment thereof, which is(are) encoded by the first and optionally the second heterologous polynucleotide(s) of the nucleic acid construct according to the invention, or of the transfer plasmid vector according to the invention, or the recombinant measles virus according to the invention or is produced within the host cell according to the invention.

As used herein, the term "*virus-like particle*" (VLP) refers to a structure that in at least one attribute resembles a virus but which has not been demonstrated to be infectious as such. VLPs in accordance with the invention do not carry genetic information encoding the proteins of the VLPs, in general, VLPs lack a viral genome and, therefore, are non-infectious and non-replicative. In accordance with the present invention, VLPs can be produced in large quantities and are expressed together with recombinant infectious MV-CoV particles. Said VLPs are VLPs of CoV.

According to a preferred embodiment of the invention, the recombinant MV vector is designed in such a way and the production process involves cells such that the virus particles produced in helper cells transfected or transformed with said vector, originated from a MV strain adapted for vaccination, enable the production of recombinant infectious replicating MV and the production of CoV-VLPs for use in immunogenic compositions, preferably protective or vaccine compositions.

Advantageously, the genome of the recombinant infectious MV-CoV particles of the invention is replication competent. By "replication competent', it is meant a nucleic acid, which when transduced into a helper cell line expressing the N, P and L proteins of a MV, is able to be transcribed and expressed in order to produce new viral particles.

Replication of the recombinant virus of the invention obtained using MV cDNA for the preparation of the recombinant genome of MV-CoV can also be achieved *in vivo* in the host, in particular the human host to which recombinant MV-CoV is administered.

Other features and advantages of the invention will be apparent from the examples which follow and will also be illustrated in the figures.

The invention also relates to the In vitro use of an antigen of the coronavirus 2019-nCoV which is the spike antigen or an immunogenic fragment thereof or an antigenic fragment thereof, in particular an antigen having the sequence of SEQ ID No. 3, 5, 7, 9, 11, 13, 15, 17, 19, 22, 23, 24, 25, 26 or 27 for the detection in a biological sample, especially a blood or a serum sample previously obtained from an individual suspected of being infected by a coronavirus, in particular by coronavirus 2019-nCoV, wherein the antigen is contacted with the biological sample to determine the presence of antibodies against the antigen.

### BRIEF DESCRIPTION OF THE FIGURES

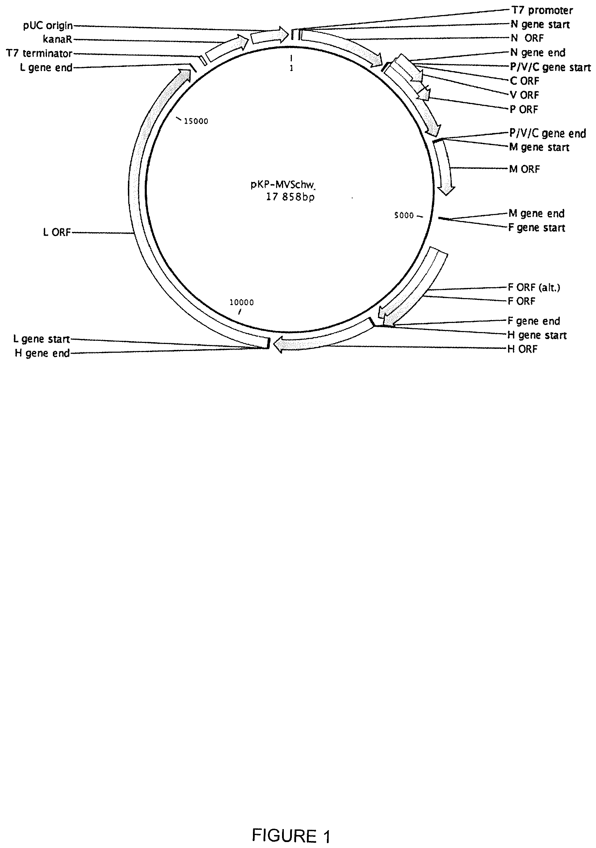

Figure 1: restriction map of plasmid pKP-MVSchw (17585bp)

### Examples

### [001] Design of specific antigenic sequences of 2019-nCoV

cDNAs encoding native spike and nucleoprotein antigens from 2019-nCoV were designed base on the Genbank MN908947 sequence publicly available from NBCBI on 20^{th} January 2020. These sequences were then processed through the Project Manager platform of GeneArt (ThermoFisher) to generate codon-optimized nucleotide sequences for high expression in mammalian and drosophila cells. Regions of very high (>80%) or low (<30%) GC content were avoided whenever possible, and cis-acting sequence motifs like internal TATA-boxes, chi-sites, ribosomal entry sites, ARE, INS, and CRS sequence elements, as well as repetitive sequences, RNA secondary structures and splice donor and acceptor sites, were avoided. Both sequences were further edited to remove MV editing (AnGn, n≥3)- and core gene end (A4CKT)-like sequences on both strands. BsiWI and BssHII restriction sites were then added at the 5' and 3' ends, respectively, of the nucleotide sequences. The resulting cDNAs respect the "rule of six", which stipulates that the number of nucleotides of the MV genome must be a multiple of 6 and have the sequences 20AAP6IP-S-2019-nCoV-opt(2x) and 20AASUIP-N-2019-nCoV-opt(2X), respectively (see below).

The resulting cDNAs have been synthesized at Geneart (ThermoFisher) facilities.

### [002] Plasmid vector constructs and vaccine candidate rescue

The MVSchw recombinant plasmid constructs have been derived from novel pKP-MVSchw-ATU1(eGFP), -ATU2 and -ATU3 plasmid vectors (abbreviated names : pKM1, pKM2, pKM3). These vectors were constructed from the original pTM-MVSchw-ATU1, -ATU2 and -ATU3 plasmid vectors, respectively (WO04/000876 and Combredet et al., J Virol, 2003). pKM and pTM plasmid vector series carry identical infectious cDNAs corresponding to the anti-genome of the Schwarz MV vaccine strain and an additional transcription unit containing unique BsiWI and BssHII restriction sites for the insertion of foreign open reading frames upstream from the N gene (ATU1), between the P and M genes (ATU2) and between the H and L genes (ATU3). First, pKM2-eGFP was obtained by transferring the whole T7 rescue cassette of the original pTM2-eGFP plasmid (17038 bp located between the two Not1 sites) into a purposively-modified version of the commercial pENTR2 minimal plasmid (ThermoFisher). Next, pKM, pKM3-eGFP and pKM1-eGFP were sequentially derived from pKM2-eGFP. The novel pKM, pKM1-, pKM2- and pKM3-eGFP vectors were verified for their capacity to rescue the corresponding measles virus and vectors with similar efficiency to that observed for the pTM plasmid vector series. It is noteworthy to highlight that viruses rescued from the novel pKM1, pKM2 and pKM3 plasmid vectors have the same genomic sequence as viruses rescued from original pTM1, pTM2 and pTM3 vectors respectively.

pKM plasmid series are suitable for use to insert a variety of viral antigens in single, dual and triple recombinant vectors which much higher cloning efficiency, stability and DNA yield than the original pTM plasmid series, making them a most useful rescue tool for the measles vaccine platform.

cDNAs encoding the 2019-nCoV nucleoprotein and spike antigens described above have been inserted into BsiWI/BssHII-digested pKM2 and pKM3 vectors and the resulting pKM-nCoV plasmids is used to rescue single recombinant MV-nCoV vaccine candidates using a helper-cell-based system as previously described (Combredet et al., J Virol, 2003).

The plasmid pKM2-nCoV_NP and any of the pKM3-nCoV_Spike constructs (fl-S, stab-S, Secto, S1, tri-Secto, stab-Secto) will be digested with Sall restriction enzyme and ligated to produce a series of double recombinant pKM-nCoV-N&S plasmids. Alternatively, double recombinant plasmids will be obtained by inserting the N and S ATU cassettes in tandem either between the P and M genes (position 3 of MV genome) or between the H and L gene (position 6 of MV genome). The resulting pKM-nCoV-N&S plasmids will be used to rescue dual recombinant MV-nCoV-N&S vaccine candidates as described above.

### [003] In cellulo characterization of the vaccine candidates

The single- and dual-recombinant vaccine candidates will be amplified on Vero-NK cells as disclosed in WO 04/000876. All viral stocks will be produced after infection at a MOI of 0.1, stored at -80°C and titrated by an endpoint limiting dilution assay on Vero-NK cell monolayers. Infectious titers will be determined as 50% tissue culture infectious doses (TCID₅₀) according to the Reed and Munsch method (Reed et al., Am. J. Hyg., 1938).

Vaccine candidates will be characterized essentially as described for MV-SARS vaccine candidates (Escriou et al., Virology, 2014) :
- growth curves of vaccine candidates and parental MVSchw will be determined on Vero-NK cells infected at a MOI of 0.1,
- expression level of 2019-nCoV antigens will be evaluated with available anti-SARS cross-reactive mouse and rabbit antibodies and anti-2019-nCoV antibodies, by indirect immunofluorescence assays (IFA) performed on VeroNK-infected cells as well as by western blot on lysates prepared from infected cells,
- genomic stability of the vaccine candidates will be assessed by serial passages in VeroNK cells and full genome NGS sequencing of the rescued and passaged viral stocks.

### Bibliography

Combredet, C., V. Labrousse, L. Mollet, C. Lorin, F. Delebecque, B. Hurtrel, H. McClure, M. B. Feinberg, M. Brahic and F. Tangy (2003). "A molecularly cloned Schwarz strain of measles virus vaccine induces strong immune responses in macaques and transgenic mice." J Virol 77(21): 11546-54.
Escriou, N., B. Callendret, V. Lorin, C. Combredet, P. Marianneau, M. Fevrier and F. Tangy (2014). "Protection from SARS coronavirus conferred by live measles vaccine expressing the spike glycoprotein." Virology 452-453: 32-41.
Reed, L. J. and H. Muench (1938). "A simple method of estimating fifty percent endpoints." Am. J. Hyg. 27: 493-497.

## Claims

1. A nucleic acid construct which comprises:
(3) a cDNA molecule encoding a full length, infectious antigenomic (+) RNA strand of a measles virus (MV);
(4) a first heterologous polynucleotide encoding at least one polypeptide of a coronavirus (CoV), in particular of coronavirus 2019-nCoV, in particular said first polynucleotide encoding at least one polypeptide consisting of the spike (S) polypeptide of a coronavirus (CoV), in particular of coronavirus 2019-nCoV, or at least one polypeptide consisting of an immunogenic fragment thereof or an antigenic fragment thereof,
and wherein the first heterologous polynucleotide is operatively cloned within an additional transcription unit (ATU) inserted within the cDNA of the antigenomic (+) RNA to provide a recombinant MV-CoV nucleic acid molecule, in particular the first heterologous polynucleotide is operatively cloned in an ATU2 or an ATU3 located between the P gene and the M gene of the MV.

2. The nucleic acid construct according to claim 1 further comprising a second heterologous polynucleotide encoding at least one polypeptide, an immunogenic fragment thereof or an antigenic fragment thereof, of a coronavirus, in particular of coronavirus 2019-nCoV, wherein said polypeptide is different from at least one polypeptide encoded by the first heterologous polynucleotide and in particular is selected from the group consisting of the nucleocapsid (N) polypeptide, the matrix (M) polypeptide, the E polypeptide, the 8a polypeptide, the 7a polypeptide and the 3A polypeptide or immunogenic or antigenic fragments thereof or mutated fragments thereof, the second heterologous polynucleotide being operatively cloned within an ATU at a location distinct from the location of the first cloned heterologous polynucleotide, in particular upstream the N gene of the MV in the ATU1, or in particular within an ATU at a location between the P gene and the M gene of the MV in the ATU2 or in particular within an ATU at a location downstream of the H gene of the MV in the ATU3.

3. The nucleic acid construct according to claim 1 or 2, wherein the first heterologous polynucleotide encodes the wild type S polypeptide of SEQ ID NO:3 or a fragment thereof consisting of the S1 domain of SEQ ID NO:11 or the S2 domain of SEQ ID NO:13 of the S polypeptide, or a fragment or a variant thereof that has 1, 2 or more amino acid residue substitution(s), in particular less than 10, or less than 5 amino acid residue substitutions, in particular a fragment or a mutated variant having amino acid sequence of SEQ ID NO: 5, 7, 9, 15, 17, 19.

4. The nucleic acid construct according to claim 2 , wherein the second heterologous polynucleotide encodes the N polypeptide of SEQ ID NO:22, an immunogenic fragment thereof or an antigenic fragment thereof, or a variant of the N polypeptide by substitution of 1, 2 or less than 10 amino acid residue(s), in particular less than 5 amino acid residues and/or the M polypeptide of sequence SEQ ID NO:24 or its endodomain, the E polypeptide of sequence SEQ ID NO:23, the ORF8 polypeptide of SEQ ID NO:25, the ORF7a polypeptide of SEQ ID NO:27 or its and/or the 3a polypeptide of SEQ ID NO:26 of coronavirus.

5. The nucleic acid construct according to any one of claims 1 to 4, wherein the heterologous polynucleotide encoding the S polypeptide, S1 polypeptide or S2 polypeptide, an immunogenic fragment thereof or an antigenic fragment thereof has the open reading frame of a wild type gene or a codon-optimized open reading frame(s) (coORF) for expression in mammalian cells and/or in drosophila cells, in particular, the heterologous polynucleotide comprises one of the following sequences:
i. SEQ ID NO: 1 or 2 or 36 which encodes the S polypeptide or,
ii. SEQ ID NO: 10 which encodes the S1 polypeptide or,
iii. SEQ ID NO: 12 which encodes the S2 polypeptide or,
iv. SEQ ID NO: 4 which encodes the stab-S polypeptide
v. SEQ ID NO: 6 which encodes the Secto polypeptide or,
vi. SEQ ID NO: 8 which encodes the stab-Secto polypeptide or,
vii. SEQ ID NO:14 which encodes the stab-S2 polypeptide or,
viii. SEQ ID NO: 16 which encodes the tri-Secto polypeptide or,
ix. SEQ ID NO: 18 which encodes the tristab-Secto polypeptide .

6. The nucleic acid construct according to any one of claims 1 to 5, which is a cDNA construct comprising from 5' to 3' end the following polynucleotides coding for ORFs:
(h) a polynucleotide encoding the N protein of the MV;
(i) a polynucleotide encoding the P protein of the MV;
(j) the first heterologous polynucleotide encoding at least one polypeptide, an immunogenic fragment thereof or an antigenic fragment thereof, of a coronavirus, in particular of coronavirus 2019-nCoV, said at least one polypeptide consisting of the S polypeptide and wherein the first heterologous polynucleotide is operatively cloned within an additional transcription unit (ATU) inserted within the cDNA of the antigenomic (+) RNA, in particular within ATU2;
(k) a polynucleotide encoding the M protein of the MV;
(l) a polynucleotide encoding the F protein of the MV;
(m) a polynucleotide encoding the H protein of the MV;
(n) a polynucleotide encoding the L protein of the MV;
and wherein said polynucleotides are operatively linked within the nucleic acid construct and are under the control of a viral replication and transcriptional regulatory elements such as MV leader and trailer sequences and are framed by a T7 promoter and a T7 terminator and additionally are framed by restrictions sites suitable for cloning in a vector to provide a recombinant MV-CoV expression cassette.

7. The nucleic acid construct according to any one of claims 1 to 6, wherein the recombinant MV-CoV nucleic acid molecule further comprises, at its 5'-end, adjacent to the first nucleotide of the nucleotide sequence encoding the full-length antigenomic (+)RNA strand of an attenuated MV strain, in particular of a Schwarz strain or of a Moraten strain, a GGG motif followed by a hammerhead ribozyme sequence and which comprises, at its 3'-end, adjacent to the last nucleotide of said nucleotide sequence encoding the full length anti-genomic (+)RNA strand, the sequence of a ribozyme in particular the sequence of the Hepatitis delta virus ribozyme (δ).

8. The nucleic acid construct according to any one of claims 2 to 7, wherein the second heterologous polynucleotide encodes the N polypeptide of 2019-nCoV, or an immunogenic fragment thereof or an antigenic fragment thereof, said second heterologous polynucleotide being cloned in a ATU at a different location from ATU2.

9. The nucleic acid construct according to any one of claims 1 to 8, wherein the measles virus is an attenuated virus strain selected from the group consisting of the Schwarz strain, the Zagreb strain, the AIK-C strain, the Moraten strain, the Philips strain, the Beckenham 4A strain, the Beckenham 16 strain, the CAM-70 strain, the TD 97 strain, the Leningrad-16 strain, the Shanghai 191 strain and the Belgrade strain, in particular the Schwarz strain.

10. A transfer vector, in particular a plasmid vector, suitable for the rescue of a recombinant Measles virus (MV) which comprises the nucleic acid construct according to any one of claims 1 to 9, in particular a transfer vector selected from the group consisting of plasmid of SEQ ID NO: 28 (pTM-MVSchwarz), plasmid of SEQ ID NO: 29 (pTM-MVSchwarz-ATU2), plasmid of SEQ ID NO: 38 (pTM-MVSchwarz-ATU3), plasmid of SEQ ID NO: 30 (pKP-MVSchwarz), plasmid of SEQ ID NO: 32 (pKP-MVSchwarz-ATU2) and plasmid of SEQ ID NO: 33 (pKP-MVSchwarz-ATU3) wherein said transfer vector is recombined with a first heterologous DNA polynucleotide encoding at least one polypeptide consisting of the spike polypeptide of a coronavirus, in particular of coronavirus 2019-nCoV, or consisting of an immunogenic fragment thereof or an antigenic fragment thereof has been operatively cloned within an additional transcription unit (ATU) inserted within the cDNA of the antigenomic (+) RNA.

11. A transfer vector which is a plasmid recombined with recombinant DNA of MC-CoV, wherein the sequence ancoding a polypeptide pf 2019-nCoV is is selected from the group consisting of:
i. SEQ ID NO: 1 or 2 or 36 (construct S);
ii. SEQ ID NO: 4 (construct stab-S);
iii. SEQ ID NO: 6 (construct Secto);
iv. SED ID NO: 8 (construct stab-Secto);
v. SEQ ID NO: 10 (construct S1),
vi. SEQ ID NO: 12 (construct S2),
vii. SEQ ID NO: 14 (construct stab-S2),
viii. SEQ ID NO: 16 (construct tri-Secto),
ix. SEQ ID NO: 18 (construct tristab-Secto) and
x. SEQ ID NO: 21 or 37 (construct N).

12. A recombinant measles virus, in particular a recombinant measles virus of the Schwarz strain, which comprises in its genome a nucleic acid construct which encodes at least one polypeptide consisting of the spike polypeptide of a coronavirus, in particular of coronavirus 2019-nCoV, or an immunogenic fragment thereof or an antigenic fragment thereof, in particular a polypeptide encoded by a nucleotide sequence as defined in any one of claims 1 to 6 and 11, in particular a nucleic acid construct which is a replicon of a transfer vector of claims 10 or 11, said nucleic acid construct being operatively linked with said genome in an expression cassette.

13. The recombinant measles virus according to claim 12, in particular a recombinant measles virus of the Schwarz strain, expressing at least one polypeptide selected from the group consisting of the S polypeptide, S1 polypeptide, S2 polypeptide of a coronavirus, in particular of the 2019-nCoV strain, or an immunogenic fragment thereof or an antigenic fragment thereof, and optionally further expressing at least one of a N polypeptide, M polypeptide, E polypeptide, ORF7a, ORF8 or ORF3 polypeptide of a coronavirus, in particular of the 2019-nCoV strain or an immunogenic fragment thereof or an antigenic fragment thereof.

14. The recombinant measles virus according to any one of claims 12 to 13 further expressing the N polypeptide of SEQ ID NO:22, an immunogenic fragment thereof or an antigenic fragment thereof, or a variant of the N polypeptide by substitution of 1, 2 or less than 10 amino acid residue(s), in particular less than 5 amino acid residues and/or the M polypeptide of sequence SEQ ID NO:24 or its endodomain, the E polypeptide of sequence SEQ ID NO:23, the ORF8 polypeptide of SEQ ID NO:25, the ORF7a polypeptide of SEQ ID NO:27 or its and/or the 3a polypeptide of SEQ ID NO:26 of 2019-nCoV, or an immunogenic fragment or an antigenic fragment thereof.

15. An immunogenic or a vaccine composition comprising (i) an effective dose of the recombinant measles virus according to any one of claims 12 to 14, and/or of the recombinant VLPs and (ii) a pharmaceutically acceptable vehicle, wherein the composition or the vaccine elicits a humoral, especially a protective, in particular a neutralizing humoral response and/or a cellular response in an animal host, especially in a human being, in particular after a single immunization, against the polypeptide(s) of the coronavirus, in particular of 2019-nCoV or their fragments, that it expresses and wherein optionally the composition is devoid of added adjuvant.

16. The composition according to claim 15 for use in the elicitation of a protective, and preferentially prophylactic, immune response against 2019-nCoV or against 2019-nCoV and against further distinct coronavirus(es), by the elicitation of antibodies recognizing coronavirus protein(s) or antigenic fragment(s) thereof, and/or by the elicitation of a cellular and/or humoral and cellular response against the Coronavirus, in a host in need thereof, in particular a human host, in particular a child.

17. A process for rescuing recombinant measles virus expressing at least one polypeptide consisting of the spike (S) polypeptide of a coronavirus, in particular of 2019-nCoV or an immunogenic fragment thereof or an antigenic fragment thereof comprising:
(e) co-transfecting cells, in particular helper cells, in particular HEK293 helper cells, stably expressing T7 RNA polymerase and measles virus N and P proteins with (i) the nucleic acid construct according to any one of claims 1 to 9 or with the transfer plasmid vector according to claim 10 or 11, and with (ii) a vector, especially a plasmid, encoding the MV L polymerase,
(f) maintaining the transfected cells in conditions suitable for the production of recombinant measles virus;
(g) infecting cells enabling propagation of the recombinant measles virus by co-cultivating them with the transfected cells of step (b), in particular VERO cells;
(h) harvesting the recombinant measles virus expressing at least the S polypeptide or an immunogenic fragment or an antigenic fragment thereof of a coronavirus, in particular of 2019-nCoV and optionally at least one of the N, M or 3A polypeptide or an immunogenic fragment or an antigenic fragment thereof of a coronavirus, in particular of 2019-nCoV.

18. A nucleic acid molecule comprising a polynucleotide selected among the group of:
i. SEQ ID NO: 1 or 2 or 36 (construct S);
ii. SEQ ID NO: 4 (construct stab-S);
iii. SEQ ID NO: 6 (construct Secto);
iv. SED ID NO: 8 (construct stab-Secto);
v. SEQ ID NO: 10 (construct S1),
vi. SEQ ID NO: 12 (construct S2),
vii. SEQ ID NO: 14 (construct stab-S2),
viii. SEQ ID NO: 16 (construct tri-Secto),
ix. SEQ ID NO: 18 (construct tristab-Secto) and
x. SEQ ID NO: 21 or 37 (construct N).

19. In vitro use of an antigen of the coronavirus 2019-nCoV which is the spike antigen or an immunogenic fragment thereof or an antigenic fragment thereof, in particular an antigen having the sequence of SEQ ID No. 3, 5, 7, 9, 11, 13, 15, 17, 19, 22, 23, 24, 25, 26 or 27 for the detection in a biological sample, especially a blood or a serum sample previously obtained from an individual suspected of being infected by a coronavirus, in particular by coronavirus 2019-nCoV, wherein the antigen is contacted with the biological sample to determine the presence of antibodies against the antigen.


SEQUENCE LISTING

<110>  INSTITUT PASTEUR

<120>  Live recombinant measles virus expressing Coronavirus antigens -
       its use in eliciting immunity against coronaviruses

<130>  B14232/AD/DP/KN

<140>  EPxxxxxxxx.x
<141>  2020-02-13

<160>  41

<170>  PatentIn version 3.5

<210>  1
<211>  3870
<212>  DNA
<213>  Artificial sequence

<220>
<223>  Native nucleotide sequence of the spike (S) polypeptide of nCoV

<400>  1
gctagcggat cccgtacggc caccatgttt gtttttcttg ttttattgcc actagtctct      60

agtcagtgtg ttaatcttac aaccagaact caattacccc ctgcatacac taattctttc     120

acacgtggtg tttattaccc tgacaaagtt ttcagatcct cagttttaca ttcaactcag     180

gacttgttct tacctttctt ttccaatgtt acttggttcc atgctataca tgtctctggg     240

accaatggta ctaagaggtt tgataaccct gtcctaccat ttaatgatgg tgtttatttt     300

gcttccactg agaagtctaa cataataaga ggctggattt ttggtactac tttagattcg     360

aagacccagt ccctacttat tgttaataac gctactaatg ttgttattaa agtctgtgaa     420

tttcaatttt gtaatgatcc atttttgggt gtttattacc acaaaaacaa caaaagttgg     480

atggaaagtg agttcagagt ttattctagt gcgaataatt gcacttttga atatgtctct     540

cagccttttc ttatggacct tgaaggaaaa cagggtaatt tcaaaaatct tagggaattt     600

gtgtttaaga atattgatgg ttattttaaa atatattcta agcacacgcc tattaattta     660

gtgcgtgatc tccctcaggg tttttcggct ttagaaccat tggtagattt gccaataggt     720

attaacatca ctaggtttca aactttactt gctttacata gaagttattt gactcctggt     780

gattcttctt caggttggac agctggtgct gcagcttatt atgtgggtta tcttcaacct     840

aggacttttc tattaaaata taatgaaaat ggaaccatta cagatgctgt agactgtgca     900

cttgaccctc tctcagaaac aaagtgtacg ttgaaatcct tcactgtaga aaaaggaatc     960

tatcaaactt ctaactttag agtccaacca acagaatcta ttgttagatt tcctaatatt    1020

acaaacttgt gcccttttgg tgaagttttt aacgccacca gatttgcatc tgtttatgct    1080

tggaacagga agagaatcag caactgtgtt gctgattatt ctgtcctata taattccgca    1140

tcattttcca cttttaagtg ttatggagtg tctcctacta aattaaatga tctctgcttt 1200

actaatgtct atgcagattc atttgtaatt agaggtgatg aagtcagaca aatcgctcca 1260

gggcaaactg gaaagattgc tgattataat tataaattac cagatgattt tacaggctgc 1320

gttatagctt ggaattctaa caatcttgat tctaaggttg gtggtaatta taattacctg 1380

tatagattgt ttaggaagtc taatctcaaa ccttttgaga gagatatttc aactgaaatc 1440

tatcaggccg gtagcacacc ttgtaatggt gttgaaggtt ttaattgtta ctttccttta 1500

caatcatatg gtttccaacc cactaatggt gttggttacc aaccatacag agtagtagta 1560

ctttcttttg aacttctaca tgcaccagca actgtttgtg gacctaaaaa gtctactaat 1620

ttggttaaaa acaaatgtgt caatttcaac ttcaatggtt taacaggcac aggtgttctt 1680

actgagtcta acaaaaagtt tctgcctttc caacaatttg gcagagacat tgctgacact 1740

actgatgctg tccgtgatcc acagacactt gagattcttg acattacacc atgttctttt 1800

ggtggtgtca gtgttataac accaggaaca aatacttcta accaggttgc tgttctttat 1860

caggatgtta actgcacaga agtccctgtt gctattcatg cagatcaact tactcctact 1920

tggcgtgttt attctacagg ttctaatgtt tttcaaacac gtgcaggctg tttaataggg 1980

gctgaacatg tcaacaactc atatgagtgt gacataccca ttggtgcagg tatatgcgct 2040

agttatcaga ctcagactaa ttctcctcgg cgggcacgta gtgtagctag tcaatccatc 2100

attgcctaca ctatgtcact tggtgcagaa aattcagttg cttactctaa taactctatt 2160

gccataccca caaattttac tattagtgtt accacagaaa ttctaccagt gtctatgacc 2220

aagacatcag tagattgtac aatgtacatt tgtggtgatt caactgaatg cagcaatctt 2280

ttgttgcaat atggcagttt ttgtacacaa ttaaaccgtg ctttaactgg aatagctgtt 2340

gaacaagaca aaaacaccca agaagttttt gcacaagtca aacaaattta caaaacacca 2400

ccaattaaag attttggtgg ttttaatttt tcacaaatat taccagatcc atcaaaacca 2460

agcaagaggt catttattga agatctactt ttcaacaaag tgacacttgc agatgctggc 2520

ttcatcaaac aatatggtga ttgccttggt gatattgctg ctagagacct catttgtgca 2580

caaaagttta acggccttac tgttttgcca cctttgctca cagatgaaat gattgctcaa 2640

tacacttctg cactgttagc gggtacaatc acttctggtt ggacctttgg tgcaggtgct 2700

gcattacaaa taccatttgc tatgcaaatg gcttataggt ttaatggtat tggagttaca 2760

cagaatgttc tctatgagaa ccaaaaattg attgccaacc aatttaatag tgctattggc 2820

aaaattcaag actcactttc ttccacagca agtgcacttg gaaaacttca agatgtggtc 2880

aaccaaaatg cacaagcttt aaacacgctt gttaaacaac ttagctccaa ttttggtgca 2940

atttcaagtg ttttaaatga tatcctttca cgtcttgaca aagttgaggc tgaagtgcaa 3000

attgataggt tgatcacagg cagacttcaa agtttgcaga catatgtgac tcaacaatta 3060

attagagctg cagaaatcag agcttctgct aatcttgctg ctactaaaat gtcagagtgt 3120

gtacttggac aatcaaaaag agttgatttt tgtggaaagg gctatcatct tatgtccttc 3180

cctcagtcag cacctcatgg tgtagtcttc ttgcatgtga cttatgtccc tgcacaagaa 3240

aagaacttca caactgctcc tgccatttgt catgatggaa aagcacactt tcctcgtgaa 3300

ggtgtctttg tttcaaatgg cacacactgg tttgtaacac aaaggaattt ttatgaacca 3360

caaatcatta ctacagacaa cacatttgtg tctggtaact gtgatgttgt aataggaatt 3420

gtcaacaaca cagtttatga tcctttgcaa cctgaattag actcattcaa ggaggagtta 3480

gataaatatt ttaagaatca tacatcacca gatgttgatt taggtgacat ctctggcatt 3540

aatgcttcag ttgtaaacat tcaaaaagaa attgaccgcc tcaatgaggt tgccaagaat 3600

ttaaatgaat ctctcatcga tctccaagaa cttggaaagt atgagcagta tataaaatgg 3660

ccatggtaca tttggctagg ttttatagct ggcttgattg ccatagtaat ggtgacaatt 3720

atgctttgct gtatgaccag ttgctgtagt tgtctcaagg gctgttgttc ttgtggatcc 3780

tgctgcaaat ttgatgaaga cgactctgag ccagtgctca aaggagtcaa attacattac 3840

acatgataac gagcgcgcct cgaggaattc 3870

<210> 2
<211> 3870
<212> DNA
<213> Artificial sequence

<220>
<223> Codon-optimized nucleotide sequence of the spike (S) polypeptide
      of nCoV

<400> 2
gctagcggat cccgtacggc caccatgttc gtgtttctgg tgctgctgcc actggtgtcc 60

agtcagtgcg tgaacctgac cacacgaaca cagctgccac cagcctacac caatagcttc 120

acccgcggag tgtactaccc cgacaaggtg ttccgcagca gcgtgctgca tagcacccag 180

gatctgtttc tgcccttctt cagcaacgtg acctggttcc acgccatcca cgtgtccggc 240

accaatggca ccaagcgctt cgataatccc gtgctgccct tcaacgatgg cgtgtacttt 300

gccagcaccg agaagtccaa tatcatccgc ggctggatct tcggcaccac actggatagc 360

aagacccaga gcctgctgat cgtgaacaac gccaccaacg tggtcatcaa agtgtgcgag 420

ttccagttct gcaacgaccc cttcctgggc gtctactacc acaagaacaa caagagctgg 480

atggaaagcg agttccgcgt gtacagcagc gccaacaact gcaccttcga gtacgtgtcc 540

cagccattcc tgatggacct ggaaggcaag cagggcaact tcaagaacct gcgcgagttc 600

gtgttcaaga acatcgacgg ctacttcaaa atctacagca agcacacccc aatcaacctc 660

gtgcgcgatc tgccacaggg attcagtgct ctggaacccc tggtggatct gcccatcggc 720

atcaacatta cccgctttca gacactgctg gccctgcacc gcagttactt gacaccaggc 780

gatagcagca gtggatggac agctggtgcc gccgcttact acgttggata tctgcagcca 840

cgcacctttc tgctgaagta caacgagaac ggcaccatca ccgacgccgt ggattgtgct 900

ctcgatcccc tgagcgagac aaagtgcacc ctgaagtcct tcaccgtcga gaagggcatc 960

taccagacca gcaatttccg cgtgcagccc accgagagca tcgtgcgctt ccccaatatc 1020

accaatctgt gccccttcgg cgaggtgttc aatgccacac gctttgcctc cgtgtacgcc 1080

tggaatcgca agcgcattag caactgcgtg gccgactact ccgtgctgta caatagcgcc 1140

agcttcagca ccttcaagtg ctacggcgtg tcacccacca agctgaacga cctgtgcttc 1200

accaatgtgt acgccgacag cttcgtgatc cgcggagatg aagtgcgaca gattgcccca 1260

ggccagaccg gcaagatcgc cgactacaat tacaagctgc ccgacgactt caccggctgc 1320

gtgatcgcct ggaacagcaa caacctggat tccaaagtcg gcggcaacta caactacctg 1380

taccgcctgt tccgcaagag caatctgaag cccttcgagc gcgacatcag caccgaaatc 1440

taccaggccg gaagcacccc atgcaacggc gtggaaggct tcaactgcta cttcccactg 1500

cagtcctacg gatttcagcc cacaaatggc gtgggctacc agccatatcg agtggtggtg 1560

ctgagcttcg aactgctgca tgctccagct accgtgtgcg gccccaagaa gagtaccaac 1620

ctggtcaaga acaaatgcgt gaacttcaac ttcaacggcc tgaccggaac cggcgtgctg 1680

accgagagta acaagaagtt cctgccattc cagcagtttg gccgcgacat tgccgataca 1740

accgatgccg ttcgcgatcc ccagaccttg gagatcctgg atattacccc atgctccttc 1800

ggcggcgtgt ccgtgattac accaggcacc aataccagca accaggtggc cgttctgtac 1860

caggatgtga attgcacaga ggtgcccgtg gccattcacg ccgatcaatt gacaccaaca 1920

tggcgcgtgt actccaccgg cagcaatgtg tttcaaaccc gcgctggatg cctgattgga 1980

gccgagcacg tgaacaatag ctacgagtgc gatatcccca tcggagccgg aatctgcgcc 2040

tcctatcaga cccagaccaa tagtccacga cgagcccgaa gtgtggccag ccagagcatc 2100

attgcctata ccatgagcct gggcgccgag aatagcgtgg cctactccaa caacagcatt 2160

gctatcccca ccaacttcac catcagcgtg accaccgaga tcctgccagt gtccatgacc 2220

aagaccagcg tggactgcac catgtacatc tgcggagata gcaccgagtg cagcaacctg 2280

ctgctgcagt acggaagttt ctgcacccag ctgaatcgcg ccctgacagg cattgccgtg 2340

gaacaggata agaacaccca agaggtgttc gcccaagtga agcaaatcta caagacccca 2400

ccaatcaagg atttcggcgg cttcaatttc agccagattc tgcccgatcc aagcaagccc 2460

agcaagcgca gcttcatcga ggacctgctg ttcaacaaag tgacactggc cgacgccgga 2520

ttcatcaagc agtatggcga ttgcctgggc gatattgccg cacgcgatct gatttgcgcc 2580

cagaagttta acggactgac cgtcctgcca ccactgctga cagatgagat gatcgcccag 2640

tacacaagtg ccctgctggc cggaaccatt accagcggat ggacatttgg agccggtgcc 2700

gctctgcaga ttcccttcgc tatgcagatg gcctaccgct tcaatggcat tggcgtgacc 2760

cagaatgtgc tgtacgagaa ccagaagctg atcgccaacc agttcaacag cgccatcggc 2820

aagattcagg acagcctgag tagtaccgcc agcgctctgg gaaagctgca ggatgtggtc 2880

aaccagaacg ctcaggccct gaacaccctg gttaagcagc tgagcagcaa cttcggcgcc 2940

atcagtagcg tgctgaacga tatcctgagc cgcctggata aggtggaagc cgaggtgcag 3000

atcgatcgcc tgattaccgg acgcctgcag tccctgcaga cctatgtgac acagcagctg 3060

atccgagccg ccgagattcg agctagtgct aatctggccg ccaccaagat gagcgaatgt 3120

gtgctgggac agagcaagcg cgtggacttt tgcggcaagg gataccacct gatgagcttc 3180

ccacagagtg ctccacacgg cgtggtgttt ctgcatgtga cctacgtgcc cgctcaagag 3240

aagaatttca ccaccgctcc agccatctgc cacgacggaa aggcccattt tccacgcgag 3300

ggcgtgttcg ttagcaacgg cactcattgg ttcgtcaccc agcgcaactt ctacgagccc 3360

cagatcatca ccaccgacaa caccttcgtc agcggcaact gcgacgtcgt gatcggcatt 3420

gtgaacaaca ccgtgtacga tccactgcag cccgagctgg acagcttcaa agaggaactg 3480

gacaagtact ttaagaacca cacaagcccc gacgtggacc tgggagacat tagcggaatc 3540

aacgccagcg tggtcaacat ccagaaagag attgaccgcc tgaacgaggt ggccaagaat 3600

ctgaacgaga gcctgatcga cctgcaagaa ctgggcaaat acgagcagta cattaagtgg 3660

ccctggtaca tctggctggg cttcattgcc ggactgattg ccatcgtgat ggtcaccatt 3720

atgctgtgct gcatgaccag ttgctgcagc tgcctgaagg gatgctgcag ttgcggaagc 3780

tgctgcaagt tcgacgagga tgatagcgag ccagtgctga agggcgtcaa gctgcactac 3840

acctgataac gagcgcgcct cgaggaattc 3870

<210> 3
<211> 1273
<212> PRT
<213> Artificial sequence

<220>
<223> Spike (S) polypeptide of nCoV

<400> 3

<210>  4
<211>  3870
<212>  DNA
<213>  Artificial sequence

<220>
<223>  Codon-optimized nucleotide sequence of a stabilized form of the
       spike (stab-S) polypeptide of nCoV

<400>  4
gctagcagat ctcgtacggc caccatgttc gtgtttctgg tgctgctgcc actggtgtcc      60

agtcagtgcg tgaacctgac cacacgaaca cagctgccac cagcctacac caatagcttc     120

acccgcggag tgtactaccc cgacaaggtg ttccgcagca gcgtgctgca tagcacccag     180

gatctgtttc tgcccttctt cagcaacgtg acctggttcc acgccatcca cgtgtccggc     240

accaatggca ccaagcgctt cgataatccc gtgctgccct tcaacgatgg cgtgtacttt     300

gccagcaccg agaagtccaa tatcatccgc ggctggatct tcggcaccac actggatagc     360

aagacccaga gcctgctgat cgtgaacaac gccaccaacg tggtcatcaa agtgtgcgag     420

ttccagttct gcaacgaccc cttcctgggc gtctactacc acaagaacaa caagagctgg     480

atggaaagcg agttccgcgt gtacagcagc gccaacaact gcaccttcga gtacgtgtcc     540

cagccattcc tgatggacct ggaaggcaag cagggcaact tcaagaacct gcgcgagttc     600

gtgttcaaga acatcgacgg ctacttcaaa atctacagca agcacacccc aatcaacctc     660

gtgcgcgatc tgccacaggg attcagtgct ctggaacccc tggtggatct gcccatcggc     720

atcaacatta cccgctttca gacactgctg gccctgcacc gcagttactt gacaccaggc     780

gatagcagca gtggatggac agctggtgcc gccgcttact acgttggata tctgcagcca     840

cgcacctttc tgctgaagta caacgagaac ggcaccatca ccgacgccgt ggattgtgct     900

ctcgatcccc tgagcgagac aaagtgcacc ctgaagtcct tcaccgtcga gaagggcatc     960

taccagacca gcaatttccg cgtgcagccc accgagagca tcgtgcgctt ccccaatatc    1020

accaatctgt gccccttcgg cgaggtgttc aatgccacac gctttgcctc cgtgtacgcc    1080

tggaatcgca agcgcattag caactgcgtg gccgactact ccgtgctgta caatagcgcc    1140

agcttcagca ccttcaagtg ctacggcgtg tcacccacca agctgaacga cctgtgcttc    1200

accaatgtgt acgccgacag cttcgtgatc cgcggagatg aagtgcgaca gattgcccca    1260

ggccagaccg gcaagatcgc cgactacaat tacaagctgc ccgacgactt caccggctgc    1320

gtgatcgcct ggaacagcaa caacctggat tccaaagtcg gcggcaacta caactacctg    1380

taccgcctgt tccgcaagag caatctgaag cccttcgagc gcgacatcag caccgaaatc    1440

taccaggccg gaagcacccc atgcaacggc gtggaaggct tcaactgcta cttcccactg    1500

cagtcctacg gatttcagcc cacaaatggc gtgggctacc agccatatcg agtggtggtg    1560

ctgagcttcg aactgctgca tgctccagct accgtgtgcg gccccaagaa gagtaccaac 1620

ctggtcaaga acaaatgcgt gaacttcaac ttcaacggcc tgaccggaac cggcgtgctg 1680

accgagagta acaagaagtt cctgccattc cagcagtttg gccgcgacat tgccgataca 1740

accgatgccg ttcgcgatcc ccagaccttg gagatcctgg atattacccc atgctccttc 1800

ggcggcgtgt ccgtgattac accaggcacc aataccagca accaggtggc cgttctgtac 1860

caggatgtga attgcacaga ggtgcccgtg gccattcacg ccgatcaatt gacaccaaca 1920

tggcgcgtgt actccaccgg cagcaatgtg tttcaaaccc gcgctggatg cctgattgga 1980

gccgagcacg tgaacaatag ctacgagtgc gatatcccca tcggagccgg aatctgcgcc 2040

tcctatcaga cccagaccaa tagtccacga cgagcccgaa gtgtggccag ccagagcatc 2100

attgcctata ccatgagcct gggcgccgag aatagcgtgg cctactccaa caacagcatt 2160

gctatcccca ccaacttcac catcagcgtg accaccgaga tcctgccagt gtccatgacc 2220

aagaccagcg tggactgcac catgtacatc tgcggagata gcaccgagtg cagcaacctg 2280

ctgctgcagt acggaagttt ctgcacccag ctgaatcgcg ccctgacagg cattgccgtg 2340

gaacaggata agaacaccca agaggtgttc gcccaagtga agcaaatcta caagacccca 2400

ccaatcaagg atttcggcgg cttcaatttc agccagattc tgcccgatcc aagcaagccc 2460

agcaagcgca gcttcatcga ggacctgctg ttcaacaaag tgacactggc cgacgccgga 2520

ttcatcaagc agtatggcga ttgcctgggc gatattgccg cacgcgatct gatttgcgcc 2580

cagaagttta acggactgac cgtcctgcca ccactgctga cagatgagat gatcgcccag 2640

tacacaagtg ccctgctggc cggaaccatt accagcggat ggacatttgg agccggtgcc 2700

gctctgcaga ttcccttcgc tatgcagatg gcctaccgct tcaatggcat tggcgtgacc 2760

cagaatgtgc tgtacgagaa ccagaagctg atcgccaacc agttcaacag cgccatcggc 2820

aagattcagg acagcctgag tagtaccgcc agcgctctgg gaaagctgca ggatgtggtc 2880

aaccagaacg ctcaggccct gaacaccctg gttaagcagc tgagcagcaa cttcggcgcc 2940

atcagtagcg tgctgaacga tatcctgagc cgcctggatc caccagaagc cgaggtgcag 3000

atcgatcgcc tgattaccgg acgcctgcag tccctgcaga cctatgtgac acagcagctg 3060

atccgagccg ccgagattcg agctagtgct aatctggccg ccaccaagat gagcgaatgt 3120

gtgctgggac agagcaagcg cgtggacttt tgcggcaagg gataccacct gatgagcttc 3180

ccacagagtg ctccacacgg cgtggtgttt ctgcatgtga cctacgtgcc cgctcaagag 3240

aagaatttca ccaccgctcc agccatctgc cacgacggaa aggcccattt tccacgcgag 3300

ggcgtgttcg ttagcaacgg cactcattgg ttcgtcaccc agcgcaactt ctacgagccc 3360

cagatcatca ccaccgacaa caccttcgtc agcggcaact gcgacgtcgt gatcggcatt 3420

gtgaacaaca ccgtgtacga tccactgcag cccgagctgg acagcttcaa agaggaactg 3480

gacaagtact ttaagaacca cacaagcccc gacgtggacc tgggagacat tagcggaatc 3540

aacgccagcg tggtcaacat ccagaaagag attgaccgcc tgaacgaggt ggccaagaat 3600

ctgaacgaga gcctgatcga cctgcaagaa ctgggcaaat acgagcagta cattaagtgg 3660

ccctggtaca tctggctggg cttcattgcc ggactgattg ccatcgtgat ggtcaccatt 3720

atgctgtgct gcatgaccag ttgctgcagc tgcctgaagg gatgctgcag ttgcggaagc 3780

tgctgcaagt tcgacgagga tgatagcgag ccagtgctga agggcgtcaa gctgcactac 3840

acctgataac gagcgcgcct cgaggaattc 3870

<210> 5
<211> 1273
<212> PRT
<213> Artificial sequence

<220>
<223> Stabilized form of the spike (stab-S) polypeptide of nCoV

<400> 5

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr

145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Pro Pro Glu Ala Glu Val Gln
            980                 985                 990

Ile Asp Arg Leu Ile Thr Gly Arg Leu Gln Ser Leu Gln Thr Tyr Val
        995                 1000                1005

Thr Gln Gln Leu Ile Arg Ala Ala Glu Ile Arg Ala Ser Ala Asn
    1010                1015                1020

Leu Ala Ala Thr Lys Met Ser Glu Cys Val Leu Gly Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn

1145                    1150                    1155

His Thr Ser Pro Asp Val Asp Leu Gly Asp Ile Ser Gly Ile Asn
    1160                    1165                    1170

Ala Ser Val Val Asn Ile Gln Lys Glu Ile Asp Arg Leu Asn Glu
    1175                    1180                    1185

Val Ala Lys Asn Leu Asn Glu Ser Leu Ile Asp Leu Gln Glu Leu
    1190                    1195                    1200

Gly Lys Tyr Glu Gln Tyr Ile Lys Trp Pro Trp Tyr Ile Trp Leu
    1205                    1210                    1215

Gly Phe Ile Ala Gly Leu Ile Ala Ile Val Met Val Thr Ile Met
    1220                    1225                    1230

Leu Cys Cys Met Thr Ser Cys Cys Ser Cys Leu Lys Gly Cys Cys
    1235                    1240                    1245

Ser Cys Gly Ser Cys Cys Lys Phe Asp Glu Asp Asp Ser Glu Pro
    1250                    1255                    1260

Val Leu Lys Gly Val Lys Leu His Tyr Thr
    1265                    1270

<210>  6
<211>  3680
<212>  DNA
<213>  Artificial sequence

<220>
<223>  Codon-optimized nucleotide sequence of the soluble and monomeric
       form of the spike (Secto) ectodomain polypeptide of nCoV

<400>  6
actagctagc ggatcccgta cggccaccat gttcgtgttt ctggtgctgc tgccactggt      60

gtccagtcag tgcgtgaacc tgaccacacg aacacagctg ccaccagcct acaccaatag     120

cttcacccgc ggagtgtact accccgacaa ggtgttccgc agcagcgtgc tgcatagcac     180

ccaggatctg tttctgccct tcttcagcaa cgtgacctgg ttccacgcca tccacgtgtc     240

cggcaccaat ggcaccaagc gcttcgataa tcccgtgctg cccttcaacg atggcgtgta     300

ctttgccagc accgagaagt ccaatatcat ccgcggctgg atcttcggca ccacactgga     360

tagcaagacc cagagcctgc tgatcgtgaa caacgccacc aacgtggtca tcaaagtgtg     420

cgagttccag ttctgcaacg accccttcct gggcgtctac taccacaaga acaacaagag     480

ctggatggaa agcgagttcc gcgtgtacag cagcgccaac aactgcacct tcgagtacgt     540

gtcccagcca ttcctgatgg acctggaagg caagcagggc aacttcaaga acctgcgcga 600

gttcgtgttc aagaacatcg acggctactt caaaatctac agcaagcaca ccccaatcaa 660

cctcgtgcgc gatctgccac agggattcag tgctctggaa cccctggtgg atctgcccat 720

cggcatcaac attacccgct ttcagacact gctggccctg caccgcagtt acttgacacc 780

aggcgatagc agcagtggat ggacagctgg tgccgccgct tactacgttg gatatctgca 840

gccacgcacc tttctgctga agtacaacga gaacggcacc atcaccgacg ccgtggattg 900

tgctctcgat cccctgagcg agacaaagtg caccctgaag tccttcaccg tcgagaaggg 960

catctaccag accagcaatt tccgcgtgca gcccaccgag agcatcgtgc gcttccccaa 1020

tatcaccaat ctgtgcccct tcggcgaggt gttcaatgcc acacgctttg cctccgtgta 1080

cgcctggaat cgcaagcgca ttagcaactg cgtggccgac tactccgtgc tgtacaatag 1140

cgccagcttc agcaccttca agtgctacgg cgtgtcaccc accaagctga acgacctgtg 1200

cttcaccaat gtgtacgccg acagcttcgt gatccgcgga gatgaagtgc gacagattgc 1260

cccaggccag accggcaaga tcgccgacta caattacaag ctgcccgacg acttcaccgg 1320

ctgcgtgatc gcctggaaca gcaacaacct ggattccaaa gtcggcggca actacaacta 1380

cctgtaccgc ctgttccgca agagcaatct gaagcccttc gagcgcgaca tcagcaccga 1440

aatctaccag gccggaagca ccccatgcaa cggcgtggaa ggcttcaact gctacttccc 1500

actgcagtcc tacggatttc agcccacaaa tggcgtgggc taccagccat atcgagtggt 1560

ggtgctgagc ttcgaactgc tgcatgctcc agctaccgtg tgcggcccca agaagagtac 1620

caacctggtc aagaacaaat gcgtgaactt caacttcaac ggcctgaccg gaaccggcgt 1680

gctgaccgag agtaacaaga agttcctgcc attccagcag tttggccgcg acattgccga 1740

tacaaccgat gccgttcgcg atccccagac cttggagatc ctggatatta ccccatgctc 1800

cttcggcggc gtgtccgtga ttacaccagg caccaatacc agcaaccagg tggccgttct 1860

gtaccaggat gtgaattgca cagaggtgcc cgtggccatt cacgccgatc aattgacacc 1920

aacatggcgc gtgtactcca ccggcagcaa tgtgtttcaa acccgcgctg gatgcctgat 1980

tggagccgag cacgtgaaca atagctacga gtgcgatatc cccatcggag ccggaatctg 2040

cgcctcctat cagacccaga ccaatagtcc acgacgagcc cgaagtgtgg ccagccagag 2100

catcattgcc tataccatga gcctgggcgc cgagaatagc gtggcctact ccaacaacag 2160

cattgctatc cccaccaact tcaccatcag cgtgaccacc gagatcctgc cagtgtccat 2220

gaccaagacc agcgtggact gcaccatgta catctgcgga gatagcaccg agtgcagcaa 2280

cctgctgctg cagtacggaa gtttctgcac ccagctgaat cgcgccctga caggcattgc 2340

cgtggaacag gataagaaca cccaagaggt gttcgcccaa gtgaagcaaa tctacaagac 2400

cccaccaatc aaggatttcg gcggcttcaa tttcagccag attctgcccg atccaagcaa 2460

gcccagcaag cgcagcttca tcgaggacct gctgttcaac aaagtgacac tggccgacgc 2520

cggattcatc aagcagtatg gcgattgcct gggcgatatt gccgcacgcg atctgatttg 2580

cgcccagaag tttaacggac tgaccgtcct gccaccactg ctgacagatg agatgatcgc 2640

ccagtacaca agtgccctgc tggccggaac cattaccagc ggatggacat ttggagccgg 2700

tgccgctctg cagattccct tcgctatgca gatggcctac cgcttcaatg gcattggcgt 2760

gacccagaat gtgctgtacg agaaccagaa gctgatcgcc aaccagttca acagcgccat 2820

cggcaagatt caggacagcc tgagtagtac cgccagcgct ctgggaaagc tgcaggatgt 2880

ggtcaaccag aacgctcagg ccctgaacac cctggttaag cagctgagca gcaacttcgg 2940

cgccatcagt agcgtgctga acgatatcct gagccgcctg gataaggtgg aagccgaggt 3000

gcagatcgat cgcctgatta ccggacgcct gcagtccctg cagacctatg tgacacagca 3060

gctgatccga gccgccgaga ttcgagctag tgctaatctg gccgccacca agatgagcga 3120

atgtgtgctg ggacagagca agcgcgtgga cttttgcggc aagggatacc acctgatgag 3180

cttcccacag agtgctccac acggcgtggt gtttctgcat gtgacctacg tgcccgctca 3240

agagaagaat ttcaccaccg ctccagccat ctgccacgac ggaaaggccc attttccacg 3300

cgagggcgtg ttcgttagca acggcactca ttggttcgtc acccagcgca acttctacga 3360

gccccagatc atcaccaccg acaacacctt cgtcagcggc aactgcgacg tcgtgatcgg 3420

cattgtgaac aacaccgtgt acgatccact gcagcccgag ctggacagct tcaaagagga 3480

actggacaag tactttaaga accacacaag ccccgacgtg gacctgggag acattagcgg 3540

aatcaacgcc agcgtggtca acatccagaa agagattgac cgcctgaacg aggtggccaa 3600

gaatctgaac gagagcctga tcgacctgca agaactgggc aaatacgagc agtacattaa 3660

gtgataacga gcgcgctact 3680

<210> 7
<211> 1211
<212> PRT
<213> Artificial sequence

<220>
<223> Soluble and monomeric form of the spike (Secto) ectodomain
 polypeptide of nCoV

<400> 7

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe

785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Thr Lys Met Ser  Glu Cys Val Leu Gly  Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys
    1205                1210

<210> 8
<211> 3680
<212> DNA
<213> Artificial sequence

<220>
<223> Codon-optimized nucleotide sequence of the stabilized form of the
spike (stab-Secto) ectodomain polypeptide of nCoV

<400> 8
actagctagc agatctcgta cggccaccat gttcgtgttt ctggtgctgc tgccactggt 60

gtccagtcag tgcgtgaacc tgaccacacg aacacagctg ccaccagcct acaccaatag 120

cttcacccgc ggagtgtact accccgacaa ggtgttccgc agcagcgtgc tgcatagcac 180

ccaggatctg tttctgccct tcttcagcaa cgtgacctgg ttccacgcca tccacgtgtc 240

cggcaccaat ggcaccaagc gcttcgataa tcccgtgctg cccttcaacg atggcgtgta 300

ctttgccagc accgagaagt ccaatatcat ccgcggctgg atcttcggca ccacactgga 360

tagcaagacc cagagcctgc tgatcgtgaa caacgccacc aacgtggtca tcaaagtgtg 420

cgagttccag ttctgcaacg accccttcct gggcgtctac taccacaaga acaacaagag 480

ctggatggaa agcgagttcc gcgtgtacag cagcgccaac aactgcacct tcgagtacgt 540

gtcccagcca ttcctgatgg acctggaagg caagcagggc aacttcaaga acctgcgcga 600

gttcgtgttc aagaacatcg acggctactt caaaatctac agcaagcaca ccccaatcaa 660

cctcgtgcgc gatctgccac agggattcag tgctctggaa cccctggtgg atctgcccat 720

cggcatcaac attacccgct ttcagacact gctggccctg caccgcagtt acttgacacc 780

aggcgatagc agcagtggat ggacagctgg tgccgccgct tactacgttg gatatctgca 840

gccacgcacc tttctgctga agtacaacga gaacggcacc atcaccgacg ccgtggattg 900

tgctctcgat cccctgagcg agacaaagtg caccctgaag tccttcaccg tcgagaaggg 960

catctaccag accagcaatt tccgcgtgca gcccaccgag agcatcgtgc gcttccccaa 1020

tatcaccaat ctgtgcccct tcggcgaggt gttcaatgcc acacgctttg cctccgtgta 1080

cgcctggaat cgcaagcgca ttagcaactg cgtggccgac tactccgtgc tgtacaatag 1140

cgccagcttc agcaccttca agtgctacgg cgtgtcaccc accaagctga acgacctgtg 1200

cttcaccaat gtgtacgccg acagcttcgt gatccgcgga gatgaagtgc gacagattgc 1260

cccaggccag accggcaaga tcgccgacta caattacaag ctgcccgacg acttcaccgg 1320

ctgcgtgatc gcctggaaca gcaacaacct ggattccaaa gtcggcggca actacaacta 1380

cctgtaccgc ctgttccgca agagcaatct gaagcccttc gagcgcgaca tcagcaccga 1440

aatctaccag gccggaagca ccccatgcaa cggcgtggaa ggcttcaact gctacttccc 1500

actgcagtcc tacggatttc agcccacaaa tggcgtgggc taccagccat atcgagtggt 1560

ggtgctgagc ttcgaactgc tgcatgctcc agctaccgtg tgcggcccca agaagagtac 1620

caacctggtc aagaacaaat gcgtgaactt caacttcaac ggcctgaccg gaaccggcgt 1680

gctgaccgag agtaacaaga agttcctgcc attccagcag tttggccgcg acattgccga 1740

tacaaccgat gccgttcgcg atccccagac cttggagatc ctggatatta ccccatgctc 1800

cttcggcggc gtgtccgtga ttacaccagg caccaatacc agcaaccagg tggccgttct 1860

gtaccaggat gtgaattgca cagaggtgcc cgtggccatt cacgccgatc aattgacacc 1920

aacatggcgc gtgtactcca ccggcagcaa tgtgtttcaa acccgcgctg gatgcctgat 1980

tggagccgag cacgtgaaca atagctacga gtgcgatatc cccatcggag ccggaatctg 2040

cgcctcctat cagacccaga ccaatagtcc acgacgagcc cgaagtgtgg ccagccagag 2100

catcattgcc tataccatga gcctgggcgc cgagaatagc gtggcctact ccaacaacag 2160

cattgctatc cccaccaact tcaccatcag cgtgaccacc gagatcctgc cagtgtccat 2220

gaccaagacc agcgtggact gcaccatgta catctgcgga gatagcaccg agtgcagcaa 2280

cctgctgctg cagtacggaa gtttctgcac ccagctgaat cgcgccctga caggcattgc 2340

cgtggaacag gataagaaca cccaagaggt gttcgcccaa gtgaagcaaa tctacaagac 2400

cccaccaatc aaggatttcg gcggcttcaa tttcagccag attctgcccg atccaagcaa 2460

gcccagcaag cgcagcttca tcgaggacct gctgttcaac aaagtgacac tggccgacgc 2520

cggattcatc aagcagtatg gcgattgcct gggcgatatt gccgcacgcg atctgatttg 2580

cgcccagaag tttaacggac tgaccgtcct gccaccactg ctgacagatg agatgatcgc 2640

ccagtacaca agtgccctgc tggccggaac cattaccagc ggatggacat ttggagccgg 2700

tgccgctctg cagattccct tcgctatgca gatggcctac cgcttcaatg gcattggcgt 2760

gacccagaat gtgctgtacg agaaccagaa gctgatcgcc aaccagttca acagcgccat 2820

cggcaagatt caggacagcc tgagtagtac cgccagcgct ctgggaaagc tgcaggatgt 2880

ggtcaaccag aacgctcagg ccctgaacac cctggttaag cagctgagca gcaacttcgg 2940

cgccatcagt agcgtgctga acgatatcct gagccgcctg gatccaccag aagccgaggt 3000

gcagatcgat cgcctgatta ccggacgcct gcagtccctg cagacctatg tgacacagca 3060

gctgatccga gccgccgaga ttcgagctag tgctaatctg gccgccacca agatgagcga 3120

atgtgtgctg ggacagagca agcgcgtgga cttttgcggc aagggatacc acctgatgag 3180

cttcccacag agtgctccac acggcgtggt gtttctgcat gtgacctacg tgcccgctca 3240

agagaagaat ttcaccaccg ctccagccat ctgccacgac ggaaaggccc attttccacg 3300

cgagggcgtg ttcgttagca acggcactca ttggttcgtc acccagcgca acttctacga 3360

gccccagatc atcaccaccg acaacacctt cgtcagcggc aactgcgacg tcgtgatcgg 3420

cattgtgaac aacaccgtgt acgatccact gcagcccgag ctggacagct tcaaagagga 3480

actggacaag tactttaaga accacacaag ccccgacgtg gacctgggag acattagcgg 3540

aatcaacgcc agcgtggtca acatccagaa agagattgac cgcctgaacg aggtggccaa 3600

gaatctgaac gagagcctga tcgacctgca agaactgggc aaatacgagc agtacattaa 3660

gtgataacga gcgcgctact 3680

<210> 9
<211> 1211
<212> PRT
<213> Artificial sequence

<220>

<223>  Stabilized form of the spike (stab-Secto) ectodomain polypeptide
      of nCoV

<400>  9

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly

                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
        740                 745                 750

Leu Leu Gln Tyr Gly Ser Phe Cys Thr Gln Leu Asn Arg Ala Leu Thr
    755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
        820                 825                 830

Phe Ile Lys Gln Tyr Gly Asp Cys Leu Gly Asp Ile Ala Ala Arg Asp
    835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
        900                 905                 910

Gln Asn Val Leu Tyr Glu Asn Gln Lys Leu Ile Ala Asn Gln Phe Asn
    915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Pro Pro Glu Ala Glu Val Gln
        980                 985                 990

Ile Asp Arg Leu Ile Thr Gly Arg Leu Gln Ser Leu Gln Thr Tyr Val
        995                 1000                1005

Thr Gln Gln Leu Ile Arg Ala Ala Glu Ile Arg Ala Ser Ala Asn
    1010                1015                1020

Leu Ala Ala Thr Lys Met Ser Glu Cys Val Leu Gly Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys
    1205                1210

<210>  10
<211>  2090

<212>  DNA
<213>  Artificial sequence

<220>
<223>  Codon-optimized nucleotide sequence of the S1 polypeptide of nCoV

<400>  10
actagctagc ggatcccgta cggccaccat gttcgtgttt ctggtgctgc tgccactggt      60

gtccagtcag tgcgtgaacc tgaccacacg aacacagctg ccaccagcct acaccaatag     120

cttcacccgc ggagtgtact accccgacaa ggtgttccgc agcagcgtgc tgcatagcac     180

ccaggatctg tttctgccct tcttcagcaa cgtgacctgg ttccacgcca tccacgtgtc     240

cggcaccaat ggcaccaagc gcttcgataa tcccgtgctg cccttcaacg atggcgtgta     300

ctttgccagc accgagaagt ccaatatcat ccgcggctgg atcttcggca ccacactgga     360

tagcaagacc cagagcctgc tgatcgtgaa caacgccacc aacgtggtca tcaaagtgtg     420

cgagttccag ttctgcaacg accccttcct gggcgtctac taccacaaga acaacaagag     480

ctggatggaa agcgagttcc gcgtgtacag cagcgccaac aactgcacct tcgagtacgt     540

gtcccagcca ttcctgatgg acctggaagg caagcagggc aacttcaaga acctgcgcga     600

gttcgtgttc aagaacatcg acggctactt caaaatctac agcaagcaca ccccaatcaa     660

cctcgtgcgc gatctgccac agggattcag tgctctggaa cccctggtgg atctgcccat     720

cggcatcaac attacccgct ttcagacact gctggccctg caccgcagtt acttgacacc     780

aggcgatagc agcagtggat ggacagctgg tgccgccgct tactacgttg gatatctgca     840

gccacgcacc tttctgctga agtacaacga gaacggcacc atcaccgacg ccgtggattg     900

tgctctcgat cccctgagcg agacaaagtg caccctgaag tccttcaccg tcgagaaggg     960

catctaccag accagcaatt tccgcgtgca gcccaccgag agcatcgtgc gcttccccaa    1020

tatcaccaat ctgtgcccct tcggcgaggt gttcaatgcc acacgctttg cctccgtgta    1080

cgcctggaat cgcaagcgca ttagcaactg cgtggccgac tactccgtgc tgtacaatag    1140

cgccagcttc agcaccttca agtgctacgg cgtgtcaccc accaagctga acgacctgtg    1200

cttcaccaat gtgtacgccg acagcttcgt gatccgcgga gatgaagtgc gacagattgc    1260

cccaggccag accggcaaga tcgccgacta caattacaag ctgcccgacg acttcaccgg    1320

ctgcgtgatc gcctggaaca gcaacaacct ggattccaaa gtcggcggca actacaacta    1380

cctgtaccgc ctgttccgca agagcaatct gaagcccttc gagcgcgaca tcagcaccga    1440

aatctaccag gccggaagca ccccatgcaa cggcgtggaa ggcttcaact gctacttccc    1500

actgcagtcc tacggatttc agcccacaaa tggcgtgggc taccagccat atcgagtggt    1560

ggtgctgagc ttcgaactgc tgcatgctcc agctaccgtg tgcggcccca agaagagtac    1620

caacctggtc aagaacaaat gcgtgaactt caacttcaac ggcctgaccg gaaccggcgt    1680

gctgaccgag agtaacaaga agttcctgcc attccagcag tttggccgcg acattgccga 1740

tacaaccgat gccgttcgcg atccccagac cttggagatc ctggatatta ccccatgctc 1800

cttcggcggc gtgtccgtga ttacaccagg caccaatacc agcaaccagg tggccgttct 1860

gtaccaggat gtgaattgca cagaggtgcc cgtggccatt cacgccgatc aattgacacc 1920

aacatggcgc gtgtactcca ccggcagcaa tgtgtttcaa acccgcgctg gatgcctgat 1980

tggagccgag cacgtgaaca atagctacga gtgcgatatc cccatcggag ccggaatctg 2040

cgcctcctat cagacccaga ccaatagtcc atgataacga gcgcgctact 2090

<210> 11
<211> 681
<212> PRT
<213> Artificial sequence

<220>
<223> S1 polypeptide of nCoV

<400> 11

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly

                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro
        675                 680

<210>  12
<211>  1860
<212>  DNA
<213>  Artificial sequence

<220>
<223>  Codon-optimized nucleotide sequence of the S2 polypeptide of nCoV

<400>  12
gctagcggat cccgtacggc caccatgttc gtgtttctgg tgctgctgcc actggtgtcc      60

agtcaggccg gcagtgtggc cagccagagc atcattgcct ataccatgag cctgggcgcc     120

gagaatagcg tggcctactc caacaacagc attgctatcc ccaccaactt caccatcagc     180

gtgaccaccg agatcctgcc agtgtccatg accaagacca gcgtggactg caccatgtac     240

atctgcggag atagcaccga gtgcagcaac ctgctgctgc agtacggaag tttctgcacc     300

cagctgaatc gcgccctgac aggcattgcc gtggaacagg ataagaacac ccaagaggtg     360

ttcgcccaag tgaagcaaat ctacaagacc ccaccaatca aggatttcgg cggcttcaat     420

ttcagccaga ttctgcccga tccaagcaag cccagcaagc gcagcttcat cgaggacctg     480

ctgttcaaca aagtgacact ggccgacgcc ggattcatca agcagtatgg cgattgcctg     540

ggcgatattg ccgcacgcga tctgatttgc gcccagaagt ttaacggact gaccgtcctg     600

ccaccactgc tgacagatga gatgatcgcc cagtacacaa gtgccctgct ggccggaacc     660

attaccagcg gatggacatt tggagccggt gccgctctgc agattccctt cgctatgcag     720

atggcctacc gcttcaatgg cattggcgtg acccagaatg tgctgtacga gaaccagaag     780

ctgatcgcca accagttcaa cagcgccatc ggcaagattc aggacagcct gagtagtacc     840

gccagcgctc tgggaaagct gcaggatgtg gtcaaccaga acgctcaggc cctgaacacc     900

ctggttaagc agctgagcag caacttcggc gccatcagta gcgtgctgaa cgatatcctg     960

agccgcctgg ataaggtgga agccgaggtg cagatcgatc gcctgattac cggacgcctg    1020

cagtccctgc agacctatgt gacacagcag ctgatccgag ccgccgagat tcgagctagt    1080

gctaatctgg ccgccaccaa gatgagcgaa tgtgtgctgg gacagagcaa gcgcgtggac    1140

ttttgcggca agggatacca cctgatgagc ttcccacaga gtgctccaca cggcgtggtg    1200

tttctgcatg tgacctacgt gcccgctcaa gagaagaatt tcaccaccgc tccagccatc    1260

tgccacgacg gaaaggccca ttttccacgc gagggcgtgt tcgttagcaa cggcactcat    1320

tggttcgtca cccagcgcaa cttctacgag ccccagatca tcaccaccga caacaccttc    1380

gtcagcggca actgcgacgt cgtgatcggc attgtgaaca acaccgtgta cgatccactg 1440

cagcccgagc tggacagctt caaagaggaa ctggacaagt actttaagaa ccacacaagc 1500

cccgacgtgg acctgggaga cattagcgga atcaacgcca gcgtggtcaa catccagaaa 1560

gagattgacc gcctgaacga ggtggccaag aatctgaacg agagcctgat cgacctgcaa 1620

gaactgggca aatacgagca gtacattaag tggccctggt acatctggct gggcttcatt 1680

gccggactga ttgccatcgt gatggtcacc attatgctgt gctgcatgac cagttgctgc 1740

agctgcctga agggatgctg cagttgcgga agctgctgca agttcgacga ggatgatagc 1800

gagccagtgc tgaagggcgt caagctgcac tacacctgat aagcgcgcct cgaggaattc 1860

<210> 13
<211> 604
<212> PRT
<213> Artificial sequence

<220>
<223> S2 polypeptide of nCoV

<400> 13

Met Phe Val Phe Leu Val Leu Leu Pro Leu Val Ser Ser Gln Ala Gly
1               5                   10                  15

Ser Val Ala Ser Gln Ser Ile Ile Ala Tyr Thr Met Ser Leu Gly Ala
            20                  25                  30

Glu Asn Ser Val Ala Tyr Ser Asn Asn Ser Ile Ala Ile Pro Thr Asn
        35                  40                  45

Phe Thr Ile Ser Val Thr Thr Glu Ile Leu Pro Val Ser Met Thr Lys
    50                  55                  60

Thr Ser Val Asp Cys Thr Met Tyr Ile Cys Gly Asp Ser Thr Glu Cys
65                  70                  75                  80

Ser Asn Leu Leu Leu Gln Tyr Gly Ser Phe Cys Thr Gln Leu Asn Arg
                85                  90                  95

Ala Leu Thr Gly Ile Ala Val Glu Gln Asp Lys Asn Thr Gln Glu Val
            100                 105                 110

Phe Ala Gln Val Lys Gln Ile Tyr Lys Thr Pro Pro Ile Lys Asp Phe
        115                 120                 125

Gly Gly Phe Asn Phe Ser Gln Ile Leu Pro Asp Pro Ser Lys Pro Ser
    130                 135                 140

Lys Arg Ser Phe Ile Glu Asp Leu Leu Phe Asn Lys Val Thr Leu Ala
145                 150                 155                 160

Asp Ala Gly Phe Ile Lys Gln Tyr Gly Asp Cys Leu Gly Asp Ile Ala
                165                 170                 175

Ala Arg Asp Leu Ile Cys Ala Gln Lys Phe Asn Gly Leu Thr Val Leu
            180                 185                 190

Pro Pro Leu Leu Thr Asp Glu Met Ile Ala Gln Tyr Thr Ser Ala Leu
        195                 200                 205

Leu Ala Gly Thr Ile Thr Ser Gly Trp Thr Phe Gly Ala Gly Ala Ala
    210                 215                 220

Leu Gln Ile Pro Phe Ala Met Gln Met Ala Tyr Arg Phe Asn Gly Ile
225                 230                 235                 240

Gly Val Thr Gln Asn Val Leu Tyr Glu Asn Gln Lys Leu Ile Ala Asn
                245                 250                 255

Gln Phe Asn Ser Ala Ile Gly Lys Ile Gln Asp Ser Leu Ser Ser Thr
            260                 265                 270

Ala Ser Ala Leu Gly Lys Leu Gln Asp Val Val Asn Gln Asn Ala Gln
        275                 280                 285

Ala Leu Asn Thr Leu Val Lys Gln Leu Ser Ser Asn Phe Gly Ala Ile
    290                 295                 300

Ser Ser Val Leu Asn Asp Ile Leu Ser Arg Leu Asp Lys Val Glu Ala
305                 310                 315                 320

Glu Val Gln Ile Asp Arg Leu Ile Thr Gly Arg Leu Gln Ser Leu Gln
                325                 330                 335

Thr Tyr Val Thr Gln Gln Leu Ile Arg Ala Ala Glu Ile Arg Ala Ser
            340                 345                 350

Ala Asn Leu Ala Ala Thr Lys Met Ser Glu Cys Val Leu Gly Gln Ser
        355                 360                 365

Lys Arg Val Asp Phe Cys Gly Lys Gly Tyr His Leu Met Ser Phe Pro
    370                 375                 380

Gln Ser Ala Pro His Gly Val Val Phe Leu His Val Thr Tyr Val Pro
385                 390                 395                 400

Ala Gln Glu Lys Asn Phe Thr Thr Ala Pro Ala Ile Cys His Asp Gly
                405                 410                 415

Lys Ala His Phe Pro Arg Glu Gly Val Phe Val Ser Asn Gly Thr His
            420                 425                 430

Trp Phe Val Thr Gln Arg Asn Phe Tyr Glu Pro Gln Ile Ile Thr Thr
        435                 440                 445

Asp Asn Thr Phe Val Ser Gly Asn Cys Asp Val Val Ile Gly Ile Val
    450                 455                 460

Asn Asn Thr Val Tyr Asp Pro Leu Gln Pro Glu Leu Asp Ser Phe Lys
465                 470                 475                 480

Glu Glu Leu Asp Lys Tyr Phe Lys Asn His Thr Ser Pro Asp Val Asp
                485                 490                 495

Leu Gly Asp Ile Ser Gly Ile Asn Ala Ser Val Val Asn Ile Gln Lys
            500                 505                 510

Glu Ile Asp Arg Leu Asn Glu Val Ala Lys Asn Leu Asn Glu Ser Leu
        515                 520                 525

Ile Asp Leu Gln Glu Leu Gly Lys Tyr Glu Gln Tyr Ile Lys Trp Pro
    530                 535                 540

Trp Tyr Ile Trp Leu Gly Phe Ile Ala Gly Leu Ile Ala Ile Val Met
545                 550                 555                 560

Val Thr Ile Met Leu Cys Cys Met Thr Ser Cys Cys Ser Cys Leu Lys
                565                 570                 575

Gly Cys Cys Ser Cys Gly Ser Cys Cys Lys Phe Asp Glu Asp Asp Ser
            580                 585                 590

Glu Pro Val Leu Lys Gly Val Lys Leu His Tyr Thr
        595                 600

<210>  14
<211>  1860
<212>  DNA
<213>  Artificial sequence

<220>
<223>  Codon-optimized nucleotide sequence of the stabilized form of the
       S2 (stab-S2) polypeptide of nCoV

<400>  14

gctagcagat ctcgtacggc caccatgttc gtgtttctgg tgctgctgcc actggtgtcc 60

agtcaggccg gcagtgtggc cagccagagc atcattgcct ataccatgag cctgggcgcc 120

gagaatagcg tggcctactc caacaacagc attgctatcc ccaccaactt caccatcagc 180

gtgaccaccg agatcctgcc agtgtccatg accaagacca gcgtggactg caccatgtac 240

atctgcggag atagcaccga gtgcagcaac ctgctgctgc agtacggaag tttctgcacc 300

cagctgaatc gcgccctgac aggcattgcc gtggaacagg ataagaacac ccaagaggtg 360

ttcgcccaag tgaagcaaat ctacaagacc ccaccaatca aggatttcgg cggcttcaat 420

ttcagccaga ttctgcccga tccaagcaag cccagcaagc gcagcttcat cgaggacctg 480

ctgttcaaca aagtgacact ggccgacgcc ggattcatca agcagtatgg cgattgcctg 540

ggcgatattg ccgcacgcga tctgatttgc gcccagaagt ttaacggact gaccgtcctg 600

ccaccactgc tgacagatga gatgatcgcc cagtacacaa gtgccctgct ggccggaacc 660

attaccagcg gatggacatt tggagccggt gccgctctgc agattccctt cgctatgcag 720

atggcctacc gcttcaatgg cattggcgtg acccagaatg tgctgtacga gaaccagaag 780

ctgatcgcca accagttcaa cagcgccatc ggcaagattc aggacagcct gagtagtacc 840

gccagcgctc tgggaaagct gcaggatgtg gtcaaccaga acgctcaggc cctgaacacc 900

ctggttaagc agctgagcag caacttcggc gccatcagta gcgtgctgaa cgatatcctg 960

agccgcctgg atccaccaga agccgaggtg cagatcgatc gcctgattac cggacgcctg 1020

cagtccctgc agacctatgt gacacagcag ctgatccgag ccgccgagat tcgagctagt 1080

gctaatctgg ccgccaccaa gatgagcgaa tgtgtgctgg gacagagcaa gcgcgtggac 1140

ttttgcggca agggatacca cctgatgagc ttcccacaga gtgctccaca cggcgtggtg 1200

tttctgcatg tgacctacgt gcccgctcaa gagaagaatt tcaccaccgc tccagccatc 1260

tgccacgacg gaaaggccca ttttccacgc gagggcgtgt tcgttagcaa cggcactcat 1320

tggttcgtca cccagcgcaa cttctacgag ccccagatca tcaccaccga caacaccttc 1380

gtcagcggca actgcgacgt cgtgatcggc attgtgaaca acaccgtgta cgatccactg 1440

cagcccgagc tggacagctt caaagaggaa ctggacaagt actttaagaa ccacacaagc 1500

cccgacgtgg acctgggaga cattagcgga atcaacgcca gcgtggtcaa catccagaaa 1560

gagattgacc gcctgaacga ggtggccaag aatctgaacg agagcctgat cgacctgcaa 1620

gaactgggca aatacgagca gtacattaag tggccctggt acatctggct gggcttcatt 1680

gccggactga ttgccatcgt gatggtcacc attatgctgt gctgcatgac cagttgctgc 1740

agctgcctga agggatgctg cagttgcgga agctgctgca agttcgacga ggatgatagc 1800

gagccagtgc tgaagggcgt caagctgcac tacacctgat aagcgcgcct cgaggaattc 1860

<210>  15
<211>  604
<212>  PRT
<213>  Artificial sequence

<220>
<223>  Stabilized form of the S2 polypeptide (stab-S2) of nCoV

<400>  15

Met Phe Val Phe Leu Val Leu Leu Pro Leu Val Ser Ser Gln Ala Gly
1               5                   10                  15

Ser Val Ala Ser Gln Ser Ile Ile Ala Tyr Thr Met Ser Leu Gly Ala
            20                  25                  30

Glu Asn Ser Val Ala Tyr Ser Asn Asn Ser Ile Ala Ile Pro Thr Asn
        35                  40                  45

Phe Thr Ile Ser Val Thr Thr Glu Ile Leu Pro Val Ser Met Thr Lys
    50                  55                  60

Thr Ser Val Asp Cys Thr Met Tyr Ile Cys Gly Asp Ser Thr Glu Cys
65                  70                  75                  80

Ser Asn Leu Leu Leu Gln Tyr Gly Ser Phe Cys Thr Gln Leu Asn Arg
                85                  90                  95

Ala Leu Thr Gly Ile Ala Val Glu Gln Asp Lys Asn Thr Gln Glu Val
            100                 105                 110

Phe Ala Gln Val Lys Gln Ile Tyr Lys Thr Pro Pro Ile Lys Asp Phe
        115                 120                 125

Gly Gly Phe Asn Phe Ser Gln Ile Leu Pro Asp Pro Ser Lys Pro Ser
    130                 135                 140

Lys Arg Ser Phe Ile Glu Asp Leu Leu Phe Asn Lys Val Thr Leu Ala
145                 150                 155                 160

Asp Ala Gly Phe Ile Lys Gln Tyr Gly Asp Cys Leu Gly Asp Ile Ala
                165                 170                 175

Ala Arg Asp Leu Ile Cys Ala Gln Lys Phe Asn Gly Leu Thr Val Leu
            180                 185                 190

Pro Pro Leu Leu Thr Asp Glu Met Ile Ala Gln Tyr Thr Ser Ala Leu
        195                 200                 205

Leu Ala Gly Thr Ile Thr Ser Gly Trp Thr Phe Gly Ala Gly Ala Ala

    210                 215                 220

Leu Gln Ile Pro Phe Ala Met Gln Met Ala Tyr Arg Phe Asn Gly Ile
225                 230                 235                 240

Gly Val Thr Gln Asn Val Leu Tyr Glu Asn Gln Lys Leu Ile Ala Asn
                245                 250                 255

Gln Phe Asn Ser Ala Ile Gly Lys Ile Gln Asp Ser Leu Ser Ser Thr
            260                 265                 270

Ala Ser Ala Leu Gly Lys Leu Gln Asp Val Val Asn Gln Asn Ala Gln
        275                 280                 285

Ala Leu Asn Thr Leu Val Lys Gln Leu Ser Ser Asn Phe Gly Ala Ile
    290                 295                 300

Ser Ser Val Leu Asn Asp Ile Leu Ser Arg Leu Asp Pro Pro Glu Ala
305                 310                 315                 320

Glu Val Gln Ile Asp Arg Leu Ile Thr Gly Arg Leu Gln Ser Leu Gln
                325                 330                 335

Thr Tyr Val Thr Gln Gln Leu Ile Arg Ala Ala Glu Ile Arg Ala Ser
            340                 345                 350

Ala Asn Leu Ala Ala Thr Lys Met Ser Glu Cys Val Leu Gly Gln Ser
        355                 360                 365

Lys Arg Val Asp Phe Cys Gly Lys Gly Tyr His Leu Met Ser Phe Pro
    370                 375                 380

Gln Ser Ala Pro His Gly Val Val Phe Leu His Val Thr Tyr Val Pro
385                 390                 395                 400

Ala Gln Glu Lys Asn Phe Thr Thr Ala Pro Ala Ile Cys His Asp Gly
                405                 410                 415

Lys Ala His Phe Pro Arg Glu Gly Val Phe Val Ser Asn Gly Thr His
            420                 425                 430

Trp Phe Val Thr Gln Arg Asn Phe Tyr Glu Pro Gln Ile Ile Thr Thr
        435                 440                 445

Asp Asn Thr Phe Val Ser Gly Asn Cys Asp Val Val Ile Gly Ile Val
    450                 455                 460

Asn Asn Thr Val Tyr Asp Pro Leu Gln Pro Glu Leu Asp Ser Phe Lys
465                 470                 475                 480

Glu Glu Leu Asp Lys Tyr Phe Lys Asn His Thr Ser Pro Asp Val Asp
                485                 490                 495

Leu Gly Asp Ile Ser Gly Ile Asn Ala Ser Val Val Asn Ile Gln Lys
            500                 505                 510

Glu Ile Asp Arg Leu Asn Glu Val Ala Lys Asn Leu Asn Glu Ser Leu
        515                 520                 525

Ile Asp Leu Gln Glu Leu Gly Lys Tyr Glu Gln Tyr Ile Lys Trp Pro
    530                 535                 540

Trp Tyr Ile Trp Leu Gly Phe Ile Ala Gly Leu Ile Ala Ile Val Met
545                 550                 555                 560

Val Thr Ile Met Leu Cys Cys Met Thr Ser Cys Cys Ser Cys Leu Lys
                565                 570                 575

Gly Cys Cys Ser Cys Gly Ser Cys Cys Lys Phe Asp Glu Asp Asp Ser
            580                 585                 590

Glu Pro Val Leu Lys Gly Val Lys Leu His Tyr Thr
        595                 600

<210>  16
<211>  3772
<212>  DNA
<213>  Artificial sequence

<220>
<223>  Codon-optimized nucleotide sequence of the soluble and trimerized
       form of the spike (tri-Secto) polypeptide of nCoV

<400>  16
actagctagc ggatcccgta cggccaccat gttcgtgttt ctggtgctgc tgccactggt      60

gtccagtcag tgcgtgaacc tgaccacacg aacacagctg ccaccagcct acaccaatag     120

cttcacccgc ggagtgtact accccgacaa ggtgttccgc agcagcgtgc tgcatagcac     180

ccaggatctg tttctgccct tcttcagcaa cgtgacctgg ttccacgcca tccacgtgtc     240

cggcaccaat ggcaccaagc gcttcgataa tcccgtgctg cccttcaacg atggcgtgta     300

ctttgccagc accgagaagt ccaatatcat ccgcggctgg atcttcggca ccacactgga     360

tagcaagacc cagagcctgc tgatcgtgaa caacgccacc aacgtggtca tcaaagtgtg     420

cgagttccag ttctgcaacg accccttcct gggcgtctac taccacaaga acaacaagag     480

ctggatggaa agcgagttcc gcgtgtacag cagcgccaac aactgcacct tcgagtacgt     540

gtcccagcca ttcctgatgg acctggaagg caagcagggc aacttcaaga acctgcgcga 600

gttcgtgttc aagaacatcg acggctactt caaaatctac agcaagcaca ccccaatcaa 660

cctcgtgcgc gatctgccac agggattcag tgctctggaa cccctggtgg atctgcccat 720

cggcatcaac attacccgct ttcagacact gctggccctg caccgcagtt acttgacacc 780

aggcgatagc agcagtggat ggacagctgg tgccgccgct tactacgttg gatatctgca 840

gccacgcacc tttctgctga agtacaacga gaacggcacc atcaccgacg ccgtggattg 900

tgctctcgat cccctgagcg agacaaagtg caccctgaag tccttcaccg tcgagaaggg 960

catctaccag accagcaatt tccgcgtgca gcccaccgag agcatcgtgc gcttccccaa 1020

tatcaccaat ctgtgcccct tcggcgaggt gttcaatgcc acacgctttg cctccgtgta 1080

cgcctggaat cgcaagcgca ttagcaactg cgtggccgac tactccgtgc tgtacaatag 1140

cgccagcttc agcaccttca agtgctacgg cgtgtcaccc accaagctga acgacctgtg 1200

cttcaccaat gtgtacgccg acagcttcgt gatccgcgga gatgaagtgc gacagattgc 1260

cccaggccag accggcaaga tcgccgacta caattacaag ctgcccgacg acttcaccgg 1320

ctgcgtgatc gcctggaaca gcaacaacct ggattccaaa gtcggcggca actacaacta 1380

cctgtaccgc ctgttccgca agagcaatct gaagcccttc gagcgcgaca tcagcaccga 1440

aatctaccag gccggaagca ccccatgcaa cggcgtggaa ggcttcaact gctacttccc 1500

actgcagtcc tacggatttc agcccacaaa tggcgtgggc taccagccat atcgagtggt 1560

ggtgctgagc ttcgaactgc tgcatgctcc agctaccgtg tgcggcccca agaagagtac 1620

caacctggtc aagaacaaat gcgtgaactt caacttcaac ggcctgaccg gaaccggcgt 1680

gctgaccgag agtaacaaga agttcctgcc attccagcag tttggccgcg acattgccga 1740

tacaaccgat gccgttcgcg atccccagac cttggagatc ctggatatta ccccatgctc 1800

cttcggcggc gtgtccgtga ttacaccagg caccaatacc agcaaccagg tggccgttct 1860

gtaccaggat gtgaattgca cagaggtgcc cgtggccatt cacgccgatc aattgacacc 1920

aacatggcgc gtgtactcca ccggcagcaa tgtgtttcaa acccgcgctg gatgcctgat 1980

tggagccgag cacgtgaaca atagctacga gtgcgatatc cccatcggag ccggaatctg 2040

cgcctcctat cagacccaga ccaatagtcc acgacgagcc cgaagtgtgg ccagccagag 2100

catcattgcc tataccatga gcctgggcgc cgagaatagc gtggcctact ccaacaacag 2160

cattgctatc cccaccaact tcaccatcag cgtgaccacc gagatcctgc cagtgtccat 2220

gaccaagacc agcgtggact gcaccatgta catctgcgga gatagcaccg agtgcagcaa 2280

cctgctgctg cagtacggaa gtttctgcac ccagctgaat cgcgccctga caggcattgc 2340

cgtggaacag gataagaaca cccaagaggt gttcgcccaa gtgaagcaaa tctacaagac 2400

cccaccaatc aaggatttcg gcggcttcaa tttcagccag attctgcccg atccaagcaa 2460

gcccagcaag cgcagcttca tcgaggacct gctgttcaac aaagtgacac tggccgacgc 2520

cggattcatc aagcagtatg gcgattgcct gggcgatatt gccgcacgcg atctgatttg 2580

cgcccagaag tttaacggac tgaccgtcct gccaccactg ctgacagatg agatgatcgc 2640

ccagtacaca agtgccctgc tggccggaac cattaccagc ggatggacat ttggagccgg 2700

tgccgctctg cagattccct tcgctatgca gatggcctac cgcttcaatg gcattggcgt 2760

gacccagaat gtgctgtacg agaaccagaa gctgatcgcc aaccagttca acagcgccat 2820

cggcaagatt caggacagcc tgagtagtac cgccagcgct ctgggaaagc tgcaggatgt 2880

ggtcaaccag aacgctcagg ccctgaacac cctggttaag cagctgagca gcaacttcgg 2940

cgccatcagt agcgtgctga acgatatcct gagccgcctg gataaggtgg aagccgaggt 3000

gcagatcgat cgcctgatta ccggacgcct gcagtccctg cagacctatg tgacacagca 3060

gctgatccga gccgccgaga ttcgagctag tgctaatctg gccgccacca agatgagcga 3120

atgtgtgctg ggacagagca agcgcgtgga cttttgcggc aagggatacc acctgatgag 3180

cttcccacag agtgctccac acggcgtggt gtttctgcat gtgacctacg tgcccgctca 3240

agagaagaat ttcaccaccg ctccagccat ctgccacgac ggaaaggccc attttccacg 3300

cgagggcgtg ttcgttagca acggcactca ttggttcgtc acccagcgca acttctacga 3360

gccccagatc atcaccaccg acaacacctt cgtcagcggc aactgcgacg tcgtgatcgg 3420

cattgtgaac aacaccgtgt acgatccact gcagcccgag ctggacagct tcaaagagga 3480

actggacaag tactttaaga accacacaag ccccgacgtg gacctgggag acattagcgg 3540

aatcaacgcc agcgtggtca acatccagaa agagattgac cgcctgaacg aggtggccaa 3600

gaatctgaac gagagcctga tcgacctgca agaactgggc aaatacgagc agtacattaa 3660

gtccggaggc tacatccccg aggccccacg cgatggccag gcctacgtgc gcaaggacgg 3720

cgagtgggtg ctgctgtcca ccttcctgtg ataagcgcgc ctcgaggaat tc 3772

<210> 17
<211> 1240
<212> PRT
<213> Artificial sequence

<220>
<223> Soluble and trimerized form of the spike (tri-Secto) polypeptide
 of nCoV

<400> 17

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe

            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Thr Lys Met Ser  Glu Cys Val Leu Gly  Gln Ser Lys

1025                    1030                    1035

Arg Val  Asp Phe Cys Gly Lys  Gly Tyr His Leu Met  Ser Phe Pro
    1040                    1045                    1050

Gln Ser  Ala Pro His Gly Val  Val Phe Leu His Val  Thr Tyr Val
    1055                    1060                    1065

Pro Ala  Gln Glu Lys Asn Phe  Thr Thr Ala Pro Ala  Ile Cys His
    1070                    1075                    1080

Asp Gly  Lys Ala His Phe Pro  Arg Glu Gly Val Phe  Val Ser Asn
    1085                    1090                    1095

Gly Thr  His Trp Phe Val Thr  Gln Arg Asn Phe Tyr  Glu Pro Gln
    1100                    1105                    1110

Ile Ile  Thr Thr Asp Asn Thr  Phe Val Ser Gly Asn  Cys Asp Val
    1115                    1120                    1125

Val Ile  Gly Ile Val Asn Asn  Thr Val Tyr Asp Pro  Leu Gln Pro
    1130                    1135                    1140

Glu Leu  Asp Ser Phe Lys Glu  Glu Leu Asp Lys Tyr  Phe Lys Asn
    1145                    1150                    1155

His Thr  Ser Pro Asp Val Asp  Leu Gly Asp Ile Ser  Gly Ile Asn
    1160                    1165                    1170

Ala Ser  Val Val Asn Ile Gln  Lys Glu Ile Asp Arg  Leu Asn Glu
    1175                    1180                    1185

Val Ala  Lys Asn Leu Asn Glu  Ser Leu Ile Asp Leu  Gln Glu Leu
    1190                    1195                    1200

Gly Lys  Tyr Glu Gln Tyr Ile  Lys Ser Gly Gly Tyr  Ile Pro Glu
    1205                    1210                    1215

Ala Pro  Arg Asp Gly Gln Ala  Tyr Val Arg Lys Asp  Gly Glu Trp
    1220                    1225                    1230

Val Leu  Leu Ser Thr Phe Leu
    1235                    1240

<210>  18
<211>  3772
<212>  DNA
<213>  Artificial sequence

<220>
<223>  Codon-optimized nucleotide sequence of the stabilized and
      trimeric form of the spike ectodomain (tristab-Secto) polypeptide
      of nCoV

<400>  18
actagctagc agatctcgta cggccaccat gttcgtgttt ctggtgctgc tgccactggt      60

gtccagtcag tgcgtgaacc tgaccacacg aacacagctg ccaccagcct acaccaatag     120

cttcacccgc ggagtgtact accccgacaa ggtgttccgc agcagcgtgc tgcatagcac     180

ccaggatctg tttctgccct tcttcagcaa cgtgacctgg ttccacgcca tccacgtgtc     240

cggcaccaat ggcaccaagc gcttcgataa tcccgtgctg cccttcaacg atggcgtgta     300

ctttgccagc accgagaagt ccaatatcat ccgcggctgg atcttcggca ccacactgga     360

tagcaagacc cagagcctgc tgatcgtgaa caacgccacc aacgtggtca tcaaagtgtg     420

cgagttccag ttctgcaacg accccttcct gggcgtctac taccacaaga acaacaagag     480

ctggatggaa agcgagttcc gcgtgtacag cagcgccaac aactgcacct tcgagtacgt     540

gtcccagcca ttcctgatgg acctggaagg caagcagggc aacttcaaga acctgcgcga     600

gttcgtgttc aagaacatcg acggctactt caaaatctac agcaagcaca ccccaatcaa     660

cctcgtgcgc gatctgccac agggattcag tgctctggaa cccctggtgg atctgcccat     720

cggcatcaac attacccgct ttcagacact gctggccctg caccgcagtt acttgacacc     780

aggcgatagc agcagtggat ggacagctgg tgccgccgct tactacgttg gatatctgca     840

gccacgcacc tttctgctga agtacaacga gaacggcacc atcaccgacg ccgtggattg     900

tgctctcgat cccctgagcg agacaaagtg caccctgaag tccttcaccg tcgagaaggg     960

catctaccag accagcaatt tccgcgtgca gcccaccgag agcatcgtgc gcttccccaa    1020

tatcaccaat ctgtgcccct tcggcgaggt gttcaatgcc acacgctttg cctccgtgta    1080

cgcctggaat cgcaagcgca ttagcaactg cgtggccgac tactccgtgc tgtacaatag    1140

cgccagcttc agcaccttca agtgctacgg cgtgtcaccc accaagctga acgacctgtg    1200

cttcaccaat gtgtacgccg acagcttcgt gatccgcgga gatgaagtgc gacagattgc    1260

cccaggccag accggcaaga tcgccgacta caattacaag ctgcccgacg acttcaccgg    1320

ctgcgtgatc gcctggaaca gcaacaacct ggattccaaa gtcggcggca actacaacta    1380

cctgtaccgc ctgttccgca agagcaatct gaagcccttc gagcgcgaca tcagcaccga    1440

aatctaccag gccggaagca ccccatgcaa cggcgtggaa ggcttcaact gctacttccc    1500

actgcagtcc tacggatttc agcccacaaa tggcgtgggc taccagccat atcgagtggt    1560

ggtgctgagc ttcgaactgc tgcatgctcc agctaccgtg tgcggcccca agaagagtac    1620

caacctggtc aagaacaaat gcgtgaactt caacttcaac ggcctgaccg gaaccggcgt    1680

gctgaccgag agtaacaaga agttcctgcc attccagcag tttggccgcg acattgccga 1740

tacaaccgat gccgttcgcg atccccagac cttggagatc ctggatatta ccccatgctc 1800

cttcggcggc gtgtccgtga ttacaccagg caccaatacc agcaaccagg tggccgttct 1860

gtaccaggat gtgaattgca cagaggtgcc cgtggccatt cacgccgatc aattgacacc 1920

aacatggcgc gtgtactcca ccggcagcaa tgtgtttcaa acccgcgctg gatgcctgat 1980

tggagccgag cacgtgaaca atagctacga gtgcgatatc cccatcggag ccggaatctg 2040

cgcctcctat cagacccaga ccaatagtcc acgacgagcc cgaagtgtgg ccagccagag 2100

catcattgcc tataccatga gcctgggcgc cgagaatagc gtggcctact ccaacaacag 2160

cattgctatc cccaccaact tcaccatcag cgtgaccacc gagatcctgc cagtgtccat 2220

gaccaagacc agcgtggact gcaccatgta catctgcgga gatagcaccg agtgcagcaa 2280

cctgctgctg cagtacggaa gtttctgcac ccagctgaat cgcgccctga caggcattgc 2340

cgtggaacag gataagaaca cccaagaggt gttcgcccaa gtgaagcaaa tctacaagac 2400

cccaccaatc aaggatttcg gcggcttcaa tttcagccag attctgcccg atccaagcaa 2460

gcccagcaag cgcagcttca tcgaggacct gctgttcaac aaagtgacac tggccgacgc 2520

cggattcatc aagcagtatg gcgattgcct gggcgatatt gccgcacgcg atctgatttg 2580

cgcccagaag tttaacggac tgaccgtcct gccaccactg ctgacagatg agatgatcgc 2640

ccagtacaca agtgccctgc tggccggaac cattaccagc ggatggacat ttggagccgg 2700

tgccgctctg cagattccct tcgctatgca gatggcctac cgcttcaatg gcattggcgt 2760

gacccagaat gtgctgtacg agaaccagaa gctgatcgcc aaccagttca acagcgccat 2820

cggcaagatt caggacagcc tgagtagtac cgccagcgct ctgggaaagc tgcaggatgt 2880

ggtcaaccag aacgctcagg ccctgaacac cctggttaag cagctgagca gcaacttcgg 2940

cgccatcagt agcgtgctga acgatatcct gagccgcctg gatccaccag aagccgaggt 3000

gcagatcgat cgcctgatta ccggacgcct gcagtccctg cagacctatg tgacacagca 3060

gctgatccga gccgccgaga ttcgagctag tgctaatctg gccgccacca agatgagcga 3120

atgtgtgctg ggacagagca agcgcgtgga cttttgcggc aagggatacc acctgatgag 3180

cttcccacag agtgctccac acggcgtggt gtttctgcat gtgacctacg tgcccgctca 3240

agagaagaat ttcaccaccg ctccagccat ctgccacgac ggaaaggccc attttccacg 3300

cgagggcgtg ttcgttagca acggcactca ttggttcgtc acccagcgca acttctacga 3360

gccccagatc atcaccaccg acaacacctt cgtcagcggc aactgcgacg tcgtgatcgg 3420

cattgtgaac aacaccgtgt acgatccact gcagcccgag ctggacagct tcaaagagga 3480

actggacaag tactttaaga accacacaag ccccgacgtg gacctgggag acattagcgg 3540

aatcaacgcc agcgtggtca acatccagaa agagattgac cgcctgaacg aggtggccaa 3600

gaatctgaac gagagcctga tcgacctgca agaactgggc aaatacgagc agtacattaa     3660

gtccggaggc tacatccccg aggccccacg cgatggccag gcctacgtgc gcaaggacgg     3720

cgagtgggtg ctgctgtcca ccttcctgtg ataagcgcgc ctcgaggaat tc             3772

<210>  19
<211>  1240
<212>  PRT
<213>  Artificial sequence

<220>
<223>  Stabilized and trimeric form of the spike ectodomain
       (tristab-Secto) polypeptide of nCoV

<400>  19

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala

930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Pro Pro Glu Ala Glu Val Gln
            980                 985                 990

Ile Asp Arg Leu Ile Thr Gly Arg Leu Gln Ser Leu Gln Thr Tyr Val
        995                 1000                1005

Thr Gln Gln Leu Ile Arg Ala Ala Glu Ile Arg Ala Ser Ala Asn
    1010                1015                1020

Leu Ala Ala Thr Lys Met Ser Glu Cys Val Leu Gly Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Ser Gly Gly Tyr Ile Pro Glu
    1205                1210                1215

Ala Pro Arg Asp Gly Gln Ala Tyr Val Arg Lys Asp Gly Glu Trp
    1220                1225                1230

Val Leu Leu Ser Thr Phe Leu
    1235                1240

<210>  20
<211>  1308
<212>  DNA
<213>  Artificial sequence

<220>
<223>  Native nucleotide sequence of the N polypeptide of CoV

<400>  20
gctagcggat cccgtacggc caccatgtct gataatggac cccaaaatca gcgaaatgca      60

ccccgcatta cgtttggtgg accctcagat tcaactggca gtaaccagaa tggagaacgc     120

agtggggcgc gatcaaaaca acgtcggccc caaggtttac ccaataatac tgcgtcttgg     180

ttcaccgctc tcactcaaca tggcaaggaa gaccttaaat tccctcgagg acaaggcgtt     240

ccaattaaca ccaatagcag tccagatgac caaattggct actaccgaag agctaccaga     300

cgaattcgtg gtggtgacgg taaaatgaaa gatctcagtc caagatggta tttctactac     360

ctaggaactg ggccagaagc tggacttccc tatggtgcta acaaagacgg catcatatgg     420

gttgcaactg agggagcctt gaatacacca aaagatcaca ttggcacccg caatcctgct     480

aacaatgctg caatcgtgct acaacttcct caaggaacaa cattgccaaa aggcttctac     540

gcagaaggga gcagaggcgg cagtcaagcc tcttctcgtt cctcatcacg tagtcgcaac     600

agttcaagaa attcaactcc aggcagcagt aggggaactt ctcctgctag aatggctggc     660

aatggcggtg atgctgctct tgctttgctg ctgcttgaca gattgaacca gcttgagagc     720

aaaatgtctg gtaaaggcca acaacaacaa ggccaaactg tcactaagaa atctgctgct     780

gaggcttcta agaagcctcg gcaaaaacgt actgccacta aagcatacaa tgtaacacaa     840

gctttcggca gacgtggtcc agaacaaacc caaggaaatt ttggggacca ggaactaatc     900

agacaaggaa ctgattacaa acattggccg caaattgcac aatttgcccc cagcgcttca     960

gcgttcttcg gaatgtcgcg cattggcatg gaagtcacac cttcgggaac gtggttgacc    1020

tacacaggtg ccatcaaatt ggatgacaaa gatccaaatt tcaaagatca agtcattttg 1080

ctgaataagc atattgacgc atacaaaaca ttcccaccaa cagagcctaa aaaggacaaa 1140

aagaagaagg ctgatgaaac tcaagcctta ccgcagagac agaagaaaca gcaaactgtg 1200

actcttcttc ctgctgcaga tttggatgat ttctccaaac aattgcaaca atccatgagc 1260

agtgctgact caactcaggc ctgataacga gcgcgcctcg aggaattc 1308

<210> 21
<211> 1308
<212> DNA
<213> Artificial sequence

<220>
<223> Codon-optimized nucleotide sequence of the N polypeptide of CoV

<400> 21
gctagcggat cccgtacggc caccatgagc gataatggac cccagaatca gcgcaacgcc 60

ccacgcatta ccttcggagg accaagcgat agcaccggca gcaatcagaa tggcgaacga 120

agcggagccc gaagcaaaca gcgacgacca cagggactgc caaacaacac cgccagttgg 180

tttaccgctc tgacccagca cggcaaagag gacctgaagt ttccacgagg acagggcgtg 240

cccatcaaca ccaatagcag cccagacgac cagatcggct actatcgacg agccacacga 300

cgcattcgag gcggcgacgg aaagatgaag gatctgagcc cacgctggta cttctactac 360

ctcggaacag gaccagaggc cggactgcca tatggtgcca acaaggatgg catcatctgg 420

gttgccaccg aaggcgccct gaacacccca aaggatcaca ttggaacccg caatccagcc 480

aacaacgccg ccattgtgct gcagttgcca cagggaacaa cactgcccaa gggattctac 540

gctgagggaa gtcgcggagg aagccaggcc agtagtcgca gtagtagccg cagccgcaat 600

agctcccgaa atagcacacc cggaagcagc cgaggaacca gtccagctcg aatggctgga 660

aatggcggag atgctgctct ggctctgctg ctgctggatc gcctgaatca gctggaaagc 720

aagatgagcg gcaagggcca gcagcaacag ggacagaccg tgacaaagaa gagtgccgcc 780

gaagccagca agaagccacg acagaagcga accgccacca aggcctataa tgtgacccag 840

gcctttggac gacgcggacc agaacagacc cagggcaatt tcggcgatca agagctgatt 900

cgccagggca ccgattacaa gcactggcca cagattgccc agtttgcccc aagcgccagc 960

gccttctttg gaatgagccg catcggaatg gaagtgaccc caagtggaac ctggctgacc 1020

tacacaggcg ccatcaagct ggatgacaag gaccccaact tcaaggacca agtgatcctg 1080

ctgaacaagc acatcgacgc ctacaagacc tttccaccaa ctgagcccaa gaaggacaag 1140

aagaagaagg ccgacgagac acaggctctg ccacagcgcc aaaagaagca gcagaccgtt 1200

acactgctgc cagccgccga cctggatgat ttttccaaac agctgcagca gtccatgagc 1260

agcgccgata gcacacaggc ctgataacga gcgcgcctcg aggaattc 1308

<210>  22
<211>  419
<212>  PRT
<213>  Artificial sequence

<220>
<223>  N polypeptide of CoV

<400>  22

Met Ser Asp Asn Gly Pro Gln Asn Gln Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Ala Arg Ser Lys Gln Arg Arg Pro Gln Gly Leu Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Pro
65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Ser Arg Asn
            180                 185                 190

Ser Ser Arg Asn Ser Thr Pro Gly Ser Ser Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Gln Leu Glu Ser Lys Met Ser Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Leu Ile Arg Gln Gly Thr Asp Tyr Lys His Trp Pro Gln Ile
    290                 295                 300

Ala Gln Phe Ala Pro Ser Ala Ser Ala Phe Phe Gly Met Ser Arg Ile
305                 310                 315                 320

Gly Met Glu Val Thr Pro Ser Gly Thr Trp Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro
        355                 360                 365

Lys Lys Asp Lys Lys Lys Lys Ala Asp Glu Thr Gln Ala Leu Pro Gln
    370                 375                 380

Arg Gln Lys Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  23
<211>  75
<212>  PRT
<213>  Artificial sequence

<220>

<223>  E polypeptide of CoV

<400>  23

Met Tyr Ser Phe Val Ser Glu Glu Thr Gly Thr Leu Ile Val Asn Ser
1               5                   10                  15

Val Leu Leu Phe Leu Ala Phe Val Val Phe Leu Leu Val Thr Leu Ala
            20                  25                  30

Ile Leu Thr Ala Leu Arg Leu Cys Ala Tyr Cys Cys Asn Ile Val Asn
        35                  40                  45

Val Ser Leu Val Lys Pro Ser Phe Tyr Val Tyr Ser Arg Val Lys Asn
    50                  55                  60

Leu Asn Ser Ser Arg Val Pro Asp Leu Leu Val
65                  70                  75

<210>  24
<211>  222
<212>  PRT
<213>  Artificial sequence

<220>
<223>  M polypeptide of CoV

<400>  24

Met Ala Asp Ser Asn Gly Thr Ile Thr Val Glu Glu Leu Lys Lys Leu
1               5                   10                  15

Leu Glu Gln Trp Asn Leu Val Ile Gly Phe Leu Phe Leu Thr Trp Ile
            20                  25                  30

Cys Leu Leu Gln Phe Ala Tyr Ala Asn Arg Asn Arg Phe Leu Tyr Ile
        35                  40                  45

Ile Lys Leu Ile Phe Leu Trp Leu Leu Trp Pro Val Thr Leu Ala Cys
    50                  55                  60

Phe Val Leu Ala Ala Val Tyr Arg Ile Asn Trp Ile Thr Gly Gly Ile
65                  70                  75                  80

Ala Ile Ala Met Ala Cys Leu Val Gly Leu Met Trp Leu Ser Tyr Phe
                85                  90                  95

Ile Ala Ser Phe Arg Leu Phe Ala Arg Thr Arg Ser Met Trp Ser Phe
            100                 105                 110

Asn Pro Glu Thr Asn Ile Leu Leu Asn Val Pro Leu His Gly Thr Ile

115                 120                 125

Leu Thr Arg Pro Leu Leu Glu Ser Glu Leu Val Ile Gly Ala Val Ile
    130                 135                 140

Leu Arg Gly His Leu Arg Ile Ala Gly His His Leu Gly Arg Cys Asp
145                 150                 155                 160

Ile Lys Asp Leu Pro Lys Glu Ile Thr Val Ala Thr Ser Arg Thr Leu
                165                 170                 175

Ser Tyr Tyr Lys Leu Gly Ala Ser Gln Arg Val Ala Gly Asp Ser Gly
            180                 185                 190

Phe Ala Ala Tyr Ser Arg Tyr Arg Ile Gly Asn Tyr Lys Leu Asn Thr
        195                 200                 205

Asp His Ser Ser Ser Ser Asp Asn Ile Ala Leu Leu Val Gln
    210                 215                 220

<210>  25
<211>  121
<212>  PRT
<213>  Artificial sequence

<220>
<223>  ORF8 polypeptide of CoV

<400>  25

Met Lys Phe Leu Val Phe Leu Gly Ile Ile Thr Thr Val Ala Ala Phe
1               5                   10                  15

His Gln Glu Cys Ser Leu Gln Ser Cys Thr Gln His Gln Pro Tyr Val
            20                  25                  30

Val Asp Asp Pro Cys Pro Ile His Phe Tyr Ser Lys Trp Tyr Ile Arg
        35                  40                  45

Val Gly Ala Arg Lys Ser Ala Pro Leu Ile Glu Leu Cys Val Asp Glu
    50                  55                  60

Ala Gly Ser Lys Ser Pro Ile Gln Tyr Ile Asp Ile Gly Asn Tyr Thr
65                  70                  75                  80

Val Ser Cys Leu Pro Phe Thr Ile Asn Cys Gln Glu Pro Lys Leu Gly
                85                  90                  95

Ser Leu Val Val Arg Cys Ser Phe Tyr Glu Asp Phe Leu Glu Tyr His
            100                 105                 110

Asp Val Arg Val Val Leu Asp Phe Ile
        115                 120

<210>  26
<211>  275
<212>  PRT
<213>  Artificial sequence

<220>
<223>  ORF3a polypeptide of CoV

<400>  26

Met Asp Leu Phe Met Arg Ile Phe Thr Ile Gly Thr Val Thr Leu Lys
1               5                   10                  15

Gln Gly Glu Ile Lys Asp Ala Thr Pro Ser Asp Phe Val Arg Ala Thr
            20                  25                  30

Ala Thr Ile Pro Ile Gln Ala Ser Leu Pro Phe Gly Trp Leu Ile Val
        35                  40                  45

Gly Val Ala Leu Leu Ala Val Phe Gln Ser Ala Ser Lys Ile Ile Thr
    50                  55                  60

Leu Lys Lys Arg Trp Gln Leu Ala Leu Ser Lys Gly Val His Phe Val
65                  70                  75                  80

Cys Asn Leu Leu Leu Leu Phe Val Thr Val Tyr Ser His Leu Leu Leu
                85                  90                  95

Val Ala Ala Gly Leu Glu Ala Pro Phe Leu Tyr Leu Tyr Ala Leu Val
            100                 105                 110

Tyr Phe Leu Gln Ser Ile Asn Phe Val Arg Ile Ile Met Arg Leu Trp
        115                 120                 125

Leu Cys Trp Lys Cys Arg Ser Lys Asn Pro Leu Leu Tyr Asp Ala Asn
    130                 135                 140

Tyr Phe Leu Cys Trp His Thr Asn Cys Tyr Asp Tyr Cys Ile Pro Tyr
145                 150                 155                 160

Asn Ser Val Thr Ser Ser Ile Val Ile Thr Ser Gly Asp Gly Thr Thr
                165                 170                 175

Ser Pro Ile Ser Glu His Asp Tyr Gln Ile Gly Gly Tyr Thr Glu Lys
            180                 185                 190

Trp Glu Ser Gly Val Lys Asp Cys Val Val Leu His Ser Tyr Phe Thr
        195                 200                 205

Ser Asp Tyr Tyr Gln Leu Tyr Ser Thr Gln Leu Ser Thr Asp Thr Gly
    210                 215                 220

Val Glu His Val Thr Phe Phe Ile Tyr Asn Lys Ile Val Asp Glu Pro
225                 230                 235                 240

Glu Glu His Val Gln Ile His Thr Ile Asp Gly Ser Ser Gly Val Val
                245                 250                 255

Asn Pro Val Met Glu Pro Ile Tyr Asp Glu Pro Thr Thr Thr Thr Ser
            260                 265                 270

Val Pro Leu
        275

<210>  27
<211>  121
<212>  PRT
<213>  Artificial sequence

<220>
<223>  ORF7a polypeptide of CoV

<400>  27

Met Lys Ile Ile Leu Phe Leu Ala Leu Ile Thr Leu Ala Thr Cys Glu
1               5                   10                  15

Leu Tyr His Tyr Gln Glu Cys Val Arg Gly Thr Thr Val Leu Leu Lys
            20                  25                  30

Glu Pro Cys Ser Ser Gly Thr Tyr Glu Gly Asn Ser Pro Phe His Pro
        35                  40                  45

Leu Ala Asp Asn Lys Phe Ala Leu Thr Cys Phe Ser Thr Gln Phe Ala
    50                  55                  60

Phe Ala Cys Pro Asp Gly Val Lys His Val Tyr Gln Leu Arg Ala Arg
65                  70                  75                  80

Ser Val Ser Pro Lys Leu Phe Ile Arg Gln Glu Glu Val Gln Glu Leu
                85                  90                  95

Tyr Ser Pro Ile Phe Leu Ile Val Ala Ala Ile Val Phe Ile Thr Leu
            100                 105                 110

Cys Phe Thr Leu Lys Arg Lys Thr Glu
        115                 120

<210>  28
<211>  18967
<212>  DNA
<213>  Artificial sequence

<220>
<223>  pTM-MVSchw

<400>  28
gcggccgcta atacgactca ctatagggcc aactttgttt ggtctgatga gtccgtgagg      60

acgaaacccg gagtcccggg tcaccaaaca aagttgggta aggatagttc aatcaatgat     120

catcttctag tgcacttagg attcaagatc ctattatcag ggacaagagc aggattaggg     180

atatccgaga tggccacact tttaaggagc ttagcattgt tcaaaagaaa caaggacaaa     240

ccacccatta catcaggatc cggtggagcc atcagaggaa tcaaacacat tattatagta     300

ccaatccctg gagattcctc aattaccact cgatccagac ttctggaccg gttggtgagg     360

ttaattggaa acccggatgt gagcgggccc aaactaacag gggcactaat aggtatatta     420

tccttatttg tggagtctcc aggtcaattg attcagagga tcaccgatga ccctgacgtt     480

agcataaggc tgttagaggt tgtccagagt gaccagtcac aatctggcct taccttcgca     540

tcaagaggta ccaacatgga ggatgaggcg gaccaatact tttcacatga tgatccaatt     600

agtagtgatc aatccaggtt cggatggttc gggaacaagg aaatctcaga tattgaagtg     660

caagaccctg agggattcaa catgattctg ggtaccatcc tagcccaaat ttgggtcttg     720

ctcgcaaagg cggttacggc cccagacacg gcagctgatt cggagctaag aaggtggata     780

aagtacaccc aacaaagaag ggtagttggt gaatttagat tggagagaaa atggttggat     840

gtggtgagga acaggattgc cgaggacctc tccttacgcc gattcatggt cgctctaatc     900

ctggatatca agagaacacc cggaaacaaa cccaggattg ctgaaatgat atgtgacatt     960

gatacatata tcgtagaggc aggattagcc agttttatcc tgactattaa gtttgggata    1020

gaaactatgt atcctgctct tggactgcat gaatttgctg gtgagttatc cacacttgag    1080

tccttgatga acctttacca gcaaatgggg gaaactgcac cctacatggt aatcctggag    1140

aactcaattc agaacaagtt cagtgcagga tcataccctc tgctctggag ctatgccatg    1200

ggagtaggag tggaacttga aaactccatg ggaggtttga actttggccg atcttacttt    1260

gatccagcat attttagatt agggcaagag atggtaagga ggtcagctgg aaaggtcagt    1320

tccacattgg catctgaact cggtatcact gccgaggatg caaggcttgt ttcagagatt    1380

gcaatgcata ctactgagga caagatcagt agagcggttg gacccagaca agcccaagta    1440

tcatttctac acggtgatca aagtgagaat gagctaccga gattgggggg caaggaagat    1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca aggtccacaa tgacagagac ctacgacttc 3540

gacaagtcgg catgggacat caaagggtcg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgattc cctagggcct 3720

ccaatcgggc gagcatttgg gttcctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gacacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgagacaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagtt tacgggaccc catatctgcg gagatatcta tccaggcttt 6240

gagctatgcg cttggaggag acatcaataa ggtgttagaa aagctcggat acagtggagg 6300

tgatttactg ggcatcttag agagcggagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagacgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taatcaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttaatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt cactgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatcacatg atgtcaccca 9240

gacatcaggc atacccacta gtgtgaaata gacatcagaa ttaagaaaaa cgtagggtcc 9300

aagtggttcc ccgttatgga ctcgctatct gtcaaccaga tcttataccc tgaagttcac 9360

ctagatagcc cgatagttac caataagata gtagccatcc tggagtatgc tcgagtccct 9420

cacgcttaca gcctggagga ccctacactg tgtcagaaca tcaagcaccg cctaaaaaac 9480

ggattttcca accaaatgat tataaacaat gtggaagttg ggaatgtcat caagtccaag 9540

cttaggagtt atccggccca ctctcatatt ccatatccaa attgtaatca ggatttattt 9600

aacatagaag acaaagagtc aacgaggaag atccgtgaac tcctcaaaaa ggggaattcg 9660

ctgtactcca aagtcagtga taaggttttc caatgcttaa gggacactaa ctcacggctt 9720

ggcctaggct ccgaattgag ggaggacatc aaggagaaag ttattaactt gggagtttac 9780

atgcacagct cccagtggtt tgagcccttt ctgttttggt ttacagtcaa gactgagatg 9840

aggtcagtga ttaaatcaca aacccatact tgccatagga ggagacacac acctgtattc 9900

ttcactggta gttcagttga gttgctaatc tctcgtgacc ttgttgctat aatcagtaaa 9960

gagtctcaac atgtatatta cctgacattt gaactggttt tgatgtattg tgatgtcata 10020

gaggggaggt taatgacaga gaccgctatg actattgatg ctaggtatac agagcttcta 10080

ggaagagtca gatacatgtg gaaactgata gatggtttct tccctgcact cgggaatcca 10140

acttatcaaa ttgtagccat gctggagcct ctttcacttg cttacctgca gctgagggat 10200

ataacagtag aactcagagg tgctttcctt aaccactgct ttactgaaat acatgatgtt 10260

cttgaccaaa acgggttttc tgatgaaggt acttatcatg agttaactga agctctagat 10320

tacattttca taactgatga catacatctg acaggggaga ttttctcatt tttcagaagt 10380

ttcggccacc ccagacttga agcagtaacg gctgctgaaa atgttaggaa atacatgaat 10440

cagcctaaag tcattgtgta tgagactctg atgaaaggtc atgccatatt ttgtggaatc 10500

ataatcaacg gctatcgtga caggcacgga ggcagttggc caccgctgac cctccccctg 10560

catgctgcag acacaatccg gaatgctcaa gcttcaggtg aagggttaac acatgagcag 10620

tgcgttgata actggaaatc ttttgctgga gtgaaatttg gctgctttat gcctcttagc 10680

ctggatagtg atctgacaat gtacctaaag gacaaggcac ttgctgctct ccaaagggaa 10740

tgggattcag tttacccgaa agagttcctg cgttacgacc ctcccaaggg aaccgggtca 10800

cggaggcttg tagatgtttt ccttaatgat tcgagctttg acccatatga tgtgataatg 10860

tatgttgtaa gtggagctta cctccatgac cctgagttca acctgtctta cagcctgaaa 10920

gaaaaggaga tcaaggaaac aggtagactt tttgctaaaa tgacttacaa aatgagggca 10980

tgccaagtga ttgctgaaaa tctaatctca aacgggattg gcaaatattt taaggacaat 11040

gggatggcca aggatgagca cgatttgact aaggcactcc acactctagc tgtctcagga 11100

gtccccaaag atctcaaaga aagtcacagg ggggggccag tcttaaaaac ctactcccga 11160

agcccagtcc acacaagtac caggaacgtg agagcagcaa aagggtttat agggttccct 11220

caagtaattc ggcaggacca agacactgat catccggaga atatggaagc ttacgagaca 11280

gtcagtgcat ttatcacgac tgatctcaag aagtactgcc ttaattggag atatgagacc 11340

atcagcttgt ttgcacagag gctaaatgag atttacggat tgccctcatt tttccagtgg 11400

ctgcataaga ggcttgagac ctctgtcctg tatgtaagtg accctcattg cccccccgac 11460

cttgacgccc atatcccgtt atataaagtc cccaatgatc aaatcttcat taagtaccct 11520

atgggaggta tagaagggta ttgtcagaag ctgtggacca tcagcaccat tccctatcta 11580

tacctggctg cttatgagag cggagtaagg attgcttcgt tagtgcaagg ggacaatcag 11640

accatagccg taacaaaaag ggtacccagc acatggccct acaaccttaa gaaacgggaa 11700

gctgctagag taactagaga ttactttgta attcttaggc aaaggctaca tgatattggc 11760

catcacctca aggcaaatga gacaattgtt tcatcacatt tttttgtcta ttcaaaagga 11820

atatattatg atgggctact tgtgtcccaa tcactcaaga gcatcgcaag atgtgtattc 11880

tggtcagaga ctatagttga tgaaacaagg gcagcatgca gtaatattgc tacaacaatg 11940

gctaaaagca tcgagagagg ttatgaccgt taccttgcat attccctgaa cgtcctaaaa 12000

gtgatacagc aaattctgat ctctcttggc ttcacaatca attcaaccat gacccgggat 12060

gtagtcatac ccctcctcac aaacaacgac ctcttaataa ggatggcact gttgcccgct 12120

cctattgggg ggatgaatta tctgaatatg agcaggctgt ttgtcagaaa catcggtgat 12180

ccagtaacat catcaattgc tgatctcaag agaatgattc tcgcctcact aatgcctgaa 12240

gagaccctcc atcaagtaat gacacaacaa ccgggggact cttcattcct agactgggct 12300

agcgaccctt actcagcaaa tcttgtatgt gtccagagca tcactagact cctcaagaac 12360

ataactgcaa ggtttgtcct gatccatagt ccaaacccaa tgttaaaagg attattccat 12420

gatgacagta aagaagagga cgagggactg gcggcattcc tcatggacag gcatattata 12480

gtacctaggg cagctcatga aatcctggat catagtgtca caggggcaag agagtctatt 12540

gcaggcatgc tggataccac aaaaggcttg attcgagcca gcatgaggaa gggggggtta 12600

acctctcgag tgataaccag attgtccaat tatgactatg aacaattcag agcagggatg 12660

gtgctattga caggaagaaa gagaaatgtc ctcattgaca aagagtcatg ttcagtgcag 12720

ctggcgagag ctctaagaag ccatatgtgg gcgaggctag ctcgaggacg gcctatttac 12780

ggccttgagg tccctgatgt actagaatct atgcgaggcc accttattcg gcgtcatgag 12840

acatgtgtca tctgcgagtg tggatcagtc aactacggat ggttttttgt cccctcgggt 12900

tgccaactgg atgatattga caaggaaaca tcatccttga gagtcccata tattggttct 12960

accactgatg agagaacaga catgaagctt gccttcgtaa gagccccaag tcgatccttg 13020

cgatctgctg ttagaatagc aacagtgtac tcatgggctt acggtgatga tgatagctct 13080

tggaacgaag cctggttgtt ggctaggcaa agggccaatg tgagcctgga ggagctaagg 13140

gtgatcactc ccatctcaac ttcgactaat ttagcgcata ggttgaggga tcgtagcact 13200

caagtgaaat actcaggtac atcccttgtc cgagtggcga ggtataccac aatctccaac 13260

gacaatctct catttgtcat atcagataag aaggttgata ctaactttat ataccaacaa 13320

ggaatgcttc tagggttggg tgttttagaa acattgtttc gactcgagaa agataccgga 13380

tcatctaaca cggtattaca tcttcacgtc gaaacagatt gttgcgtgat cccgatgata 13440

gatcatccca ggatacccag ctcccgcaag ctagagctga gggcagagct atgtaccaac 13500

ccattgatat atgataatgc acctttaatt gacagagatg caacaaggct atacacccag 13560

agccatagga ggcaccttgt ggaatttgtt acatggtcca caccccaact atatcacatt 13620

ttagctaagt ccacagcact atctatgatt gacctggtaa caaaatttga gaaggaccat 13680

atgaatgaaa tttcagctct cataggggat gacgatatca atagtttcat aactgagttt 13740

ctgctcatag agccaagatt attcactatc tacttgggcc agtgtgcggc catcaattgg 13800

gcatttgatg tacattatca tagaccatca gggaaatatc agatgggtga gctgttgtca 13860

tcgttccttt ctagaatgag caaaggagtg tttaaggtgc ttgtcaatgc tctaagccac 13920

ccaaagatct acaagaaatt ctggcattgt ggtattatag agcctatcca tggtccttca 13980

cttgatgctc aaaacttgca cacaactgtg tgcaacatgg tttacacatg ctatatgacc 14040

tacctcgacc tgttgttgaa tgaagagtta gaagagttca catttctctt gtgtgaaagc 14100

gacgaggatg tagtaccgga cagattcgac aacatccagg caaaacactt atgtgttctg 14160

gcagatttgt actgtcaacc agggacctgc ccaccaattc gaggtctaag accggtagag 14220

aaatgtgcag ttctaaccga ccatatcaag gcagaggcta tgttatctcc agcaggatct 14280

tcgtggaaca taaatccaat tattgtagac cattactcat gctctctgac ttatctccgg 14340

cgaggatcga tcaaacagat aagattgaga gttgatccag gattcatttt cgacgccctc 14400

gctgaggtaa atgtcagtca gccaaagatc ggcagcaaca acatctcaaa tatgagcatc 14460

aaggctttca gacccccaca cgatgatgtt gcaaaattgc tcaaagatat caacacaagc 14520

aagcacaatc ttcccatttc agggggcaat ctcgccaatt atgaaatcca tgctttccgc 14580

agaatcgggt tgaactcatc tgcttgctac aaagctgttg agatatcaac attaattagg 14640

agatgccttg agccagggga ggacggcttg ttcttgggtg agggatcggg ttctatgttg 14700

atcacttata aagagatact taaactaaac aagtgcttct ataatagtgg ggtttccgcc 14760

aattctagat ctggtcaaag ggaattagca ccctatccct ccgaagttgg ccttgtcgaa 14820

cacagaatgg gagtaggtaa tattgtcaaa gtgctcttta acgggaggcc cgaagtcacg 14880

tgggtaggca gtgtagattg cttcaatttc atagttagta atatccctac ctctagtgtg 14940

gggtttatcc attcagatat agagaccttg cctgacaaag atactataga gaagctagag 15000

gaattggcag ccatcttatc gatggctctg ctcctgggca aaataggatc aatactggtg 15060

attaagctta tgcctttcag cggggatttt gttcagggat ttataagtta tgtagggtct 15120

cattatagag aagtgaacct tgtataccct agatacagca acttcatctc tactgaatct 15180

tatttggtta tgacagatct caaggctaac cggctaatga atcctgaaaa gattaagcag 15240

cagataattg aatcatctgt gaggacttca cctggactta taggtcacat cctatccatt 15300

aagcaactaa gctgcataca agcaattgtg ggagacgcag ttagtagagg tgatatcaat 15360

cctactctga aaaaacttac acctatagag caggtgctga tcaattgcgg gttggcaatt 15420

aacggaccta agctgtgcaa agaattgatc caccatgatg ttgcctcagg gcaagatgga 15480

ttgcttaatt ctatactcat cctctacagg gagttggcaa gattcaaaga caaccaaaga 15540

agtcaacaag ggatgttcca cgcttacccc gtattggtaa gtagcaggca acgagaactt 15600

atatctagga tcacccgcaa attctggggg cacattcttc tttactccgg gaacaaaaag 15660

ttgataaata agtttatcca gaatctcaag tccggctatc tgatactaga cttacaccag 15720

aatatcttcg ttaagaatct atccaagtca gagaaacaga ttattatgac gggggggttg 15780

aaacgtgagt gggtttttaa ggtaacagtc aaggagacca aagaatggta taagttagtc 15840

ggatacagtg ccctgattaa ggactaattg gttgaactcc ggaaccctaa tcctgcccta 15900

ggtggttagg cattatttgc aatatattaa agaaaacttt gaaaatacga agtttctatt 15960

cccagctttg tctggtggcc ggcatggtcc cagcctcctc gctggcgccg gctgggcaac 16020

attccgaggg gaccgtcccc tcggtaatgg cgaatgggac gcggccgatc cggctgctaa 16080

caaagcccga aaggaagctg agttggctgc tgccaccgct gagcaataac tagcataacc 16140

ccttggggcc tctaaacggg tcttgagggg ttttttgctg aaaggaggaa ctatatccgg 16200

atgcggccgc gggccctatg gtacccagct tttgttccct ttagtgaggg ttaattccga 16260

gcttggcgta atcatggtca tagctgtttc ctgtgtgaaa ttgttatccg ctcacaattc 16320

cacacaacat aggagccgga agcataaagt gtaaagcctg gggtgcctaa tgagtgaggt 16380

aactcacatt aattgcgttg cgctcactgc ccgctttcca gtcgggaaac ctgtcgtgcc 16440

agctgcatta atgaatcggc caacgcgcgg ggagaggcgg tttgcgtatt gggcgctctt 16500

ccgcttcctc gctcactgac tcgctgcgct cggtcgttcg gctgcggcga gcggtatcag 16560

ctcactcaaa ggcggtaata cggttatcca cagaatcagg ggataacgca ggaaagaaca 16620

tgtgagcaaa aggccagcaa aaggccagga accgtaaaaa ggccgcgttg ctggcgtttt 16680

tccataggct ccgcccccct gacgagcatc acaaaaatcg acgctcaagt cagaggtggc 16740

gaaacccgac aggactataa agataccagg cgtttccccc tggaagctcc ctcgtgcgct 16800

ctcctgttcc gaccctgccg cttaccggat acctgtccgc ctttctccct tcgggaagcg 16860

tggcgctttc tcaatgctca cgctgtaggt atctcagttc ggtgtaggtc gttcgctcca 16920

agctgggctg tgtgcacgaa ccccccgttc agcccgaccg ctgcgcctta tccggtaact 16980

atcgtcttga gtccaacccg gtaagacacg acttatcgcc actggcagca gccactggta 17040

acaggattag cagagcgagg tatgtaggcg gtgctacaga gttcttgaag tggtggccta 17100

actacggcta cactagaagg acagtatttg gtatctgcgc tctgctgaag ccagttacct 17160

tcggaaaaag agttggtagc tcttgatccg gcaaacaaac caccgctggt agcggtggtt 17220

tttttgtttg caagcagcag attacgcgca gaaaaaaagg atctcaagaa gatcctttga 17280

tcttttctac ggggtctgac gctcagtgga acgaaaactc acgttaaggg attttggtca 17340

tgagattatc aaaaaggatc ttcacctaga tccttttaaa ttaaaaatga agttttaaat 17400

caatctaaag tatatatgag taaacttggt ctgacagtta ccaatgctta atcagtgagg 17460

cacctatctc agcgatctgt ctatttcgtt catccatagt tgcctgactg cccgtcgtgt 17520

agataactac gatacgggag ggcttaccat ctggccccag tgctgcaatg ataccgcgag 17580

acccacgctc accggctcca gatttatcag caataaacca gccagccgga agggccgagc 17640

gcagaagtgg tcctgcaact ttatccgcct ccatccagtc tattaattgt tgccgggaag 17700

ctagagtaag tagttcgcca gttaatagtt tgcgcaacgt tgttgccatt gctacaggca 17760

tcgtggtgtc acgctcgtcg tttggtatgg cttcattcag ctccggttcc caacgatcaa 17820

ggcgagttac atgatccccc atgttgtgaa aaaaagcggt tagctccttc ggtcctccga 17880

tcgttgtcag aagtaagttg gccgcagtgt tatcactcat gcttatggca gcactgcata 17940

attctcttac tgtcatgcca tccgtaagat gcttttctgt gactggtgag tactcaacca 18000

agtcattctg agaatagtgt atgcggcgac cgagttgctc ttgcccggcg tcaatacggg 18060

ataataccgc gccacatagc agaactttaa aagtgctcat cattggaaaa cgttcttcgg 18120

ggcgaaaact ctcaaggatc ttaccgctgt tgagatccag ttcgatgtaa cccactcgtg 18180

cacccaactg atcttcagca tcttttactt tcaccagcgt ttctgggtga gcaaaaacag 18240

gaaggcaaaa tgccgcaaaa aagggaataa gggcgacacg gaaatgttga atactcatac 18300

tcttcctttt tcaatattat tgaagcattt atcagggtta ttgtctcatg agcggataca 18360

tatttgaatg tatttagaaa aataaacaaa taggggttcc gcgcacattt ccccgaaaag 18420

tgccacctga aattgtaaac gttaatattt tgttaaaatt cgcgttaaat ttttgttaaa 18480

tcagctcatt ttttaaccaa taggccgaaa tcggcaaaat cccttataaa tcaaaagaat 18540

agaccgagat agggttgagt gttgttccag tttggaacaa gagtccacta ttaaagaacg 18600

tggactccaa cgtcaaaggg cgaaaaaccg tctatcaggg cgatggccca ctacgtgaac 18660

catcacccta atcaagtttt ttggggtcga ggtgccgtaa agcactaaat cggaacccta 18720

aagggagccc ccgatttaga gcttgacggg gaaagccggc gaacgtggcg agaaaggaag 18780

ggaagaaagc gaaaggagcg ggcgctaggg cgctggcaag tgtagcggtc acgctgcgcg 18840

taaccaccac acccgccgcg cttaatgcgc cgctacaggg cgcgtcccat tcgccattca 18900

ggctgcgcaa ctgttgggaa gggcgatcgg tgcgggcctc ttcgctatta cgccagccac 18960

cgcggtg 18967

<210> 29
<211> 19795
<212> DNA
<213> Artificial sequence

<220>
<223> pTM2-MVSchw-gfp

<400> 29
gcggccgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac gatggtgagc 3540

aagggcgagg agctgttcac cggggtggtg cccatcctgg tcgagctgga cggcgacgta 3600

aacggccaca agttcagcgt gtccggcgag ggcgagggcg atgccaccta cggcaagctg 3660

accctgaagt tcatctgcac caccggcaag ctgcccgtgc cctggcccac cctcgtgacc 3720

accctgacct acggcgtgca gtgcttcagc cgctaccccg accacatgaa gcagcacgac 3780

ttcttcaagt ccgccatgcc cgaaggctac gtccaggagc gcaccatctt cttcaaggac 3840

gacggcaact acaagacccg cgccgaggtg aagttcgagg gcgacaccct ggtgaaccgc 3900

atcgagctga agggcatcga cttcaaggag gacggcaaca tcctggggca caagctggag 3960

tacaactaca acagccacaa cgtctatatc atggccgaca agcagaagaa cggcatcaag 4020

gtgaacttca agatccgcca caacatcgag gacggcagcg tgcagctcgc cgaccactac 4080

cagcagaaca cccccatcgg cgacggcccc gtgctgctgc ccgacaacca ctacctgagc 4140

acccagtccg ccctgagcaa agaccccaac gagaagcgcg atcacatggt cctgctggag 4200

ttcgtgaccg ccgccgggat cactctcggc atggacgagc tgtacaagta ggcgcgcagc 4260

gcttagacgt ctcgcgatcg atactagtac aacctaaatc cattataaaa aacttaggag 4320

caaagtgatt gcctcccaag gtccacaatg acagagacct acgacttcga caagtcggca 4380

tgggacatca aagggtcgat cgctccgata caacccacca cctacagtga tggcaggctg 4440

gtgccccagg tcagagtcat agatcctggt ctaggcgaca ggaaggatga atgctttatg 4500

tacatgtttc tgctgggggt tgttgaggac agcgattccc tagggcctcc aatcgggcga 4560

gcatttgggt tcctgccctt aggtgttggc agatccacag caaagcccga aaaactcctc 4620

aaagaggcca ctgagcttga catagttgtt agacgtacag cagggctcaa tgaaaaactg 4680

gtgttctaca acaacacccc actaactctc ctcacacctt ggagaaaggt cctaacaaca 4740

gggagtgtct tcaacgcaaa ccaagtgtgc aatgcggtta atctgatacc gctcgatacc 4800

ccgcagaggt tccgtgttgt ttatatgagc atcacccgtc tttcggataa cgggtattac 4860

accgttccta gaagaatgct ggaattcaga tcggtcaatg cagtggcctt caacctgctg 4920

gtgaccctta ggattgacaa ggcgataggc cctgggaaga tcatcgacaa tacagagcaa 4980

cttcctgagg caacatttat ggtccacatc gggaacttca ggagaaagaa gagtgaagtc 5040

tactctgccg attattgcaa aatgaaaatc gaaaagatgg gcctggtttt tgcacttggt 5100

gggatagggg gcaccagtct tcacattaga agcacaggca aaatgagcaa gactctccat 5160

gcacaactcg ggttcaagaa gaccttatgt tacccgctga tggatatcaa tgaagacctt 5220

aatcgattac tctggaggag cagatgcaag atagtaagaa tccaggcagt tttgcagcca 5280

tcagttcctc aagaattccg catttacgac gacgtgatca taaatgatga ccaaggacta 5340

ttcaaagttc tgtagaccgt agtgcccagc aatgcccgaa aacgaccccc ctcacaatga 5400

cagccagaag gcccggacaa aaaagccccc tccgaaagac tccacggacc aagcgagagg 5460

ccagccagca gccgacggca agcgcgaaca ccaggcggcc ccagcacaga acagccctga 5520

cacaaggcca ccaccagcca ccccaatctg catcctcctc gtgggacccc cgaggaccaa 5580

cccccaaggc tgcccccgat ccaaaccacc aaccgcatcc ccaccacccc cgggaaagaa 5640

acccccagca attggaaggc ccctccccct cttcctcaac acaagaactc cacaaccgaa 5700

ccgcacaagc gaccgaggtg acccaaccgc aggcatccga ctccctagac agatcctctc 5760

tccccggcaa actaaacaaa acttagggcc aaggaacata cacacccaac agaacccaga 5820

ccccggccca cggcgccgcg cccccaaccc ccgacaacca gagggagccc ccaaccaatc 5880

ccgccggctc ccccggtgcc cacaggcagg gacaccaacc cccgaacaga cccagcaccc 5940

aaccatcgac aatccaagac gggggggccc ccccaaaaaa aggcccccag gggccgacag 6000

ccagcaccgc gaggaagccc acccacccca cacacgacca cggcaaccaa accagaaccc 6060

agaccaccct gggccaccag ctcccagact cggccatcac cccgcagaaa ggaaaggcca 6120

caacccgcgc accccagccc cgatccggcg gggagccacc caacccgaac cagcacccaa 6180

gagcgatccc cgaaggaccc ccgaaccgca aaggacatca gtatcccaca gcctctccaa 6240

gtcccccggt ctcctcctct tctcgaaggg accaaaagat caatccacca cacccgacga 6300

cactcaactc cccaccccta aaggagacac cgggaatccc agaatcaaga ctcatccaat 6360

gtccatcatg ggtctcaagg tgaacgtctc tgccatattc atggcagtac tgttaactct 6420

ccaaacaccc accggtcaaa tccattgggg caatctctct aagatagggg tggtaggaat 6480

aggaagtgca agctacaaag ttatgactcg ttccagccat caatcattag tcataaaatt 6540

aatgcccaat ataactctcc tcaataactg cacgaggtta gagattgcag aatacaggag 6600

actactgaga acagttttgg aaccaattag agatgcactt aatgcaatga cccagaatat 6660

aagaccggtt cagagtgtag cttcaagtag gagacacaag agatttgcgg gagtagtcct 6720

ggcaggtgcg gccctaggcg ttgccacagc tgctcagata acagccggca ttgcacttca 6780

ccagtccatg ctgaactctc aagccatcga caatctgaga gcgagcctgg aaactactaa 6840

tcaggcaatt gagacaatca gacaagcagg gcaggagatg atattggctg ttcagggtgt 6900

ccaagactac atcaataatg agctgatacc gtctatgaac caactatctt gtgatttaat 6960

cggccagaag ctcgggctca aattgctcag atactataca gaaatcctgt cattatttgg 7020

ccccagttta cgggacccca tatctgcgga gatatctatc caggctttga gctatgcgct 7080

tggaggagac atcaataagg tgttagaaaa gctcggatac agtggaggtg atttactggg 7140

catcttagag agcggaggaa taaaggcccg gataactcac gtcgacacag agtcctactt 7200

cattgtcctc agtatagcct atccgacgct gtccgagatt aagggggtga ttgtccaccg 7260

gctagagggg gtctcgtaca acataggctc tcaagagtgg tataccactg tgcccaagta 7320

tgttgcaacc caagggtacc ttatctcgaa ttttgatgag tcatcgtgta ctttcatgcc 7380

agaggggact gtgtgcagcc aaaatgcctt gtacccgatg agtcctctgc tccaagaatg 7440

cctccggggg tacaccaagt cctgtgctcg tacactcgta tccgggtctt ttgggaaccg 7500

gttcatttta tcacaaggga acctaatagc caattgtgca tcaatccttt gcaagtgtta 7560

cacaacagga acgatcatta atcaagaccc tgacaagatc ctaacataca ttgctgccga 7620

tcactgcccg gtagtcgagg tgaacggcgt gaccatccaa gtcgggagca ggaggtatcc 7680

agacgctgtg tacttgcaca gaattgacct cggtcctccc atatcattgg agaggttgga 7740

cgtagggaca aatctgggga atgcaattgc taagttggag gatgccaagg aattgttgga 7800

gtcatcggac cagatattga ggagtatgaa aggtttatcg agcactagca tagtctacat 7860

cctgattgca gtgtgtcttg gagggttgat agggatcccc gctttaatat gttgctgcag 7920

ggggcgttgt aacaaaaagg gagaacaagt tggtatgtca agaccaggcc taaagcctga 7980

tcttacggga acatcaaaat cctatgtaag gtcgctctga tcctctacaa ctcttgaaac 8040

acaaatgtcc cacaagtctc ctcttcgtca tcaagcaacc accgcaccca gcatcaagcc 8100

cacctgaaat tatctccggc ttccctctgg ccgaacaata tcggtagtta atcaaaactt 8160

agggtgcaag atcatccaca atgtcaccac aacgagaccg gataaatgcc ttctacaaag 8220

ataaccccca tcccaaggga agtaggatag tcattaacag agaacatctt atgattgata 8280

gaccttatgt tttgctggct gttctgtttg tcatgtttct gagcttgatc gggttgctag 8340

ccattgcagg cattagactt catcgggcag ccatctacac cgcagagatc cataaaagcc 8400

tcagcaccaa tctagatgta actaactcaa tcgagcatca ggtcaaggac gtgctgacac 8460

cactcttcaa aatcatcggt gatgaagtgg gcctgaggac acctcagaga ttcactgacc 8520

tagtgaaatt aatctctgac aagattaaat tccttaatcc ggatagggag tacgacttca 8580

gagatctcac ttggtgtatc aacccgccag agagaatcaa attggattat gatcaatact 8640

gtgcagatgt ggctgctgaa gagctcatga atgcattggt gaactcaact ctactggaga 8700

ccagaacaac caatcagttc ctagctgtct caaagggaaa ctgctcaggg cccactacaa 8760

tcagaggtca attctcaaac atgtcgctgt ccctgttaga cttgtattta ggtcgaggtt 8820

acaatgtgtc atctatagtc actatgacat cccagggaat gtatggggga acttacctag 8880

tggaaaagcc taatctgagc agcaaaaggt cagagttgtc acaactgagc atgtaccgag 8940

tgtttgaagt aggtgttatc agaaatccgg gtttgggggc tccggtgttc catatgacaa 9000

actatcttga gcaaccagtc agtaatgatc tcagcaactg tatggtggct ttgggggagc 9060

tcaaactcgc agccctttgt cacggggaag attctatcac aattccctat cagggatcag 9120

ggaaaggtgt cagcttccag ctcgtcaagc taggtgtctg gaaatcccca accgacatgc 9180

aatcctgggt ccccttatca acggatgatc cagtgataga caggctttac ctctcatctc 9240

acagaggtgt tatcgctgac aatcaagcaa aatgggctgt cccgacaaca cgaacagatg 9300

acaagttgcg aatggagaca tgcttccaac aggcgtgtaa gggtaaaatc caagcactct 9360

gcgagaatcc cgagtgggca ccattgaagg ataacaggat tccttcatac ggggtcttgt 9420

ctgttgatct gagtctgaca gttgagctta aaatcaaaat tgcttcggga ttcgggccat 9480

tgatcacaca cggttcaggg atggacctat acaaatccaa ccacaacaat gtgtattggc 9540

tgactatccc gccaatgaag aacctagcct taggtgtaat caacacattg gagtggatac 9600

cgagattcaa ggttagtccc tacctcttca ctgtcccaat taaggaagca ggcgaagact 9660

gccatgcccc aacataccta cctgcggagg tggatggtga tgtcaaactc agttccaatc 9720

tggtgattct acctggtcaa gatctccaat atgttttggc aacctacgat acttccaggg 9780

ttgaacatgc tgtggtttat tacgtttaca gcccaagccg ctcattttct tacttttatc 9840

cttttaggtt gcctataaag ggggtcccca tcgaattaca agtggaatgc ttcacatggg 9900

accaaaaact ctggtgccgt cacttctgtg tgcttgcgga ctcagaatct ggtggacata 9960

tcactcactc tgggatggtg ggcatgggag tcagctgcac agtcacccgg gaagatggaa 10020

ccaatcgcag atagggctgc tagtgaacca atcacatgat gtcacccaga catcaggcat 10080

acccactagt gtgaaataga catcagaatt aagaaaaacg tagggtccaa gtggttcccc 10140

gttatggact cgctatctgt caaccagatc ttataccctg aagttcacct agatagcccg 10200

atagttacca ataagatagt agccatcctg gagtatgctc gagtccctca cgcttacagc 10260

ctggaggacc ctacactgtg tcagaacatc aagcaccgcc taaaaaacgg attttccaac 10320

caaatgatta taaacaatgt ggaagttggg aatgtcatca agtccaagct taggagttat 10380

ccggcccact ctcatattcc atatccaaat tgtaatcagg atttatttaa catagaagac 10440

aaagagtcaa cgaggaagat ccgtgaactc ctcaaaaagg ggaattcgct gtactccaaa 10500

gtcagtgata aggttttcca atgcttaagg gacactaact cacggcttgg cctaggctcc 10560

gaattgaggg aggacatcaa ggagaaagtt attaacttgg gagtttacat gcacagctcc 10620

cagtggtttg agccctttct gttttggttt acagtcaaga ctgagatgag gtcagtgatt 10680

aaatcacaaa cccatacttg ccataggagg agacacacac ctgtattctt cactggtagt 10740

tcagttgagt tgctaatctc tcgtgacctt gttgctataa tcagtaaaga gtctcaacat 10800

gtatattacc tgacatttga actggttttg atgtattgtg atgtcataga ggggaggtta 10860

atgacagaga ccgctatgac tattgatgct aggtatacag agcttctagg aagagtcaga 10920

tacatgtgga aactgataga tggtttcttc cctgcactcg ggaatccaac ttatcaaatt 10980

gtagccatgc tggagcctct ttcacttgct tacctgcagc tgagggatat aacagtagaa 11040

ctcagaggtg ctttccttaa ccactgcttt actgaaatac atgatgttct tgaccaaaac 11100

gggttttctg atgaaggtac ttatcatgag ttaactgaag ctctagatta cattttcata 11160

actgatgaca tacatctgac aggggagatt ttctcatttt tcagaagttt cggccacccc 11220

agacttgaag cagtaacggc tgctgaaaat gttaggaaat acatgaatca gcctaaagtc 11280

attgtgtatg agactctgat gaaaggtcat gccatatttt gtggaatcat aatcaacggc 11340

tatcgtgaca ggcacggagg cagttggcca ccgctgaccc tccccctgca tgctgcagac 11400

acaatccgga atgctcaagc ttcaggtgaa gggttaacac atgagcagtg cgttgataac 11460

tggaaatctt ttgctggagt gaaatttggc tgctttatgc ctcttagcct ggatagtgat 11520

ctgacaatgt acctaaagga caaggcactt gctgctctcc aaagggaatg ggattcagtt 11580

tacccgaaag agttcctgcg ttacgaccct cccaagggaa ccgggtcacg gaggcttgta 11640

gatgttttcc ttaatgattc gagctttgac ccatatgatg tgataatgta tgttgtaagt 11700

ggagcttacc tccatgaccc tgagttcaac ctgtcttaca gcctgaaaga aaaggagatc 11760

aaggaaacag gtagactttt tgctaaaatg acttacaaaa tgagggcatg ccaagtgatt 11820

gctgaaaatc taatctcaaa cgggattggc aaatatttta aggacaatgg gatggccaag 11880

gatgagcacg atttgactaa ggcactccac actctagctg tctcaggagt ccccaaagat 11940

ctcaaagaaa gtcacagggg ggggccagtc ttaaaaacct actcccgaag cccagtccac 12000

acaagtacca ggaacgtgag agcagcaaaa gggtttatag ggttccctca agtaattcgg 12060

caggaccaag acactgatca tccggagaat atggaagctt acgagacagt cagtgcattt 12120

atcacgactg atctcaagaa gtactgcctt aattggagat atgagaccat cagcttgttt 12180

gcacagaggc taaatgagat ttacggattg ccctcatttt tccagtggct gcataagagg 12240

cttgagacct ctgtcctgta tgtaagtgac cctcattgcc cccccgacct tgacgcccat 12300

atcccgttat ataaagtccc caatgatcaa atcttcatta agtaccctat gggaggtata 12360

gaagggtatt gtcagaagct gtggaccatc agcaccattc cctatctata cctggctgct 12420

tatgagagcg gagtaaggat tgcttcgtta gtgcaagggg acaatcagac catagccgta 12480

acaaaaaggg tacccagcac atggccctac aaccttaaga aacgggaagc tgctagagta 12540

actagagatt actttgtaat tcttaggcaa aggctacatg atattggcca tcacctcaag 12600

gcaaatgaga caattgtttc atcacatttt tttgtctatt caaaaggaat atattatgat 12660

gggctacttg tgtcccaatc actcaagagc atcgcaagat gtgtattctg gtcagagact 12720

atagttgatg aaacaagggc agcatgcagt aatattgcta caacaatggc taaaagcatc 12780

gagagaggtt atgaccgtta ccttgcatat tccctgaacg tcctaaaagt gatacagcaa 12840

attctgatct ctcttggctt cacaatcaat tcaaccatga cccgggatgt agtcataccc 12900

ctcctcacaa acaacgacct cttaataagg atggcactgt tgcccgctcc tattgggggg 12960

atgaattatc tgaatatgag caggctgttt gtcagaaaca tcggtgatcc agtaacatca 13020

tcaattgctg atctcaagag aatgattctc gcctcactaa tgcctgaaga gaccctccat 13080

caagtaatga cacaacaacc ggggactctt tcattcctag actgggctag cgacccttac 13140

tcagcaaatc ttgtatgtgt ccagagcatc actagactcc tcaagaacat aactgcaagg 13200

tttgtcctga tccatagtcc aaacccaatg ttaaaaggat tattccatga tgacagtaaa 13260

gaagaggacg agggactggc ggcattcctc atggacaggc atattatagt acctagggca 13320

gctcatgaaa tcctggatca tagtgtcaca ggggcaagag agtctattgc aggcatgctg 13380

gataccacaa aaggcttgat tcgagccagc atgaggaagg gggggttaac ctctcgagtg 13440

ataaccagat tgtccaatta tgactatgaa caattcagag cagggatggt gctattgaca 13500

ggaagaaaga gaaatgtcct cattgacaaa gagtcatgtt cagtgcagct ggcgagagct 13560

ctaagaagcc atatgtgggc gaggctagct cgaggacggc ctatttacgg ccttgaggtc 13620

cctgatgtac tagaatctat gcgaggccac cttattcggc gtcatgagac atgtgtcatc 13680

tgcgagtgtg gatcagtcaa ctacggatgg ttttttgtcc cctcgggttg ccaactggat 13740

gatattgaca aggaaacatc atccttgaga gtcccatata ttggttctac cactgatgag 13800

agaacagaca tgaagcttgc cttcgtaaga gccccaagtc gatccttgcg atctgctgtt 13860

agaatagcaa cagtgtactc atgggcttac ggtgatgatg atagctcttg gaacgaagcc 13920

tggttgttgg ctaggcaaag ggccaatgtg agcctggagg agctaagggt gatcactccc 13980

atctcaactt cgactaattt agcgcatagg ttgagggatc gtagcactca agtgaaatac 14040

tcaggtacat cccttgtccg agtggcgagg tataccacaa tctccaacga caatctctca 14100

tttgtcatat cagataagaa ggttgatact aactttatat accaacaagg aatgcttcta 14160

gggttgggtg ttttagaaac attgtttcga ctcgagaaag ataccggatc atctaacacg 14220

gtattacatc ttcacgtcga aacagattgt tgcgtgatcc cgatgataga tcatcccagg 14280

atacccagct cccgcaagct agagctgagg gcagagctat gtaccaaccc attgatatat 14340

gataatgcac ctttaattga cagagatgca acaaggctat acacccagag ccataggagg 14400

caccttgtgg aatttgttac atggtccaca ccccaactat atcacatttt agctaagtcc 14460

acagcactat ctatgattga cctggtaaca aaatttgaga aggaccatat gaatgaaatt 14520

tcagctctca taggggatga cgatatcaat agtttcataa ctgagtttct gctcatagag 14580

ccaagattat tcactatcta cttgggccag tgtgcggcca tcaattgggc atttgatgta 14640

cattatcata gaccatcagg gaaatatcag atgggtgagc tgttgtcatc gttcctttct 14700

agaatgagca aaggagtgtt taaggtgctt gtcaatgctc taagccaccc aaagatctac 14760

aagaaattct ggcattgtgg tattatagag cctatccatg gtccttcact tgatgctcaa 14820

aacttgcaca caactgtgtg caacatggtt tacacatgct atatgaccta cctcgacctg 14880

ttgttgaatg aagagttaga agagttcaca tttctcttgt gtgaaagcga cgaggatgta 14940

gtaccggaca gattcgacaa catccaggca aaacacttat gtgttctggc agatttgtac 15000

tgtcaaccag ggacctgccc accaattcga ggtctaagac cggtagagaa atgtgcagtt 15060

ctaaccgacc atatcaaggc agaggctatg ttatctccag caggatcttc gtggaacata 15120

aatccaatta ttgtagacca ttactcatgc tctctgactt atctccggcg aggatcgatc 15180

aaacagataa gattgagagt tgatccagga ttcattttcg acgccctcgc tgaggtaaat 15240

gtcagtcagc caaagatcgg cagcaacaac atctcaaata tgagcatcaa ggctttcaga 15300

cccccacacg atgatgttgc aaaattgctc aaagatatca acacaagcaa gcacaatctt 15360

cccatttcag ggggcaatct cgccaattat gaaatccatg ctttccgcag aatcgggttg 15420

aactcatctg cttgctacaa agctgttgag atatcaacat taattaggag atgccttgag 15480

ccaggggagg acggcttgtt cttgggtgag ggatcgggtt ctatgttgat cacttataaa 15540

gagatactta aactaaacaa gtgcttctat aatagtgggg tttccgccaa ttctagatct 15600

ggtcaaaggg aattagcacc ctatccctcc gaagttggcc ttgtcgaaca cagaatggga 15660

gtaggtaata ttgtcaaagt gctctttaac gggaggcccg aagtcacgtg ggtaggcagt 15720

gtagattgct tcaatttcat agttagtaat atccctacct ctagtgtggg gtttatccat 15780

tcagatatag agaccttgcc tgacaaagat actatagaga agctagagga attggcagcc 15840

atcttatcga tggctctgct cctgggcaaa ataggatcaa tactggtgat taagcttatg 15900

cctttcagcg gggattttgt tcagggattt ataagttatg tagggtctca ttatagagaa 15960

gtgaaccttg tataccctag atacagcaac ttcatctcta ctgaatctta tttggttatg 16020

acagatctca aggctaaccg gctaatgaat cctgaaaaga ttaagcagca gataattgaa 16080

tcatctgtga ggacttcacc tggacttata ggtcacatcc tatccattaa gcaactaagc 16140

tgcatacaag caattgtggg agacgcagtt agtagaggtg atatcaatcc tactctgaaa 16200

aaacttacac ctatagagca ggtgctgatc aattgcgggt tggcaattaa cggacctaag 16260

ctgtgcaaag aattgatcca ccatgatgtt gcctcagggc aagatggatt gcttaattct 16320

atactcatcc tctacaggga gttggcaaga ttcaaagaca accaaagaag tcaacaaggg 16380

atgttccacg cttaccccgt attggtaagt agcaggcaac gagaacttat atctaggatc 16440

acccgcaaat tctgggggca cattcttctt tactccggga acaaaaagtt gataaataag 16500

tttatccaga atctcaagtc cggctatctg atactagact tacaccagaa tatcttcgtt 16560

aagaatctat ccaagtcaga gaaacagatt attatgacgg ggggtttgaa acgtgagtgg 16620

gtttttaagg taacagtcaa ggagaccaaa gaatggtata agttagtcgg atacagtgcc 16680

ctgattaagg actaattggt tgaactccgg aaccctaatc ctgccctagg tggttaggca 16740

ttatttgcaa tatattaaag aaaactttga aaatacgaag tttctattcc cagctttgtc 16800

tggtggccgg catggtccca gcctcctcgc tggcgccggc tgggcaacat tccgagggga 16860

ccgtcccctc ggtaatggcg aatgggacgc ggccgatccg gctgctaaca aagcccgaaa 16920

ggaagctgag ttggctgctg ccaccgctga gcaataacta gcataacccc ttggggcctc 16980

taaacgggtc ttgaggggtt ttttgctgaa aggaggaact atatccggat gcggccgcgg 17040

gccctatggt acccagcttt tgttcccttt agtgagggtt aattccgagc ttggcgtaat 17100

catggtcata gctgtttcct gtgtgaaatt gttatccgct cacaattcca cacaacatag 17160

gagccggaag cataaagtgt aaagcctggg gtgcctaatg agtgaggtaa ctcacattaa 17220

ttgcgttgcg ctcactgccc gctttccagt cgggaaacct gtcgtgccag ctgcattaat 17280

gaatcggcca acgcgcgggg agaggcggtt tgcgtattgg gcgctcttcc gcttcctcgc 17340

tcactgactc gctgcgctcg gtcgttcggc tgcggcgagc ggtatcagct cactcaaagg 17400

cggtaatacg gttatccaca gaatcagggg ataacgcagg aaagaacatg tgagcaaaag 17460

gccagcaaaa ggccaggaac cgtaaaaagg ccgcgttgct ggcgtttttc cataggctcg 17520

gcccccctga cgagcatcac aaaaatcgac gctcaagtca gaggtggcga aacccgacag 17580

gactataaag ataccaggcg tttccccctg gaagctccct cgtgcgctct cctgttccga 17640

ccctgccgct taccggatac ctgtccgcct ttctcccttc gggaagcgtg gcgctttctc 17700

aatgctcacg ctgtaggtat ctcagttcgg tgtaggtcgt tcgctccaag ctgggctgtg 17760

tgcacgaacc ccccgttcag cccgaccgct gcgccttatc cggtaactat cgtcttgagt 17820

ccaacccggt aagacacgac ttatcgccac tggcagcagc cactggtaac aggattagca 17880

gagcgaggta tgtaggcggt gctacagagt tcttgaagtg gtggcctaac tacggctaca 17940

ctagaaggac agtatttggt atctgcgctc tgctgaagcc agttaccttc ggaaaaagag 18000

ttggtagctc ttgatccggc aaacaaacca ccgctggtag cggtggtttt tttgtttgca 18060

agcagcagat tacgcgcaga aaaaaaggat ctcaagaaga tcctttgatc ttttctacgg 18120

ggtctgacgc tcagtggaac gaaaactcac gttaagggat tttggtcatg agattatcaa 18180

aaaggatctt cacctagatc cttttaaatt aaaaatgaag ttttaaatca atctaaagta 18240

tatatgagta aacttggtct gacagttacc aatgcttaat cagtgaggca cctatctcag 18300

cgatctgtct atttcgttca tccatagttg cctgactgcc cgtcgtgtag ataactacga 18360

tacgggaggg cttaccatct ggccccagtg ctgcaatgat accgcgagac ccacgctcac 18420

cggctccaga tttatcagca ataaaccagc cagccggaag ggccgagcgc agaagtggtc 18480

ctgcaacttt atccgcctcc atccagtcta ttaattgttg ccgggaagct agagtaagta 18540

gttcgccagt taatagtttg cgcaacgttg ttgccattgc tacaggcatc gtggtgtcac 18600

gctcgtcgtt tggtatggct tcattcagct ccggttccca acgatcaagg cgagttacat 18660

gatcccccat gttgtgaaaa aaagcggtta gctccttcgg tcctccgatc gttgtcagaa 18720

gtaagttggc cgcagtgtta tcactcatgc ttatggcagc actgcataat tctcttactg 18780

tcatgccatc cgtaagatgc ttttctgtga ctggtgagta ctcaaccaag tcattctgag 18840

aatagtgtat gcggcgaccg agttgctctt gcccggcgtc aatacgggat aataccgcgc 18900

cacatagcag aactttaaaa gtgctcatca ttggaaaacg ttcttcgggg cgaaaactct 18960

caaggatctt accgctgttg agatccagtt cgatgtaacc cactcgtgca cccaactgat 19020

cttcagcatc ttttactttc accagcgttt ctgggtgagc aaaaacagga aggcaaaatg 19080

ccgcaaaaaa gggaataagg gcgacacgga aatgttgaat actcatactc ttcctttttc 19140

aatattattg aagcatttat cagggttatt gtctcatgag cggatacata tttgaatgta 19200

tttagaaaaa taaacaaata ggggttccgc gcacatttcc ccgaaaagtg ccacctgaaa 19260

ttgtaaacgt taatattttg ttaaaattcg cgttaaattt ttgttaaatc agctcatttt 19320

ttaaccaata ggccgaaatc ggcaaaatcc cttataaatc aaaagaatag accgagatag 19380

ggttgagtgt tgttccagtt tggaacaaga gtccactatt aaagaacgtg gactccaacg 19440

tcaaagggcg aaaaaccgtc tatcagggcg atggcccact acgtgaacca tcaccctaat 19500

caagtttttt ggggtcgagg tgccgtaaag cactaaatcg gaaccctaaa gggagccccc 19560

gatttagagc ttgacgggga aagccggcga acgtggcgag aaaggaaggg aagaaagcga 19620

aaggagcggg cgctagggcg ctggcaagtg tagcggtcac gctgcgcgta accaccacac 19680

ccgccgcgct taatgcgccg ctacagggcg cgtcccattc gccattcagg ctgcgcaact 19740

gttgggaagg gcgatcggtg cgggcctctt cgctattacg ccagccaccg cggtg 19795

<210> 30
<211> 17858
<212> DNA
<213> Artificial sequence

<220>
<223>  pKP-MVSchw

<400>  30
gcgatcgcta atacgactca ctatagggcc aactttgttt ggtctgatga gtccgtgagg      60

acgaaacccg gagtcccggg tcaccaaaca aagttgggta aggatagttc aatcaatgat     120

catcttctag tgcacttagg attcaagatc ctattatcag ggacaagagc aggattaggg     180

atatccgaga tggccacact tttaaggagc ttagcattgt tcaaaagaaa caaggacaaa     240

ccacccatta catcaggatc cggtggagcc atcagaggaa tcaaacacat tattatagta     300

ccaatccctg gagattcctc aattaccact cgatccagac ttctggaccg gttggtgagg     360

ttaattggaa acccggatgt gagcgggccc aaactaacag gggcactaat aggtatatta     420

tccttatttg tggagtctcc aggtcaattg attcagagga tcaccgatga ccctgacgtt     480

agcataaggc tgttagaggt tgtccagagt gaccagtcac aatctggcct taccttcgca     540

tcaagaggta ccaacatgga ggatgaggcg gaccaatact tttcacatga tgatccaatt     600

agtagtgatc aatccaggtt cggatggttc gggaacaagg aaatctcaga tattgaagtg     660

caagaccctg agggattcaa catgattctg ggtaccatcc tagcccaaat ttgggtcttg     720

ctcgcaaagg cggttacggc cccagacacg gcagctgatt cggagctaag aaggtggata     780

aagtacaccc aacaaagaag ggtagttggt gaatttagat tggagagaaa atggttggat     840

gtggtgagga acaggattgc cgaggacctc tccttacgcc gattcatggt cgctctaatc     900

ctggatatca agagaacacc cggaaacaaa cccaggattg ctgaaatgat atgtgacatt     960

gatacatata tcgtagaggc aggattagcc agttttatcc tgactattaa gtttgggata    1020

gaaactatgt atcctgctct tggactgcat gaatttgctg gtgagttatc cacacttgag    1080

tccttgatga acctttacca gcaaatgggg gaaactgcac cctacatggt aatcctggag    1140

aactcaattc agaacaagtt cagtgcagga tcataccctc tgctctggag ctatgccatg    1200

ggagtaggag tggaacttga aaactccatg ggaggtttga actttggccg atcttacttt    1260

gatccagcat attttagatt agggcaagag atggtaagga ggtcagctgg aaaggtcagt    1320

tccacattgg catctgaact cggtatcact gccgaggatg caaggcttgt ttcagagatt    1380

gcaatgcata ctactgagga caagatcagt agagcggttg gacccagaca agcccaagta    1440

tcatttctac acggtgatca aagtgagaat gagctaccga gattgggggg caaggaagat    1500

aggagggtca aacagagtcg aggagaagcc agggagagct acagagaaac cgggcccagc    1560

agagcaagtg atgcgagagc tgcccatctt ccaaccggca cacccctaga cattgacact    1620

gcaacggagt ccagccaaga tccgcaggac agtcgaaggt cagctgacgc cctgcttagg    1680

ctgcaagcca tggcaggaat ctcggaagaa caaggctcag acacggacac ccctatagtg    1740

tacaatgaca gaaatcttct agactaggtg cgagaggccg agggccagaa caacatccgc    1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca aggtccacaa tgacagagac ctacgacttc 3540

gacaagtcgg catgggacat caaagggtcg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgattc cctagggcct 3720

ccaatcgggc gagcatttgg gttcctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gacacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgagacaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagtt tacgggaccc catatctgcg gagatatcta tccaggcttt 6240

gagctatgcg cttggaggag acatcaataa ggtgttagaa aagctcggat acagtggagg 6300

tgatttactg ggcatcttag agagcggagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagacgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taatcaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttaatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt cactgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatcacatg atgtcaccca 9240

gacatcaggc atacccacta gtgtgaaata gacatcagaa ttaagaaaaa cgtagggtcc 9300

aagtggttcc ccgttatgga ctcgctatct gtcaaccaga tcttataccc tgaagttcac 9360

ctagatagcc cgatagttac caataagata gtagccatcc tggagtatgc tcgagtccct 9420

cacgcttaca gcctggagga ccctacactg tgtcagaaca tcaagcaccg cctaaaaaac 9480

ggattttcca accaaatgat tataaacaat gtggaagttg ggaatgtcat caagtccaag 9540

cttaggagtt atccggccca ctctcatatt ccatatccaa attgtaatca ggatttattt 9600

aacatagaag acaaagagtc aacgaggaag atccgtgaac tcctcaaaaa ggggaattcg 9660

ctgtactcca aagtcagtga taaggttttc caatgcttaa gggacactaa ctcacggctt 9720

ggcctaggct ccgaattgag ggaggacatc aaggagaaag ttattaactt gggagtttac 9780

atgcacagct cccagtggtt tgagcccttt ctgttttggt ttacagtcaa gactgagatg 9840

aggtcagtga ttaaatcaca aacccatact tgccatagga ggagacacac acctgtattc 9900

ttcactggta gttcagttga gttgctaatc tctcgtgacc ttgttgctat aatcagtaaa 9960

gagtctcaac atgtatatta cctgacattt gaactggttt tgatgtattg tgatgtcata 10020

gaggggaggt taatgacaga gaccgctatg actattgatg ctaggtatac agagcttcta 10080

ggaagagtca gatacatgtg gaaactgata gatggtttct tccctgcact cgggaatcca 10140

acttatcaaa ttgtagccat gctggagcct ctttcacttg cttacctgca gctgagggat 10200

ataacagtag aactcagagg tgctttcctt aaccactgct ttactgaaat acatgatgtt 10260

cttgaccaaa acgggttttc tgatgaaggt acttatcatg agttaactga agctctagat 10320

tacattttca taactgatga catacatctg acaggggaga ttttctcatt tttcagaagt 10380

ttcggccacc ccagacttga agcagtaacg gctgctgaaa atgttaggaa atacatgaat 10440

cagcctaaag tcattgtgta tgagactctg atgaaaggtc atgccatatt ttgtggaatc 10500

ataatcaacg gctatcgtga caggcacgga ggcagttggc caccgctgac cctccccctg 10560

catgctgcag acacaatccg gaatgctcaa gcttcaggtg aagggttaac acatgagcag 10620

tgcgttgata actggaaatc ttttgctgga gtgaaatttg gctgctttat gcctcttagc 10680

ctggatagtg atctgacaat gtacctaaag gacaaggcac ttgctgctct ccaaagggaa 10740

tgggattcag tttacccgaa agagttcctg cgttacgacc ctcccaaggg aaccgggtca 10800

cggaggcttg tagatgtttt ccttaatgat tcgagctttg acccatatga tgtgataatg 10860

tatgttgtaa gtggagctta cctccatgac cctgagttca acctgtctta cagcctgaaa 10920

gaaaaggaga tcaaggaaac aggtagactt tttgctaaaa tgacttacaa aatgagggca 10980

tgccaagtga ttgctgaaaa tctaatctca aacgggattg gcaaatattt taaggacaat 11040

gggatggcca aggatgagca cgatttgact aaggcactcc acactctagc tgtctcagga 11100

gtccccaaag atctcaaaga aagtcacagg ggggggccag tcttaaaaac ctactcccga 11160

agcccagtcc acacaagtac caggaacgtg agagcagcaa aagggtttat agggttccct 11220

caagtaattc ggcaggacca agacactgat catccggaga atatggaagc ttacgagaca 11280

gtcagtgcat ttatcacgac tgatctcaag aagtactgcc ttaattggag atatgagacc 11340

atcagcttgt ttgcacagag gctaaatgag atttacggat tgccctcatt tttccagtgg 11400

ctgcataaga ggcttgagac ctctgtcctg tatgtaagtg accctcattg cccccccgac 11460

cttgacgccc atatcccgtt atataaagtc cccaatgatc aaatcttcat taagtaccct 11520

atgggaggta tagaagggta ttgtcagaag ctgtggacca tcagcaccat tccctatcta 11580

tacctggctg cttatgagag cggagtaagg attgcttcgt tagtgcaagg ggacaatcag 11640

accatagccg taacaaaaag ggtacccagc acatggccct acaaccttaa gaaacgggaa 11700

gctgctagag taactagaga ttactttgta attcttaggc aaaggctaca tgatattggc 11760

catcacctca aggcaaatga gacaattgtt tcatcacatt tttttgtcta ttcaaaagga 11820

atatattatg atgggctact tgtgtcccaa tcactcaaga gcatcgcaag atgtgtattc 11880

tggtcagaga ctatagttga tgaaacaagg gcagcatgca gtaatattgc tacaacaatg 11940

gctaaaagca tcgagagagg ttatgaccgt taccttgcat attccctgaa cgtcctaaaa 12000

gtgatacagc aaattctgat ctctcttggc ttcacaatca attcaaccat gacccgggat 12060

gtagtcatac ccctcctcac aaacaacgac ctcttaataa ggatggcact gttgcccgct 12120

cctattgggg ggatgaatta tctgaatatg agcaggctgt ttgtcagaaa catcggtgat 12180

ccagtaacat catcaattgc tgatctcaag agaatgattc tcgcctcact aatgcctgaa 12240

gagaccctcc atcaagtaat gacacaacaa ccgggggact cttcattcct agactgggct 12300

agcgaccctt actcagcaaa tcttgtatgt gtccagagca tcactagact cctcaagaac 12360

ataactgcaa ggtttgtcct gatccatagt ccaaacccaa tgttaaaagg attattccat 12420

gatgacagta aagaagagga cgagggactg gcggcattcc tcatggacag gcatattata 12480

gtacctaggg cagctcatga aatcctggat catagtgtca caggggcaag agagtctatt 12540

gcaggcatgc tggataccac aaaaggcttg attcgagcca gcatgaggaa gggggggtta 12600

acctctcgag tgataaccag attgtccaat tatgactatg aacaattcag agcagggatg 12660

gtgctattga caggaagaaa gagaaatgtc ctcattgaca aagagtcatg ttcagtgcag 12720

ctggcgagag ctctaagaag ccatatgtgg gcgaggctag ctcgaggacg gcctatttac 12780

ggccttgagg tccctgatgt actagaatct atgcgaggcc accttattcg gcgtcatgag 12840

acatgtgtca tctgcgagtg tggatcagtc aactacggat ggttttttgt cccctcgggt 12900

tgccaactgg atgatattga caaggaaaca tcatccttga gagtcccata tattggttct 12960

accactgatg agagaacaga catgaagctt gccttcgtaa gagccccaag tcgatccttg 13020

cgatctgctg ttagaatagc aacagtgtac tcatgggctt acggtgatga tgatagctct 13080

tggaacgaag cctggttgtt ggctaggcaa agggccaatg tgagcctgga ggagctaagg 13140

gtgatcactc ccatctcaac ttcgactaat ttagcgcata ggttgaggga tcgtagcact 13200

caagtgaaat actcaggtac atcccttgtc cgagtggcga ggtataccac aatctccaac 13260

gacaatctct catttgtcat atcagataag aaggttgata ctaactttat ataccaacaa 13320

ggaatgcttc tagggttggg tgttttagaa acattgtttc gactcgagaa agataccgga 13380

tcatctaaca cggtattaca tcttcacgtc gaaacagatt gttgcgtgat cccgatgata 13440

gatcatccca ggatacccag ctcccgcaag ctagagctga gggcagagct atgtaccaac 13500

ccattgatat atgataatgc acctttaatt gacagagatg caacaaggct atacacccag 13560

agccatagga ggcaccttgt ggaatttgtt acatggtcca caccccaact atatcacatt 13620

ttagctaagt ccacagcact atctatgatt gacctggtaa caaaatttga gaaggaccat 13680

atgaatgaaa tttcagctct cataggggat gacgatatca atagtttcat aactgagttt 13740

ctgctcatag agccaagatt attcactatc tacttgggcc agtgtgcggc catcaattgg 13800

gcatttgatg tacattatca tagaccatca gggaaatatc agatgggtga gctgttgtca 13860

tcgttccttt ctagaatgag caaaggagtg tttaaggtgc ttgtcaatgc tctaagccac 13920

ccaaagatct acaagaaatt ctggcattgt ggtattatag agcctatcca tggtccttca 13980

cttgatgctc aaaacttgca cacaactgtg tgcaacatgg tttacacatg ctatatgacc 14040

tacctcgacc tgttgttgaa tgaagagtta gaagagttca catttctctt gtgtgaaagc 14100

gacgaggatg tagtaccgga cagattcgac aacatccagg caaaacactt atgtgttctg 14160

gcagatttgt actgtcaacc agggacctgc ccaccaattc gaggtctaag accggtagag 14220

aaatgtgcag ttctaaccga ccatatcaag gcagaggcta tgttatctcc agcaggatct 14280

tcgtggaaca taaatccaat tattgtagac cattactcat gctctctgac ttatctccgg 14340

cgaggatcga tcaaacagat aagattgaga gttgatccag gattcatttt cgacgccctc 14400

gctgaggtaa atgtcagtca gccaaagatc ggcagcaaca acatctcaaa tatgagcatc 14460

aaggctttca gacccccaca cgatgatgtt gcaaaattgc tcaaagatat caacacaagc 14520

aagcacaatc ttcccatttc agggggcaat ctcgccaatt atgaaatcca tgctttccgc 14580

agaatcgggt tgaactcatc tgcttgctac aaagctgttg agatatcaac attaattagg 14640

agatgccttg agccagggga ggacggcttg ttcttgggtg agggatcggg ttctatgttg 14700

atcacttata aagagatact taaactaaac aagtgcttct ataatagtgg ggtttccgcc 14760

aattctagat ctggtcaaag ggaattagca ccctatccct ccgaagttgg ccttgtcgaa 14820

cacagaatgg gagtaggtaa tattgtcaaa gtgctcttta acgggaggcc cgaagtcacg 14880

tgggtaggca gtgtagattg cttcaatttc atagttagta atatccctac ctctagtgtg 14940

gggtttatcc attcagatat agagaccttg cctgacaaag atactataga gaagctagag 15000

gaattggcag ccatcttatc gatggctctg ctcctgggca aaataggatc aatactggtg 15060

attaagctta tgcctttcag cggggatttt gttcagggat ttataagtta tgtagggtct 15120

cattatagag aagtgaacct tgtataccct agatacagca acttcatctc tactgaatct 15180

tatttggtta tgacagatct caaggctaac cggctaatga atcctgaaaa gattaagcag 15240

cagataattg aatcatctgt gaggacttca cctggactta taggtcacat cctatccatt 15300

aagcaactaa gctgcataca agcaattgtg ggagacgcag ttagtagagg tgatatcaat 15360

cctactctga aaaaacttac acctatagag caggtgctga tcaattgcgg gttggcaatt 15420

aacggaccta agctgtgcaa agaattgatc caccatgatg ttgcctcagg gcaagatgga 15480

ttgcttaatt ctatactcat cctctacagg gagttggcaa gattcaaaga caaccaaaga 15540

agtcaacaag ggatgttcca cgcttacccc gtattggtaa gtagcaggca acgagaactt 15600

atatctagga tcacccgcaa attctggggg cacattcttc tttactccgg gaacaaaaag 15660

ttgataaata agtttatcca gaatctcaag tccggctatc tgatactaga cttacaccag 15720

aatatcttcg ttaagaatct atccaagtca gagaaacaga ttattatgac gggggggttg 15780

aaacgtgagt gggtttttaa ggtaacagtc aaggagacca aagaatggta taagttagtc 15840

ggatacagtg ccctgattaa ggactaattg gttgaactcc ggaaccctaa tcctgcccta 15900

ggtggttagg cattatttgc aatatattaa agaaaacttt gaaaatacga agtttctatt 15960

cccagctttg tctggtggcc ggcatggtcc cagcctcctc gctggcgccg gctgggcaac 16020

attccgaggg gaccgtcccc tcggtaatgg cgaatgggac gcggccgatc cggctgctaa 16080

caaagcccga aaggaagctg agttggctgc tgccaccgct gagcaataac tagcataacc 16140

ccttggggcc tctaaacggg tcttgagggg ttttttgctg aaaggaggaa ctatatccgg 16200

atgcggccgc catgaacaat aaaactgtct gcttacataa acagtaatac aaggggtgtt 16260

atgagccata ttcaacggga aacgtcgagg ccgcgattaa attccaacat ggatgctgat 16320

ttatatgggt ataaatgggc tcgcgataat gtcgggcaat caggtgcgac aatctatcgc 16380

ttgtatggga agcccgatgc gccagagttg tttctgaaac atggcaaagg tagcgttgcc 16440

aatgatgtta cagatgagat ggtcagacta aactggctga cggaatttat gcctcttccg 16500

accatcaagc attttatccg tactcctgat gatgcatggt tactcaccac tgcgatcccc 16560

ggaaaaacag cattccaggt attagaagaa tatcctgatt caggtgaaaa tattgttgat 16620

gcgctggcag tgttcctgcg ccggttgcat tcgattcctg tttgtaattg tccttttaac 16680

agcgatcgtg tatttcgtct cgctcaggcg caatcacgaa tgaataacgg tttggttgat 16740

gcgagtgatt ttgatgacga gcgtaatggc tggcctgttg aacaagtctg gaaagaaatg 16800

cataaacttt tgccattctc accggattca gtcgtcactc atggtgattt ctcacttgat 16860

aaccttattt ttgacgaggg gaaattaata ggttgtattg atgttggacg agtcggaatc 16920

gcagaccgat accaggatct tgccatccta tggaactgcc tcggtgagtt ttctccttca 16980

ttacagaaac ggctttttca aaaatatggt attgataatc ctgatatgaa taaattgcag 17040

tttcatttga tgctcgatga gtttttctaa tcagaattgg ttaattggtt gtaacattat 17100

tcagattggg ccccgttcca ctgagcgtca gaccccgtag aaaagatcaa aggatcttct 17160

tgagatcctt tttttctgcg cgtaatctgc tgcttgcaaa caaaaaaacc accgctacca 17220

gcggtggttt gtttgccgga tcaagagcta ccaactcttt ttccgaaggt aactggcttc 17280

agcagagcgc agataccaaa tactgttctt ctagtgtagc cgtagttagg ccaccacttc 17340

aagaactctg tagcaccgcc tacatacctc gctctgctaa tcctgttacc agtggctgct 17400

gccagtggcg ataagtcgtg tcttaccggg ttggactcaa gacgatagtt accggataag 17460

gcgcagcggt cgggctgaac ggggggttcg tgcacacagc ccagcttgga gcgaacgacc 17520

tacaccgaac tgagatacct acagcgtgag ctatgagaaa gcgccacgct tcccgaaggg 17580

agaaaggcgg acaggtatcc ggtaagcggc agggtcggaa caggagagcg cacgagggag 17640

cttccagggg gaaacgcctg gtatctttat agtcctgtcg ggtttcgcca cctctgactt 17700

gagcgtcgat ttttgtgatg ctcgtcaggg gggcggagcc tatggaaaaa cgccagcaac 17760

gcggcctttt tacggttcct ggccttttgc tggccttttg ctcacatgtt ctttcctgcg 17820

ttatcccctg attctgtgga taaccgtatt accgctag 17858

<210> 31
<211> 18734
<212> DNA
<213> Artificial sequence

<220>
<223> pKP-MVSchw-ATU1(eGFP)

<400> 31
gcgatcgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga cgcgtacgat ggtgagcaag ggcgaggagc tgttcaccgg ggtggtgccc 240

atcctggtcg agctggacgg cgacgtaaac ggccacaagt tcagcgtgtc cggcgagggc 300

gagggcgatg ccacctacgg caagctgacc ctgaagttca tctgcaccac cggcaagctg 360

cccgtgccct ggcccaccct cgtgaccacc ctgacctacg gcgtgcagtg cttcagccgc 420

taccccgacc acatgaagca gcacgacttc ttcaagtccg ccatgcccga aggctacgtc 480

caggagcgca ccatcttctt caaggacgac ggcaactaca agacccgcgc cgaggtgaag 540

ttcgagggcg acaccctggt gaaccgcatc gagctgaagg gcatcgactt caaggaggac 600

ggcaacatcc tggggcacaa gctggagtac aactacaaca gccacaacgt ctatatcatg 660

gccgacaagc agaagaacgg catcaaggtg aacttcaaga tccgccacaa catcgaggac 720

ggcagcgtgc agctcgccga ccactaccag cagaacaccc ccatcggcga cggccccgtg 780

ctgctgcccg acaaccacta cctgagcacc cagtccgccc tgagcaaaga ccccaacgag 840

aagcgcgatc acatggtcct gctggagttc gtgaccgccg ccgggatcac tctcggcatg 900

gacgagctgt acaagtaggc gcgcagcgct tagacgtctc gcgatcgatt agtgcgagag 960

gccgagggcc agaacaacat ccgcctacca tccatcattg ttataaaaaa cttaggattc 1020

aagatcctat tatcagggac aagagcagga ttagggatat ccgagatggc cacactttta 1080

aggagcttag cattgttcaa aagaaacaag gacaaaccac ccattacatc aggatccggt 1140

ggagccatca gaggaatcaa acacattatt atagtaccaa tccctggaga ttcctcaatt 1200

accactcgat ccagacttct ggaccggttg gtgaggttaa ttggaaaccc ggatgtgagc 1260

gggcccaaac taacaggggc actaataggt atattatcct tatttgtgga gtctccaggt 1320

caattgattc agaggatcac cgatgaccct gacgttagca taaggctgtt agaggttgtc 1380

cagagtgacc agtcacaatc tggccttacc ttcgcatcaa gaggtaccaa catggaggat 1440

gaggcggacc aatacttttc acatgatgat ccaattagta gtgatcaatc caggttcgga 1500

tggttcggga acaaggaaat ctcagatatt gaagtgcaag accctgaggg attcaacatg 1560

attctgggta ccatcctagc ccaaatttgg gtcttgctcg caaaggcggt tacggcccca 1620

gacacggcag ctgattcgga gctaagaagg tggataaagt acacccaaca aagaagggta 1680

gttggtgaat ttagattgga gagaaaatgg ttggatgtgg tgaggaacag gattgccgag 1740

gacctctcct tacgccgatt catggtcgct ctaatcctgg atatcaagag aacacccgga 1800

aacaaaccca ggattgctga aatgatatgt gacattgata catatatcgt agaggcagga 1860

ttagccagtt ttatcctgac tattaagttt gggatagaaa ctatgtatcc tgctcttgga 1920

ctgcatgaat ttgctggtga gttatccaca cttgagtcct tgatgaacct ttaccagcaa 1980

atgggggaaa ctgcacccta catggtaatc ctggagaact caattcagaa caagttcagt 2040

gcaggatcat accctctgct ctggagctat gccatgggag taggagtgga acttgaaaac 2100

tccatgggag gtttgaactt tggccgatct tactttgatc cagcatattt tagattaggg 2160

caagagatgg taaggaggtc agctggaaag gtcagttcca cattggcatc tgaactcggt 2220

atcactgccg aggatgcaag gcttgtttca gagattgcaa tgcatactac tgaggacaag 2280

atcagtagag cggttggacc cagacaagcc caagtatcat ttctacacgg tgatcaaagt 2340

gagaatgagc taccgagatt ggggggcaag gaagatagga gggtcaaaca gagtcgagga 2400

gaagccaggg agagctacag agaaaccggg cccagcagag caagtgatgc gagagctgcc 2460

catcttccaa ccggcacacc cctagacatt gacactgcaa cggagtccag ccaagatccg 2520

caggacagtc gaaggtcagc tgacgccctg cttaggctgc aagccatggc aggaatctcg 2580

gaagaacaag gctcagacac ggacacccct atagtgtaca atgacagaaa tcttctagac 2640

taggtgcgag aggccgaggg ccagaacaac atccgcctac catccatcat tgttataaaa 2700

aacttaggaa ccaggtccac acagccgcca gcccatcaac catccactcc cacgattgga 2760

gccaatggca gaagagcagg cacgccatgt caaaaacgga ctggaatgca tccgggctct 2820

caaggccgag cccatcggct cactggccat cgaggaagct atggcagcat ggtcagaaat 2880

atcagacaac ccaggacagg agcgagccac ctgcagggaa gagaaggcag gcagttcggg 2940

tctcagcaaa ccatgcctct cagcaattgg atcaactgaa ggcggtgcac ctcgcatccg 3000

cggtcaggga cctggagaga gcgatgacga cgctgaaact ttgggaatcc ccccaagaaa 3060

tctccaggca tcaagcactg ggttacagtg ttattacgtt tatgatcaca gcggtgaagc 3120

ggttaaggga atccaagatg ctgactctat catggttcaa tcaggccttg atggtgatag 3180

caccctctca ggaggagaca atgaatctga aaacagcgat gtggatattg gcgaacctga 3240

taccgaggga tatgctatca ctgaccgggg atctgctccc atctctatgg ggttcagggc 3300

ttctgatgtt gaaactgcag aaggagggga gatccacgag ctcctgagac tccaatccag 3360

aggcaacaac tttccgaagc ttgggaaaac tctcaatgtt cctccgcccc cggaccccgg 3420

tagggccagc acttccggga cacccattaa aaagggcaca gacgcgagat tagcctcatt 3480

tggaacggag atcgcgtctt tattgacagg tggtgcaacc caatgtgctc gaaagtcacc 3540

ctcggaacca tcagggccag gtgcacctgc ggggaatgtc cccgagtgtg tgagcaatgc 3600

cgcactgata caggagtgga cacccgaatc tggtaccaca atctccccga gatcccagaa 3660

taatgaagaa gggggagact attatgatga tgagctgttc tctgatgtcc aagatattaa 3720

aacagccttg gccaaaatac acgaggataa tcagaagata atctccaagc tagaatcact 3780

gctgttattg aagggagaag ttgagtcaat taagaagcag atcaacaggc aaaatatcag 3840

catatccacc ctggaaggac acctctcaag catcatgatc gccattcctg gacttgggaa 3900

ggatcccaac gaccccactg cagatgtcga aatcaatccc gacttgaaac ccatcatagg 3960

cagagattca ggccgagcac tggccgaagt tctcaagaaa cccgttgcca gccgacaact 4020

ccaaggaatg acaaatggac ggaccagttc cagaggacag ctgctgaagg aatttcagct 4080

aaagccgatc gggaaaaaga tgagctcagc cgtcgggttt gttcctgaca ccggccctgc 4140

atcacgcagt gtaatccgct ccattataaa atccagccgg ctagaggagg atcggaagcg 4200

ttacctgatg actctccttg atgatatcaa aggagccaat gatcttgcca agttccacca 4260

gatgctgatg aagataataa tgaagtagct acagctcaac ttacctgcca accccatgcc 4320

agtcgaccca actagtacaa cctaaatcca ttataaaaaa cttaggagca aagtgattgc 4380

ctcccaaggt ccacaatgac agagacctac gacttcgaca agtcggcatg ggacatcaaa 4440

gggtcgatcg ctccgataca acccaccacc tacagtgatg gcaggctggt gccccaggtc 4500

agagtcatag atcctggtct aggcgacagg aaggatgaat gctttatgta catgtttctg 4560

ctgggggttg ttgaggacag cgattcccta gggcctccaa tcgggcgagc atttgggttc 4620

ctgcccttag gtgttggcag atccacagca aagcccgaaa aactcctcaa agaggccact 4680

gagcttgaca tagttgttag acgtacagca gggctcaatg aaaaactggt gttctacaac 4740

aacaccccac taactctcct cacaccttgg agaaaggtcc taacaacagg gagtgtcttc 4800

aacgcaaacc aagtgtgcaa tgcggttaat ctgataccgc tcgatacccc gcagaggttc 4860

cgtgttgttt atatgagcat cacccgtctt tcggataacg ggtattacac cgttcctaga 4920

agaatgctgg aattcagatc ggtcaatgca gtggccttca acctgctggt gacccttagg 4980

attgacaagg cgataggccc tgggaagatc atcgacaata cagagcaact tcctgaggca 5040

acatttatgg tccacatcgg gaacttcagg agaaagaaga gtgaagtcta ctctgccgat 5100

tattgcaaaa tgaaaatcga aaagatgggc ctggtttttg cacttggtgg gatagggggc 5160

accagtcttc acattagaag cacaggcaaa atgagcaaga ctctccatgc acaactcggg 5220

ttcaagaaga ccttatgtta cccgctgatg gatatcaatg aagaccttaa tcgattactc 5280

tggaggagca gatgcaagat agtaagaatc caggcagttt tgcagccatc agttcctcaa 5340

gaattccgca tttacgacga cgtgatcata aatgatgacc aaggactatt caaagttctg 5400

tagaccgtag tgcccagcaa tgcccgaaaa cgacccccct cacaatgaca gccagaaggc 5460

ccggacaaaa aagccccctc cgaaagactc cacggaccaa gcgagaggcc agccagcagc 5520

cgacggcaag cgcgaacacc aggcggcccc agcacagaac agccctgaca caaggccacc 5580

accagccacc ccaatctgca tcctcctcgt gggacccccg aggaccaacc cccaaggctg 5640

cccccgatcc aaaccaccaa ccgcatcccc accacccccg ggaaagaaac ccccagcaat 5700

tggaaggccc ctccccctct tcctcaacac aagaactcca caaccgaacc gcacaagcga 5760

ccgaggtgac ccaaccgcag gcatccgact ccctagacag atcctctctc cccggcaaac 5820

taaacaaaac ttagggccaa ggaacataca cacccaacag aacccagacc ccggcccacg 5880

gcgccgcgcc cccaaccccc gacaaccaga gggagccccc aaccaatccc gccggctccc 5940

ccggtgccca caggcaggga caccaacccc cgaacagacc cagcacccaa ccatcgacaa 6000

tccaagacgg gggggccccc ccaaaaaaag gcccccaggg gccgacagcc agcaccgcga 6060

ggaagcccac ccaccccaca cacgaccacg gcaaccaaac cagaacccag accaccctgg 6120

gccaccagct cccagactcg gccatcaccc cgcagaaagg aaaggccaca acccgcgcac 6180

cccagccccg atccggcggg gagccaccca acccgaacca gcacccaaga gcgatccccg 6240

aaggaccccc gaaccgcaaa ggacatcagt atcccacagc ctctccaagt cccccggtct 6300

cctcctcttc tcgaagggac caaaagatca atccaccaca cccgacgaca ctcaactccc 6360

cacccctaaa ggagacaccg ggaatcccag aatcaagact catccaatgt ccatcatggg 6420

tctcaaggtg aacgtctctg ccatattcat ggcagtactg ttaactctcc aaacacccac 6480

cggtcaaatc cattggggca atctctctaa gataggggtg gtaggaatag gaagtgcaag 6540

ctacaaagtt atgactcgtt ccagccatca atcattagtc ataaaattaa tgcccaatat 6600

aactctcctc aataactgca cgagggtaga gattgcagaa tacaggagac tactgagaac 6660

agttttggaa ccaattagag atgcacttaa tgcaatgacc cagaatataa gaccggttca 6720

gagtgtagct tcaagtagga gacacaagag atttgcggga gtagtcctgg caggtgcggc 6780

cctaggcgtt gccacagctg ctcagataac agccggcatt gcacttcacc agtccatgct 6840

gaactctcaa gccatcgaca atctgagagc gagcctggaa actactaatc aggcaattga 6900

gacaatcaga caagcagggc aggagatgat attggctgtt cagggtgtcc aagactacat 6960

caataatgag ctgataccgt ctatgaacca actatcttgt gatttaatcg gccagaagct 7020

cgggctcaaa ttgctcagat actatacaga aatcctgtca ttatttggcc ccagtttacg 7080

ggaccccata tctgcggaga tatctatcca ggctttgagc tatgcgcttg gaggagacat 7140

caataaggtg ttagaaaagc tcggatacag tggaggtgat ttactgggca tcttagagag 7200

cggaggaata aaggcccgga taactcacgt cgacacagag tcctacttca ttgtcctcag 7260

tatagcctat ccgacgctgt ccgagattaa gggggtgatt gtccaccggc tagagggggt 7320

ctcgtacaac ataggctctc aagagtggta taccactgtg cccaagtatg ttgcaaccca 7380

agggtacctt atctcgaatt ttgatgagtc atcgtgtact ttcatgccag aggggactgt 7440

gtgcagccaa aatgccttgt acccgatgag tcctctgctc caagaatgcc tccgggggta 7500

caccaagtcc tgtgctcgta cactcgtatc cgggtctttt gggaaccggt tcattttatc 7560

acaagggaac ctaatagcca attgtgcatc aatcctttgc aagtgttaca caacaggaac 7620

gatcattaat caagaccctg acaagatcct aacatacatt gctgccgatc actgcccggt 7680

agtcgaggtg aacggcgtga ccatccaagt cgggagcagg aggtatccag acgctgtgta 7740

cttgcacaga attgacctcg gtcctcccat atcattggag aggttggacg tagggacaaa 7800

tctggggaat gcaattgcta agttggagga tgccaaggaa ttgttggagt catcggacca 7860

gatattgagg agtatgaaag gtttatcgag cactagcata gtctacatcc tgattgcagt 7920

gtgtcttgga gggttgatag ggatccccgc tttaatatgt tgctgcaggg ggcgttgtaa 7980

caaaaaggga gaacaagttg gtatgtcaag accaggccta aagcctgatc ttacgggaac 8040

atcaaaatcc tatgtaaggt cgctctgatc ctctacaact cttgaaacac aaatgtccca 8100

caagtctcct cttcgtcatc aagcaaccac cgcacccagc atcaagccca cctgaaatta 8160

tctccggctt ccctctggcc gaacaatatc ggtagttaat caaaacttag ggtgcaagat 8220

catccacaat gtcaccacaa cgagaccgga taaatgcctt ctacaaagat aacccccatc 8280

ccaagggaag taggatagtc attaacagag aacatcttat gattgataga ccttatgttt 8340

tgctggctgt tctgtttgtc atgtttctga gcttgatcgg gttgctagcc attgcaggca 8400

ttagacttca tcgggcagcc atctacaccg cagagatcca taaaagcctc agcaccaatc 8460

tagatgtaac taactcaatc gagcatcagg tcaaggacgt gctgacacca ctcttcaaaa 8520

tcatcggtga tgaagtgggc ctgaggacac ctcagagatt cactgaccta gtgaaattaa 8580

tctctgacaa gattaaattc cttaatccgg atagggagta cgacttcaga gatctcactt 8640

ggtgtatcaa cccgccagag agaatcaaat tggattatga tcaatactgt gcagatgtgg 8700

ctgctgaaga gctcatgaat gcattggtga actcaactct actggagacc agaacaacca 8760

atcagttcct agctgtctca aagggaaact gctcagggcc cactacaatc agaggtcaat 8820

tctcaaacat gtcgctgtcc ctgttagact tgtatttagg tcgaggttac aatgtgtcat 8880

ctatagtcac tatgacatcc cagggaatgt atgggggaac ttacctagtg gaaaagccta 8940

atctgagcag caaaaggtca gagttgtcac aactgagcat gtaccgagtg tttgaagtag 9000

gtgttatcag aaatccgggt ttgggggctc cggtgttcca tatgacaaac tatcttgagc 9060

aaccagtcag taatgatctc agcaactgta tggtggcttt gggggagctc aaactcgcag 9120

ccctttgtca cggggaagat tctatcacaa ttccctatca gggatcaggg aaaggtgtca 9180

gcttccagct cgtcaagcta ggtgtctgga aatccccaac cgacatgcaa tcctgggtcc 9240

ccttatcaac ggatgatcca gtgatagaca ggctttacct ctcatctcac agaggtgtta 9300

tcgctgacaa tcaagcaaaa tgggctgtcc cgacaacacg aacagatgac aagttgcgaa 9360

tggagacatg cttccaacag gcgtgtaagg gtaaaatcca agcactctgc gagaatcccg 9420

agtgggcacc attgaaggat aacaggattc cttcatacgg ggtcttgtct gttgatctga 9480

gtctgacagt tgagcttaaa atcaaaattg cttcgggatt cgggccattg atcacacacg 9540

gttcagggat ggacctatac aaatccaacc acaacaatgt gtattggctg actatcccgc 9600

caatgaagaa cctagcctta ggtgtaatca acacattgga gtggataccg agattcaagg 9660

ttagtcccta cctcttcact gtcccaatta aggaagcagg cgaagactgc catgccccaa 9720

catacctacc tgcggaggtg gatggtgatg tcaaactcag ttccaatctg gtgattctac 9780

ctggtcaaga tctccaatat gttttggcaa cctacgatac ttccaggggt gaacatgctg 9840

tggtttatta cgtttacagc ccaagccgct cattttctta cttttatcct tttaggttgc 9900

ctataaaggg ggtccccatc gaattacaag tggaatgctt cacatgggac caaaaactct 9960

ggtgccgtca cttctgtgtg cttgcggact cagaatctgg tggacatatc actcactctg 10020

ggatggtggg catgggagtc agctgcacag tcacccggga agatggaacc aatcgcagat 10080

agggctgcta gtgaaccaat cacatgatgt cacccagaca tcaggcatac ccactagtgt 10140

gaaatagaca tcagaattaa gaaaaacgta gggtccaagt ggttccccgt tatggactcg 10200

ctatctgtca accagatctt ataccctgaa gttcacctag atagcccgat agttaccaat 10260

aagatagtag ccatcctgga gtatgctcga gtccctcacg cttacagcct ggaggaccct 10320

acactgtgtc agaacatcaa gcaccgccta aaaaacggat tttccaacca aatgattata 10380

aacaatgtgg aagttgggaa tgtcatcaag tccaagctta ggagttatcc ggcccactct 10440

catattccat atccaaattg taatcaggat ttatttaaca tagaagacaa agagtcaacg 10500

aggaagatcc gtgaactcct caaaaagggg aattcgctgt actccaaagt cagtgataag 10560

gttttccaat gcttaaggga cactaactca cggcttggcc taggctccga attgagggag 10620

gacatcaagg agaaagttat taacttggga gtttacatgc acagctccca gtggtttgag 10680

ccctttctgt tttggtttac agtcaagact gagatgaggt cagtgattaa atcacaaacc 10740

catacttgcc ataggaggag acacacacct gtattcttca ctggtagttc agttgagttg 10800

ctaatctctc gtgaccttgt tgctataatc agtaaagagt ctcaacatgt atattacctg 10860

acatttgaac tggttttgat gtattgtgat gtcatagagg ggaggttaat gacagagacc 10920

gctatgacta ttgatgctag gtatacagag cttctaggaa gagtcagata catgtggaaa 10980

ctgatagatg gtttcttccc tgcactcggg aatccaactt atcaaattgt agccatgctg 11040

gagcctcttt cacttgctta cctgcagctg agggatataa cagtagaact cagaggtgct 11100

ttccttaacc actgctttac tgaaatacat gatgttcttg accaaaacgg gttttctgat 11160

gaaggtactt atcatgagtt aactgaagct ctagattaca ttttcataac tgatgacata 11220

catctgacag gggagatttt ctcatttttc agaagtttcg gccaccccag acttgaagca 11280

gtaacggctg ctgaaaatgt taggaaatac atgaatcagc ctaaagtcat tgtgtatgag 11340

actctgatga aaggtcatgc catattttgt ggaatcataa tcaacggcta tcgtgacagg 11400

cacggaggca gttggccacc gctgaccctc cccctgcatg ctgcagacac aatccggaat 11460

gctcaagctt caggtgaagg gttaacacat gagcagtgcg ttgataactg gaaatctttt 11520

gctggagtga aatttggctg ctttatgcct cttagcctgg atagtgatct gacaatgtac 11580

ctaaaggaca aggcacttgc tgctctccaa agggaatggg attcagttta cccgaaagag 11640

ttcctgcgtt acgaccctcc caagggaacc gggtcacgga ggcttgtaga tgttttcctt 11700

aatgattcga gctttgaccc atatgatgtg ataatgtatg ttgtaagtgg agcttacctc 11760

catgaccctg agttcaacct gtcttacagc ctgaaagaaa aggagatcaa ggaaacaggt 11820

agactttttg ctaaaatgac ttacaaaatg agggcatgcc aagtgattgc tgaaaatcta 11880

atctcaaacg ggattggcaa atattttaag gacaatggga tggccaagga tgagcacgat 11940

ttgactaagg cactccacac tctagctgtc tcaggagtcc ccaaagatct caaagaaagt 12000

cacagggggg ggccagtctt aaaaacctac tcccgaagcc cagtccacac aagtaccagg 12060

aacgtgagag cagcaaaagg gtttataggg ttccctcaag taattcggca ggaccaagac 12120

actgatcatc cggagaatat ggaagcttac gagacagtca gtgcatttat cacgactgat 12180

ctcaagaagt actgccttaa ttggagatat gagaccatca gcttgtttgc acagaggcta 12240

aatgagattt acggattgcc ctcatttttc cagtggctgc ataagaggct tgagacctct 12300

gtcctgtatg taagtgaccc tcattgcccc cccgaccttg acgcccatat cccgttatat 12360

aaagtcccca atgatcaaat cttcattaag taccctatgg gaggtataga agggtattgt 12420

cagaagctgt ggaccatcag caccattccc tatctatacc tggctgctta tgagagcgga 12480

gtaaggattg cttcgttagt gcaaggggac aatcagacca tagccgtaac aaaaagggta 12540

cccagcacat ggccctacaa ccttaagaaa cgggaagctg ctagagtaac tagagattac 12600

tttgtaattc ttaggcaaag gctacatgat attggccatc acctcaaggc aaatgagaca 12660

attgtttcat cacatttttt tgtctattca aaaggaatat attatgatgg gctacttgtg 12720

tcccaatcac tcaagagcat cgcaagatgt gtattctggt cagagactat agttgatgaa 12780

acaagggcag catgcagtaa tattgctaca acaatggcta aaagcatcga gagaggttat 12840

gaccgttacc ttgcatattc cctgaacgtc ctaaaagtga tacagcaaat tctgatctct 12900

cttggcttca caatcaattc aaccatgacc cgggatgtag tcatacccct cctcacaaac 12960

aacgacctct taataaggat ggcactgttg cccgctccta ttggggggat gaattatctg 13020

aatatgagca ggctgtttgt cagaaacatc ggtgatccag taacatcatc aattgctgat 13080

ctcaagagaa tgattctcgc ctcactaatg cctgaagaga ccctccatca agtaatgaca 13140

caacaaccgg gggactcttc attcctagac tgggctagcg acccttactc agcaaatctt 13200

gtatgtgtcc agagcatcac tagactcctc aagaacataa ctgcaaggtt tgtcctgatc 13260

catagtccaa acccaatgtt aaaaggatta ttccatgatg acagtaaaga agaggacgag 13320

ggactggcgg cattcctcat ggacaggcat attatagtac ctagggcagc tcatgaaatc 13380

ctggatcata gtgtcacagg ggcaagagag tctattgcag gcatgctgga taccacaaaa 13440

ggcttgattc gagccagcat gaggaagggg gggttaacct ctcgagtgat aaccagattg 13500

tccaattatg actatgaaca attcagagca gggatggtgc tattgacagg aagaaagaga 13560

aatgtcctca ttgacaaaga gtcatgttca gtgcagctgg cgagagctct aagaagccat 13620

atgtgggcga ggctagctcg aggacggcct atttacggcc ttgaggtccc tgatgtacta 13680

gaatctatgc gaggccacct tattcggcgt catgagacat gtgtcatctg cgagtgtgga 13740

tcagtcaact acggatggtt ttttgtcccc tcgggttgcc aactggatga tattgacaag 13800

gaaacatcat ccttgagagt cccatatatt ggttctacca ctgatgagag aacagacatg 13860

aagcttgcct tcgtaagagc cccaagtcga tccttgcgat ctgctgttag aatagcaaca 13920

gtgtactcat gggcttacgg tgatgatgat agctcttgga acgaagcctg gttgttggct 13980

aggcaaaggg ccaatgtgag cctggaggag ctaagggtga tcactcccat ctcaacttcg 14040

actaatttag cgcataggtt gagggatcgt agcactcaag tgaaatactc aggtacatcc 14100

cttgtccgag tggcgaggta taccacaatc tccaacgaca atctctcatt tgtcatatca 14160

gataagaagg ttgatactaa ctttatatac caacaaggaa tgcttctagg gttgggtgtt 14220

ttagaaacat tgtttcgact cgagaaagat accggatcat ctaacacggt attacatctt 14280

cacgtcgaaa cagattgttg cgtgatcccg atgatagatc atcccaggat acccagctcc 14340

cgcaagctag agctgagggc agagctatgt accaacccat tgatatatga taatgcacct 14400

ttaattgaca gagatgcaac aaggctatac acccagagcc ataggaggca ccttgtggaa 14460

tttgttacat ggtccacacc ccaactatat cacattttag ctaagtccac agcactatct 14520

atgattgacc tggtaacaaa atttgagaag gaccatatga atgaaatttc agctctcata 14580

ggggatgacg atatcaatag tttcataact gagtttctgc tcatagagcc aagattattc 14640

actatctact tgggccagtg tgcggccatc aattgggcat ttgatgtaca ttatcataga 14700

ccatcaggga aatatcagat gggtgagctg ttgtcatcgt tcctttctag aatgagcaaa 14760

ggagtgttta aggtgcttgt caatgctcta agccacccaa agatctacaa gaaattctgg 14820

cattgtggta ttatagagcc tatccatggt ccttcacttg atgctcaaaa cttgcacaca 14880

actgtgtgca acatggttta cacatgctat atgacctacc tcgacctgtt gttgaatgaa 14940

gagttagaag agttcacatt tctcttgtgt gaaagcgacg aggatgtagt accggacaga 15000

ttcgacaaca tccaggcaaa acacttatgt gttctggcag atttgtactg tcaaccaggg 15060

acctgcccac caattcgagg tctaagaccg gtagagaaat gtgcagttct aaccgaccat 15120

atcaaggcag aggctatgtt atctccagca ggatcttcgt ggaacataaa tccaattatt 15180

gtagaccatt actcatgctc tctgacttat ctccggcgag gatcgatcaa acagataaga 15240

ttgagagttg atccaggatt cattttcgac gccctcgctg aggtaaatgt cagtcagcca 15300

aagatcggca gcaacaacat ctcaaatatg agcatcaagg ctttcagacc cccacacgat 15360

gatgttgcaa aattgctcaa agatatcaac acaagcaagc acaatcttcc catttcaggg 15420

ggcaatctcg ccaattatga aatccatgct ttccgcagaa tcgggttgaa ctcatctgct 15480

tgctacaaag ctgttgagat atcaacatta attaggagat gccttgagcc aggggaggac 15540

ggcttgttct tgggtgaggg atcgggttct atgttgatca cttataaaga gatacttaaa 15600

ctaaacaagt gcttctataa tagtggggtt tccgccaatt ctagatctgg tcaaagggaa 15660

ttagcaccct atccctccga agttggcctt gtcgaacaca gaatgggagt aggtaatatt 15720

gtcaaagtgc tctttaacgg gaggcccgaa gtcacgtggg taggcagtgt agattgcttc 15780

aatttcatag ttagtaatat ccctacctct agtgtggggt ttatccattc agatatagag 15840

accttgcctg acaaagatac tatagagaag ctagaggaat tggcagccat cttatcgatg 15900

gctctgctcc tgggcaaaat aggatcaata ctggtgatta agcttatgcc tttcagcggg 15960

gattttgttc agggatttat aagttatgta gggtctcatt atagagaagt gaaccttgta 16020

taccctagat acagcaactt catctctact gaatcttatt tggttatgac agatctcaag 16080

gctaaccggc taatgaatcc tgaaaagatt aagcagcaga taattgaatc atctgtgagg 16140

acttcacctg gacttatagg tcacatccta tccattaagc aactaagctg catacaagca 16200

attgtgggag acgcagttag tagaggtgat atcaatccta ctctgaaaaa acttacacct 16260

atagagcagg tgctgatcaa ttgcgggttg gcaattaacg gacctaagct gtgcaaagaa 16320

ttgatccacc atgatgttgc ctcagggcaa gatggattgc ttaattctat actcatcctc 16380

tacagggagt tggcaagatt caaagacaac caaagaagtc aacaagggat gttccacgct 16440

taccccgtat tggtaagtag caggcaacga gaacttatat ctaggatcac ccgcaaattc 16500

tgggggcaca ttcttcttta ctccgggaac aaaaagttga taaataagtt tatccagaat 16560

ctcaagtccg gctatctgat actagactta caccagaata tcttcgttaa gaatctatcc 16620

aagtcagaga aacagattat tatgacgggg ggtttgaaac gtgagtgggt ttttaaggta 16680

acagtcaagg agaccaaaga atggtataag ttagtcggat acagtgccct gattaaggac 16740

taattggttg aactccggaa ccctaatcct gccctaggtg gttaggcatt atttgcaata 16800

tattaaagaa aactttgaaa atacgaagtt tctattccca gctttgtctg gtggccggca 16860

tggtcccagc ctcctcgctg gcgccggctg ggcaacattc cgaggggacc gtcccctcgg 16920

taatggcgaa tgggacgcgg ccgatccggc tgctaacaaa gcccgaaagg aagctgagtt 16980

ggctgctgcc accgctgagc aataactagc ataacccctt ggggcctcta aacgggtctt 17040

gaggggtttt ttgctgaaag gaggaactat atccggatgc ggccgccatg aacaataaaa 17100

ctgtctgctt acataaacag taatacaagg ggtgttatga gccatattca acgggaaacg 17160

tcgaggccgc gattaaattc caacatggat gctgatttat atgggtataa atgggctcgc 17220

gataatgtcg ggcaatcagg tgcgacaatc tatcgcttgt atgggaagcc cgatgcgcca 17280

gagttgtttc tgaaacatgg caaaggtagc gttgccaatg atgttacaga tgagatggtc 17340

agactaaact ggctgacgga atttatgcct cttccgacca tcaagcattt tatccgtact 17400

cctgatgatg catggttact caccactgcg atccccggaa aaacagcatt ccaggtatta 17460

gaagaatatc ctgattcagg tgaaaatatt gttgatgcgc tggcagtgtt cctgcgccgg 17520

ttgcattcga ttcctgtttg taattgtcct tttaacagcg atcgtgtatt tcgtctcgct 17580

caggcgcaat cacgaatgaa taacggtttg gttgatgcga gtgattttga tgacgagcgt 17640

aatggctggc ctgttgaaca agtctggaaa gaaatgcata aacttttgcc attctcaccg 17700

gattcagtcg tcactcatgg tgatttctca cttgataacc ttatttttga cgaggggaaa 17760

ttaataggtt gtattgatgt tggacgagtc ggaatcgcag accgatacca ggatcttgcc 17820

atcctatgga actgcctcgg tgagttttct ccttcattac agaaacggct ttttcaaaaa 17880

tatggtattg ataatcctga tatgaataaa ttgcagtttc atttgatgct cgatgagttt 17940

ttctaatcag aattggttaa ttggttgtaa cattattcag attgggcccc gttccactga 18000

gcgtcagacc ccgtagaaaa gatcaaagga tcttcttgag atcctttttt tctgcgcgta 18060

atctgctgct tgcaaacaaa aaaaccaccg ctaccagcgg tggtttgttt gccggatcaa 18120

gagctaccaa ctctttttcc gaaggtaact ggcttcagca gagcgcagat accaaatact 18180

gttcttctag tgtagccgta gttaggccac cacttcaaga actctgtagc accgcctaca 18240

tacctcgctc tgctaatcct gttaccagtg gctgctgcca gtggcgataa gtcgtgtctt 18300

accgggttgg actcaagacg atagttaccg gataaggcgc agcggtcggg ctgaacgggg 18360

ggttcgtgca cacagcccag cttggagcga acgacctaca ccgaactgag atacctacag 18420

cgtgagctat gagaaagcgc cacgcttccc gaagggagaa aggcggacag gtatccggta 18480

agcggcaggg tcggaacagg agagcgcacg agggagcttc cagggggaaa cgcctggtat 18540

ctttatagtc ctgtcgggtt tcgccacctc tgacttgagc gtcgattttt gtgatgctcg 18600

tcaggggggc ggagcctatg gaaaaacgcc agcaacgcgg cctttttacg gttcctggcc 18660

ttttgctggc cttttgctca catgttcttt cctgcgttat cccctgattc tgtggataac 18720

cgtattaccg ctag 18734

<210> 32
<211> 18686
<212> DNA
<213> Artificial sequence

<220>
<223> pKP-MVSchw-ATU2(eGFP)

<400> 32
gcgatcgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480
agcataaggc tgttagaggt tgtccagagt gaccagtcac aatctggcct taccttcgca 540
tcaagaggta ccaacatgga ggatgaggcg gaccaatact tttcacatga tgatccaatt 600
agtagtgatc aatccaggtt cggatggttc gggaacaagg aaatctcaga tattgaagtg 660
caagaccctg agggattcaa catgattctg ggtaccatcc tagcccaaat ttgggtcttg 720
ctcgcaaagg cggttacggc cccagacacg gcagctgatt cggagctaag aaggtggata 780
aagtacaccc aacaaagaag ggtagttggt gaatttagat tggagagaaa atggttggat 840
gtggtgagga acaggattgc cgaggacctc tccttacgcc gattcatggt cgctctaatc 900
ctggatatca agagaacacc cggaaacaaa cccaggattg ctgaaatgat atgtgacatt 960
gatacatata tcgtagaggc aggattagcc agttttatcc tgactattaa gtttgggata 1020
gaaactatgt atcctgctct tggactgcat gaatttgctg gtgagttatc cacacttgag 1080
tccttgatga acctttacca gcaaatgggg gaaactgcac cctacatggt aatcctggag 1140
aactcaattc agaacaagtt cagtgcagga tcataccctc tgctctggag ctatgccatg 1200
ggagtaggag tggaacttga aaactccatg ggaggtttga actttggccg atcttacttt 1260
gatccagcat attttagatt agggcaagag atggtaagga ggtcagctgg aaaggtcagt 1320
tccacattgg catctgaact cggtatcact gccgaggatg caaggcttgt ttcagagatt 1380
gcaatgcata ctactgagga caagatcagt agagcggttg gacccagaca agcccaagta 1440
tcatttctac acggtgatca aagtgagaat gagctaccga gattgggggg caaggaagat 1500
aggagggtca aacagagtcg aggagaagcc agggagagct acagagaaac cgggcccagc 1560
agagcaagtg atgcgagagc tgcccatctt ccaaccggca cacccctaga cattgacact 1620
gcaacggagt ccagccaaga tccgcaggac agtcgaaggt cagctgacgc cctgcttagg 1680
ctgcaagcca tggcaggaat ctcggaagaa caaggctcag acacggacac ccctatagtg 1740
tacaatgaca gaaatcttct agactaggtg cgagaggccg agggccagaa caacatccgc 1800
ctaccatcca tcattgttat aaaaaactta ggaaccaggt ccacacagcc gccagcccat 1860
caaccatcca ctcccacgat tggagccaat ggcagaagag caggcacgcc atgtcaaaaa 1920
cggactggaa tgcatccggg ctctcaaggc cgagcccatc ggctcactgg ccatcgagga 1980
agctatggca gcatggtcag aaatatcaga caacccagga caggagcgag ccacctgcag 2040
ggaagagaag gcaggcagtt cgggtctcag caaaccatgc ctctcagcaa ttggatcaac 2100
tgaaggcggt gcacctcgca tccgcggtca gggacctgga gagagcgatg acgacgctga 2160
aactttggga atccccccaa gaaatctcca ggcatcaagc actgggttac agtgttatta 2220
cgtttatgat cacagcggtg aagcggttaa gggaatccaa gatgctgact ctatcatggt 2280
tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac gatggtgagc 3540

aagggcgagg agctgttcac cggggtggtg cccatcctgg tcgagctgga cggcgacgta 3600

aacggccaca agttcagcgt gtccggcgag ggcgagggcg atgccaccta cggcaagctg 3660

accctgaagt tcatctgcac caccggcaag ctgcccgtgc cctggcccac cctcgtgacc 3720

accctgacct acggcgtgca gtgcttcagc cgctaccccg accacatgaa gcagcacgac 3780

ttcttcaagt ccgccatgcc cgaaggctac gtccaggagc gcaccatctt cttcaaggac 3840

gacggcaact acaagacccg cgccgaggtg aagttcgagg gcgacaccct ggtgaaccgc 3900

atcgagctga agggcatcga cttcaaggag gacggcaaca tcctggggca caagctggag 3960

tacaactaca acagccacaa cgtctatatc atggccgaca agcagaagaa cggcatcaag 4020

gtgaacttca agatccgcca caacatcgag gacggcagcg tgcagctcgc cgaccactac 4080

cagcagaaca cccccatcgg cgacggcccc gtgctgctgc ccgacaacca ctacctgagc 4140

acccagtccg ccctgagcaa agaccccaac gagaagcgcg atcacatggt cctgctggag 4200

ttcgtgaccg ccgccgggat cactctcggc atggacgagc tgtacaagta ggcgcgcagc 4260

gcttagacgt ctcgcgatcg atactagtac aacctaaatc cattataaaa aacttaggag 4320

caaagtgatt gcctcccaag gtccacaatg acagagacct acgacttcga caagtcggca 4380

tgggacatca aagggtcgat cgctccgata caacccacca cctacagtga tggcaggctg 4440

gtgccccagg tcagagtcat agatcctggt ctaggcgaca ggaaggatga atgctttatg 4500

tacatgtttc tgctgggggt tgttgaggac agcgattccc tagggcctcc aatcgggcga 4560

gcatttgggt tcctgccctt aggtgttggc agatccacag caaagcccga aaaactcctc 4620

aaagaggcca ctgagcttga catagttgtt agacgtacag cagggctcaa tgaaaaactg 4680

gtgttctaca acaacacccc actaactctc ctcacacctt ggagaaaggt cctaacaaca 4740

gggagtgtct tcaacgcaaa ccaagtgtgc aatgcggtta atctgatacc gctcgatacc 4800

ccgcagaggt tccgtgttgt ttatatgagc atcacccgtc tttcggataa cgggtattac 4860

accgttccta gaagaatgct ggaattcaga tcggtcaatg cagtggcctt caacctgctg 4920

gtgaccctta ggattgacaa ggcgataggc cctgggaaga tcatcgacaa tacagagcaa 4980

cttcctgagg caacatttat ggtccacatc gggaacttca ggagaaagaa gagtgaagtc 5040

tactctgccg attattgcaa aatgaaaatc gaaaagatgg gcctggtttt tgcacttggt 5100

gggatagggg gcaccagtct tcacattaga agcacaggca aaatgagcaa gactctccat 5160

gcacaactcg ggttcaagaa gaccttatgt tacccgctga tggatatcaa tgaagacctt 5220

aatcgattac tctggaggag cagatgcaag atagtaagaa tccaggcagt tttgcagcca 5280

tcagttcctc aagaattccg catttacgac gacgtgatca taaatgatga ccaaggacta 5340

ttcaaagttc tgtagaccgt agtgcccagc aatgcccgaa aacgaccccc ctcacaatga 5400

cagccagaag gcccggacaa aaaagccccc tccgaaagac tccacggacc aagcgagagg 5460

ccagccagca gccgacggca agcgcgaaca ccaggcggcc ccagcacaga acagccctga 5520

cacaaggcca ccaccagcca ccccaatctg catcctcctc gtgggacccc cgaggaccaa 5580

cccccaaggc tgcccccgat ccaaaccacc aaccgcatcc ccaccacccc cgggaaagaa 5640

acccccagca attggaaggc ccctccccct cttcctcaac acaagaactc cacaaccgaa 5700

ccgcacaagc gaccgaggtg acccaaccgc aggcatccga ctccctagac agatcctctc 5760

tccccggcaa actaaacaaa acttagggcc aaggaacata cacacccaac agaacccaga 5820

ccccggccca cggcgccgcg cccccaaccc ccgacaacca gagggagccc ccaaccaatc 5880

ccgccggctc ccccggtgcc cacaggcagg gacaccaacc cccgaacaga cccagcaccc 5940

aaccatcgac aatccaagac gggggggccc ccccaaaaaa aggcccccag gggccgacag 6000

ccagcaccgc gaggaagccc acccacccca cacacgacca cggcaaccaa accagaaccc 6060

agaccaccct gggccaccag ctcccagact cggccatcac cccgcagaaa ggaaaggcca 6120

caacccgcgc accccagccc cgatccggcg gggagccacc caacccgaac cagcacccaa 6180

gagcgatccc cgaaggaccc ccgaaccgca aaggacatca gtatcccaca gcctctccaa 6240

gtcccccggt ctcctcctct tctcgaaggg accaaaagat caatccacca cacccgacga 6300

cactcaactc cccaccccta aaggagacac cgggaatccc agaatcaaga ctcatccaat 6360

gtccatcatg ggtctcaagg tgaacgtctc tgccatattc atggcagtac tgttaactct 6420

ccaaacaccc accggtcaaa tccattgggg caatctctct aagatagggg tggtaggaat 6480

aggaagtgca agctacaaag ttatgactcg ttccagccat caatcattag tcataaaatt 6540

aatgcccaat ataactctcc tcaataactg cacgagggta gagattgcag aatacaggag 6600

actactgaga acagttttgg aaccaattag agatgcactt aatgcaatga cccagaatat 6660

aagaccggtt cagagtgtag cttcaagtag gagacacaag agatttgcgg gagtagtcct 6720

ggcaggtgcg gccctaggcg ttgccacagc tgctcagata acagccggca ttgcacttca 6780

ccagtccatg ctgaactctc aagccatcga caatctgaga gcgagcctgg aaactactaa 6840

tcaggcaatt gagacaatca gacaagcagg gcaggagatg atattggctg ttcagggtgt 6900

ccaagactac atcaataatg agctgatacc gtctatgaac caactatctt gtgatttaat 6960

cggccagaag ctcgggctca aattgctcag atactataca gaaatcctgt cattatttgg 7020

ccccagttta cggaccccca tatctgcgga gatatctatc caggctttga gctatgcgct 7080

tggaggagac atcaataagg tgttagaaaa gctcggatac agtggaggtg atttactggg 7140

catcttagag agcggaggaa taaaggcccg gataactcac gtcgacacag agtcctactt 7200

cattgtcctc agtatagcct atccgacgct gtccgagatt aagggggtga ttgtccaccg 7260

gctagagggg gtctcgtaca acataggctc tcaagagtgg tataccactg tgcccaagta 7320

tgttgcaacc caagggtacc ttatctcgaa ttttgatgag tcatcgtgta ctttcatgcc 7380

agaggggact gtgtgcagcc aaaatgcctt gtacccgatg agtcctctgc tccaagaatg 7440

cctccggggg tacaccaagt cctgtgctcg tacactcgta tccgggtctt ttgggaaccg 7500

gttcatttta tcacaaggga acctaatagc caattgtgca tcaatccttt gcaagtgtta 7560

cacaacagga acgatcatta atcaagaccc tgacaagatc ctaacataca ttgctgccga 7620

tcactgcccg gtagtcgagg tgaacggcgt gaccatccaa gtcgggagca ggaggtatcc 7680

agacgctgtg tacttgcaca gaattgacct cggtcctccc atatcattgg agaggttgga 7740

cgtagggaca aatctgggga atgcaattgc taagttggag gatgccaagg aattgttgga 7800

gtcatcggac cagatattga ggagtatgaa aggtttatcg agcactagca tagtctacat 7860

cctgattgca gtgtgtcttg gagggttgat agggatcccc gctttaatat gttgctgcag 7920

ggggcgttgt aacaaaaagg gagaacaagt tggtatgtca agaccaggcc taaagcctga 7980

tcttacggga acatcaaaat cctatgtaag gtcgctctga tcctctacaa ctcttgaaac 8040
acaaatgtcc cacaagtctc ctcttcgtca tcaagcaacc accgcaccca gcatcaagcc 8100
cacctgaaat tatctccggc ttccctctgg ccgaacaata tcggtagtta atcaaaactt 8160
agggtgcaag atcatccaca atgtcaccac aacgagaccg gataaatgcc ttctacaaag 8220
ataaccccca tcccaaggga agtaggatag tcattaacag agaacatctt atgattgata 8280
gaccttatgt tttgctggct gttctgtttg tcatgtttct gagcttgatc gggttgctag 8340
ccattgcagg cattagactt catcgggcag ccatctacac cgcagagatc cataaaagcc 8400
tcagcaccaa tctagatgta actaactcaa tcgagcatca ggtcaaggac gtgctgacac 8460
cactcttcaa aatcatcggt gatgaagtgg gcctgaggac acctcagaga ttcactgacc 8520
tagtgaaatt aatctctgac aagattaaat tccttaatcc ggatagggag tacgacttca 8580
gagatctcac ttggtgtatc aacccgccag agagaatcaa attggattat gatcaatact 8640
gtgcagatgt ggctgctgaa gagctcatga atgcattggt gaactcaact ctactggaga 8700
ccagaacaac caatcagttc ctagctgtct caaagggaaa ctgctcaggg cccactacaa 8760
tcagaggtca attctcaaac atgtcgctgt ccctgttaga cttgtattta ggtcgaggtt 8820
acaatgtgtc atctatagtc actatgacat cccagggaat gtatggggga acttacctag 8880
tggaaaagcc taatctgagc agcaaaaggt cagagttgtc acaactgagc atgtaccgag 8940
tgtttgaagt aggtgttatc agaaatccgg gtttgggggc tccggtgttc catatgacaa 9000
actatcttga gcaaccagtc agtaatgatc tcagcaactg tatggtggct ttgggggagc 9060
tcaaactcgc agccctttgt cacggggaag attctatcac aattccctat cagggatcag 9120
ggaaaggtgt cagcttccag ctcgtcaagc taggtgtctg gaaatcccca accgacatgc 9180
aatcctgggt ccccttatca acggatgatc cagtgataga caggctttac ctctcatctc 9240
acagaggtgt tatcgctgac aatcaagcaa aatgggctgt cccgacaaca cgaacagatg 9300
acaagttgcg aatggagaca tgcttccaac aggcgtgtaa gggtaaaatc caagcactct 9360
gcgagaatcc cgagtgggca ccattgaagg ataacaggat tccttcatac ggggtcttgt 9420
ctgttgatct gagtctgaca gttgagctta aaatcaaaat tgcttcggga ttcgggccat 9480
tgatcacaca cggttcaggg atggacctat acaaatccaa ccacaacaat gtgtattggc 9540
tgactatccc gccaatgaag aacctagcct taggtgtaat caacacattg gagtggatac 9600
cgagattcaa ggttagtccc tacctcttca ctgtcccaat taaggaagca ggcgaagact 9660
gccatgcccc aacataccta cctgcggagg tggatggtga tgtcaaactc agttccaatc 9720
tggtgattct acctggtcaa gatctccaat atgttttggc aacctacgat acttccaggg 9780
ttgaacatgc tgtggtttat tacgtttaca gcccaagccg ctcattttct tacttttatc 9840
cttttaggtt gcctataaag ggggtcccca tcgaattaca agtggaatgc ttcacatggg 9900

accaaaaact ctggtgccgt cacttctgtg tgcttgcgga ctcagaatct ggtggacata 9960

tcactcactc tgggatggtg ggcatgggag tcagctgcac agtcacccgg gaagatggaa 10020

ccaatcgcag atagggctgc tagtgaacca atcacatgat gtcacccaga catcaggcat 10080

acccactagt gtgaaataga catcagaatt aagaaaaacg tagggtccaa gtggttcccc 10140

gttatggact cgctatctgt caaccagatc ttataccctg aagttcacct agatagcccg 10200

atagttacca ataagatagt agccatcctg gagtatgctc gagtccctca cgcttacagc 10260

ctggaggacc ctacactgtg tcagaacatc aagcaccgcc taaaaaacgg attttccaac 10320

caaatgatta taaacaatgt ggaagttggg aatgtcatca agtccaagct taggagttat 10380

ccggcccact ctcatattcc atatccaaat tgtaatcagg atttatttaa catagaagac 10440

aaagagtcaa cgaggaagat ccgtgaactc ctcaaaaagg ggaattcgct gtactccaaa 10500

gtcagtgata aggttttcca atgcttaagg gacactaact cacggcttgg cctaggctcc 10560

gaattgaggg aggacatcaa ggagaaagtt attaacttgg gagtttacat gcacagctcc 10620

cagtggtttg agccctttct gttttggttt acagtcaaga ctgagatgag gtcagtgatt 10680

aaatcacaaa cccatacttg ccataggagg agacacacac ctgtattctt cactggtagt 10740

tcagttgagt tgctaatctc tcgtgacctt gttgctataa tcagtaaaga gtctcaacat 10800

gtatattacc tgacatttga actggttttg atgtattgtg atgtcataga ggggaggtta 10860

atgacagaga ccgctatgac tattgatgct aggtatacag agcttctagg aagagtcaga 10920

tacatgtgga aactgataga tggtttcttc cctgcactcg ggaatccaac ttatcaaatt 10980

gtagccatgc tggagcctct ttcacttgct tacctgcagc tgagggatat aacagtagaa 11040

ctcagaggtg ctttccttaa ccactgcttt actgaaatac atgatgttct tgaccaaaac 11100

gggttttctg atgaaggtac ttatcatgag ttaactgaag ctctagatta cattttcata 11160

actgatgaca tacatctgac aggggagatt ttctcatttt tcagaagttt cggccacccc 11220

agacttgaag cagtaacggc tgctgaaaat gttaggaaat acatgaatca gcctaaagtc 11280

attgtgtatg agactctgat gaaaggtcat gccatatttt gtggaatcat aatcaacggc 11340

tatcgtgaca ggcacggagg cagttggcca ccgctgaccc tccccctgca tgctgcagac 11400

acaatccgga atgctcaagc ttcaggtgaa gggttaacac atgagcagtg cgttgataac 11460

tggaaatctt ttgctggagt gaaatttggc tgctttatgc ctcttagcct ggatagtgat 11520

ctgacaatgt acctaaagga caaggcactt gctgctctcc aaagggaatg ggattcagtt 11580

tacccgaaag agttcctgcg ttacgaccct cccaagggaa ccgggtcacg gaggcttgta 11640

gatgttttcc ttaatgattc gagctttgac ccatatgatg tgataatgta tgttgtaagt 11700

ggagcttacc tccatgaccc tgagttcaac ctgtcttaca gcctgaaaga aaaggagatc 11760

aaggaaacag gtagactttt tgctaaaatg acttacaaaa tgagggcatg ccaagtgatt 11820

gctgaaaatc taatctcaaa cgggattggc aaatatttta aggacaatgg gatggccaag 11880

gatgagcacg atttgactaa ggcactccac actctagctg tctcaggagt ccccaaagat 11940

ctcaaagaaa gtcacagggg ggggccagtc ttaaaaacct actcccgaag cccagtccac 12000

acaagtacca ggaacgtgag agcagcaaaa gggtttatag ggttccctca agtaattcgg 12060

caggaccaag acactgatca tccggagaat atggaagctt acgagacagt cagtgcattt 12120

atcacgactg atctcaagaa gtactgcctt aattggagat atgagaccat cagcttgttt 12180

gcacagaggc taaatgagat ttacggattg ccctcatttt tccagtggct gcataagagg 12240

cttgagacct ctgtcctgta tgtaagtgac cctcattgcc cccccgacct tgacgcccat 12300

atcccgttat ataaagtccc caatgatcaa atcttcatta agtaccctat gggaggtata 12360

gaagggtatt gtcagaagct gtggaccatc agcaccattc cctatctata cctggctgct 12420

tatgagagcg gagtaaggat tgcttcgtta gtgcaagggg acaatcagac catagccgta 12480

acaaaaaggg tacccagcac atggccctac aaccttaaga aacgggaagc tgctagagta 12540

actagagatt actttgtaat tcttaggcaa aggctacatg atattggcca tcacctcaag 12600

gcaaatgaga caattgtttc atcacatttt tttgtctatt caaaaggaat atattatgat 12660

gggctacttg tgtcccaatc actcaagagc atcgcaagat gtgtattctg gtcagagact 12720

atagttgatg aaacaagggc agcatgcagt aatattgcta caacaatggc taaaagcatc 12780

gagagaggtt atgaccgtta ccttgcatat tccctgaacg tcctaaaagt gatacagcaa 12840

attctgatct ctcttggctt cacaatcaat tcaaccatga cccgggatgt agtcataccc 12900

ctcctcacaa acaacgacct cttaataagg atggcactgt tgcccgctcc tattgggggg 12960

atgaattatc tgaatatgag caggctgttt gtcagaaaca tcggtgatcc agtaacatca 13020

tcaattgctg atctcaagag aatgattctc gcctcactaa tgcctgaaga gaccctccat 13080

caagtaatga cacaacaacc gggggactct tcattcctag actgggctag cgacccttac 13140

tcagcaaatc ttgtatgtgt ccagagcatc actagactcc tcaagaacat aactgcaagg 13200

tttgtcctga tccatagtcc aaacccaatg ttaaaaggat tattccatga tgacagtaaa 13260

gaagaggacg agggactggc ggcattcctc atggacaggc atattatagt acctagggca 13320

gctcatgaaa tcctggatca tagtgtcaca ggggcaagag agtctattgc aggcatgctg 13380

gataccacaa aaggcttgat tcgagccagc atgaggaagg gggggttaac ctctcgagtg 13440

ataaccagat tgtccaatta tgactatgaa caattcagag cagggatggt gctattgaca 13500

ggaagaaaga gaaatgtcct cattgacaaa gagtcatgtt cagtgcagct ggcgagagct 13560

ctaagaagcc atatgtgggc gaggctagct cgaggacggc ctatttacgg ccttgaggtc 13620

cctgatgtac tagaatctat gcgaggccac cttattcggc gtcatgagac atgtgtcatc 13680

tgcgagtgtg gatcagtcaa ctacggatgg ttttttgtcc cctcgggttg ccaactggat 13740

gatattgaca aggaaacatc atccttgaga gtcccatata ttggttctac cactgatgag 13800

agaacagaca tgaagcttgc cttcgtaaga gccccaagtc gatccttgcg atctgctgtt 13860

agaatagcaa cagtgtactc atgggcttac ggtgatgatg atagctcttg gaacgaagcc 13920

tggttgttgg ctaggcaaag ggccaatgtg agcctggagg agctaagggt gatcactccc 13980

atctcaactt cgactaattt agcgcatagg ttgagggatc gtagcactca agtgaaatac 14040

tcaggtacat cccttgtccg agtggcgagg tataccacaa tctccaacga caatctctca 14100

tttgtcatat cagataagaa ggttgatact aactttatat accaacaagg aatgcttcta 14160

gggttgggtg ttttagaaac attgtttcga ctcgagaaag ataccggatc atctaacacg 14220

gtattacatc ttcacgtcga aacagattgt tgcgtgatcc cgatgataga tcatcccagg 14280

atacccagct cccgcaagct agagctgagg gcagagctat gtaccaaccc attgatatat 14340

gataatgcac ctttaattga cagagatgca acaaggctat acacccagag ccataggagg 14400

caccttgtgg aatttgttac atggtccaca ccccaactat atcacatttt agctaagtcc 14460

acagcactat ctatgattga cctggtaaca aaatttgaga aggaccatat gaatgaaatt 14520

tcagctctca taggggatga cgatatcaat agtttcataa ctgagtttct gctcatagag 14580

ccaagattat tcactatcta cttgggccag tgtgcggcca tcaattgggc atttgatgta 14640

cattatcata gaccatcagg gaaatatcag atgggtgagc tgttgtcatc gttcctttct 14700

agaatgagca aaggagtgtt taaggtgctt gtcaatgctc taagccaccc aaagatctac 14760

aagaaattct ggcattgtgg tattatagag cctatccatg gtccttcact tgatgctcaa 14820

aacttgcaca caactgtgtg caacatggtt tacacatgct atatgaccta cctcgacctg 14880

ttgttgaatg aagagttaga agagttcaca tttctcttgt gtgaaagcga cgaggatgta 14940

gtaccggaca gattcgacaa catccaggca aaacacttat gtgttctggc agatttgtac 15000

tgtcaaccag ggacctgccc accaattcga ggtctaagac cggtagagaa atgtgcagtt 15060

ctaaccgacc atatcaaggc agaggctatg ttatctccag caggatcttc gtggaacata 15120

aatccaatta ttgtagacca ttactcatgc tctctgactt atctccggcg aggatcgatc 15180

aaacagataa gattgagagt tgatccagga ttcattttcg acgccctcgc tgaggtaaat 15240

gtcagtcagc caaagatcgg cagcaacaac atctcaaata tgagcatcaa ggctttcaga 15300

cccccacacg atgatgttgc aaaattgctc aaagatatca acacaagcaa gcacaatctt 15360

cccatttcag ggggcaatct cgccaattat gaaatccatg ctttccgcag aatcgggttg 15420

aactcatctg cttgctacaa agctgttgag atatcaacat taattaggag atgccttgag 15480

ccaggggagg acggcttgtt cttgggtgag ggatcgggtt ctatgttgat cacttataaa 15540

gagatactta aactaaacaa gtgcttctat aatagtgggg tttccgccaa ttctagatct 15600

ggtcaaaggg aattagcacc ctatccctcc gaagttggcc ttgtcgaaca cagaatggga 15660

gtaggtaata ttgtcaaagt gctctttaac gggaggcccg aagtcacgtg ggtaggcagt 15720

gtagattgct tcaatttcat agttagtaat atccctacct ctagtgtggg gtttatccat 15780

tcagatatag agaccttgcc tgacaaagat actatagaga agctagagga attggcagcc 15840

atcttatcga tggctctgct cctgggcaaa ataggatcaa tactggtgat taagcttatg 15900

cctttcagcg gggattttgt tcagggattt ataagttatg tagggtctca ttatagagaa 15960

gtgaaccttg tataccctag atacagcaac ttcatctcta ctgaatctta tttggttatg 16020

acagatctca aggctaaccg gctaatgaat cctgaaaaga ttaagcagca gataattgaa 16080

tcatctgtga ggacttcacc tggacttata ggtcacatcc tatccattaa gcaactaagc 16140

tgcatacaag caattgtggg agacgcagtt agtagaggtg atatcaatcc tactctgaaa 16200

aaacttacac ctatagagca ggtgctgatc aattgcgggt tggcaattaa cggacctaag 16260

ctgtgcaaag aattgatcca ccatgatgtt gcctcagggc aagatggatt gcttaattct 16320

atactcatcc tctacaggga gttggcaaga ttcaaagaca accaaagaag tcaacaaggg 16380

atgttccacg cttaccccgt attggtaagt agcaggcaac gagaacttat atctaggatc 16440

acccgcaaat tctgggggca cattcttctt tactccggga acaaaaagtt gataaataag 16500

tttatccaga atctcaagtc cggctatctg atactagact tacaccagaa tatcttcgtt 16560

aagaatctat ccaagtcaga gaaacagatt attatgacgg ggggtttgaa acgtgagtgg 16620

gtttttaagg taacagtcaa ggagaccaaa gaatggtata agttagtcgg atacagtgcc 16680

ctgattaagg actaattggt tgaactccgg aaccctaatc ctgccctagg tggttaggca 16740

ttatttgcaa tatattaaag aaaactttga aaatacgaag tttctattcc cagctttgtc 16800

tggtggccgg catggtccca gcctcctcgc tggcgccggc tgggcaacat tccgagggga 16860

ccgtcccctc ggtaatggcg aatgggacgc ggccgatccg gctgctaaca aagcccgaaa 16920

ggaagctgag ttggctgctg ccaccgctga gcaataacta gcataacccc ttggggcctc 16980

taaacgggtc ttgaggggtt ttttgctgaa aggaggaact atatccggat gcggccgcca 17040

tgaacaataa aactgtctgc ttacataaac agtaatacaa ggggtgttat gagccatatt 17100

caacgggaaa cgtcgaggcc gcgattaaat tccaacatgg atgctgattt atatgggtat 17160

aaatgggctc gcgataatgt cgggcaatca ggtgcgacaa tctatcgctt gtatgggaag 17220

cccgatgcgc cagagttgtt tctgaaacat ggcaaaggta gcgttgccaa tgatgttaca 17280

gatgagatgg tcagactaaa ctggctgacg gaatttatgc ctcttccgac catcaagcat 17340

tttatccgta ctcctgatga tgcatggtta ctcaccactg cgatccccgg aaaaacagca 17400

ttccaggtat tagaagaata tcctgattca ggtgaaaata ttgttgatgc gctggcagtg 17460

ttcctgcgcc ggttgcattc gattcctgtt tgtaattgtc cttttaacag cgatcgtgta 17520

tttcgtctcg ctcaggcgca atcacgaatg aataacggtt tggttgatgc gagtgatttt 17580

gatgacgagc gtaatggctg gcctgttgaa caagtctgga aagaaatgca taaacttttg 17640

ccattctcac cggattcagt cgtcactcat ggtgatttct cacttgataa ccttattttt 17700

gacgagggga aattaatagg ttgtattgat gttggacgag tcggaatcgc agaccgatac 17760

caggatcttg ccatcctatg gaactgcctc ggtgagtttt ctccttcatt acagaaacgg 17820

ctttttcaaa aatatggtat tgataatcct gatatgaata aattgcagtt tcatttgatg 17880

ctcgatgagt ttttctaatc agaattggtt aattggttgt aacattattc agattgggcc 17940

ccgttccact gagcgtcaga ccccgtagaa aagatcaaag gatcttcttg agatcctttt 18000

tttctgcgcg taatctgctg cttgcaaaca aaaaaaccac cgctaccagc ggtggtttgt 18060

ttgccggatc aagagctacc aactcttttt ccgaaggtaa ctggcttcag cagagcgcag 18120

ataccaaata ctgttcttct agtgtagccg tagttaggcc accacttcaa gaactctgta 18180

gcaccgccta catacctcgc tctgctaatc ctgttaccag tggctgctgc cagtggcgat 18240

aagtcgtgtc ttaccgggtt ggactcaaga cgatagttac cggataaggc gcagcggtcg 18300

ggctgaacgg ggggttcgtg cacacagccc agcttggagc gaacgaccta caccgaactg 18360

agatacctac agcgtgagct atgagaaagc gccacgcttc ccgaagggag aaaggcggac 18420

aggtatccgg taagcggcag ggtcggaaca ggagagcgca cgagggagct tccaggggga 18480

aacgcctggt atctttatag tcctgtcggg tttcgccacc tctgacttga gcgtcgattt 18540

ttgtgatgct cgtcaggggg gcggagccta tggaaaaacg ccagcaacgc ggccttttta 18600

cggttcctgg ccttttgctg gccttttgct cacatgttct ttcctgcgtt atcccctgat 18660

tctgtggata accgtattac cgctag 18686

<210> 33
<211> 18686
<212> DNA
<213> Artificial sequence

<220>
<223> pKP-MVSchw-ATU3(eGFP)

<400> 33
gcgatcgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact ttaaggagc ttagcattgt tcaaagagaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca aggtccacaa tgacagagac ctacgacttc 3540

gacaagtcgg catgggacat caaagggtcg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgattc cctagggcct 3720

ccaatcgggc gagcatttgg gttcctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gacacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgagacaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagtt tacgggaccc catatctgcg gagatatcta tccaggcttt 6240

gagctatgcg cttggaggag acatcaataa ggtgttagaa aagctcggat acagtggagg 6300

tgatttactg ggcatcttag agagcggagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagacgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taatcaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttaatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt cactgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatcacatg atgtcaccca 9240

gacatcaggc atacccacta gtctaccctc catcattgtt ataaaaaact taggaaccag 9300

gtccacacag ccgccagccc atcaacgcgt acgatggtga gcaagggcga ggagctgttc 9360

accggggtgg tgcccatcct ggtcgagctg gacggcgacg taaacggcca caagttcagc 9420

gtgtccggcg agggcgaggg cgatgccacc tacggcaagc tgaccctgaa gttcatctgc 9480

accaccggca agctgcccgt gccctggccc accctcgtga ccaccctgac ctacggcgtg 9540

cagtgcttca gccgctaccc cgaccacatg aagcagcacg acttcttcaa gtccgccatg 9600

cccgaaggct acgtccagga gcgcaccatc ttcttcaagg acgacggcaa ctacaagacc 9660

cgcgccgagg tgaagttcga gggcgacacc ctggtgaacc gcatcgagct gaagggcatc 9720

gacttcaagg aggacggcaa catcctgggg cacaagctgg agtacaacta caacagccac 9780

aacgtctata tcatggccga caagcagaag aacggcatca aggtgaactt caagatccgc 9840

cacaacatcg aggacggcag cgtgcagctc gccgaccact accagcagaa cacccccatc 9900

ggcgacggcc ccgtgctgct gcccgacaac cactacctga gcacccagtc cgccctgagc 9960

aaagacccca acgagaagcg cgatcacatg gtcctgctgg agttcgtgac cgccgccggg 10020

atcactctcg gcatggacga gctgtacaag taggcgcgca gcgcttagac gtctcgcgat 10080

cgatgctagt gtgaaataga catcagaatt aagaaaaacg tagggtccaa gtggttcccc 10140

gttatggact cgctatctgt caaccagatc ttataccctg aagttcacct agatagcccg 10200

atagttacca ataagatagt agccatcctg gagtatgctc gagtccctca cgcttacagc 10260

ctggaggacc ctacactgtg tcagaacatc aagcaccgcc taaaaaacgg attttccaac 10320

caaatgatta taaacaatgt ggaagttggg aatgtcatca agtccaagct taggagttat 10380

ccggcccact ctcatattcc atatccaaat tgtaatcagg atttatttaa catagaagac 10440

aaagagtcaa cgaggaagat ccgtgaactc ctcaaaaagg ggaattcgct gtactccaaa 10500

gtcagtgata aggttttcca atgcttaagg gacactaact cacggcttgg cctaggctcc 10560

gaattgaggg aggacatcaa ggagaaagtt attaacttgg gagtttacat gcacagctcc 10620

cagtggtttg agccctttct gttttggttt acagtcaaga ctgagatgag gtcagtgatt 10680

aaatcacaaa cccatacttg ccataggagg agacacacac ctgtattctt cactggtagt 10740

tcagttgagt tgctaatctc tcgtgacctt gttgctataa tcagtaaaga gtctcaacat 10800

gtatattacc tgacatttga actggttttg atgtattgtg atgtcataga ggggaggtta 10860

atgacagaga ccgctatgac tattgatgct aggtatacag agcttctagg aagagtcaga 10920

tacatgtgga aactgataga tggtttcttc cctgcactcg ggaatccaac ttatcaaatt 10980

gtagccatgc tggagcctct ttcacttgct tacctgcagc tgagggatat aacagtagaa 11040

ctcagaggtg ctttccttaa ccactgcttt actgaaatac atgatgttct tgaccaaaac 11100

gggttttctg atgaaggtac ttatcatgag ttaactgaag ctctagatta cattttcata 11160

actgatgaca tacatctgac aggggagatt ttctcatttt tcagaagttt cggccacccc 11220

agacttgaag cagtaacggc tgctgaaaat gttaggaaat acatgaatca gcctaaagtc 11280

attgtgtatg agactctgat gaaaggtcat gccatatttt gtggaatcat aatcaacggc 11340

tatcgtgaca ggcacggagg cagttggcca ccgctgaccc tccccctgca tgctgcagac 11400

acaatccgga atgctcaagc ttcaggtgaa gggttaacac atgagcagtg cgttgataac 11460

tggaaatctt ttgctggagt gaaatttggc tgctttatgc ctcttagcct ggatagtgat 11520

ctgacaatgt acctaaagga caaggcactt gctgctctcc aaagggaatg ggattcagtt 11580

tacccgaaag agttcctgcg ttacgaccct cccaagggaa ccgggtcacg gaggcttgta 11640

gatgttttcc ttaatgattc gagctttgac ccatatgatg tgataatgta tgttgtaagt 11700
ggagcttacc tccatgaccc tgagttcaac ctgtcttaca gcctgaaaga aaaggagatc 11760
aaggaaacag gtagactttt tgctaaaatg acttacaaaa tgagggcatg ccaagtgatt 11820
gctgaaaatc taatctcaaa cgggattggc aaatatttta aggacaatgg gatggccaag 11880
gatgagcacg atttgactaa ggcactccac actctagctg tctcaggagt ccccaaagat 11940
ctcaaagaaa gtcacagggg ggggccagtc ttaaaaacct actcccgaag cccagtccac 12000
acaagtacca ggaacgtgag agcagcaaaa gggtttatag ggttccctca agtaattcgg 12060
caggaccaag acactgatca tccggagaat atggaagctt acgagacagt cagtgcattt 12120
atcacgactg atctcaagaa gtactgcctt aattggagat atgagaccat cagcttgttt 12180
gcacagaggc taaatgagat ttacggattg ccctcatttt tccagtggct gcataagagg 12240
cttgagacct ctgtcctgta tgtaagtgac cctcattgcc cccccgacct tgacgcccat 12300
atcccgttat ataaagtccc caatgatcaa atcttcatta agtaccctat gggaggtata 12360
gaagggtatt gtcagaagct gtggaccatc agcaccattc cctatctata cctggctgct 12420
tatgagagcg gagtaaggat tgcttcgtta gtgcaagggg acaatcagac catagccgta 12480
acaaaaaggg tacccagcac atggccctac aaccttaaga aacgggaagc tgctagagta 12540
actagagatt actttgtaat tcttaggcaa aggctacatg atattggcca tcacctcaag 12600
gcaaatgaga caattgtttc atcacatttt tttgtctatt caaaaggaat atattatgat 12660
gggctacttg tgtcccaatc actcaagagc atcgcaagat gtgtattctg gtcagagact 12720
atagttgatg aaacaagggc agcatgcagt aatattgcta caacaatggc taaaagcatc 12780
gagagaggtt atgaccgtta ccttgcatat tccctgaacg tcctaaaagt gatacagcaa 12840
attctgatct ctcttggctt cacaatcaat tcaaccatga cccgggatgt agtcataccc 12900
ctcctcacaa acaacgacct cttaataagg atggcactgt tgcccgctcc tattgggggg 12960
atgaattatc tgaatatgag caggctgttt gtcagaaaca tcggtgatcc agtaacatca 13020
tcaattgctg atctcaagag aatgattctc gcctcactaa tgcctgaaga gaccctccat 13080
caagtaatga cacaacaacc gggggactct tcattcctag actgggctag cgacccttac 13140
tcagcaaatc ttgtatgtgt ccagagcatc actagactcc tcaagaacat aactgcaagg 13200
tttgtcctga tccatagtcc aaacccaatg ttaaaaggat tattccatga tgacagtaaa 13260
gaagaggacg agggactggc ggcattcctc atggacaggc atattatagt acctagggca 13320
gctcatgaaa tcctggatca tagtgtcaca ggggcaagag agtctattgc aggcatgctg 13380
gataccacaa aaggcttgat tcgagccagc atgaggaagg gggggttaac ctctcgagtg 13440
ataaccagat tgtccaatta tgactatgaa caattcagag cagggatggt gctattgaca 13500
ggaagaaaga gaaatgtcct cattgacaaa gagtcatgtt cagtgcagct ggcgagagct 13560

ctaagaagcc atatgtgggc gaggctagct cgaggacggc ctatttacgg ccttgaggtc 13620

cctgatgtac tagaatctat gcgaggccac cttattcggc gtcatgagac atgtgtcatc 13680

tgcgagtgtg gatcagtcaa ctacggatgg ttttttgtcc cctcgggttg ccaactggat 13740

gatattgaca aggaaacatc atccttgaga gtcccatata ttggttctac cactgatgag 13800

agaacagaca tgaagcttgc cttcgtaaga gccccaagtc gatccttgcg atctgctgtt 13860

agaatagcaa cagtgtactc atgggcttac ggtgatgatg atagctcttg gaacgaagcc 13920

tggttgttgg ctaggcaaag ggccaatgtg agcctggagg agctaagggt gatcactccc 13980

atctcaactt cgactaattt agcgcatagg ttgagggatc gtagcactca agtgaaatac 14040

tcaggtacat cccttgtccg agtggcgagg tataccacaa tctccaacga caatctctca 14100

tttgtcatat cagataagaa ggttgatact aactttatat accaacaagg aatgcttcta 14160

gggttgggtg ttttagaaac attgtttcga ctcgagaaag ataccggatc atctaacacg 14220

gtattacatc ttcacgtcga aacagattgt tgcgtgatcc cgatgataga tcatcccagg 14280

atacccagct cccgcaagct agagctgagg gcagagctat gtaccaaccc attgatatat 14340

gataatgcac ctttaattga cagagatgca acaaggctat acacccagag ccataggagg 14400

caccttgtgg aatttgttac atggtccaca ccccaactat atcacatttt agctaagtcc 14460

acagcactat ctatgattga cctggtaaca aaatttgaga aggaccatat gaatgaaatt 14520

tcagctctca taggggatga cgatatcaat agtttcataa ctgagtttct gctcatagag 14580

ccaagattat tcactatcta cttgggccag tgtgcggcca tcaattgggc atttgatgta 14640

cattatcata gaccatcagg gaaatatcag atgggtgagc tgttgtcatc gttcctttct 14700

agaatgagca aaggagtgtt taaggtgctt gtcaatgctc taagccaccc aaagatctac 14760

aagaaattct ggcattgtgg tattatagag cctatccatg gtccttcact tgatgctcaa 14820

aacttgcaca caactgtgtg caacatggtt tacacatgct atatgaccta cctcgacctg 14880

ttgttgaatg aagagttaga agagttcaca tttctcttgt gtgaaagcga cgaggatgta 14940

gtaccggaca gattcgacaa catccaggca aaacacttat gtgttctggc agatttgtac 15000

tgtcaaccag ggacctgccc accaattcga ggtctaagac cggtagagaa atgtgcagtt 15060

ctaaccgacc atatcaaggc agaggctatg ttatctccag caggatcttc gtggaacata 15120

aatccaatta ttgtagacca ttactcatgc tctctgactt atctccggcg aggatcgatc 15180

aaacagataa gattgagagt tgatccagga ttcattttcg acgccctcgc tgaggtaaat 15240

gtcagtcagc caaagatcgg cagcaacaac atctcaaata tgagcatcaa ggctttcaga 15300

cccccacacg atgatgttgc aaaattgctc aaagatatca acacaagcaa gcacaatctt 15360

cccatttcag ggggcaatct cgccaattat gaaatccatg ctttccgcag aatcgggttg 15420

aactcatctg cttgctacaa agctgttgag atatcaacat taattaggag atgccttgag 15480

ccaggggagg acggcttgtt cttgggtgag ggatcgggtt ctatgttgat cacttataaa 15540

gagatactta aactaaacaa gtgcttctat aatagtgggg tttccgccaa ttctagatct 15600

ggtcaaaggg aattagcacc ctatccctcc gaagttggcc ttgtcgaaca cagaatggga 15660

gtaggtaata ttgtcaaagt gctctttaac gggaggcccg aagtcacgtg ggtaggcagt 15720

gtagattgct tcaatttcat agttagtaat atccctacct ctagtgtggg gtttatccat 15780

tcagatatag agaccttgcc tgacaaagat actatagaga agctagagga attggcagcc 15840

atcttatcga tggctctgct cctgggcaaa ataggatcaa tactggtgat taagcttatg 15900

cctttcagcg gggattttgt tcagggattt ataagttatg tagggtctca ttatagagaa 15960

gtgaaccttg tataccctag atacagcaac ttcatctcta ctgaatctta tttggttatg 16020

acagatctca aggctaaccg gctaatgaat cctgaaaaga ttaagcagca gataattgaa 16080

tcatctgtga ggacttcacc tggacttata ggtcacatcc tatccattaa gcaactaagc 16140

tgcatacaag caattgtggg agacgcagtt agtagaggtg atatcaatcc tactctgaaa 16200

aaacttacac ctatagagca ggtgctgatc aattgcgggt tggcaattaa cggacctaag 16260

ctgtgcaaag aattgatcca ccatgatgtt gcctcagggc aagatggatt gcttaattct 16320

atactcatcc tctacaggga gttggcaaga ttcaaagaca accaaagaag tcaacaaggg 16380

atgttccacg cttaccccgt attggtaagt agcaggcaac gagaacttat atctaggatc 16440

acccgcaaat tctgggggca cattcttctt tactccggga acaaaaagtt gataaataag 16500

tttatccaga atctcaagtc cggctatctg atactagact tacaccagaa tatcttcgtt 16560

aagaatctat ccaagtcaga gaaacagatt attatgacgg ggggtttgaa acgtgagtgg 16620

gtttttaagg taacagtcaa ggagaccaaa gaatggtata agttagtcgg atacagtgcc 16680

ctgattaagg actaattggt tgaactccgg aaccctaatc ctgccctagg tggttaggca 16740

ttatttgcaa tatattaaag aaaactttga aaatacgaag tttctattcc cagctttgtc 16800

tggtggccgg catggtccca gcctcctcgc tggcgccggc tgggcaacat tccgagggga 16860

ccgtcccctc ggtaatggcg aatgggacgc ggccgatccg gctgctaaca aagcccgaaa 16920

ggaagctgag ttggctgctg ccaccgctga gcaataacta gcataacccc ttggggcctc 16980

taaacgggtc ttgaggggtt ttttgctgaa aggaggaact atatccggat gcggccgcca 17040

tgaacaataa aactgtctgc ttacataaac agtaatacaa ggggtgttat gagccatatt 17100

caacgggaaa cgtcgaggcc gcgattaaat tccaacatgg atgctgattt atatgggtat 17160

aaatgggctc gcgataatgt cgggcaatca ggtgcgacaa tctatcgctt gtatgggaag 17220

cccgatgcgc cagagttgtt tctgaaacat ggcaaaggta gcgttgccaa tgatgttaca 17280

gatgagatgg tcagactaaa ctggctgacg gaatttatgc ctcttccgac catcaagcat 17340

tttatccgta ctcctgatga tgcatggtta ctcaccactg cgatccccgg aaaaacagca 17400

ttccaggtat tagaagaata tcctgattca ggtgaaaata ttgttgatgc gctggcagtg 17460

ttcctgcgcc ggttgcattc gattcctgtt tgtaattgtc cttttaacag cgatcgtgta 17520

tttcgtctcg ctcaggcgca atcacgaatg aataacggtt tggttgatgc gagtgatttt 17580

gatgacgagc gtaatggctg gcctgttgaa caagtctgga aagaaatgca taaacttttg 17640

ccattctcac cggattcagt cgtcactcat ggtgatttct cacttgataa ccttattttt 17700

gacgagggga aattaatagg ttgtattgat gttggacgag tcggaatcgc agaccgatac 17760

caggatcttg ccatcctatg gaactgcctc ggtgagtttt ctccttcatt acagaaacgg 17820

ctttttcaaa aatatggtat tgataatcct gatatgaata aattgcagtt tcatttgatg 17880

ctcgatgagt ttttctaatc agaattggtt aattggttgt aacattattc agattgggcc 17940

ccgttccact gagcgtcaga ccccgtagaa aagatcaaag gatcttcttg agatcctttt 18000

tttctgcgcg taatctgctg cttgcaaaca aaaaaaccac cgctaccagc ggtggtttgt 18060

ttgccggatc aagagctacc aactcttttt ccgaaggtaa ctggcttcag cagagcgcag 18120

ataccaaata ctgttcttct agtgtagccg tagttaggcc accacttcaa gaactctgta 18180

gcaccgccta catacctcgc tctgctaatc ctgttaccag tggctgctgc cagtggcgat 18240

aagtcgtgtc ttaccgggtt ggactcaaga cgatagttac cggataaggc gcagcggtcg 18300

ggctgaacgg ggggttcgtg cacacagccc agcttggagc gaacgaccta caccgaactg 18360

agatacctac agcgtgagct atgagaaagc gccacgcttc ccgaagggag aaaggcggac 18420

aggtatccgg taagcggcag ggtcggaaca ggagagcgca cgagggagct tccaggggga 18480

aacgcctggt atctttatag tcctgtcggg tttcgccacc tctgacttga gcgtcgattt 18540

ttgtgatgct cgtcaggggg gcggagccta tggaaaaacg ccagcaacgc ggccttttta 18600

cggttcctgg ccttttgctg gccttttgct cacatgttct ttcctgcgtt atcccctgat 18660

tctgtggata accgtattac cgctag 18686

<210> 34
<211> 21800
<212> DNA
<213> Artificial sequence

<220>
<223> pKM-ATU2-S_2019-nCoV (optimized sequence)

<400> 34
gcgatcgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagc ctaccctcca tcattgttat 3480

aaaaaactta ggaaccaggt ccacacagcc gccagcccat caacgcgtac ggccaccatg 3540

ttcgtgtttc tggtgctgct gccactggtg tccagtcagt gcgtgaacct gaccacacga 3600

acacagctgc caccagccta caccaatagc ttcacccgcg gagtgtacta ccccgacaag 3660

gtgttccgca gcagcgtgct gcatagcacc caggatctgt ttctgccctt cttcagcaac 3720

gtgacctggt tccacgccat ccacgtgtcc ggcaccaatg gcaccaagcg cttcgataat 3780

cccgtgctgc ccttcaacga tggcgtgtac tttgccagca ccgagaagtc caatatcatc 3840

cgcggctgga tcttcggcac cacactggat agcaagaccc agagcctgct gatcgtgaac 3900

aacgccacca acgtggtcat caaagtgtgc gagttccagt tctgcaacga ccccttcctg 3960

ggcgtctact accacaagaa caacaagagc tggatggaaa gcgagttccg cgtgtacagc 4020

agcgccaaca actgcacctt cgagtacgtg tcccagccat tcctgatgga cctggaaggc 4080

aagcagggca acttcaagaa cctgcgcgag ttcgtgttca agaacatcga cggctacttc 4140

aaaatctaca gcaagcacac cccaatcaac ctcgtgcgcg atctgccaca gggattcagt 4200

gctctggaac ccctggtgga tctgcccatc ggcatcaaca ttacccgctt tcagacactg 4260

ctggccctgc accgcagtta cttgacacca ggcgatagca gcagtggatg gacagctggt 4320

gccgccgctt actacgttgg atatctgcag ccacgcacct ttctgctgaa gtacaacgag 4380

aacggcacca tcaccgacgc cgtggattgt gctctcgatc ccctgagcga gacaaagtgc 4440

accctgaagt ccttcaccgt cgagaagggc atctaccaga ccagcaattt ccgcgtgcag 4500

cccaccgaga gcatcgtgcg cttccccaat atcaccaatc tgtgcccctt cggcgaggtg 4560

ttcaatgcca cacgctttgc ctccgtgtac gcctggaatc gcaagcgcat tagcaactgc 4620

gtggccgact actccgtgct gtacaatagc gccagcttca gcaccttcaa gtgctacggc 4680

gtgtcaccca ccaagctgaa cgacctgtgc ttcaccaatg tgtacgccga cagcttcgtg 4740

atccgcggag atgaagtgcg acagattgcc ccaggccaga ccggcaagat cgccgactac 4800

aattacaagc tgcccgacga cttcaccggc tgcgtgatcg cctggaacag caacaacctg 4860

gattccaaag tcggcggcaa ctacaactac ctgtaccgcc tgttccgcaa gagcaatctg 4920

aagcccttcg agcgcgacat cagcaccgaa atctaccagg ccggaagcac cccatgcaac 4980

ggcgtggaag gcttcaactg ctacttccca ctgcagtcct acggatttca gcccacaaat 5040

ggcgtgggct accagccata tcgagtggtg gtgctgagct tcgaactgct gcatgctcca 5100

gctaccgtgt gcggccccaa gaagagtacc aacctggtca agaacaaatg cgtgaacttc 5160

aacttcaacg gcctgaccgg aaccggcgtg ctgaccgaga gtaacaagaa gttcctgcca 5220

ttccagcagt ttggccgcga cattgccgat acaaccgatg ccgttcgcga tccccagacc 5280

ttggagatcc tggatattac cccatgctcc ttcggcggcg tgtccgtgat tacaccaggc 5340

accaatacca gcaaccaggt ggccgttctg taccaggatg tgaattgcac agaggtgccc 5400

gtggccattc acgccgatca attgacacca acatggcgcg tgtactccac cggcagcaat 5460

gtgtttcaaa cccgcgctgg atgcctgatt ggagccgagc acgtgaacaa tagctacgag 5520

tgcgatatcc ccatcggagc cggaatctgc gcctcctatc agacccagac caatagtcca 5580

cgacgagccc gaagtgtggc cagccagagc atcattgcct ataccatgag cctgggcgcc 5640

gagaatagcg tggcctactc caacaacagc attgctatcc ccaccaactt caccatcagc 5700

gtgaccaccg agatcctgcc agtgtccatg accaagacca gcgtggactg caccatgtac 5760

atctgcggag atagcaccga gtgcagcaac ctgctgctgc agtacggaag tttctgcacc 5820

cagctgaatc gcgccctgac aggcattgcc gtggaacagg ataagaacac ccaagaggtg 5880

ttcgcccaag tgaagcaaat ctacaagacc ccaccaatca aggatttcgg cggcttcaat 5940

ttcagccaga ttctgcccga tccaagcaag cccagcaagc gcagcttcat cgaggacctg 6000

ctgttcaaca aagtgacact ggccgacgcc ggattcatca agcagtatgg cgattgcctg 6060

ggcgatattg ccgcacgcga tctgatttgc gcccagaagt ttaacggact gaccgtcctg 6120

ccaccactgc tgacagatga gatgatcgcc cagtacacaa gtgccctgct ggccggaacc 6180

attaccagcg gatggacatt tggagccggt gccgctctgc agattccctt cgctatgcag 6240

atggcctacc gcttcaatgg cattggcgtg acccagaatg tgctgtacga gaaccagaag 6300

ctgatcgcca accagttcaa cagcgccatc ggcaagattc aggacagcct gagtagtacc 6360

gccagcgctc tgggaaagct gcaggatgtg gtcaaccaga acgctcaggc cctgaacacc 6420

ctggttaagc agctgagcag caacttcggc gccatcagta gcgtgctgaa cgatatcctg 6480

agccgcctgg ataaggtgga agccgaggtg cagatcgatc gcctgattac cggacgcctg 6540

cagtccctgc agacctatgt gacacagcag ctgatccgag ccgccgagat tcgagctagt 6600

gctaatctgg ccgccaccaa gatgagcgaa tgtgtgctgg gacagagcaa gcgcgtggac 6660

ttttgcggca agggatacca cctgatgagc ttcccacaga gtgctccaca cggcgtggtg 6720

tttctgcatg tgacctacgt gcccgctcaa gagaagaatt tcaccaccgc tccagccatc 6780

tgccacgacg gaaaggccca ttttccacgc gagggcgtgt tcgttagcaa cggcactcat 6840

tggttcgtca cccagcgcaa cttctacgag ccccagatca tcaccaccga caacaccttc 6900

gtcagcggca actgcgacgt cgtgatcggc attgtgaaca acaccgtgta cgatccactg 6960

cagcccgagc tggacagctt caaagaggaa ctggacaagt actttaagaa ccacacaagc 7020

cccgacgtgg acctgggaga cattagcgga atcaacgcca gcgtggtcaa catccagaaa 7080

gagattgacc gcctgaacga ggtggccaag aatctgaacg agagcctgat cgacctgcaa 7140

gaactgggca aatacgagca gtacattaag tggccctggt acatctggct gggcttcatt 7200

gccggactga ttgccatcgt gatggtcacc attatgctgt gctgcatgac cagttgctgc 7260

agctgcctga agggatgctg cagttgcgga agctgctgca agttcgacga ggatgatagc 7320

gagccagtgc tgaagggcgt caagctgcac tacacctgat aacgagcgcg cagcgcttag 7380

acgtctcgcg atcgatacta gtacaaccta aatccattat aaaaaactta ggagcaaagt 7440

gattgcctcc caaggtccac aatgacagag acctacgact tcgacaagtc ggcatgggac 7500

atcaaagggt cgatcgctcc gatacaaccc accacctaca gtgatggcag gctggtgccc 7560

caggtcagag tcatagatcc tggtctaggc gacaggaagg atgaatgctt tatgtacatg 7620

tttctgctgg gggttgttga ggacagcgat tccctagggc ctccaatcgg gcgagcattt 7680

gggttcctgc ccttaggtgt tggcagatcc acagcaaagc ccgaaaaact cctcaaagag 7740

gccactgagc ttgacatagt tgttagacgt acagcagggc tcaatgaaaa actggtgttc 7800

tacaacaaca ccccactaac tctcctcaca ccttggagaa aggtcctaac aacagggagt 7860

gtcttcaacg caaaccaagt gtgcaatgcg gttaatctga taccgctcga taccccgcag 7920

aggttccgtg ttgtttatat gagcatcacc cgtctttcgg ataacgggta ttacaccgtt 7980

cctagaagaa tgctggaatt cagatcggtc aatgcagtgg ccttcaacct gctggtgacc 8040

cttaggattg acaaggcgat aggccctggg aagatcatcg acaatacaga gcaacttcct 8100

gaggcaacat ttatggtcca catcgggaac ttcaggagaa agaagagtga agtctactct 8160

gccgattatt gcaaaatgaa aatcgaaaag atgggcctgg tttttgcact tggtgggata 8220

gggggcacca gtcttcacat tagaagcaca ggcaaaatga gcaagactct ccatgcacaa 8280

ctcgggttca agaagacctt atgttacccg ctgatggata tcaatgaaga ccttaatcga 8340

ttactctgga ggagcagatg caagatagta agaatccagg cagttttgca gccatcagtt 8400

cctcaagaat tccgcattta cgacgacgtg atcataaatg atgaccaagg actattcaaa 8460

gttctgtaga ccgtagtgcc cagcaatgcc cgaaaacgac ccccctcaca atgacagcca 8520

gaaggcccgg acaaaaaagc cccctccgaa agactccacg gaccaagcga gaggccagcc 8580

agcagccgac ggcaagcgcg aacaccaggc ggccccagca cagaacagcc ctgacacaag 8640

gccaccacca gccaccccaa tctgcatcct cctcgtggga cccccgagga ccaaccccca 8700

aggctgcccc cgatccaaac caccaaccgc atccccacca cccccgggaa agaaaccccc 8760

agcaattgga aggcccctcc ccctcttcct caacacaaga actccacaac cgaaccgcac 8820

aagcgaccga ggtgacccaa ccgcaggcat ccgactccct agacagatcc tctctccccg 8880

gcaaactaaa caaaacttag ggccaaggaa catacacacc caacagaacc cagaccccgg 8940

cccacggcgc cgcgccccca acccccgaca accagaggga gcccccaacc aatcccgccg 9000

gctcccccgg tgcccacagg cagggacacc aacccccgaa cagacccagc acccaaccat 9060

cgacaatcca agacgggggg gcccccccaa aaaaaggccc ccaggggccg acagccagca 9120

ccgcgaggaa gcccacccac cccacacacg accacggcaa ccaaaccaga acccagacca 9180

ccctgggcca ccagctccca gactcggcca tcaccccgca gaaaggaaag gccacaaccc 9240

gcgcacccca gccccgatcc ggcggggagc cacccaaccc gaaccagcac ccaagagcga 9300

tccccgaagg acccccgaac cgcaaaggac atcagtatcc cacagcctct ccaagtcccc 9360

cggtctcctc ctcttctcga agggaccaaa agatcaatcc accacacccg acgacactca 9420

actccccacc cctaaaggag acaccgggaa tcccagaatc aagactcatc caatgtccat 9480

catgggtctc aaggtgaacg tctctgccat attcatggca gtactgttaa ctctccaaac 9540

acccaccggt caaatccatt ggggcaatct ctctaagata ggggtggtag gaataggaag 9600

tgcaagctac aaagttatga ctcgttccag ccatcaatca ttagtcataa aattaatgcc 9660

caatataact ctcctcaata actgcacgag ggtagagatt gcagaataca ggagactact 9720

gagaacagtt ttggaaccaa ttagagatgc acttaatgca atgacccaga atataagacc 9780

ggttcagagt gtagcttcaa gtaggagaca caagagattt gcgggagtag tcctggcagg 9840

tgcggcccta ggcgttgcca cagctgctca gataacagcc ggcattgcac ttcaccagtc 9900

catgctgaac tctcaagcca tcgacaatct gagagcgagc ctggaaacta ctaatcaggc 9960

aattgagaca atcagacaag cagggcagga gatgatattg gctgttcagg gtgtccaaga 10020

ctacatcaat aatgagctga taccgtctat gaaccaacta tcttgtgatt taatcggcca 10080

gaagctcggg ctcaaattgc tcagatacta tacagaaatc ctgtcattat ttggccccag 10140

tttacgggac cccatatctg cggagatatc tatccaggct ttgagctatg cgcttggagg 10200

agacatcaat aaggtgttag aaaagctcgg atacagtgga ggtgatttac tgggcatctt 10260

agagagcgga ggaataaagg cccggataac tcacgtcgac acagagtcct acttcattgt 10320

cctcagtata gcctatccga cgctgtccga gattaagggg gtgattgtcc accggctaga 10380

gggggtctcg tacaacatag gctctcaaga gtggtatacc actgtgccca agtatgttgc 10440

aacccaaggg taccttatct cgaattttga tgagtcatcg tgtactttca tgccagaggg 10500

gactgtgtgc agccaaaatg ccttgtaccc gatgagtcct ctgctccaag aatgcctccg 10560

ggggtacacc aagtcctgtg ctcgtacact cgtatccggg tcttttggga accggttcat 10620

tttatcacaa gggaacctaa tagccaattg tgcatcaatc ctttgcaagt gttacacaac 10680

aggaacgatc attaatcaag accctgacaa gatcctaaca tacattgctg ccgatcactg 10740

cccggtagtc gaggtgaacg gcgtgaccat ccaagtcggg agcaggaggt atccagacgc 10800

tgtgtacttg cacagaattg acctcggtcc tcccatatca ttggagaggt tggacgtagg 10860

gacaaatctg gggaatgcaa ttgctaagtt ggaggatgcc aaggaattgt tggagtcatc 10920

ggaccagata ttgaggagta tgaaaggttt atcgagcact agcatagtct acatcctgat 10980

tgcagtgtgt cttggagggt tgatagggat ccccgcttta atatgttgct gcagggggcg 11040

ttgtaacaaa aagggagaac aagttggtat gtcaagacca ggcctaaagc ctgatcttac 11100

gggaacatca aaatcctatg taaggtcgct ctgatcctct acaactcttg aaacacaaat 11160

gtcccacaag tctcctcttc gtcatcaagc aaccaccgca cccagcatca agcccacctg 11220

aaattatctc cggcttccct ctggccgaac aatatcggta gttaatcaaa acttagggtg 11280

caagatcatc cacaatgtca ccacaacgag accggataaa tgccttctac aaagataacc 11340

cccatcccaa gggaagtagg atagtcatta acagagaaca tcttatgatt gatagacctt 11400

atgttttgct ggctgttctg tttgtcatgt ttctgagctt gatcgggttg ctagccattg 11460

caggcattag acttcatcgg gcagccatct acaccgcaga gatccataaa agcctcagca 11520

ccaatctaga tgtaactaac tcaatcgagc atcaggtcaa ggacgtgctg acaccactct 11580

tcaaaatcat cggtgatgaa gtgggcctga ggacacctca gagattcact gacctagtga 11640

aattaatctc tgacaagatt aaattcctta atccggatag ggagtacgac ttcagagatc 11700

tcacttggtg tatcaacccg ccagagagaa tcaaattgga ttatgatcaa tactgtgcag 11760

atgtggctgc tgaagagctc atgaatgcat tggtgaactc aactctactg gagaccagaa 11820

caaccaatca gttcctagct gtctcaaagg gaaactgctc agggcccact acaatcagag 11880

gtcaattctc aaacatgtcg ctgtccctgt tagacttgta tttaggtcga ggttacaatg 11940

tgtcatctat agtcactatg acatcccagg gaatgtatgg gggaacttac ctagtggaaa 12000

agcctaatct gagcagcaaa aggtcagagt tgtcacaact gagcatgtac cgagtgtttg 12060

aagtaggtgt tatcagaaat ccgggtttgg gggctccggt gttccatatg acaaactatc 12120

ttgagcaacc agtcagtaat gatctcagca actgtatggt ggctttgggg gagctcaaac 12180

tcgcagccct ttgtcacggg gaagattcta tcacaattcc ctatcaggga tcagggaaag 12240

gtgtcagctt ccagctcgtc aagctaggtg tctggaaatc cccaaccgac atgcaatcct 12300

gggtcccctt atcaacggat gatccagtga tagacaggct ttacctctca tctcacagag 12360

gtgttatcgc tgacaatcaa gcaaaatggg ctgtcccgac aacacgaaca gatgacaagt 12420

tgcgaatgga gacatgcttc caacaggcgt gtaagggtaa aatccaagca ctctgcgaga 12480

atcccgagtg ggcaccattg aaggataaca ggattccttc atacggggtc ttgtctgttg 12540

atctgagtct gacagttgag cttaaaatca aaattgcttc gggattcggg ccattgatca 12600

cacacggttc agggatggac ctatacaaat ccaaccacaa caatgtgtat tggctgacta 12660

tcccgccaat gaagaaccta gccttaggtg taatcaacac attggagtgg ataccgagat 12720

tcaaggttag tccctacctc ttcactgtcc caattaagga agcaggcgaa gactgccatg 12780

ccccaacata cctacctgcg gaggtggatg gtgatgtcaa actcagttcc aatctggtga 12840

ttctacctgg tcaagatctc caatatgttt tggcaaccta cgatacttcc agggttgaac 12900

atgctgtggt ttattacgtt tacagcccaa gccgctcatt ttcttacttt tatcctttta 12960

ggttgcctat aaagggggtc cccatcgaat tacaagtgga atgcttcaca tgggaccaaa 13020

aactctggtg ccgtcacttc tgtgtgcttg cggactcaga atctggtgga catatcactc 13080

actctgggat ggtgggcatg ggagtcagct gcacagtcac ccgggaagat ggaaccaatc 13140

gcagataggg ctgctagtga accaatcaca tgatgtcacc cagacatcag gcatacccac 13200

tagtgtgaaa tagacatcag aattaagaaa aacgtagggt ccaagtggtt ccccgttatg 13260

gactcgctat ctgtcaacca gatcttatac cctgaagttc acctagatag cccgatagtt 13320

accaataaga tagtagccat cctggagtat gctcgagtcc ctcacgctta cagcctggag 13380

gaccctacac tgtgtcagaa catcaagcac cgcctaaaaa acggattttc caaccaaatg 13440

attataaaca atgtggaagt tgggaatgtc atcaagtcca agcttaggag ttatccggcc 13500

cactctcata ttccatatcc aaattgtaat caggatttat ttaacataga agacaaagag 13560

tcaacgagga agatccgtga actcctcaaa aaggggaatt cgctgtactc caaagtcagt 13620

gataaggttt tccaatgctt aagggacact aactcacggc ttggcctagg ctccgaattg 13680

agggaggaca tcaaggagaa agttattaac ttgggagttt acatgcacag ctcccagtgg 13740

tttgagccct ttctgttttg gtttacagtc aagactgaga tgaggtcagt gattaaatca 13800

caaacccata cttgccatag gaggagacac acacctgtat tcttcactgg tagttcagtt 13860

gagttgctaa tctctcgtga ccttgttgct ataatcagta aagagtctca acatgtatat 13920

tacctgacat ttgaactggt tttgatgtat tgtgatgtca tagaggggag gttaatgaca 13980

gagaccgcta tgactattga tgctaggtat acagagcttc taggaagagt cagatacatg 14040

tggaaactga tagatggttt cttccctgca ctcgggaatc caacttatca aattgtagcc 14100

atgctggagc ctctttcact tgcttacctg cagctgaggg atataacagt agaactcaga 14160

ggtgctttcc ttaaccactg ctttactgaa atacatgatg ttcttgacca aaacgggttt 14220

tctgatgaag gtacttatca tgagttaact gaagctctag attacatttt cataactgat 14280

gacatacatc tgacagggga gattttctca tttttcagaa gtttcggcca ccccagactt 14340

gaagcagtaa cggctgctga aaatgttagg aaatacatga atcagcctaa agtcattgtg 14400

tatgagactc tgatgaaagg tcatgccata ttttgtggaa tcataatcaa cggctatcgt 14460

gacaggcacg gaggcagttg gccaccgctg accctccccc tgcatgctgc agacacaatc 14520

cggaatgctc aagcttcagg tgaagggtta acacatgagc agtgcgttga taactggaaa 14580

tcttttgctg gagtgaaatt tggctgcttt atgcctctta gcctggatag tgatctgaca 14640

atgtacctaa aggacaaggc acttgctgct ctccaaaggg aatgggattc agtttacccg 14700

aaagagttcc tgcgttacga ccctcccaag ggaaccgggt cacggaggct tgtagatgtt 14760

ttccttaatg attcgagctt tgacccatat gatgtgataa tgtatgttgt aagtggagct 14820

tacctccatg accctgagtt caacctgtct tacagcctga aagaaaagga gatcaaggaa 14880

acaggtagac tttttgctaa aatgacttac aaaatgaggg catgccaagt gattgctgaa 14940

aatctaatct caaacgggat tggcaaatat tttaaggaca atgggatggc caaggatgag 15000

cacgatttga ctaaggcact ccacactcta gctgtctcag gagtccccaa agatctcaaa 15060

gaaagtcaca ggggggggcc agtcttaaaa acctactccc gaagcccagt ccacacaagt 15120

accaggaacg tgagagcagc aaaagggttt atagggttcc ctcaagtaat tcggcaggac 15180

caagacactg atcatccgga gaatatggaa gcttacgaga cagtcagtgc atttatcacg 15240

actgatctca agaagtactg ccttaattgg agatatgaga ccatcagctt gtttgcacag 15300

aggctaaatg agatttacgg attgccctca tttttccagt ggctgcataa gaggcttgag 15360

acctctgtcc tgtatgtaag tgaccctcat tgcccccccg accttgacgc ccatatcccg 15420

ttatataaag tccccaatga tcaaatcttc attaagtacc ctatgggagg tatagaaggg 15480

tattgtcaga agctgtggac catcagcacc attccctatc tatacctggc tgcttatgag 15540

agcggagtaa ggattgcttc gttagtgcaa ggggacaatc agaccatagc cgtaacaaaa 15600

agggtaccca gcacatggcc ctacaacctt aagaaacggg aagctgctag agtaactaga 15660

gattactttg taattcttag gcaaaggcta catgatattg gccatcacct caaggcaaat 15720

gagacaattg tttcatcaca tttttttgtc tattcaaaag gaatatatta tgatgggcta 15780

cttgtgtccc aatcactcaa gagcatcgca agatgtgtat tctggtcaga gactatagtt 15840

gatgaaacaa gggcagcatg cagtaatatt gctacaacaa tggctaaaag catcgagaga 15900

ggttatgacc gttaccttgc atattccctg aacgtcctaa aagtgataca gcaaattctg 15960

atctctcttg gcttcacaat caattcaacc atgacccggg atgtagtcat acccctcctc 16020

acaaacaacg acctcttaat aaggatggca ctgttgcccg ctcctattgg ggggatgaat 16080

tatctgaata tgagcaggct gtttgtcaga aacatcggtg atccagtaac atcatcaatt 16140

gctgatctca agagaatgat tctcgcctca ctaatgcctg aagagaccct ccatcaagta 16200

atgacacaac aaccggggga ctcttcattc ctagactggg ctagcgaccc ttactcagca 16260

aatcttgtat gtgtccagag catcactaga ctcctcaaga acataactgc aaggtttgtc 16320

ctgatccata gtccaaaccc aatgttaaaa ggattattcc atgatgacag taaagaagag 16380

gacgagggac tggcggcatt cctcatggac aggcatatta tagtacctag ggcagctcat 16440

gaaatcctgg atcatagtgt cacaggggca agagagtcta ttgcaggcat gctggatacc 16500

acaaaaggct tgattcgagc cagcatgagg aagggggggt taacctctcg agtgataacc 16560

agattgtcca attatgacta tgaacaattc agagcaggga tggtgctatt gacaggaaga 16620

aagagaaatg tcctcattga caaagagtca tgttcagtgc agctggcgag agctctaaga 16680

agccatatgt gggcgaggct agctcgagga cggcctattt acggccttga ggtccctgat 16740

gtactagaat ctatgcgagg ccaccttatt cggcgtcatg agacatgtgt catctgcgag 16800

tgtggatcag tcaactacgg atggtttttt gtcccctcgg gttgccaact ggatgatatt 16860

gacaaggaaa catcatcctt gagagtccca tatattggtt ctaccactga tgagagaaca 16920

gacatgaagc ttgccttcgt aagagcccca agtcgatcct tgcgatctgc tgttagaata 16980

gcaacagtgt actcatgggc ttacggtgat gatgatagct cttggaacga agcctggttg 17040

ttggctaggc aaagggccaa tgtgagcctg gaggagctaa gggtgatcac tcccatctca 17100

acttcgacta atttagcgca taggttgagg gatcgtagca ctcaagtgaa atactcaggt 17160

acatcccttg tccgagtggc gaggtatacc acaatctcca acgacaatct ctcatttgtc 17220

atatcagata agaaggttga tactaacttt atataccaac aaggaatgct tctagggttg 17280

ggtgttttag aaacattgtt tcgactcgag aaagataccg gatcatctaa cacggtatta 17340

catcttcacg tcgaaacaga ttgttgcgtg atcccgatga tagatcatcc caggataccc 17400

agctcccgca agctagagct gagggcagag ctatgtacca acccattgat atatgataat 17460

gcacctttaa ttgacagaga tgcaacaagg ctatacaccc agagccatag gaggcacctt 17520

gtggaatttg ttacatggtc cacaccccaa ctatatcaca ttttagctaa gtccacagca 17580

ctatctatga ttgacctggt aacaaaattt gagaaggacc atatgaatga aatttcagct 17640

ctcatagggg atgacgatat caatagtttc ataactgagt ttctgctcat agagccaaga 17700

ttattcacta tctacttggg ccagtgtgcg gccatcaatt gggcatttga tgtacattat 17760

catagaccat cagggaaata tcagatgggt gagctgttgt catcgttcct ttctagaatg 17820

agcaaaggag tgtttaaggt gcttgtcaat gctctaagcc acccaaagat ctacaagaaa 17880

ttctggcatt gtggtattat agagcctatc catggtcctt cacttgatgc tcaaaacttg 17940

cacacaactg tgtgcaacat ggtttacaca tgctatatga cctacctcga cctgttgttg 18000

aatgaagagt tagaagagtt cacatttctc ttgtgtgaaa gcgacgagga tgtagtaccg 18060

gacagattcg acaacatcca ggcaaaacac ttatgtgttc tggcagattt gtactgtcaa 18120

ccagggacct gcccaccaat tcgaggtcta agaccggtag agaaatgtgc agttctaacc 18180

gaccatatca aggcagaggc tatgttatct ccagcaggat cttcgtggaa cataaatcca 18240

attattgtag accattactc atgctctctg acttatctcc ggcgaggatc gatcaaacag 18300

ataagattga gagttgatcc aggattcatt ttcgacgccc tcgctgaggt aaatgtcagt 18360

cagccaaaga tcggcagcaa caacatctca aatatgagca tcaaggcttt cagaccccca 18420

cacgatgatg ttgcaaaatt gctcaaagat atcaacacaa gcaagcacaa tcttcccatt 18480

tcagggggca atctcgccaa ttatgaaatc catgctttcc gcagaatcgg gttgaactca 18540

tctgcttgct acaaagctgt tgagatatca acattaatta ggagatgcct tgagccaggg 18600

gaggacggct tgttcttggg tgagggatcg ggttctatgt tgatcactta taaagagata 18660

cttaaactaa acaagtgctt ctataatagt ggggtttccg ccaattctag atctggtcaa 18720

agggaattag caccctatcc ctccgaagtt ggccttgtcg aacacagaat gggagtaggt 18780

aatattgtca aagtgctctt taacgggagg cccgaagtca cgtgggtagg cagtgtagat 18840

tgcttcaatt tcatagttag taatatccct acctctagtg tggggtttat ccattcagat 18900

atagagacct tgcctgacaa agatactata gagaagctag aggaattggc agccatctta 18960

tcgatggctc tgctcctggg caaaatagga tcaatactgg tgattaagct tatgcctttc 19020

agcggggatt ttgttcaggg atttataagt tatgtagggt ctcattatag agaagtgaac 19080

cttgtatacc ctagatacag caacttcatc tctactgaat cttatttggt tatgacagat 19140

ctcaaggcta accggctaat gaatcctgaa aagattaagc agcagataat tgaatcatct 19200

gtgaggactt cacctggact tataggtcac atcctatcca ttaagcaact aagctgcata 19260

caagcaattg tgggagacgc agttagtaga ggtgatatca atcctactct gaaaaaactt 19320

acacctatag agcaggtgct gatcaattgc gggttggcaa ttaacggacc taagctgtgc 19380

aaagaattga tccaccatga tgttgcctca gggcaagatg gattgcttaa ttctatactc 19440

atcctctaca gggagttggc aagattcaaa gacaaccaaa gaagtcaaca agggatgttc 19500

cacgcttacc ccgtattggt aagtagcagg caacgagaac ttatatctag gatcacccgc 19560

aaattctggg ggcacattct tctttactcc gggaacaaaa agttgataaa taagtttatc 19620

cagaatctca agtccggcta tctgatacta gacttacacc agaatatctt cgttaagaat 19680

ctatccaagt cagagaaaca gattattatg acggggggtt tgaaacgtga gtgggttttt 19740

aaggtaacag tcaaggagac caaagaatgg tataagttag tcggatacag tgccctgatt 19800

aaggactaat tggttgaact ccggaaccct aatcctgccc taggtggtta ggcattattt 19860

gcaatatatt aaagaaaact ttgaaaatac gaagtttcta ttcccagctt tgtctggtgg 19920

ccggcatggt cccagcctcc tcgctggcgc cggctgggca acattccgag gggaccgtcc 19980

cctcggtaat ggcgaatggg acgcggccga tccggctgct aacaaagccc gaaaggaagc 20040

tgagttggct gctgccaccg ctgagcaata actagcataa ccccttgggg cctctaaacg 20100

ggtcttgagg ggttttttgc tgaaaggagg aactatatcc ggatgcggcc gccatgaaca 20160

ataaaactgt ctgcttacat aaacagtaat acaaggggtg ttatgagcca tattcaacgg 20220

gaaacgtcga ggccgcgatt aaattccaac atggatgctg atttatatgg gtataaatgg 20280

gctcgcgata atgtcgggca atcaggtgcg acaatctatc gcttgtatgg gaagcccgat 20340

gcgccagagt tgtttctgaa acatggcaaa ggtagcgttg ccaatgatgt tacagatgag 20400

atggtcagac taaactggct gacggaattt atgcctcttc cgaccatcaa gcattttatc 20460

cgtactcctg atgatgcatg gttactcacc actgcgatcc ccggaaaaac agcattccag 20520

gtattagaag aatatcctga ttcaggtgaa aatattgttg atgcgctggc agtgttcctg 20580

cgccggttgc attcgattcc tgtttgtaat tgtcctttta acagcgatcg tgtatttcgt 20640

ctcgctcagg cgcaatcacg aatgaataac ggtttggttg atgcgagtga ttttgatgac 20700

gagcgtaatg gctggcctgt tgaacaagtc tggaaagaaa tgcataaact tttgccattc 20760

tcaccggatt cagtcgtcac tcatggtgat ttctcacttg ataaccttat ttttgacgag 20820

gggaaattaa taggttgtat tgatgttgga cgagtcggaa tcgcagaccg ataccaggat 20880

cttgccatcc tatggaactg cctcggtgag ttttctcctt cattacagaa acggcttttt 20940

caaaaatatg gtattgataa tcctgatatg aataaattgc agtttcattt gatgctcgat 21000

gagtttttct aatcagaatt ggttaattgg ttgtaacatt attcagattg ggccccgttc 21060

cactgagcgt cagaccccgt agaaaagatc aaaggatctt cttgagatcc tttttttctg 21120

cgcgtaatct gctgcttgca aacaaaaaaa ccaccgctac cagcggtggt ttgtttgccg 21180

gatcaagagc taccaactct ttttccgaag gtaactggct tcagcagagc gcagatacca 21240

aatactgttc ttctagtgta gccgtagtta ggccaccact tcaagaactc tgtagcaccg 21300

cctacatacc tcgctctgct aatcctgtta ccagtggctg ctgccagtgg cgataagtcg 21360

tgtcttaccg ggttggactc aagacgatag ttaccggata aggcgcagcg gtcgggctga 21420

acggggggtt cgtgcacaca gcccagcttg gagcgaacga cctacaccga actgagatac 21480

ctacagcgtg agctatgaga aagcgccacg cttcccgaag ggagaaaggc ggacaggtat 21540

ccggtaagcg gcagggtcgg aacaggagag cgcacgaggg agcttccagg gggaaacgcc 21600

tggtatcttt atagtcctgt cgggtttcgc cacctctgac ttgagcgtcg atttttgtga 21660

tgctcgtcag gggggcggag cctatggaaa aacgccagca acgcggcctt tttacggttc 21720

ctggcctttt gctggccttt tgctcacatg ttctttcctg cgttatcccc tgattctgtg 21780

gataaccgta ttaccgctag 21800

<210> 35
<211> 21800
<212> DNA
<213> Artificial sequence

<220>
<223> pKM-ATU3-S_2019-nCoV (optimized sequence)

<400> 35
gcgatcgcta atacgactca ctatagggcc aactttgttt ggtctgatga gtccgtgagg 60

acgaaacccg gagtcccggg tcaccaaaca aagttgggta aggatagttc aatcaatgat 120

catcttctag tgcacttagg attcaagatc ctattatcag ggacaagagc aggattaggg 180

atatccgaga tggccacact tttaaggagc ttagcattgt tcaaaagaaa caaggacaaa 240

ccacccatta catcaggatc cggtggagcc atcagaggaa tcaaacacat tattatagta 300

ccaatccctg gagattcctc aattaccact cgatccagac ttctggaccg gttggtgagg 360

ttaattggaa acccggatgt gagcgggccc aaactaacag gggcactaat aggtatatta 420

tccttatttg tggagtctcc aggtcaattg attcagagga tcaccgatga ccctgacgtt 480

agcataaggc tgttagaggt tgtccagagt gaccagtcac aatctggcct taccttcgca 540

tcaagaggta ccaacatgga ggatgaggcg gaccaatact tttcacatga tgatccaatt 600

agtagtgatc aatccaggtt cggatggttc gggaacaagg aaatctcaga tattgaagtg 660

caagaccctg agggattcaa catgattctg ggtaccatcc tagcccaaat ttgggtcttg 720

ctcgcaaagg cggttacggc cccagacacg gcagctgatt cggagctaag aaggtggata 780

aagtacaccc aacaaagaag ggtagttggt gaatttagat tggagagaaa atggttggat 840

gtggtgagga acaggattgc cgaggacctc tccttacgcc gattcatggt cgctctaatc 900

ctggatatca agagaacacc cggaaacaaa cccaggattg ctgaaatgat atgtgacatt 960

gatacatata tcgtagaggc aggattagcc agttttatcc tgactattaa gtttgggata 1020

gaaactatgt atcctgctct tggactgcat gaatttgctg gtgagttatc cacacttgag 1080

tccttgatga acctttacca gcaaatgggg gaaactgcac cctacatggt aatcctggag 1140

aactcaattc agaacaagtt cagtgcagga tcataccctc tgctctggag ctatgccatg 1200

ggagtaggag tggaacttga aaactccatg ggaggtttga actttggccg atcttacttt 1260

gatccagcat attttagatt agggcaagag atggtaagga ggtcagctgg aaaggtcagt 1320

tccacattgg catctgaact cggtatcact gccgaggatg caaggcttgt ttcagagatt 1380

gcaatgcata ctactgagga caagatcagt agagcggttg gacccagaca agcccaagta 1440

tcatttctac acggtgatca aagtgagaat gagctaccga gattgggggg caaggaagat 1500

aggagggtca aacagagtcg aggagaagcc agggagagct acagagaaac cgggcccagc 1560

agagcaagtg atgcgagagc tgcccatctt ccaaccggca cacccctaga cattgacact 1620

gcaacggagt ccagccaaga tccgcaggac agtcgaaggt cagctgacgc cctgcttagg 1680

ctgcaagcca tggcaggaat ctcggaagaa caaggctcag acacggacac ccctatagtg 1740

tacaatgaca gaaatcttct agactaggtg cgagaggccg agggccagaa caacatccgc 1800

ctaccatcca tcattgttat aaaaaactta ggaaccaggt ccacacagcc gccagcccat 1860

caaccatcca ctcccacgat tggagccaat ggcagaagag caggcacgcc atgtcaaaaa 1920

cggactggaa tgcatccggg ctctcaaggc cgagcccatc ggctcactgg ccatcgagga 1980

agctatggca gcatggtcag aaatatcaga caacccagga caggagcgag ccacctgcag 2040

ggaagagaag gcaggcagtt cgggtctcag caaaccatgc ctctcagcaa ttggatcaac 2100

tgaaggcggt gcacctcgca tccgcggtca gggacctgga gagagcgatg acgacgctga 2160

aactttggga atccccccaa gaaatctcca ggcatcaagc actgggttac agtgttatta 2220

cgtttatgat cacagcggtg aagcggttaa gggaatccaa gatgctgact ctatcatggt 2280

tcaatcaggc cttgatggtg atagcaccct ctcaggagga gacaatgaat ctgaaaacag 2340

cgatgtggat attggcgaac ctgataccga gggatatgct atcactgacc ggggatctgc 2400

tcccatctct atggggttca gggcttctga tgttgaaact gcagaaggag gggagatcca 2460

cgagctcctg agactccaat ccagaggcaa caactttccg aagcttggga aaactctcaa 2520

tgttcctccg cccccggacc ccggtagggc cagcacttcc gggacaccca ttaaaaaggg 2580

cacagacgcg agattagcct catttggaac ggagatcgcg tctttattga caggtggtgc 2640

aacccaatgt gctcgaaagt caccctcgga accatcaggg ccaggtgcac ctgcggggaa 2700

tgtccccgag tgtgtgagca atgccgcact gatacaggag tggacacccg aatctggtac 2760

cacaatctcc ccgagatccc agaataatga agaaggggga gactattatg atgatgagct 2820

gttctctgat gtccaagata ttaaaacagc cttggccaaa atacacgagg ataatcagaa 2880

gataatctcc aagctagaat cactgctgtt attgaaggga gaagttgagt caattaagaa 2940

gcagatcaac aggcaaaata tcagcatatc caccctggaa ggacacctct caagcatcat 3000

gatcgccatt cctggacttg ggaaggatcc caacgacccc actgcagatg tcgaaatcaa 3060

tcccgacttg aaacccatca taggcagaga ttcaggccga gcactggccg aagttctcaa 3120

gaaacccgtt gccagccgac aactccaagg aatgacaaat ggacggacca gttccagagg 3180

acagctgctg aaggaatttc agctaaagcc gatcgggaaa aagatgagct cagccgtcgg 3240

gtttgttcct gacaccggcc ctgcatcacg cagtgtaatc cgctccatta taaaatccag 3300

ccggctagag gaggatcgga agcgttacct gatgactctc cttgatgata tcaaaggagc 3360

caatgatctt gccaagttcc accagatgct gatgaagata ataatgaagt agctacagct 3420

caacttacct gccaacccca tgccagtcga cccaactagt acaacctaaa tccattataa 3480

aaaacttagg agcaaagtga ttgcctccca aggtccacaa tgacagagac ctacgacttc 3540

gacaagtcgg catgggacat caaagggtcg atcgctccga tacaacccac cacctacagt 3600

gatggcaggc tggtgcccca ggtcagagtc atagatcctg gtctaggcga caggaaggat 3660

gaatgcttta tgtacatgtt tctgctgggg gttgttgagg acagcgattc cctagggcct 3720

ccaatcgggc gagcatttgg gttcctgccc ttaggtgttg gcagatccac agcaaagccc 3780

gaaaaactcc tcaaagaggc cactgagctt gacatagttg ttagacgtac agcagggctc 3840

aatgaaaaac tggtgttcta caacaacacc ccactaactc tcctcacacc ttggagaaag 3900

gtcctaacaa cagggagtgt cttcaacgca aaccaagtgt gcaatgcggt taatctgata 3960

ccgctcgata ccccgcagag gttccgtgtt gtttatatga gcatcacccg tctttcggat 4020

aacgggtatt acaccgttcc tagaagaatg ctggaattca gatcggtcaa tgcagtggcc 4080

ttcaacctgc tggtgaccct taggattgac aaggcgatag gccctgggaa gatcatcgac 4140

aatacagagc aacttcctga ggcaacattt atggtccaca tcgggaactt caggagaaag 4200

aagagtgaag tctactctgc cgattattgc aaaatgaaaa tcgaaaagat gggcctggtt 4260

tttgcacttg gtgggatagg gggcaccagt cttcacatta gaagcacagg caaaatgagc 4320

aagactctcc atgcacaact cgggttcaag aagaccttat gttacccgct gatggatatc 4380

aatgaagacc ttaatcgatt actctggagg agcagatgca agatagtaag aatccaggca 4440

gttttgcagc catcagttcc tcaagaattc cgcatttacg acgacgtgat cataaatgat 4500

gaccaaggac tattcaaagt tctgtagacc gtagtgccca gcaatgcccg aaaacgaccc 4560

ccctcacaat gacagccaga aggcccggac aaaaaagccc cctccgaaag actccacgga 4620

ccaagcgaga ggccagccag cagccgacgg caagcgcgaa caccaggcgg ccccagcaca 4680

gaacagccct gacacaaggc caccaccagc caccccaatc tgcatcctcc tcgtgggacc 4740

cccgaggacc aacccccaag gctgcccccg atccaaacca ccaaccgcat ccccaccacc 4800

cccgggaaag aaacccccag caattggaag gcccctcccc ctcttcctca acacaagaac 4860

tccacaaccg aaccgcacaa gcgaccgagg tgacccaacc gcaggcatcc gactccctag 4920

acagatcctc tctccccggc aaactaaaca aaacttaggg ccaaggaaca tacacaccca 4980

acagaaccca gaccccggcc cacggcgccg cgcccccaac ccccgacaac cagagggagc 5040

ccccaaccaa tcccgccggc tcccccggtg cccacaggca gggacaccaa cccccgaaca 5100

gacccagcac ccaaccatcg acaatccaag acgggggggc ccccccaaaa aaaggccccc 5160

aggggccgac agccagcacc gcgaggaagc ccacccaccc cacacacgac cacggcaacc 5220

aaaccagaac ccagaccacc ctgggccacc agctcccaga ctcggccatc accccgcaga 5280

aaggaaaggc cacaacccgc gcaccccagc cccgatccgg cggggagcca cccaacccga 5340

accagcaccc aagagcgatc cccgaaggac ccccgaaccg caaaggacat cagtatccca 5400

cagcctctcc aagtcccccg gtctcctcct cttctcgaag ggaccaaaag atcaatccac 5460

cacacccgac gacactcaac tccccacccc taaaggagac accgggaatc ccagaatcaa 5520

gactcatcca atgtccatca tgggtctcaa ggtgaacgtc tctgccatat tcatggcagt 5580

actgttaact ctccaaacac ccaccggtca aatccattgg ggcaatctct ctaagatagg 5640

ggtggtagga ataggaagtg caagctacaa agttatgact cgttccagcc atcaatcatt 5700

agtcataaaa ttaatgccca atataactct cctcaataac tgcacgaggg tagagattgc 5760

agaatacagg agactactga gaacagtttt ggaaccaatt agagatgcac ttaatgcaat 5820

gacccagaat ataagaccgg ttcagagtgt agcttcaagt aggagacaca agagatttgc 5880

gggagtagtc ctggcaggtg cggccctagg cgttgccaca gctgctcaga taacagccgg 5940

cattgcactt caccagtcca tgctgaactc tcaagccatc gacaatctga gagcgagcct 6000

ggaaactact aatcaggcaa ttgagacaat cagacaagca gggcaggaga tgatattggc 6060

tgttcagggt gtccaagact acatcaataa tgagctgata ccgtctatga accaactatc 6120

ttgtgattta atcggccaga agctcgggct caaattgctc agatactata cagaaatcct 6180

gtcattattt ggccccagtt tacgggaccc catatctgcg gagatatcta tccaggcttt 6240

gagctatgcg cttggaggag acatcaataa ggtgttagaa aagctcggat acagtggagg 6300

tgatttactg ggcatcttag agagcggagg aataaaggcc cggataactc acgtcgacac 6360

agagtcctac ttcattgtcc tcagtatagc ctatccgacg ctgtccgaga ttaagggggt 6420

gattgtccac cggctagagg gggtctcgta caacataggc tctcaagagt ggtataccac 6480

tgtgcccaag tatgttgcaa cccaagggta ccttatctcg aattttgatg agtcatcgtg 6540

tactttcatg ccagagggga ctgtgtgcag ccaaaatgcc ttgtacccga tgagtcctct 6600

gctccaagaa tgcctccggg ggtacaccaa gtcctgtgct cgtacactcg tatccgggtc 6660

ttttgggaac cggttcattt tatcacaagg gaacctaata gccaattgtg catcaatcct 6720

ttgcaagtgt tacacaacag gaacgatcat taatcaagac cctgacaaga tcctaacata 6780

cattgctgcc gatcactgcc cggtagtcga ggtgaacggc gtgaccatcc aagtcgggag 6840

caggaggtat ccagacgctg tgtacttgca cagaattgac ctcggtcctc ccatatcatt 6900

ggagaggttg gacgtaggga caaatctggg gaatgcaatt gctaagttgg aggatgccaa 6960

ggaattgttg gagtcatcgg accagatatt gaggagtatg aaaggtttat cgagcactag 7020

catagtctac atcctgattg cagtgtgtct tggagggttg atagggatcc ccgctttaat 7080

atgttgctgc agggggcgtt gtaacaaaaa gggagaacaa gttggtatgt caagaccagg 7140

cctaaagcct gatcttacgg gaacatcaaa atcctatgta aggtcgctct gatcctctac 7200

aactcttgaa acacaaatgt cccacaagtc tcctcttcgt catcaagcaa ccaccgcacc 7260

cagcatcaag cccacctgaa attatctccg gcttccctct ggccgaacaa tatcggtagt 7320

taatcaaaac ttagggtgca agatcatcca caatgtcacc acaacgagac cggataaatg 7380

ccttctacaa agataacccc catcccaagg gaagtaggat agtcattaac agagaacatc 7440

ttatgattga tagaccttat gttttgctgg ctgttctgtt tgtcatgttt ctgagcttga 7500

tcgggttgct agccattgca ggcattagac ttcatcgggc agccatctac accgcagaga 7560

tccataaaag cctcagcacc aatctagatg taactaactc aatcgagcat caggtcaagg 7620

acgtgctgac accactcttc aaaatcatcg gtgatgaagt gggcctgagg acacctcaga 7680

gattcactga cctagtgaaa ttaatctctg acaagattaa attccttaat ccggataggg 7740

agtacgactt cagagatctc acttggtgta tcaacccgcc agagagaatc aaattggatt 7800

atgatcaata ctgtgcagat gtggctgctg aagagctcat gaatgcattg gtgaactcaa 7860

ctctactgga gaccagaaca accaatcagt tcctagctgt ctcaaaggga aactgctcag 7920

ggcccactac aatcagaggt caattctcaa acatgtcgct gtccctgtta gacttgtatt 7980

taggtcgagg ttacaatgtg tcatctatag tcactatgac atcccaggga atgtatgggg 8040

gaacttacct agtggaaaag cctaatctga gcagcaaaag gtcagagttg tcacaactga 8100

gcatgtaccg agtgtttgaa gtaggtgtta tcagaaatcc gggtttgggg gctccggtgt 8160

tccatatgac aaactatctt gagcaaccag tcagtaatga tctcagcaac tgtatggtgg 8220

ctttggggga gctcaaactc gcagcccttt gtcacgggga agattctatc acaattccct 8280

atcagggatc agggaaaggt gtcagcttcc agctcgtcaa gctaggtgtc tggaaatccc 8340

caaccgacat gcaatcctgg gtccccttat caacggatga tccagtgata gacaggcttt 8400

acctctcatc tcacagaggt gttatcgctg acaatcaagc aaaatgggct gtcccgacaa 8460

cacgaacaga tgacaagttg cgaatggaga catgcttcca acaggcgtgt aagggtaaaa 8520

tccaagcact ctgcgagaat cccgagtggg caccattgaa ggataacagg attccttcat 8580

acggggtctt gtctgttgat ctgagtctga cagttgagct taaaatcaaa attgcttcgg 8640

gattcgggcc attgatcaca cacggttcag ggatggacct atacaaatcc aaccacaaca 8700

atgtgtattg gctgactatc ccgccaatga agaacctagc cttaggtgta atcaacacat 8760

tggagtggat accgagattc aaggttagtc cctacctctt cactgtccca attaaggaag 8820

caggcgaaga ctgccatgcc ccaacatacc tacctgcgga ggtggatggt gatgtcaaac 8880

tcagttccaa tctggtgatt ctacctggtc aagatctcca atatgttttg gcaacctacg 8940

atacttccag ggttgaacat gctgtggttt attacgttta cagcccaagc cgctcatttt 9000

cttactttta tccttttagg ttgcctataa agggggtccc catcgaatta caagtggaat 9060

gcttcacatg ggaccaaaaa ctctggtgcc gtcacttctg tgtgcttgcg gactcagaat 9120

ctggtggaca tatcactcac tctgggatgg tgggcatggg agtcagctgc acagtcaccc 9180

gggaagatgg aaccaatcgc agatagggct gctagtgaac caatcacatg atgtcaccca 9240

gacatcaggc atacccacta gtctaccctc catcattgtt ataaaaaact taggaaccag 9300

gtccacacag ccgccagccc atcaacgcgt acggccacca tgttcgtgtt tctggtgctg 9360

ctgccactgg tgtccagtca gtgcgtgaac ctgaccacac gaacacagct gccaccagcc 9420

tacaccaata gcttcacccg cggagtgtac taccccgaca aggtgttccg cagcagcgtg 9480

ctgcatagca cccaggatct gtttctgccc ttcttcagca acgtgacctg gttccacgcc 9540

atccacgtgt ccggcaccaa tggcaccaag cgcttcgata atcccgtgct gcccttcaac 9600

gatggcgtgt actttgccag caccgagaag tccaatatca tccgcggctg gatcttcggc 9660

accacactgg atagcaagac ccagagcctg ctgatcgtga acaacgccac caacgtggtc 9720

atcaaagtgt gcgagttcca gttctgcaac gaccccttcc tgggcgtcta ctaccacaag 9780

aacaacaaga gctggatgga aagcgagttc cgcgtgtaca gcagcgccaa caactgcacc 9840

ttcgagtacg tgtcccagcc attcctgatg gacctggaag gcaagcaggg caacttcaag 9900

aacctgcgcg agttcgtgtt caagaacatc gacggctact tcaaaatcta cagcaagcac 9960

accccaatca acctcgtgcg cgatctgcca cagggattca gtgctctgga acccctggtg 10020

gatctgccca tcggcatcaa cattacccgc tttcagacac tgctggccct gcaccgcagt 10080

tacttgacac caggcgatag cagcagtgga tggacagctg gtgccgccgc ttactacgtt 10140

ggatatctgc agccacgcac ctttctgctg aagtacaacg agaacggcac catcaccgac 10200

gccgtggatt gtgctctcga tcccctgagc gagacaaagt gcaccctgaa gtccttcacc 10260

gtcgagaagg gcatctacca gaccagcaat ttccgcgtgc agcccaccga gagcatcgtg 10320

cgcttcccca atatcaccaa tctgtgcccc ttcggcgagg tgttcaatgc cacacgcttt 10380

gcctccgtgt acgcctggaa tcgcaagcgc attagcaact gcgtggccga ctactccgtg 10440

ctgtacaata gcgccagctt cagcaccttc aagtgctacg gcgtgtcacc caccaagctg 10500

aacgacctgt gcttcaccaa tgtgtacgcc gacagcttcg tgatccgcgg agatgaagtg 10560

cgacagattg ccccaggcca gaccggcaag atcgccgact acaattacaa gctgcccgac 10620

gacttcaccg gctgcgtgat cgcctggaac agcaacaacc tggattccaa agtcggcggc 10680

aactacaact acctgtaccg cctgttccgc aagagcaatc tgaagccctt cgagcgcgac 10740

atcagcaccg aaatctacca ggccggaagc accccatgca acggcgtgga aggcttcaac 10800

tgctacttcc cactgcagtc ctacggattt cagcccacaa atggcgtggg ctaccagcca 10860

tatcgagtgg tggtgctgag cttcgaactg ctgcatgctc cagctaccgt gtgcggcccc 10920

aagaagagta ccaacctggt caagaacaaa tgcgtgaact tcaacttcaa cggcctgacc 10980

ggaaccggcg tgctgaccga gagtaacaag aagttcctgc cattccagca gtttggccgc 11040

gacattgccg atacaaccga tgccgttcgc gatccccaga ccttggagat cctggatatt 11100

accccatgct ccttcggcgg cgtgtccgtg attacaccag gcaccaatac cagcaaccag 11160

gtggccgttc tgtaccagga tgtgaattgc acagaggtgc ccgtggccat tcacgccgat 11220

caattgacac caacatggcg cgtgtactcc accggcagca atgtgtttca aacccgcgct 11280

ggatgcctga ttggagccga gcacgtgaac aatagctacg agtgcgatat ccccatcgga 11340

gccggaatct gcgcctccta tcagacccag accaatagtc cacgacgagc ccgaagtgtg 11400

gccagccaga gcatcattgc ctataccatg agcctgggcg ccgagaatag cgtggcctac 11460

tccaacaaca gcattgctat ccccaccaac ttcaccatca gcgtgaccac cgagatcctg 11520

ccagtgtcca tgaccaagac cagcgtggac tgcaccatgt acatctgcgg agatagcacc 11580

gagtgcagca acctgctgct gcagtacgga agtttctgca cccagctgaa tcgcgccctg 11640

acaggcattg ccgtggaaca ggataagaac acccaagagg tgttcgccca agtgaagcaa 11700

atctacaaga ccccaccaat caaggatttc ggcggcttca atttcagcca gattctgccc 11760

gatccaagca agcccagcaa gcgcagcttc atcgaggacc tgctgttcaa caaagtgaca 11820

ctggccgacg ccggattcat caagcagtat ggcgattgcc tgggcgatat tgccgcacgc 11880

gatctgattt gcgcccagaa gtttaacgga ctgaccgtcc tgccaccact gctgacagat 11940

gagatgatcg cccagtacac aagtgccctg ctggccggaa ccattaccag cggatggaca 12000

tttggagccg gtgccgctct gcagattccc ttcgctatgc agatggccta ccgcttcaat 12060

ggcattggcg tgacccagaa tgtgctgtac gagaaccaga agctgatcgc caaccagttc 12120

aacagcgcca tcggcaagat tcaggacagc ctgagtagta ccgccagcgc tctgggaaag 12180

ctgcaggatg tggtcaacca gaacgctcag gccctgaaca ccctggttaa gcagctgagc 12240

agcaacttcg gcgccatcag tagcgtgctg aacgatatcc tgagccgcct ggataaggtg 12300

gaagccgagg tgcagatcga tcgcctgatt accggacgcc tgcagtccct gcagacctat 12360

gtgacacagc agctgatccg agccgccgag attcgagcta gtgctaatct ggccgccacc 12420

aagatgagcg aatgtgtgct gggacagagc aagcgcgtgg acttttgcgg caagggatac 12480

cacctgatga gcttcccaca gagtgctcca cacggcgtgg tgtttctgca tgtgacctac 12540

gtgcccgctc aagagaagaa tttcaccacc gctccagcca tctgccacga cggaaaggcc 12600

cattttccac gcgagggcgt gttcgttagc aacggcactc attggttcgt cacccagcgc 12660

aacttctacg agccccagat catcaccacc gacaacacct tcgtcagcgg caactgcgac 12720

gtcgtgatcg gcattgtgaa caacaccgtg tacgatccac tgcagcccga gctggacagc 12780

ttcaaagagg aactggacaa gtactttaag aaccacacaa gccccgacgt ggacctggga 12840

gacattagcg gaatcaacgc cagcgtggtc aacatccaga aagagattga ccgcctgaac 12900

gaggtggcca agaatctgaa cgagagcctg atcgacctgc aagaactggg caaatacgag 12960

cagtacatta agtggccctg gtacatctgg ctgggcttca ttgccggact gattgccatc 13020

gtgatggtca ccattatgct gtgctgcatg accagttgct gcagctgcct gaagggatgc 13080

tgcagttgcg gaagctgctg caagttcgac gaggatgata gcgagccagt gctgaagggc 13140

gtcaagctgc actacacctg ataacgagcg cgcagcgctt agacgtctcg cgatcgatgc 13200

tagtgtgaaa tagacatcag aattaagaaa aacgtagggt ccaagtggtt ccccgttatg 13260

gactcgctat ctgtcaacca gatcttatac cctgaagttc acctagatag cccgatagtt 13320

accaataaga tagtagccat cctggagtat gctcgagtcc ctcacgctta cagcctggag 13380

gaccctacac tgtgtcagaa catcaagcac cgcctaaaaa acggattttc caaccaaatg 13440

attataaaca atgtggaagt tgggaatgtc atcaagtcca agcttaggag ttatccggcc 13500

cactctcata ttccatatcc aaattgtaat caggatttat ttaacataga agacaaagag 13560

tcaacgagga agatccgtga actcctcaaa aaggggaatt cgctgtactc caaagtcagt 13620

gataaggttt tccaatgctt aagggacact aactcacggc ttggcctagg ctccgaattg 13680

agggaggaca tcaaggagaa agttattaac ttgggagttt acatgcacag ctcccagtgg 13740

tttgagccct ttctgttttg gtttacagtc aagactgaga tgaggtcagt gattaaatca 13800

caaacccata cttgccatag gaggagacac acacctgtat tcttcactgg tagttcagtt 13860

gagttgctaa tctctcgtga ccttgttgct ataatcagta aagagtctca acatgtatat 13920

tacctgacat ttgaactggt tttgatgtat tgtgatgtca tagaggggag gttaatgaca 13980

gagaccgcta tgactattga tgctaggtat acagagcttc taggaagagt cagatacatg 14040

tggaaactga tagatggttt cttccctgca ctcgggaatc caacttatca aattgtagcc 14100

atgctggagc ctctttcact tgcttacctg cagctgaggg atataacagt agaactcaga 14160

ggtgctttcc ttaaccactg ctttactgaa atacatgatg ttcttgacca aaacgggttt 14220

tctgatgaag gtacttatca tgagttaact gaagctctag attacatttt cataactgat 14280

gacatacatc tgacagggga gattttctca tttttcagaa gtttcggcca ccccagactt 14340

gaagcagtaa cggctgctga aaatgttagg aaatacatga atcagcctaa agtcattgtg 14400

tatgagactc tgatgaaagg tcatgccata ttttgtggaa tcataatcaa cggctatcgt 14460

gacaggcacg gaggcagttg gccaccgctg accctccccc tgcatgctgc agacacaatc 14520

cggaatgctc aagcttcagg tgaaggttta acacatgagc agtgcgttga taactggaaa 14580

tcttttgctg gagtgaaatt tggctgcttt atgcctctta gcctggatag tgatctgaca 14640

atgtacctaa aggacaaggc acttgctgct ctccaaaggg aatgggattc agtttacccg 14700

aaagagttcc tgcgttacga ccctcccaag ggaaccgggt cacggaggct tgtagatgtt 14760

ttccttaatg attcgagctt tgacccatat gatgtgataa tgtatgttgt aagtggagct 14820

tacctccatg accctgagtt caacctgtct tacagcctga aagaaaagga gatcaaggaa 14880

acaggtagac tttttgctaa aatgacttac aaaatgaggg catgccaagt gattgctgaa 14940

aatctaatct caaacgggat tggcaaatat tttaaggaca atgggatggc caaggatgag 15000

cacgatttga ctaaggcact ccacactcta gctgtctcag gagtccccaa agatctcaaa 15060

gaaagtcaca ggggggggcc agtcttaaaa acctactccc gaagcccagt ccacacaagt 15120

accaggaacg tgagagcagc aaaagggttt atagggttcc ctcaagtaat tcggcaggac 15180

caagacactg atcatccgga gaatatggaa gcttacgaga cagtcagtgc atttatcacg 15240

actgatctca agaagtactg ccttaattgg agatatgaga ccatcagctt gtttgcacag 15300

aggctaaatg agatttacgg attgccctca tttttccagt ggctgcataa gaggcttgag 15360

acctctgtcc tgtatgtaag tgaccctcat tgcccccccg accttgacgc ccatatcccg 15420

ttatataaag tccccaatga tcaaatcttc attaagtacc ctatggaggg tatagaaggg 15480

tattgtcaga agctgtggac catcagcacc attccctatc tatacctggc tgcttatgag 15540

agcggagtaa ggattgcttc gttagtgcaa ggggacaatc agaccatagc cgtaacaaaa 15600

agggtaccca gcacatggcc ctacaacctt aagaaacggg aagctgctag agtaactaga 15660

gattactttg taattcttag gcaaaggcta catgatattg gccatcacct caaggcaaat 15720

gagacaattg tttcatcaca tttttttgtc tattcaaaag gaatatatta tgatgggcta 15780

cttgtgtccc aatcactcaa gagcatcgca agatgtgtat tctggtcaga gactatagtt 15840

gatgaaacaa gggcagcatg cagtaatatt gctacaacaa tggctaaaag catcgagaga 15900

ggttatgacc gttaccttgc atattccctg aacgtcctaa aagtgataca gcaaattctg 15960

atctctcttg gcttcacaat caattcaacc atgacccggg atgtagtcat acccctcctc 16020

acaaacaacg acctcttaat aaggatggca ctgttgcccg ctcctattgg ggggatgaat 16080

tatctgaata tgagcaggct gtttgtcaga aacatcggtg atccagtaac atcatcaatt 16140

gctgatctca agagaatgat tctcgcctca ctaatgcctg aagagaccct ccatcaagta 16200

atgacacaac aaccggggga ctcttcattc ctagactggg ctagcgaccc ttactcagca 16260

aatcttgtat gtgtccagag catcactaga ctcctcaaga acataactgc aaggtttgtc 16320

ctgatccata gtccaaaccc aatgttaaaa ggattattcc atgatgacag taaagaagag 16380

gacgagggac tggcggcatt cctcatggac aggcatatta tagtacctag ggcagctcat 16440

gaaatcctgg atcatagtgt cacaggggca agagagtcta ttgcaggcat gctggatacc 16500

acaaaaggct tgattcgagc cagcatgagg aagggggggt taacctctcg agtgataacc 16560

agattgtcca attatgacta tgaacaattc agagcaggga tggtgctatt gacaggaaga 16620

aagagaaatg tcctcattga caaagagtca tgttcagtgc agctggcgag agctctaaga 16680

agccatatgt gggcgaggct agctcgagga cggcctattt acggccttga ggtccctgat 16740

gtactagaat ctatgcgagg ccaccttatt cggcgtcatg agacatgtgt catctgcgag 16800

tgtggatcag tcaactacgg atggtttttt gtcccctcgg gttgccaact ggatgatatt 16860

gacaaggaaa catcatcctt gagagtccca tatattggtt ctaccactga tgagagaaca 16920

gacatgaagc ttgccttcgt aagagcccca agtcgatcct tgcgatctgc tgttagaata 16980

gcaacagtgt actcatgggc ttacggtgat gatgatagct cttggaacga agcctggttg 17040

ttggctaggc aaagggccaa tgtgagcctg gaggagctaa gggtgatcac tcccatctca 17100

acttcgacta atttagcgca taggttgagg gatcgtagca ctcaagtgaa atactcaggt 17160

acatcccttg tccgagtggc gaggtatacc acaatctcca acgacaatct ctcatttgtc 17220

atatcagata agaaggttga tactaacttt atataccaac aaggaatgct tctagggttg 17280

ggtgttttag aaacattgtt tcgactcgag aaagataccg gatcatctaa cacggtatta 17340

catcttcacg tcgaaacaga ttgttgcgtg atcccgatga tagatcatcc caggataccc 17400

agctcccgca agctagagct gagggcagag ctatgtacca acccattgat atatgataat 17460

gcacctttaa ttgacagaga tgcaacaagg ctatacaccc agagccatag gaggcacctt 17520

gtggaatttg ttacatggtc cacaccccaa ctatatcaca ttttagctaa gtccacagca 17580

ctatctatga ttgacctggt aacaaaattt gagaaggacc atatgaatga aatttcagct 17640

ctcatagggg atgacgatat caatagtttc ataactgagt ttctgctcat agagccaaga 17700

ttattcacta tctacttggg ccagtgtgcg gccatcaatt gggcatttga tgtacattat 17760

catagaccat cagggaaata tcagatgggt gagctgttgt catcgttcct ttctagaatg 17820

agcaaaggag tgtttaaggt gcttgtcaat gctctaagcc acccaaagat ctacaagaaa 17880

ttctggcatt gtggtattat agagcctatc catggtcctt cacttgatgc tcaaaacttg 17940

cacacaactg tgtgcaacat ggtttacaca tgctatatga cctacctcga cctgttgttg 18000

aatgaagagt tagaagagtt cacatttctc ttgtgtgaaa gcgacgagga tgtagtaccg 18060

gacagattcg acaacatcca ggcaaaacac ttatgtgttc tggcagattt gtactgtcaa 18120

ccagggacct gcccaccaat tcgaggtcta agaccggtag agaaatgtgc agttctaacc 18180

gaccatatca aggcagaggc tatgttatct ccagcaggat cttcgtggaa cataaatcca 18240

attattgtag accattactc atgctctctg acttatctcc ggcgaggatc gatcaaacag 18300

ataagattga gagttgatcc aggattcatt ttcgacgccc tcgctgaggt aaatgtcagt 18360

cagccaaaga tcggcagcaa caacatctca aatatgagca tcaaggcttt cagaccccca 18420

cacgatgatg ttgcaaaatt gctcaaagat atcaacacaa gcaagcacaa tcttcccatt 18480

tcagggggca atctcgccaa ttatgaaatc catgctttcc gcagaatcgg gttgaactca 18540

tctgcttgct acaaagctgt tgagatatca acattaatta ggagatgcct tgagccaggg 18600

gaggacggct tgttcttggg tgagggatcg ggttctatgt tgatcactta taaagagata 18660

cttaaactaa acaagtgctt ctataatagt ggggtttccg ccaattctag atctggtcaa 18720

agggaattag caccctatcc ctccgaagtt ggccttgtcg aacacagaat gggagtaggt 18780

aatattgtca aagtgctctt taacgggagg cccgaagtca cgtgggtagg cagtgtagat 18840

tgcttcaatt tcatagttag taatatccct acctctagtg tggggtttat ccattcagat 18900

atagagacct tgcctgacaa agatactata gagaagctag aggaattggc agccatctta 18960

tcgatggctc tgctcctggg caaaatagga tcaatactgg tgattaagct tatgcctttc 19020

agcggggatt ttgttcaggg atttataagt tatgtagggt ctcattatag agaagtgaac 19080

cttgtatacc ctagatacag caacttcatc tctactgaat cttatttggt tatgacagat 19140

ctcaaggcta accggctaat gaatcctgaa aagattaagc agcagataat tgaatcatct 19200

gtgaggactt cacctggact tataggtcac atcctatcca ttaagcaact aagctgcata 19260

caagcaattg tgggagacgc agttagtaga ggtgatatca atcctactct gaaaaaactt 19320

acacctatag agcaggtgct gatcaattgc gggttggcaa ttaacggacc taagctgtgc 19380

aaagaattga tccaccatga tgttgcctca gggcaagatg gattgcttaa ttctatactc 19440

atcctctaca gggagttggc aagattcaaa gacaaccaaa gaagtcaaca agggatgttc 19500

cacgcttacc ccgtattggt aagtagcagg caacgagaac ttatatctag gatcacccgc 19560

aaattctggg ggcacattct tctttactcc gggaacaaaa agttgataaa taagtttatc 19620

cagaatctca agtccggcta tctgatacta gacttacacc agaatatctt cgttaagaat 19680

ctatccaagt cagagaaaca gattattatg acggggggtt tgaaacgtga gtgggttttt 19740

aaggtaacag tcaaggagac caaagaatgg tataagttag tcggatacag tgccctgatt 19800

aaggactaat tggttgaact ccggaaccct aatcctgccc taggtggtta ggcattattt 19860

gcaatatatt aaagaaaact ttgaaaatac gaagtttcta ttcccagctt tgtctggtgg 19920

ccggcatggt cccagcctcc tcgctggcgc cggctgggca acattccgag gggaccgtcc 19980

cctcggtaat ggcgaatggg acgcggccga tccggctgct aacaaagccc gaaaggaagc 20040

tgagttggct gctgccaccg ctgagcaata actagcataa ccccttgggg cctctaaacg 20100

ggtcttgagg ggttttttgc tgaaaggagg aactatatcc ggatgcggcc gccatgaaca 20160

ataaaactgt ctgcttacat aaacagtaat acaaggggtg ttatgagcca tattcaacgg 20220

gaaacgtcga ggccgcgatt aaattccaac atggatgctg atttatatgg gtataaatgg 20280

gctcgcgata atgtcgggca atcaggtgcg acaatctatc gcttgtatgg gaagcccgat 20340

gcgccagagt tgtttctgaa acatggcaaa ggtagcgttg ccaatgatgt tacagatgag 20400

atggtcagac taaactggct gacggaattt atgcctcttc cgaccatcaa gcattttatc 20460

cgtactcctg atgatgcatg gttactcacc actgcgatcc ccggaaaaac agcattccag 20520

gtattagaag aatatcctga ttcaggtgaa aatattgttg atgcgctggc agtgttcctg 20580

cgccggttgc attcgattcc tgtttgtaat tgtcctttta acagcgatcg tgtatttcgt 20640

ctcgctcagg cgcaatcacg aatgaataac ggtttggttg atgcgagtga ttttgatgac 20700

gagcgtaatg gctggcctgt tgaacaagtc tggaaagaaa tgcataaact tttgccattc 20760

tcaccggatt cagtcgtcac tcatggtgat ttctcacttg ataaccttat ttttgacgag 20820

gggaaattaa taggttgtat tgatgttgga cgagtcggaa tcgcagaccg ataccaggat 20880

cttgccatcc tatggaactg cctcggtgag ttttctcctt cattacagaa acggcttttt 20940

caaaaatatg gtattgataa tcctgatatg aataaattgc agtttcattt gatgctcgat 21000

gagtttttct aatcagaatt ggttaattgg ttgtaacatt attcagattg ggccccgttc 21060

cactgagcgt cagaccccgt agaaaagatc aaaggatctt cttgagatcc tttttttctg 21120

cgcgtaatct gctgcttgca aacaaaaaaa ccaccgctac cagcggtggt ttgtttgccg 21180

gatcaagagc taccaactct ttttccgaag gtaactggct tcagcagagc gcagatacca 21240

aatactgttc ttctagtgta gccgtagtta ggccaccact tcaagaactc tgtagcaccg 21300

cctacatacc tcgctctgct aatcctgtta ccagtggctg ctgccagtgg cgataagtcg 21360

tgtcttaccg ggttggactc aagacgatag ttaccggata aggcgcagcg gtcgggctga 21420

acggggggtt cgtgcacaca gcccagcttg gagcgaacga cctacaccga actgagatac 21480

ctacagcgtg agctatgaga aagcgccacg cttcccgaag ggagaaaggc ggacaggtat 21540

ccggtaagcg gcagggtcgg aacaggagag cgcacgaggg agcttccagg gggaaacgcc 21600

tggtatcttt atagtcctgt cgggtttcgc cacctctgac ttgagcgtcg atttttgtga 21660

tgctcgtcag gggggcggag cctatggaaa aacgccagca acgcggcctt tttacggttc 21720

ctggcctttt gctggccttt tgctcacatg ttctttcctg cgttatcccc tgattctgtg 21780

gataaccgta ttaccgctag 21800

<210> 36
<211> 3870
<212> DNA
<213> MV-optimized nucleotide sequence of the spike (S) polypeptide of nCoV

<400> 36
gctagcggat cccgtacggc caccatgttt gtgtttttgg ttttgttgcc actggtgtcc 60

agtcagtgtg ttaatttgac aacaagaaca cagctgccac cagcctacac aaatagtttt 120

acaaggggag tgtactaccc agataaggtg ttcagaagta gtgttctgca tagcacacag 180

gatctgtttc tgccattctt tagcaatgtg acctggttcc atgctattca tgtttccggt 240

acaaatggca ccaagcgctt tgataatcca gtgttgccat tcaatgatgg cgtgtacttt 300

gccagtaccg agaagagtaa tatcattcgc ggatggattt tcggaaccac actggatagt 360

aaaacccaga gtctgctgat tgtgaacaat gccaccaatg tggttatcaa agtgtgcgag 420

tttcagttct gcaatgatcc atttctgggc gtctactacc ataagaacaa caagagttgg 480

atggaaagcg agtttagagt gtacagtagt gccaacaatt gcacctttga atacgttagc 540

cagccattct tgatggattt ggagggaaag cagggcaatt tcaagaatct gcgcgagttc 600

gtgttcaaga atatcgacgg atacttcaaa atctacagca agcacacacc aatcaatctt 660

gttagagatt tgccacaggg attcagtgct ttggaaccat tggttgatct gccaatcgga 720

atcaatatta ccagatttca aacactgctg gccctgcata gaagttattt gacaccaggc 780

gattcaagta gtggatggac agctggtgct gctgcttatt atgttggata tctgcaacca 840

cgaacctttc tgctgaagta caatgagaat ggaacaatca cagatgctgt ggattgtgct 900

cttgatccac tgagtgaaac aaagtgcacc ctgaagagtt ttaccgttga gaagggaatc 960

taccagacca gtaattttcg agtgcagcca acagaaagta tcgtgcgctt cccaaatatt 1020

acaaatctgt gcccattcgg cgaagtgttt aatgctacaa gatttgctag tgtgtacgcc 1080

tggaatcgca agagaattag taattgcgtg gccgattaca gcgtgctgta taatagtgcc 1140

agctttagca cctttaagtg ctatggtgtt agtcccacaa agctgaatga tctgtgcttt 1200

accaatgtgt atgccgatag ttttgtgatc aggggagatg aagttcgaca aattgctcca 1260

ggacagactg gaaagattgc cgattataac tacaagctgc cagatgattt caccggatgt 1320

gtgattgcct ggaacagtaa caatctggat agcaaagttg gcggcaacta caattatctg 1380

tatcgcctgt ttcgcaagag caatctgaaa ccatttgagc gcgatatcag caccgaaatc 1440

tatcaagctg gaagtactcc atgcaatggc gtggaaggtt ttaattgcta ctttccactg 1500

cagtcctacg gatttcaacc tactaatggt gttggatacc agccatatag agtggtggtt 1560

ctgagttttg aattgctgca tgctccagct acagtgtgtg gaccaaagaa aagtaccaac 1620

ctggttaaga acaaatgcgt gaacttcaac ttcaatggac tgactggtac aggcgttctg 1680

actgagagta acaagaaatt cctgccattt cagcagtttg gacgagatat tgctgataca 1740

acagatgcag ttagagatcc ccaaacactg gaaattctgg atattacccc atgcagtttt 1800

ggcggagtta gtgttattac acccggcaca aataccagta atcaagttgc tgtgctgtac 1860

caggatgtga attgcacaga agttccagtt gcaattcatg cagatcagtt gacaccaaca 1920

tggcgagtgt atagtaccgg aagtaatgtt tttcagacac gagctggatg tctgattgga 1980

gccgaacatg ttaacaatag ctacgagtgc gatattccaa ttggtgctgg aatttgcgct 2040

agttatcaga cccaaacaaa tagtccaaga cgagcaagaa gtgtggccag tcagagtatt 2100

attgcctata caatgagtct gggagccgag aatagtgtgg cctatagtaa caattccatt 2160

gctatcccca ccaactttac cattagtgtg acaacagaga ttctgccagt gtccatgaca 2220

aagacaagtg tggattgcac catgtacatt tgcggagata gtacagaatg cagtaatctg 2280

ctgctgcagt atggaagttt ctgtacacaa ctgaatagag ccctgactgg aattgctgtg 2340

gaacaggata agaatactca agaggtgttc gcccaagtga agcaaatcta taagacccca 2400

cctattaagg atttcggcgg attcaatttc agtcagattt tgccagatcc aagcaagcca 2460

tccaaacgca gttttattga ggacctgctg ttcaacaaag tgacattggc tgatgctggc 2520

tttattaagc aatatggcga ttgtctggga gatatagctg ccagagattt gatttgcgcc 2580

caaaagttta atggattgac agttttgcca ccactgctga cagatgagat gattgctcag 2640

tatacaagtg ctctgctggc cggaacaatt acatcaggat ggacatttgg agctggtgca 2700

gcattgcaaa taccatttgc tatgcagatg gcctaccgct ttaatggtat tggagttacc 2760

cagaacgtgc tgtacgaaaa tcagaagctg atcgccaatc agttcaatag cgccattgga 2820

aaaattcagg atagcctgag tagtactgct agtgctcttg gaaaactgca ggatgtggtt 2880

aatcagaatg ctcaggctct gaatactctg gttaagcagt tgagttccaa tttcggagct 2940

attagcagtg tgctgaacga tattctgagt cgtttggata aggttgaggc cgaagttcag 3000

attgatagac tgattacagg acgactgcag agcttgcaga catatgttac acaacaactg 3060

attcgagccg ctgaaattag agctagtgct aatttggctg ccacaaagat gagtgaatgt 3120

gtgcttggac agagcaaaag agtggatttt tgcggcaaag gataccatct gatgagcttt 3180

ccacaaagtg ctccacatgg tgttgtgttt ctgcatgtta cttatgtgcc cgctcaagag 3240

aagaatttca caacagctcc agccatttgc catgatggaa aagctcattt tccacgagaa 3300

ggcgtgttcg ttagtaatgg tactcattgg ttcgtgaccc agcgcaattt ttacgagcca 3360

cagattatta ccaccgacaa tactttcgtg tccggcaatt gtgatgttgt gattggaatc 3420

gtgaacaaca cagtgtacga tccattgcag ccagagctgg atagttttaa agaggaactg 3480

gacaagtact tcaagaacca tacaagtcca gatgtggatc tgggcgatat tagtggaatc 3540

aatgcctccg tggtcaacat ccagaaagaa attgatcgac tgaacgaagt ggccaagaat 3600

ttgaatgaga gcctgattga tctgcaagag ctgggcaaat acgagcagta tattaagtgg 3660

ccttggtaca tttggctggg attcattgct ggactgattg ccattgtgat ggttaccatt 3720

atgctgtgtt gcatgacaag ttgttgcagt tgcctgaaag gatgctgtag ttgcggtagt 3780

tgctgcaagt ttgatgagga tgatagtgaa ccagtgctga aaggcgtgaa gctgcattat 3840

acttgataac gagcgcgcct cgaggaattc 3870

<210> 37
<211> 1308
<212> DNA
<213> MV-optimized nucleotide sequence of the N polypeptide of CoV

<400> 37
gctagcggat cccgtacggc caccatgagt gataatggac cacaaaatca acgcaatgcc 60

ccaagaatta catttggagg accaagtgat agcaccggaa gtaatcaaaa tggcgaaaga 120

agtggtgcta gaagtaaaca aagacgacca caaggactgc caaacaatac tgctagttgg 180

tttacagctc tgacccagca tggaaaagag gatttgaaat ttccaagagg acagggcgtg 240

ccaatcaata ccaatagtag tccagatgat cagattggct attatagacg agctacaaga 300

agaatacgag gcggagatgg aaaaatgaag gatttgagtc cacgctggta cttctattat 360

cttggaacag gaccagaggc tggattgcca tatggtgcta acaaagatgg aattatttgg 420

gttgcaacag aaggtgccct gaatacccca aaagatcata ttggaacacg caatcctgcc 480

aacaatgctg caattgtgtt gcaattgcca cagggaacaa cactgcctaa aggattctat 540

gctgagggaa gtagaggtgg aagtcaagct agttcaagaa gtagtagtcg cagtagaaat 600

agcagtcgaa atagtacacc tggaagtagt agaggaacaa gtccagctag aatggctgga 660

aatggtggtg atgctgcttt ggctttgttg ctgttggata gactgaatca gctggaaagc 720

aaaatgagtg gcaaaggaca acaacagcag ggacagacag ttacaaagaa aagtgctgct 780

gaagcaagca agaaaccacg tcaaaaacga acagccacaa aggcctataa tgtgacacaa 840

gcttttggac gaagaggacc agaacagaca cagggaaatt ttggagatca agaactgatt 900

cgccagggaa cagattacaa acattggcca caaattgccc agtttgctcc aagtgctagt 960

gctttctttg gaatgagtcg aattggaatg gaagtgacac caagtggaac atggttgaca 1020

tatacaggtg ccatcaagct ggatgataag gacccaaatt tcaaggatca agtgatcctg 1080

ctgaacaaac acatcgacgc ctataagaca tttccaccaa cagaacccaa gaaggataag 1140

aagaagaaag ctgacgaaac acaagctctg ccacagagac aaaagaaaca gcaaacagtt 1200

actctgctgc cagctgctga tctggatgat ttcagtaaac aactgcagca gagcatgagt 1260

agtgctgata gtacacaagc ctgataacga gcgcgcctcg aggaattc 1308

<210> 38
<211> 19795
<212> DNA
<213> pTM3-MVSchw-gfp

<400> 38
accaaacaaa gttgggtaag gatagttcaa tcaatgatca tcttctagtg cacttaggat 60

tcaagatcct attatcaggg acaagagcag gattagggat atccgagatg gccacacttt 120

taaggagctt agcattgttc aaaagaaaca aggacaaacc acccattaca tcaggatccg 180

gtggagccat cagaggaatc aaacacatta ttatagtacc aatccctgga gattcctcaa 240

ttaccactcg atccagactt ctggaccggt tggtgaggtt aattggaaac ccggatgtga 300

gcgggcccaa actaacaggg gcactaatag gtatattatc cttatttgtg gagtctccag 360

gtcaattgat tcagaggatc accgatgacc ctgacgttag cataaggctg ttagaggttg 420

tccagagtga ccagtcacaa tctggcctta ccttcgcatc aagaggtacc aacatggagg 480

atgaggcgga ccaatacttt tcacatgatg atccaattag tagtgatcaa tccaggttcg 540

gatggttcgg gaacaaggaa atctcagata ttgaagtgca agaccctgag ggattcaaca 600

tgattctggg taccatccta gcccaaattt gggtcttgct cgcaaaggcg gttacggccc 660

cagacacggc agctgattcg gagctaagaa ggtggataaa gtacacccaa caaagaaggg 720

tagttggtga atttagattg gagagaaaat ggttggatgt ggtgaggaac aggattgccg 780

aggacctctc cttacgccga ttcatggtcg ctctaatcct ggatatcaag agaacacccg 840

gaaacaaacc caggattgct gaaatgatat gtgacattga tacatatatc gtagaggcag 900

gattagccag ttttatcctg actattaagt ttgggataga aactatgtat cctgctcttg 960

gactgcatga atttgctggt gagttatcca cacttgagtc cttgatgaac ctttaccagc 1020

aaatggggga aactgcaccc tacatggtaa tcctggagaa ctcaattcag aacaagttca 1080

gtgcaggatc ataccctctg ctctggagct atgccatggg agtaggagtg gaacttgaaa 1140

actccatggg aggtttgaac tttggccgat cttactttga tccagcatat tttagattag 1200

ggcaagagat ggtaaggagg tcagctggaa aggtcagttc cacattggca tctgaactcg 1260

gtatcactgc cgaggatgca aggcttgttt cagagattgc aatgcatact actgaggaca 1320

agatcagtag agcggttgga cccagacaag cccaagtatc atttctacac ggtgatcaaa 1380

gtgagaatga gctaccgaga ttggggggca aggaagatag gagggtcaaa cagagtcgag 1440

gagaagccag ggagagctac agagaaaccg ggcccagcag agcaagtgat gcgagagctg 1500

cccatcttcc aaccggcaca cccctagaca ttgacactgc aacggagtcc agccaagatc 1560

cgcaggacag tcgaaggtca gctgacgccc tgcttaggct gcaagccatg gcaggaatct 1620

cggaagaaca aggctcagac acggacaccc ctatagtgta caatgacaga aatcttctag 1680

actaggtgcg agaggccgag ggccagaaca acatccgcct accatccatc attgttataa 1740

aaaacttagg aaccaggtcc acacagccgc cagcccatca accatccact cccacgattg 1800

gagccaatgg cagaagagca ggcacgccat gtcaaaaacg gactggaatg catccgggct 1860

ctcaaggccg agcccatcgg ctcactggcc atcgaggaag ctatggcagc atggtcagaa 1920

atatcagaca acccaggaca ggagcgagcc acctgcaggg aagagaaggc aggcagttcg 1980

ggtctcagca aaccatgcct ctcagcaatt ggatcaactg aaggcggtgc acctcgcatc 2040

cgcggtcagg gacctggaga gagcgatgac gacgctgaaa ctttgggaat ccccccaaga 2100

aatctccagg catcaagcac tgggttacag tgttattacg tttatgatca cagcggtgaa 2160

gcggttaagg gaatccaaga tgctgactct atcatggttc aatcaggcct tgatggtgat 2220

agcaccctct caggaggaga caatgaatct gaaaacagcg atgtggatat tggcgaacct 2280

gataccgagg gatatgctat cactgaccgg ggatctgctc ccatctctat ggggttcagg 2340

gcttctgatg ttgaaactgc agaaggaggg gagatccacg agctcctgag actccaatcc 2400

agaggcaaca actttccgaa gcttgggaaa actctcaatg ttcctccgcc cccggacccc 2460

ggtagggcca gcacttccgg gacacccatt aaaaagggca cagacgcgag attagcctca 2520

tttggaacgg agatcgcgtc tttattgaca ggtggtgcaa cccaatgtgc tcgaaagtca 2580

ccctcggaac catcagggcc aggtgcacct gcggggaatg tccccgagtg tgtgagcaat 2640

gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagtac aacctaaatc cattataaaa aacttaggag caaagtgatt 3420

gcctcccaag gtccacaatg acagagacct acgacttcga caagtcggca tgggacatca 3480

aagggtcgat cgctccgata caacccacca cctacagtga tggcaggctg gtgccccagg 3540

tcagagtcat agatcctggt ctaggcgaca ggaaggatga atgctttatg tacatgtttc 3600

tgctgggggt tgttgaggac agcgattccc tagggcctcc aatcgggcga gcatttgggt 3660

tcctgccctt aggtgttggc agatccacag caaagcccga aaaactcctc aaagaggcca 3720

ctgagcttga catagttgtt agacgtacag cagggctcaa tgaaaaactg gtgttctaca 3780

acaacacccc actaactctc ctcacacctt ggagaaaggt cctaacaaca gggagtgtct 3840

tcaacgcaaa ccaagtgtgc aatgcggtta atctgatacc gctcgatacc ccgcagaggt 3900

tccgtgttgt ttatatgagc atcacccgtc tttcggataa cgggtattac accgttccta 3960

gaagaatgct ggaattcaga tcggtcaatg cagtggcctt caacctgctg gtgaccctta 4020

ggattgacaa ggcgataggc cctgggaaga tcatcgacaa tacagagcaa cttcctgagg 4080

caacatttat ggtccacatc gggaacttca ggagaaagaa gagtgaagtc tactctgccg 4140

attattgcaa aatgaaaatc gaaaagatgg gcctggtttt tgcacttggt gggatagggg 4200

gcaccagtct tcacattaga agcacaggca aaatgagcaa gactctccat gcacaactcg 4260

ggttcaagaa gaccttatgt tacccgctga tggatatcaa tgaagacctt aatcgattac 4320

tctggaggag cagatgcaag atagtaagaa tccaggcagt tttgcagcca tcagttcctc 4380

aagaattccg catttacgac gacgtgatca taaatgatga ccaaggacta ttcaaagttc 4440

tgtagaccgt agtgcccagc aatgcccgaa aacgaccccc ctcacaatga cagccagaag 4500

gcccggacaa aaaagccccc tccgaaagac tccacggacc aagcgagagg ccagccagca 4560

gccgacggca agcgcgaaca ccaggcggcc ccagcacaga acagccctga cacaaggcca 4620

ccaccagcca ccccaatctg catcctcctc gtgggacccc cgaggaccaa cccccaaggc 4680

tgcccccgat ccaaaccacc aaccgcatcc ccaccacccc cgggaaagaa acccccagca 4740

attggaaggc ccctccccct cttcctcaac acaagaactc cacaaccgaa ccgcacaagc 4800

gaccgaggtg acccaaccgc aggcatccga ctccctagac agatcctctc tccccggcaa 4860

actaaacaaa acttagggcc aaggaacata cacacccaac agaacccaga ccccggccca 4920

cggcgccgcg cccccaaccc ccgacaacca gagggagccc ccaaccaatc ccgccggctc 4980

ccccggtgcc cacaggcagg gacaccaacc cccgaacaga cccagcaccc aaccatcgac 5040

aatccaagac gggggggccc ccccaaaaaa aggcccccag gggccgacag ccagcaccgc 5100

gaggaagccc acccacccca cacacgacca cggcaaccaa accagaaccc agaccaccct 5160

gggccaccag ctcccagact cggccatcac cccgcagaaa ggaaaggcca caacccgcgc 5220

accccagccc cgatccggcg gggagccacc caacccgaac cagcacccaa gagcgatccc 5280

cgaaggaccc ccgaaccgca aaggacatca gtatcccaca gcctctccaa gtcccccggt 5340

ctcctcctct tctcgaaggg accaaaagat caatccacca cacccgacga cactcaactc 5400

cccaccccta aaggagacac cgggaatccc agaatcaaga ctcatccaat gtccatcatg 5460

ggtctcaagg tgaacgtctc tgccatattc atggcagtac tgttaactct ccaaacaccc 5520

accggtcaaa tccattgggg caatctctct aagatagggg tggtaggaat aggaagtgca 5580

agctacaaag ttatgactcg ttccagccat caatcattag tcataaaatt aatgcccaat 5640

ataactctcc tcaataactg cacgagggta gagattgcag aatacaggag actactgaga 5700

acagttttgg aaccaattag agatgcactt aatgcaatga cccagaatat aagaccggtt 5760

cagagtgtag cttcaagtag gagacacaag agatttgcgg gagtagtcct ggcaggtgcg 5820

gccctaggcg ttgccacagc tgctcagata acagccggca ttgcacttca ccagtccatg 5880

ctgaactctc aagccatcga caatctgaga gcgagcctgg aaactactaa tcaggcaatt 5940

gagacaatca gacaagcagg gcaggagatg atattggctg ttcagggtgt ccaagactac 6000

atcaataatg agctgatacc gtctatgaac caactatctt gtgatttaat cggccagaag 6060

ctcgggctca aattgctcag atactataca gaaatcctgt cattatttgg ccccagttta 6120

cgggacccca tatctgcgga gatatctatc caggctttga gctatgcgct tggaggagac 6180

atcaataagg tgttagaaaa gctcggatac agtggaggtg atttactggg catcttagag 6240

agcggaggaa taaaggcccg gataactcac gtcgacacag agtcctactt cattgtcctc 6300

agtatagcct atccgacgct gtccgagatt aagggggtga ttgtccaccg gctagagggg 6360

gtctcgtaca acataggctc tcaagagtgg tataccactg tgcccaagta tgttgcaacc 6420

caagggtacc ttatctcgaa ttttgatgag tcatcgtgta ctttcatgcc agaggggact 6480

gtgtgcagcc aaaatgcctt gtacccgatg agtcctctgc tccaagaatg cctccggggg 6540

tacaccaagt cctgtgctcg tacactcgta tccgggtctt ttgggaaccg gttcatttta 6600

tcacaaggga acctaatagc caattgtgca tcaatccttt gcaagtgtta cacaacagga 6660

acgatcatta atcaagaccc tgacaagatc ctaacataca ttgctgccga tcactgcccg 6720

gtagtcgagg tgaacggcgt gaccatccaa gtcgggagca ggaggtatcc agacgctgtg 6780

tacttgcaca gaattgacct cggtcctccc atatcattgg agaggttgga cgtagggaca 6840

aatctgggga atgcaattgc taagttggag gatgccaagg aattgttgga gtcatcggac 6900

cagatattga ggagtatgaa aggtttatcg agcactagca tagtctacat cctgattgca 6960

gtgtgtcttg gagggttgat agggatcccc gctttaatat gttgctgcag ggggcgttgt 7020

aacaaaaagg gagaacaagt tggtatgtca agaccaggcc taaagcctga tcttacggga 7080

acatcaaaat cctatgtaag gtcgctctga tcctctacaa ctcttgaaac acaaatgtcc 7140

cacaagtctc ctcttcgtca tcaagcaacc accgcaccca gcatcaagcc cacctgaaat 7200

tatctccggc ttccctctgg ccgaacaata tcggtagtta atcaaaactt agggtgcaag 7260

atcatccaca atgtcaccac aacgagaccg gataaatgcc ttctacaaag ataaccccca 7320

tcccaaggga agtaggatag tcattaacag agaacatctt atgattgata gaccttatgt 7380

tttgctggct gttctgtttg tcatgtttct gagcttgatc gggttgctag ccattgcagg 7440

cattagactt catcgggcag ccatctacac cgcagagatc cataaaagcc tcagcaccaa 7500

tctagatgta actaactcaa tcgagcatca ggtcaaggac gtgctgacac cactcttcaa 7560

aatcatcggt gatgaagtgg gcctgaggac acctcagaga ttcactgacc tagtgaaatt 7620

aatctctgac aagattaaat tccttaatcc ggatagggag tacgacttca gagatctcac 7680

ttggtgtatc aacccgccag agagaatcaa attggattat gatcaatact gtgcagatgt 7740

ggctgctgaa gagctcatga atgcattggt gaactcaact ctactggaga ccagaacaac 7800

caatcagttc ctagctgtct caaagggaaa ctgctcaggg cccactacaa tcagaggtca 7860

attctcaaac atgtcgctgt ccctgttaga cttgtattta ggtcgaggtt acaatgtgtc 7920

atctatagtc actatgacat cccagggaat gtatggggga acttacctag tggaaaagcc 7980

taatctgagc agcaaaaggt cagagttgtc acaactgagc atgtaccgag tgtttgaagt 8040

aggtgttatc agaaatccgg gtttgggggc tccggtgttc catatgacaa actatcttga 8100

gcaaccagtc agtaatgatc tcagcaactg tatggtggct ttgggggagc tcaaactcgc 8160

agccctttgt cacggggaag attctatcac aattccctat cagggatcag ggaaaggtgt 8220

cagcttccag ctcgtcaagc taggtgtctg gaaatcccca accgacatgc aatcctgggt 8280

ccccttatca acggatgatc cagtgataga caggctttac ctctcatctc acagaggtgt 8340

tatcgctgac aatcaagcaa aatgggctgt cccgacaaca cgaacagatg acaagttgcg 8400

aatggagaca tgcttccaac aggcgtgtaa gggtaaaatc caagcactct gcgagaatcc 8460

cgagtgggca ccattgaagg ataacaggat tccttcatac ggggtcttgt ctgttgatct 8520

gagtctgaca gttgagctta aaatcaaaat tgcttcggga ttcgggccat tgatcacaca 8580

cggttcaggg atggacctat acaaatccaa ccacaacaat gtgtattggc tgactatccc 8640

gccaatgaag aacctagcct taggtgtaat caacacattg gagtggatac cgagattcaa 8700

ggttagtccc tacctcttca ctgtcccaat taaggaagca ggcgaagact gccatgcccc 8760

aacataccta cctgcggagg tggatggtga tgtcaaactc agttccaatc tggtgattct 8820

acctggtcaa gatctccaat atgttttggc aacctacgat acttccaggg ttgaacatgc 8880

tgtggtttat tacgtttaca gcccaagccg ctcattttct tacttttatc cttttaggtt 8940

gcctataaag ggggtcccca tcgaattaca agtggaatgc ttcacatggg accaaaaact 9000

ctggtgccgt cacttctgtg tgcttgcgga ctcagaatct ggtggacata tcactcactc 9060

tgggatggtg ggcatgggag tcagctgcac agtcacccgg gaagatggaa ccaatcgcag 9120

atagggctgc tagtgaacca atcacatgat gtcacccaga catcaggcat acccactagt 9180

ctaccctcca tcattgttat aaaaaactta ggaaccaggt ccacacagcc gccagcccat 9240

caacgcgtac gatggtgagc aagggcgagg agctgttcac cggggtggtg cccatcctgg 9300

tcgagctgga cggcgacgta aacggccaca agttcagcgt gtccggcgag ggcgagggcg 9360

atgccaccta cggcaagctg accctgaagt tcatctgcac caccggcaag ctgcccgtgc 9420

cctggcccac cctcgtgacc accctgacct acggcgtgca gtgcttcagc cgctaccccg 9480

accacatgaa gcagcacgac ttcttcaagt ccgccatgcc cgaaggctac gtccaggagc 9540

gcaccatctt cttcaaggac gacggcaact acaagacccg cgccgaggtg aagttcgagg 9600

gcgacaccct ggtgaaccgc atcgagctga agggcatcga cttcaaggag gacggcaaca 9660

tcctggggca caagctggag tacaactaca acagccacaa cgtctatatc atggccgaca 9720

agcagaagaa cggcatcaag gtgaacttca agatccgcca caacatcgag gacggcagcg 9780

tgcagctcgc cgaccactac cagcagaaca cccccatcgg cgacggcccc gtgctgctgc 9840

ccgacaacca ctacctgagc acccagtccg ccctgagcaa agaccccaac gagaagcgcg 9900

atcacatggt cctgctggag ttcgtgaccg ccgccgggat cactctcggc atggacgagc 9960

tgtacaagta ggcgcgcagc gcttagacgt ctcgcgatcg atgctagtgt gaaatagaca 10020

tcagaattaa gaaaaacgta gggtccaagt ggttccccgt tatggactcg ctatctgtca 10080

accagatctt ataccctgaa gttcacctag atagcccgat agttaccaat aagatagtag 10140

ccatcctgga gtatgctcga gtccctcacg cttacagcct ggaggaccct acactgtgtc 10200

agaacatcaa gcaccgccta aaaaacggat tttccaacca aatgattata aacaatgtgg 10260

aagttgggaa tgtcatcaag tccaagctta ggagttatcc ggcccactct catattccat 10320

atccaaattg taatcaggat ttatttaaca tagaagacaa agagtcaacg aggaagatcc 10380

gtgaactcct caaaaagggg aattcgctgt actccaaagt cagtgataag gttttccaat 10440

gcttaaggga cactaactca cggcttggcc taggctccga attgagggag gacatcaagg 10500

agaaagttat taacttggga gtttacatgc acagctccca gtggtttgag ccctttctgt 10560

tttggtttac agtcaagact gagatgaggt cagtgattaa atcacaaacc catacttgcc 10620

ataggaggag acacacacct gtattcttca ctggtagttc agttgagttg ctaatctctc 10680

gtgaccttgt tgctataatc agtaaagagt ctcaacatgt atattacctg acatttgaac 10740

tggttttgat gtattgtgat gtcatagagg ggaggttaat gacagagacc gctatgacta 10800

ttgatgctag gtatacagag cttctaggaa gagtcagata catgtggaaa ctgatagatg 10860

gtttcttccc tgcactcggg aatccaactt atcaaattgt agccatgctg gagcctcttt 10920

cacttgctta cctgcagctg agggatataa cagtagaact cagaggtgct ttccttaacc 10980

actgctttac tgaaatacat gatgttcttg accaaaacgg gttttctgat gaaggtactt 11040

atcatgagtt aactgaagct ctagattaca ttttcataac tgatgacata catctgacag 11100

gggagatttt ctcatttttc agaagtttcg gccaccccag acttgaagca gtaacggctg 11160

ctgaaaatgt taggaaatac atgaatcagc ctaaagtcat tgtgtatgag actctgatga 11220

aaggtcatgc catattttgt ggaatcataa tcaacggcta tcgtgacagg cacggaggca 11280

gttggccacc gctgaccctc cccctgcatg ctgcagacac aatccggaat gctcaagctt 11340

caggtgaagg gttaacacat gagcagtgcg ttgataactg gaaatctttt gctggagtga 11400

aatttggctg ctttatgcct cttagcctgg atagtgatct gacaatgtac ctaaaggaca 11460

aggcacttgc tgctctccaa agggaatggg attcagttta cccgaaagag ttcctgcgtt 11520

acgaccctcc caagggaacc gggtcacgga ggcttgtaga tgttttcctt aatgattcga 11580

gctttgaccc atatgatgtg ataatgtatg ttgtaagtgg agcttacctc catgaccctg 11640

agttcaacct gtcttacagc ctgaaagaaa aggagatcaa ggaaacaggt agactttttg 11700

ctaaaatgac ttacaaaatg agggcatgcc aagtgattgc tgaaaatcta atctcaaacg 11760

ggattggcaa atattttaag gacaatggga tggccaagga tgagcacgat ttgactaagg 11820

cactccacac tctagctgtc tcaggagtcc ccaaagatct caaagaaagt cacagggggg 11880

ggccagtctt aaaaacctac tcccgaagcc cagtccacac aagtaccagg aacgtgagag 11940

cagcaaaagg gtttataggg ttccctcaag taattcggca ggaccaagac actgatcatc 12000

cggagaatat ggaagcttac gagacagtca gtgcatttat cacgactgat ctcaagaagt 12060

actgccttaa ttggagatat gagaccatca gcttgtttgc acagaggcta aatgagattt 12120

acggattgcc ctcatttttc cagtggctgc ataagaggct tgagacctct gtcctgtatg 12180

taagtgaccc tcattgcccc cccgaccttg acgcccatat cccgttatat aaagtcccca 12240

atgatcaaat cttcattaag taccctatgg gaggtataga agggtattgt cagaagctgt 12300

ggaccatcag caccattccc tatctatacc tggctgctta tgagagcgga gtaaggattg 12360

cttcgttagt gcaaggggac aatcagacca tagccgtaac aaaaagggta cccagcacat 12420

ggccctacaa ccttaagaaa cgggaagctg ctagagtaac tagagattac tttgtaattc 12480

ttaggcaaag gctacatgat attggccatc acctcaaggc aaatgagaca attgtttcat 12540

cacatttttt tgtctattca aaaggaatat attatgatgg gctacttgtg tcccaatcac 12600

tcaagagcat cgcaagatgt gtattctggt cagagactat agttgatgaa acaagggcag 12660

catgcagtaa tattgctaca acaatggcta aaagcatcga gagaggttat gaccgttacc 12720

ttgcatattc cctgaacgtc ctaaaagtga tacagcaaat tctgatctct cttggcttca 12780

caatcaattc aaccatgacc cgggatgtag tcatacccct cctcacaaac aacgacctct 12840

taataaggat ggcactgttg cccgctccta ttggggggat gaattatctg aatatgagca 12900

ggctgtttgt cagaaacatc ggtgatccag taacatcatc aattgctgat ctcaagagaa 12960

tgattctcgc ctcactaatg cctgaagaga ccctccatca agtaatgaca caacaaccgg 13020

gggactcttc attcctagac tgggctagcg acccttactc agcaaatctt gtatgtgtcc 13080

agagcatcac tagactcctc aagaacataa ctgcaaggtt tgtcctgatc catagtccaa 13140

acccaatgtt aaaaggatta ttccatgatg acagtaaaga agaggacgag ggactggcgg 13200

cattcctcat ggacaggcat attatagtac ctagggcagc tcatgaaatc ctggatcata 13260

gtgtcacagg ggcaagagag tctattgcag gcatgctgga taccacaaaa ggcttgattc 13320

gagccagcat gaggaagggg gggttaacct ctcgagtgat aaccagattg tccaattatg 13380

actatgaaca attcagagca gggatggtgc tattgacagg aagaaagaga aatgtcctca 13440

ttgacaaaga gtcatgttca gtgcagctgg cgagagctct aagaagccat atgtgggcga 13500

ggctagctcg aggacggcct atttacggcc ttgaggtccc tgatgtacta gaatctatgc 13560

gaggccacct tattcggcgt catgagacat gtgtcatctg cgagtgtgga tcagtcaact 13620

acggatggtt ttttgtcccc tcgggttgcc aactggatga tattgacaag gaaacatcat 13680

ccttgagagt cccatatatt ggttctacca ctgatgagag aacagacatg aagcttgcct 13740

tcgtaagagc cccaagtcga tccttgcgat ctgctgttag aatagcaaca gtgtactcat 13800

gggcttacgg tgatgatgat agctcttgga acgaagcctg gttgttggct aggcaaaggg 13860

ccaatgtgag cctggaggag ctaagggtga tcactcccat ctcaacttcg actaatttag 13920

cgcataggtt gagggatcgt agcactcaag tgaaatactc aggtacatcc cttgtccgag 13980

tggcgaggta taccacaatc tccaacgaca atctctcatt tgtcatatca gataagaagg 14040

ttgatactaa ctttatatac caacaaggaa tgcttctagg gttgggtgtt ttagaaacat 14100

tgtttcgact cgagaaagat accggatcat ctaacacggt attacatctt cacgtcgaaa 14160

cagattgttg cgtgatcccg atgatagatc atcccaggat acccagctcc cgcaagctag 14220

agctgagggc agagctatgt accaacccat tgatatatga taatgcacct ttaattgaca 14280

gagatgcaac aaggctatac acccagagcc ataggaggca ccttgtggaa tttgttacat 14340

ggtccacacc ccaactatat cacattttag ctaagtccac agcactatct atgattgacc 14400

tggtaacaaa atttgagaag gaccatatga atgaaatttc agctctcata ggggatgacg 14460

atatcaatag tttcataact gagtttctgc tcatagagcc aagattattc actatctact 14520

tgggccagtg tgcggccatc aattgggcat ttgatgtaca ttatcataga ccatcaggga 14580

aatatcagat gggtgagctg ttgtcatcgt tcctttctag aatgagcaaa ggagtgttta 14640

aggtgcttgt caatgctcta agccacccaa agatctacaa gaaattctgg cattgtggta 14700

ttatagagcc tatccatggt ccttcacttg atgctcaaaa cttgcacaca actgtgtgca 14760

acatggttta cacatgctat atgacctacc tcgacctgtt gttgaatgaa gagttagaag 14820

agttcacatt tctcttgtgt gaaagcgacg aggatgtagt accggacaga ttcgacaaca 14880

tccaggcaaa acacttatgt gttctggcag atttgtactg tcaaccaggg acctgcccac 14940

caattcgagg tctaagaccg gtagagaaat gtgcagttct aaccgaccat atcaaggcag 15000

aggctatgtt atctccagca ggatcttcgt ggaacataaa tccaattatt gtagaccatt 15060

actcatgctc tctgacttat ctccggcgag gatcgatcaa acagataaga ttgagagttg 15120

atccaggatt cattttcgac gccctcgctg aggtaaatgt cagtcagcca aagatcggca 15180

gcaacaacat ctcaaatatg agcatcaagg ctttcagacc cccacacgat gatgttgcaa 15240

aattgctcaa agatatcaac acaagcaagc acaatcttcc catttcaggg ggcaatctcg 15300

ccaattatga aatccatgct ttccgcagaa tcgggttgaa ctcatctgct tgctacaaag 15360

ctgttgagat atcaacatta attaggagat gccttgagcc aggggaggac ggcttgttct 15420

tgggtgaggg atcgggttct atgttgatca cttataaaga gatacttaaa ctaaacaagt 15480

gcttctataa tagtggggtt tccgccaatt ctagatctgg tcaaagggaa ttagcaccct 15540

atccctccga agttggcctt gtcgaacaca gaatgggagt aggtaatatt gtcaaagtgc 15600

tctttaacgg gaggcccgaa gtcacgtggg taggcagtgt agattgcttc aatttcatag 15660

ttagtaatat ccctacctct agtgtggggt ttatccattc agatatagag accttgcctg 15720

acaaagatac tatagagaag ctagaggaat tggcagccat cttatcgatg gctctgctcc 15780

tgggcaaaat aggatcaata ctggtgatta agcttatgcc tttcagcggg gattttgttc 15840

agggatttat aagttatgta gggtctcatt atagagaagt gaaccttgta taccctagat 15900

acagcaactt catctctact gaatcttatt tggttatgac agatctcaag gctaaccggc 15960

taatgaatcc tgaaaagatt aagcagcaga taattgaatc atctgtgagg acttcacctg 16020

gacttatagg tcacatccta tccattaagc aactaagctg catacaagca attgtgggag 16080

acgcagttag tagaggtgat atcaatccta ctctgaaaaa acttacacct atagagcagg 16140

tgctgatcaa ttgcgggttg gcaattaacg gacctaagct gtgcaaagaa ttgatccacc 16200

atgatgttgc ctcagggcaa gatggattgc ttaattctat actcatcctc tacagggagt 16260

tggcaagatt caaagacaac caaagaagtc aacaagggat gttccacgct taccccgtat 16320

tggtaagtag caggcaacga gaacttatat ctaggatcac ccgcaaattc tgggggcaca 16380

ttcttcttta ctccgggaac aaaaagttga taaataagtt tatccagaat ctcaagtccg 16440

gctatctgat actagactta caccagaata tcttcgttaa gaatctatcc aagtcagaga 16500

aacagattat tatgacgggg ggtttgaaac gtgagtgggt ttttaaggta acagtcaagg 16560

agaccaaaga atggtataag ttagtcggat acagtgccct gattaaggac taattggttg 16620

aactccggaa ccctaatcct gccctaggtg gttaggcatt atttgcaata tattaaagaa 16680

aactttgaaa atacgaagtt tctattccca gctttgtctg gtggccggca tggtcccagc 16740

ctcctcgctg gcgccggctg ggcaacattc cgaggggacc gtcccctcgg taatggcgaa 16800

tgggacgcgg ccgatccggc tgctaacaaa gcccgaaagg aagctgagtt ggctgctgcc 16860

accgctgagc aataactagc ataacccctt ggggcctcta aacgggtctt gaggggtttt 16920

ttgctgaaag gaggaactat atccggatgc ggccgcgggc cctatggtac ccagcttttg 16980

ttccctttag tgagggttaa ttccgagctt ggcgtaatca tggtcatagc tgtttcctgt 17040

gtgaaattgt tatccgctca caattccaca caacatagga gccggaagca taaagtgtaa 17100

agcctggggt gcctaatgag tgaggtaact cacattaatt gcgttgcgct cactgcccgc 17160

tttccagtcg ggaaacctgt cgtgccagct gcattaatga atcggccaac gcgcggggag 17220

aggcggtttg cgtattgggc gctcttccgc ttcctcgctc actgactcgc tgcgctcggt 17280

cgttcggctg cggcgagcgg tatcagctca ctcaaaggcg gtaatacggt tatccacaga 17340

atcaggggat aacgcaggaa agaacatgtg agcaaaaggc cagcaaaagg ccaggaaccg 17400

taaaaaggcc gcgttgctgg cgtttttcca taggctcggc ccccctgacg agcatcacaa 17460

aaatcgacgc tcaagtcaga ggtggcgaaa cccgacagga ctataaagat accaggcgtt 17520

tccccctgga agctccctcg tgcgctctcc tgttccgacc ctgccgctta ccggatacct 17580

gtccgccttt ctcccttcgg gaagcgtggc gctttctcaa tgctcacgct gtaggtatct 17640

cagttcggtg taggtcgttc gctccaagct gggctgtgtg cacgaacccc ccgttcagcc 17700

cgaccgctgc gccttatccg gtaactatcg tcttgagtcc aacccggtaa gacacgactt 17760

atcgccactg gcagcagcca ctggtaacag gattagcaga gcgaggtatg taggcggtgc 17820

tacagagttc ttgaagtggt ggcctaacta cggctacact agaaggacag tatttggtat 17880

ctgcgctctg ctgaagccag ttaccttcgg aaaaagagtt ggtagctctt gatccggcaa 17940

acaaaccacc gctggtagcg gtggtttttt tgtttgcaag cagcagatta cgcgcagaaa 18000

aaaaggatct caagaagatc ctttgatctt ttctacgggg tctgacgctc agtggaacga 18060

aaactcacgt taagggattt tggtcatgag attatcaaaa aggatcttca cctagatcct 18120

tttaaattaa aaatgaagtt ttaaatcaat ctaaagtata tatgagtaaa cttggtctga 18180

cagttaccaa tgcttaatca gtgaggcacc tatctcagcg atctgtctat ttcgttcatc 18240

catagttgcc tgactgcccg tcgtgtagat aactacgata cgggagggct taccatctgg 18300

ccccagtgct gcaatgatac cgcgagaccc acgctcaccg gctccagatt tatcagcaat 18360

aaaccagcca gccggaaggg ccgagcgcag aagtggtcct gcaactttat ccgcctccat 18420

ccagtctatt aattgttgcc gggaagctag agtaagtagt tcgccagtta atagtttgcg 18480

caacgttgtt gccattgcta caggcatcgt ggtgtcacgc tcgtcgtttg gtatggcttc 18540

attcagctcc ggttcccaac gatcaaggcg agttacatga tcccccatgt tgtgaaaaaa 18600

agcggttagc tccttcggtc ctccgatcgt tgtcagaagt aagttggccg cagtgttatc 18660

actcatgctt atggcagcac tgcataattc tcttactgtc atgccatccg taagatgctt 18720

ttctgtgact ggtgagtact caaccaagtc attctgagaa tagtgtatgc ggcgaccgag 18780

ttgctcttgc ccggcgtcaa tacgggataa taccgcgcca catagcagaa ctttaaaagt 18840

gctcatcatt ggaaaacgtt cttcggggcg aaaactctca aggatcttac cgctgttgag 18900

atccagttcg atgtaaccca ctcgtgcacc caactgatct tcagcatctt ttactttcac 18960

cagcgtttct gggtgagcaa aaacaggaag gcaaaatgcc gcaaaaaagg gaataagggc 19020

gacacggaaa tgttgaatac tcatactctt cctttttcaa tattattgaa gcatttatca 19080

gggttattgt ctcatgagcg gatacatatt tgaatgtatt tagaaaaata aacaaatagg 19140

ggttccgcgc acatttcccc gaaaagtgcc acctgaaatt gtaaacgtta atattttgtt 19200

aaaattcgcg ttaaattttt gttaaatcag ctcatttttt aaccaatagg ccgaaatcgg 19260

caaaatccct tataaatcaa aagaatagac cgagataggg ttgagtgttg ttccagtttg 19320

gaacaagagt ccactattaa agaacgtgga ctccaacgtc aaagggcgaa aaaccgtcta 19380

tcagggcgat ggcccactac gtgaaccatc accctaatca agttttttgg ggtcgaggtg 19440

ccgtaaagca ctaaatcgga accctaaagg gagcccccga tttagagctt gacggggaaa 19500

gccggcgaac gtggcgagaa aggaagggaa gaaagcgaaa ggagcgggcg ctagggcgct 19560

ggcaagtgta gcggtcacgc tgcgcgtaac caccacaccc gccgcgctta atgcgccgct 19620

acagggcgcg tcccattcgc cattcaggct gcgcaactgt tgggaagggc gatcggtgcg 19680

ggcctcttcg ctattacgcc agccaccgcg gtggcggccg ctaatacgac tcactatagg 19740

gccaactttg tttggtctga tgagtccgtg aggacgaaac ccggagtccc gggtc 19795

<210> 39
<211> 876
<212> DNA
<213> ATU1(eGFP)

<400> 39
cttaggattc aagatcctat tatcagggac aagagcagga ttagggatat ccgagacgcg 60

tacgatggtg agcaagggcg aggagctgtt caccggggtg gtgcccatcc tggtcgagct 120

ggacggcgac gtaaacggcc acaagttcag cgtgtccggc gagggcgagg gcgatgccac 180

ctacggcaag ctgaccctga agttcatctg caccaccggc aagctgcccg tgccctggcc 240

caccctcgtg accaccctga cctacggcgt gcagtgcttc agccgctacc ccgaccacat 300

gaagcagcac gacttcttca agtccgccat gcccgaaggc tacgtccagg agcgcaccat 360

cttcttcaag gacgacggca actacaagac ccgcgccgag gtgaagttcg agggcgacac 420

cctggtgaac cgcatcgagc tgaagggcat cgacttcaag gaggacggca acatcctggg 480

gcacaagctg gagtacaact acaacagcca caacgtctat atcatggccg acaagcagaa 540

gaacggcatc aaggtgaact tcaagatccg ccacaacatc gaggacggca gcgtgcagct 600

cgccgaccac taccagcaga acacccccat cggcgacggc cccgtgctgc tgcccgacaa 660

ccactacctg agcacccagt ccgccctgag caaagacccc aacgagaagc gcgatcacat 720

ggtcctgctg gagttcgtga ccgccgccgg gatcactctc ggcatggacg agctgtacaa 780

gtaggcgcgc agcgcttaga cgtctcgcga tcgattagtg cgagaggccg agggccagaa 840

caacatccgc ctaccatcca tcattgttat aaaaaa 876

<210> 40
<211> 828
<212> DNA
<213> ATU2(eGFP)

<400> 40
cctaccctcc atcattgtta taaaaaactt aggaaccagg tccacacagc cgccagccca 60

tcaacgcgta cgatggtgag caagggcgag gagctgttca ccggggtggt gcccatcctg 120

gtcgagctgg acggcgacgt aaacggccac aagttcagcg tgtccggcga gggcgagggc 180

gatgccacct acggcaagct gaccctgaag ttcatctgca ccaccggcaa gctgcccgtg 240

ccctggccca ccctcgtgac caccctgacc tacggcgtgc agtgcttcag ccgctacccc 300

gaccacatga agcagcacga cttcttcaag tccgccatgc ccgaaggcta cgtccaggag 360

cgcaccatct tcttcaagga cgacggcaac tacaagaccc gcgccgaggt gaagttcgag 420

ggcgacaccc tggtgaaccg catcgagctg aagggcatcg acttcaagga ggacggcaac 480

atcctggggc acaagctgga gtacaactac aacagccaca acgtctatat catggccgac 540

aagcagaaga acggcatcaa ggtgaacttc aagatccgcc acaacatcga ggacggcagc 600

gtgcagctcg ccgaccacta ccagcagaac acccccatcg gcgacggccc cgtgctgctg 660

cccgacaacc actacctgag cacccagtcc gccctgagca aagaccccaa cgagaagcgc 720

gatcacatgg tcctgctgga gttcgtgacc gccgccggga tcactctcgg catggacgag 780

ctgtacaagt aggcgcgcag cgcttagacg tctcgcgatc gatactag 828

<210> 41
<211> 828
<212> DNA
<213> ATU3(eGFP)

<400> 41

ctagtctacc ctccatcatt gttataaaaa acttaggaac caggtccaca cagccgccag 60

cccatcaacg cgtacgatgg tgagcaaggg cgaggagctg ttcaccgggg tggtgcccat 120

cctggtcgag ctggacggcg acgtaaacgg ccacaagttc agcgtgtccg gcgagggcga 180

gggcgatgcc acctacggca agctgaccct gaagttcatc tgcaccaccg gcaagctgcc 240

cgtgccctgg cccaccctcg tgaccaccct gacctacggc gtgcagtgct tcagccgcta 300

ccccgaccac atgaagcagc acgacttctt caagtccgcc atgcccgaag gctacgtcca 360

ggagcgcacc atcttcttca aggacgacgg caactacaag acccgcgccg aggtgaagtt 420

cgagggcgac accctggtga accgcatcga gctgaagggc atcgacttca aggaggacgg 480

caacatcctg gggcacaagc tggagtacaa ctacaacagc cacaacgtct atatcatggc 540

cgacaagcag aagaacggca tcaaggtgaa cttcaagatc cgccacaaca tcgaggacgg 600

cagcgtgcag ctcgccgacc actaccagca gaacaccccc atcggcgacg gccccgtgct 660

gctgcccgac aaccactacc tgagcaccca gtccgccctg agcaaagacc ccaacgagaa 720

gcgcgatcac atggtcctgc tggagttcgt gaccgccgcc gggatcactc tcggcatgga 780

cgagctgtac aagtaggcgc gcagcgctta gacgtctcgc gatcgatg 828